# EUROPEAN PATENT APPLICATION

(11) **EP 4 818 176 A1**
(43) Date of publication of application: **07.10.2026**
(21) Application number: 24896782.0
(22) Date of filing: 02.12.2024
(51) Int. Cl.: C07D 519/00, A61K 31/407, A61K 31/4353, A61K 31/4985, A61P 35/00

(54) **TETRACYCLIC DERIVATIVE INHIBITOR, AND PREPARATION METHOD AND USE THEREFOR**

(30) Priority: 01.12.2023 CN 202311642205; 12.01.2024 CN 202410052748; 06.02.2024 CN 202410172322; 23.02.2024 CN 202410205545; 29.02.2024 CN 202410232845; 03.04.2024 CN 202410405908; 15.04.2024 CN 202410453210; 31.05.2024 CN 202410705858; 20.06.2024 CN 202410806927; 27.06.2024 CN 202410855447; 03.07.2024 CN 202410890783; 26.07.2024 CN 202411022696; 16.08.2024 CN 202411138112; 27.09.2024 CN 202411369508
(71) Applicant: Shanghai Hansoh Biomedical Co., Ltd., Shanghai 201203 (CN); Jiangsu Hansoh Pharmaceutical Group Co., Ltd., Lianyungang, Jiangsu 222069 (CN)
(72) Inventor: WU, Jianrui, Shanghai 201203 (CN); GAO, Peng, Shanghai 201203 (CN); DONG, Jiaqiang, Shanghai 201203 (CN); ZHANG, Yang, Shanghai 201203 (CN); GONG, Zhen, Shanghai 201203 (CN)
(74) Representative: De Clercq & Partners
(86) International application number: PCT/CN2024/136094
(87) International publication number: WO 2025/113703

(57) **Abstract**

The present invention relates to a tetracyclic derivative inhibitor, and a preparation method and use therefor. In particular, the present invention relates to a tetracyclic derivative compound, a preparation method therefor and a pharmaceutical composition containing the compound, and a use thereof in treating cancer.

## Description

### Technical Field

The present invention belongs to the field of drug synthesis and specifically relates to a tetracyclic derivative inhibitor, and a preparation method therefor and the use thereof.

### Background Art

Protein arginine methyltransferases (PRMTs) are classified into three major types based on their catalytic activity and product types: Types I, II, and III. Type I mainly comprises PRMT1/2/3/4/6/8, which catalyse the formation of asymmetric dimethylarginine (ADMA) on substrates; Type II comprises PRMT5/9, which catalyse the formation of symmetric dimethylarginine (SDMA) on substrates; Type III comprises only PRMT7,whichis responsible for catalysing the formation of monomethylarginine (MMA) on substrates. PRMT5 uses S-adenosyl-L-methionine (SAM) as a methyl donor to transfer methyl groups to substrates such as DNA, RNA and histones. Symmetric dimethylation of arginine residues on the substrates generates SDMA, thereby regulating multiple key cellular processes including transcription, translation and DNA repair to maintain cellular homeostasis. In addition, PRMT5 is also involved in regulating the growth and survival pathways of tumour cells and promoting tumourigenesis and progression. Elevated expression of PRMT5 has also been demonstrated to be associated with poor prognosis in a variety of cancers, making it a highly promising epigenetic target.

Methylthioadenosine phosphorylase (MTAP) catalyses the conversion of methylthioadenosine (MTA) to methionine, which is essential for maintaining normal cell functions. Deletion mutations in the MTAP gene lead to the accumulation of MTA in cells. MTA competes with PRMT5's substrate SAM, thereby reducing PRMT5 activity and resulting in abundant formation of PRMT5-MTA complexes. Deletion of the MTAP gene increases tumour dependency on PRMT5, and inhibition of PRMT5 in MTAP-deficient tumours can exert a "synthetic lethal" effect. PARP inhibitors based on the theory of "synthetic lethality" have achieved great success in the field of precision oncology. The MTAP gene is adjacent to CDKN2A, the most common tumour suppressor gene in human cancers, and is often co-deleted with CDKN2A. This co-deletion occurs in 10%-15% of all cancers, mainly in non-small cell lung cancer (12%-20%), glioma (53%), pancreatic cancer (30%), and DLBCL (20%), representing significant market potential.

Currently, no PRMT5 inhibitor has been approved for marketing. Early PRMT5 inhibitors were all non-selective, SAM-competitive inhibitors, which exhibited severe haematological toxicity and a narrow therapeutic window in clinical settings. The clinical progress of the first-generation PRMT5 inhibitors GSK-3326595, JNJ-64619178 and PF-06939999 has been suboptimal. The next-generation PRMT5 inhibitors targeting the PRMT5-MTA complex are effective specifically against MTAP-deficient, MTA-enriched tumourswhile exhibiting high selectivity for MTAP wild-type tumours. This mechanism reduces haematological toxicity, as has been validated in preclinical studies, and is expected to significantly widen the therapeutic window.

The present patent relates to a novel selective PRMT5-MTA inhibitor that exhibits activity specifically against MTAP-deficient cells while showing weak inhibition against MTAP wild-type cells, thereby avoiding the haematological toxicity and other side effectsassociated with the inhibition of MTAP wild-type cells by non-selective PRMT5 inhibitors in clinical settings. Highly selective PRMT5-MTA inhibitors, as novel PRMT5-MTA inhibitors, can be used for the treatment of various tumours, cancers and other diseases.

### Summary of the Invention

The objective of the present invention is to provide a compound represented by general formula (I), or a stereoisomer or a pharmaceutically acceptable salt thereof, wherein the compound represented by general formula (I) has the following structure: 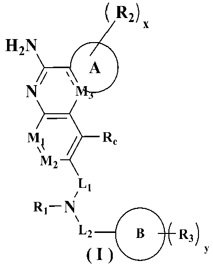
M₁ is selected from -N- or -CRₐ-; M₂ is selected from -N- or -CR_{b}-; preferably -CR_{b}-; M₃ is selected from N or C;
ring A is selected from C₃₋₈ cycloalkyl, 3- to 8-membered heterocyclyl, C₆₋₁₀ aryl or 5- to 10-membered heteroaryl; preferably C₃₋₆ cycloalkyl, 3- to 6-membered heterocyclyl, phenyl or 5- to 6-membered heteroaryl;
ring B is selected from C₃₋₁₄ cycloalkyl, 3- to 14-membered heterocyclyl, C₆₋₁₄ aryl or 5- to 14-membered heteroaryl; preferably C₃₋₁₂ cycloalkyl, 3- to 12-membered heterocyclyl, C₆₋₁₂ aryl or 5- to 12-membered heteroaryl; preferably C₃₋₆ cycloalkyl, 3- to 6-membered heterocyclyl, C₆₋₁₀ fused cycloalkyl, 6- to 10-membered fused heterocyclyl, C₆₋₁₀ aryl or 5- to 10-membered heteroaryl;
L₁ is selected from a bond, -(CRₐₐR_{bb})ₘ₂-, -(CRₐₐR_{bb})ₘ₂C(O)-, - (CRₐₐR_{bb})ₘ₂C(S)-, -(CRₐₐR_{bb})ₘ₂C(NR_{cc})-, -(CRₐₐR_{bb})ₘ₂NR_{cc}C(O)-, - (CRₐₐR_{bb})ₘ₂S(O)ₘ₁-, -(CRₐₐR_{bb})ₘ₂NR_{cc}-, -(CRₐₐR_{bb})ₘ₂P(O)₂-, - (CRₐₐR_{bb})ₘ₂P(O)(OR_{cc})-, C₃₋₁₂ cycloalkylene, 3- to 12-membered heterocyclylene, C₆₋₁₂ arylene or 5- to 12-membered heteroarylene, wherein the C₃₋₁₂ cycloalkylene, 3- to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably -CRₐₐR_{bb}-, - C(O)-, -S(O)ₘ₁- or NR_{cc};
L₂ is selected from a bond, -(CRₐₐR_{bb})ₘ₂-, -(CRₐₐR_{bb})ₘ₂C(O)-, - (CRₐₐR_{bb})ₘ₂NR_{cc}C(O), -(CRₐₐR_{bb})ₘ₂S(O)ₘ₁- or -(CRₐₐR_{bb})ₘ₂NR_{cc}-; preferably - CRₐₐR_{bb}-, -C(O)-, -S(O)ₘ₁- or NR_{cc};
R₁ is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, -(CR_{cc}R_{dd})ₙ₁-C₃₋₁₂ cycloalkyl, - (CR_{cc}R_{dd})ₙ₁-3- to 12-membered heterocyclyl, -(CR_{cc}R_{dd})ₙ₁-C₆₋₁₂ aryl, -(CR_{cc}R_{dd})ₙ₁-5- to 12-membered heteroaryl, -SF₅, -ORₑ, -NRₑR_{f}, -C(O)Rₑ, -C(O)ORₑ, - C(O)NRₑR_{f}, -N=S(O)RₑR_{f}, -S(O)Rₑ(=NR_{f}) or -P(O)RₑR_{f}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, -(CR_{cc}R_{dd})ₙ₁-C₃₋₈ cycloalkyl, -(CR_{cc}R_{dd})ₙ₁-3- to 8-membered heterocyclyl, -(CR_{cc}R_{dd})ₙ₁-C₆₋₁₀ aryl, -(CR_{cc}R_{dd})ₙ₁-5- to 10-membered heteroaryl, -ORₑ, -NRₑR_{f}, -C(O)Rₑ, -C(O)NRₑR_{f} or -P(O)RₑR_{f}, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3-to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
alternatively, R₁ and Rₐ, R_{b} or R_{c} are connected to form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably form C₃₋₈ cycloalkyl, 3- to 8-membered heterocyclyl, C₆₋₁₀ aryl and 5- to 10-membered heteroaryl, which are optionally further substituted with one or more substituents selected from deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R₂ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff}, -(CH₂)ₙ₂P(O)RₑₑR_{ff} or =CRₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -Y₁-C₃₋₁₂ cycloalkyl, -Y₁-3- to 12-membered heterocyclyl, -Y₁-C₆₋₁₂ aryl, -Y₁-5- to 12-membered heteroaryl, -SF₅, -OR_{g}, -NR_{g}Rₕ, -C(O)R_{g}, -C(O)OR_{g}, -C(O)NR_{g}Rₕ, - N=S(O)R_{g}Rₕ, -S(O)R_{g}(=NRₕ), -P(O)R_{g}Rₕ, -C(=NRᵢ)NR_{g}Rₕ or =R_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f}; preferably hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, - C(O)NR_{g}Rₕ, -N=S(O)R_{g}Rₕ, -S(O)R_{g}(=NRₕ), -P(O)R_{g}Rₕ or =R_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f};
alternatively, any two R₃, together with adjacent atoms to which they are attached, form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably form C₃₋₈ cycloalkyl, 3- to 8-membered heterocyclyl, C₆₋₁₀ aryl and 5- to 10-membered heteroaryl, which are optionally further substituted with one or more substituents selected from deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₐ, R_{b}, R_{c}, Rₑ and R_{f} are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, - (CH₂)ₙ₂NRₑₑR_{ff}, -(CH₂)ₙ₂C(O)Rₑₑ, -(CH₂)ₙ₂C(O)ORₑₑ, -(CH₂)ₙ₂C(O)NRₑₑR_{ff}, - (CH₂)ₙ₂N=S(O)RₑₑR_{ff}, -(CH₂)ₙ₂S(O)Rₑₑ(=NR_{ff}) or -(CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
alternatively, Rₐ and R_{b} are connected to form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Y₁ is selected from a bond, -O-, -S-, -C(O), -NRⱼ-, -C(O)NRⱼ-, -NRⱼC(O)-, - S(O)₂NRj-, -NRⱼS(O)₂-, C₁₋₆ alkylene, -O-C₁₋₆ alkylene-, -C₁₋₆ alkylene-O-, -NRⱼ-C₁₋₆ alkylene-, -C₁₋₆ alkylene-NRⱼ-, C₂₋₆ alkenylene or C₂₋₆ alkynylene, wherein the C₁₋₆ alkylene, C₂₋₆ alkenylene and C₂₋₆ alkynylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{g}, Rₕ, Rᵢ and Rⱼ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₐₐ, R_{bb}, R_{cc} and R_{dd} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₑₑ and R_{ff} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R_{gg} and Rₕₕ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
x is selected from 0, 1, 2, 3, 4, 5 or 6; y is selected from 0, 1, 2, 3, 4, 5 or 6;
m1 is selected from 0, 1 or 2; m2 is selected from 0, 1 or 2; n1 is selected from 0, 1, 2, 3 or 4; and
n2 is selected from 0, 1, 2, 3 or 4.

In a preferred embodiment of the present invention, the compound, or the stereoisomer or the pharmaceutically acceptable salt thereof, is characterised in that the 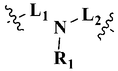 is selected from 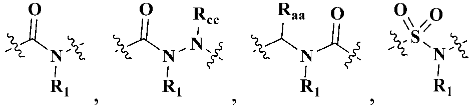 or

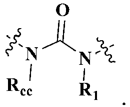

The present invention further provides a compound represented by general formula (A), or a stereoisomer or a pharmaceutically acceptable salt thereof: 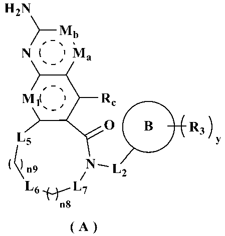
M₁ is selected from -N- or -CRₐ-; Mₐ is selected from CR₂ₐ, NR₂ₐ or N; M_{b} is selected from CR_{2b}, NR_{2b} or N;
alternatively, R₂ₐ and R_{2b} are connected to form ring A;
ring A is selected from C₃₋₈ cycloalkyl, 3- to 8-membered heterocyclyl, C₆₋₁₀ aryl or 5- to 10-membered heteroaryl, which is optionally substituted with 0, 1, 2, 3, 4, 5 or 6 R_{2c};
ring B is selected from C₃₋₁₄ cycloalkyl, 3- to 14-membered heterocyclyl, C₆₋₁₄ aryl or 5- to 14-membered heteroaryl;
L₂ is selected from a bond, -(CRₐₐR_{bb})ₘ₂-, -(CRₐₐR_{bb})ₘ₂C(O)-, - (CRₐₐR_{bb})ₘ₂NR_{cc}C(O), -(CRₐₐR_{bb})ₘ₂S(O)ₘ₁- or -(CRₐₐR_{bb})ₘ₂NR_{cc}-;
L₅ is selected from a bond, -O(CR₉ₐR_{9b})ₙ₁₀-, -(CR₉ₐR_{9b})ₙ₁₀O-, -S(CR₉ₐR_{9b})ₙ₁₀-, -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, =CR₉ₐR_{9b}, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
L₆ is selected from a bond, -O(CR₉ₐR_{9b})ₙ₁₀-, -(CR₉ₐR_{9b})ₙ₁₀O-, -S(CR₉₂R_{9b})ₙ₁₀-, -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, =CR₉ₐR_{9b}, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
L₇ is selected from a bond, -O(CR₉ₐR_{9b})ₙ₁₀-, -(CR₉ₐR_{9b})ₙ₁₀O-, -S(CR₉ₐR_{9b})ₙ₁₀-, -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, =CR₉ₐR_{9b}, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₂ₐ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{2b} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{2c} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff}, -(CH₂)ₙ₂P(O)RₑₑR_{ff} or =CRₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₃ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, -C(O)NR_{g}Rₕ, -N=S(O)R_{g}Rₕ, - S(O)R_{g}(=NRₕ), -P(O)R_{g}Rₕ or =R_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f};
R₉ₐ and R_{9b} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Rₐ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff} or -(CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{c} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff} or -(CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Rₑ and R_{f} are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{g} and Rₙ are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{N} is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, - C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Rₐₐ, R_{bb} and R_{cc} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₑₑ and R_{ff} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
y is selected from 0, 1, 2, 3, 4, 5 or 6; ml is selected from 0, 1 or 2; m2 is selected from 0, 1 or 2; n2 is selected from 0, 1, 2, 3 or 4; n8 is selected from 0, 1, 2, 3 or 4; n9 is selected from 0, 1, 2, 3 or 4; n10 is selected from 0, 1, 2, 3 or 4.

In a preferred embodiment of the present invention, the compound, or the stereoisomer or the pharmaceutically acceptable salt thereof, is characterised in that the compound is further as represented by general formula (III-E): 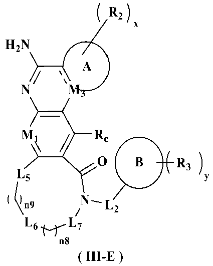
L₅ is selected from a bond, -O(CR₉ₐR_{9b})ₙ₁₀-, -(CR₉ₐR_{9b})ₙ₁₀O-, -S(CR₉ₐR_{9b})ₙ₁₀-, -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, C(=CR₉ₐR_{9b}), C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably a bond, -O-, - S-, -C(O), -NR_{N}-, -C(O)NR_{N}-, -NR_{N}C(O)-, -S(O)₂NR_{N}-, -NR_{N}S(O)₂-, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3- to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
L₆ is selected from a bond, -O(CR₉ₐR_{9b})ₙ₁₀-, -(CR₉ₐR_{9b})ₙ₁₀O-, -S(CR₉ₐR_{9b})ₙ₁₀-₃ -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, C(=CR₉ₐR_{9b}), C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably a bond, -O-, - S-, -C(O), -NR_{N}-, -C(O)NR_{N}-, -NR_{N}C(O)-, -S(O)₂NR_{N}-, -NR_{N}S(O)₂-, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3- to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
L₇ is selected from a bond, -O(CR₉ₐR_{9b})ₙ₁₀-, -(CR₉ₐR_{9b})ₙ₁₀O-, -S(CR₉ₐR_{9b})ₙ₁₀-, -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, C(=CR₉ₐR_{9b}), C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably a bond, -O-, - S-, -C(O), -NR_{N}-, -C(O)NR_{N}-, -NR_{N}C(O)-, -S(O)₂NRₙ-, -NR_{N}S(O)₂-, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3- to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
ring A, ring B, L₂, M₁, M₃, R_{c}, R₂, R₃, x and y are as defined in any one of the above embodiments.

In a preferred embodiment of the present invention, the compound, or the stereoisomer or the pharmaceutically acceptable salt thereof, is characterised in that the compound is further as represented by general formula (IV-E): 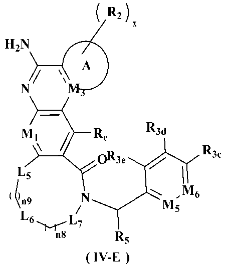
M₅ is selected from N or CR₃ₐ; M₆ is selected from N or CR_{3b};
R₅ is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, - C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₃ₐ, R_{3b}, R_{3d} and R₃ₑ are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, -C(O)NR_{g}Rₕ, -N=S(O)R_{g}Rₕ, -S(O)R_{g}(=NRₕ), -P(O)R_{g}Rₕ or - C(=NRᵢ)NR_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5-to 12-membered heteroaryl and =CRₑR_{f};
R_{3c} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, -C(O)NR_{g}Rₕ, -N=S(O)R_{g}Rₕ, - S(O)R_{g}(=NRₕ), -P(O)R_{g}Rₕ or -C(=NRᵢ)NR_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f};
ring A, M₁, M₃, R_{c}, R₂, x, L₅, L₆, L₇, n8 and n9 are as defined in any one of the above embodiments.

In a preferred embodiment of the present invention, ring A is selected from 3-to 12-membered heterocyclyl or 5- to 12-membered heteroaryl; preferably 5-membered heterocyclyl, 6-membered heterocyclyl, 5-membered heteroaryl or 6-membered heteroaryl; more preferably 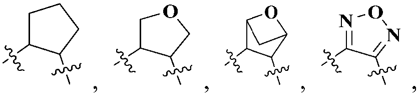 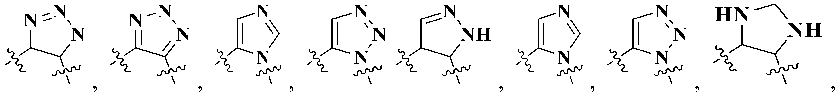 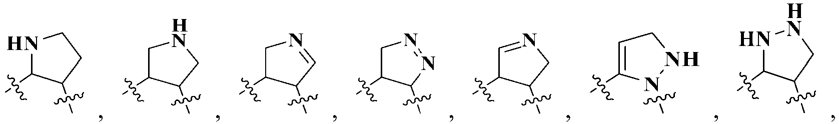 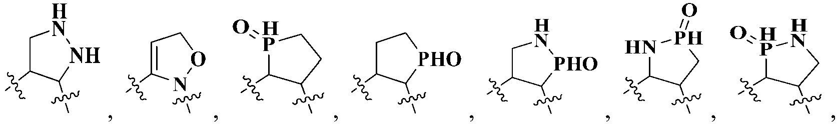 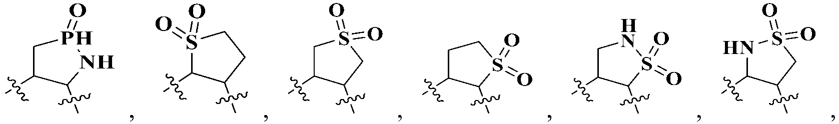 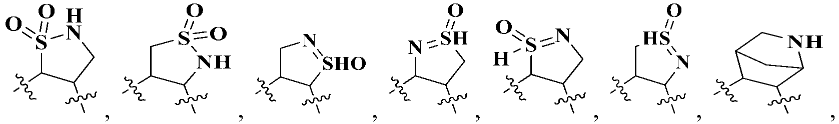 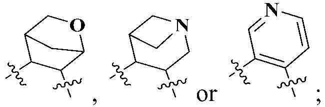 more preferably 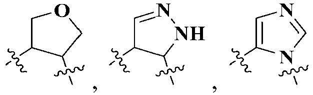 or

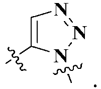

In a preferred embodiment of the present invention, ring B is selected from 3-to 6-membered heterocyclyl fused phenyl or 3- to 6-membered heterocyclyl fused 5- to 6-membered heteroaryl; preferably 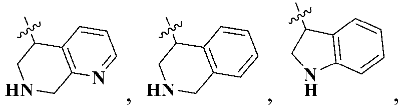 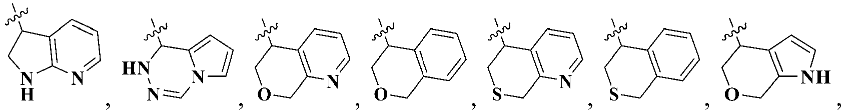 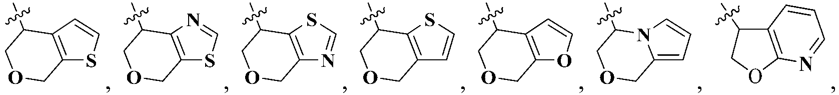 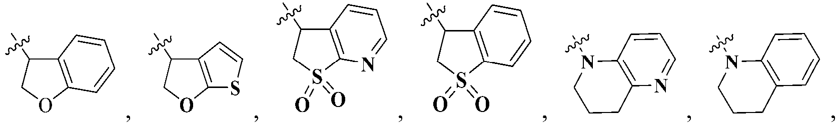 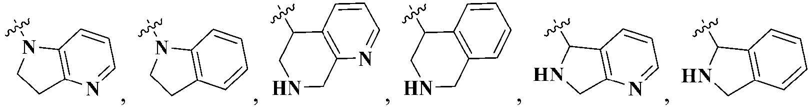 or

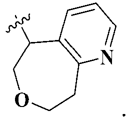

In a preferred embodiment of the present invention, ring B is 6- to 14-membered tricyclic heterocyclyl; preferably 6- to 14-membered tricyclic spiro heterocyclyl or 6- to 14-membered tricyclic fused heterocyclyl; more preferably

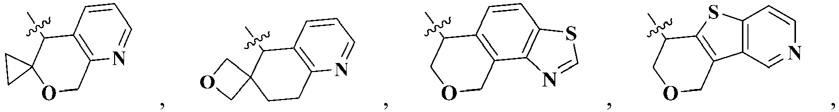

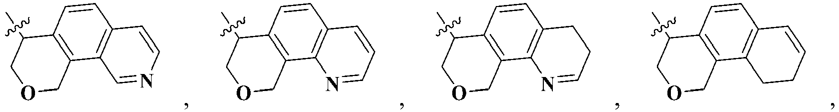

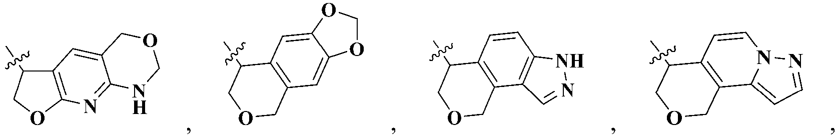

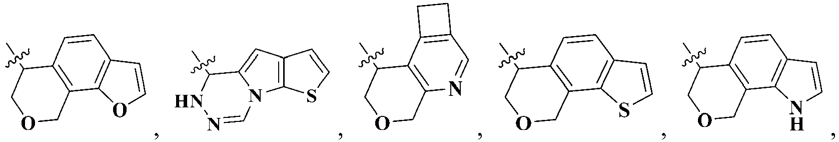

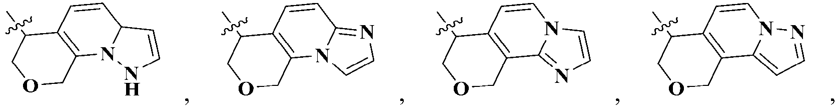

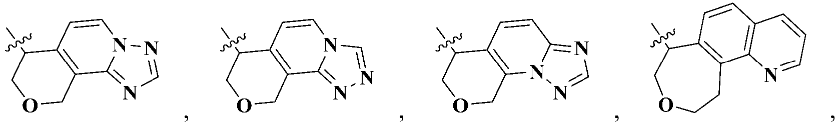

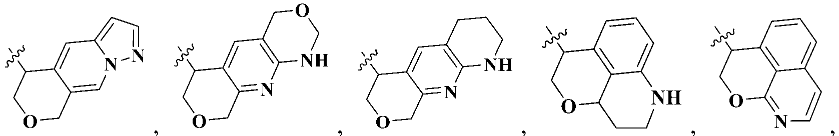

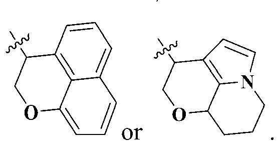

In a preferred embodiment of the present invention, the compound, or the stereoisomer or the pharmaceutically acceptable salt thereof, is characterised in that the compound is further as represented by general formula (VI-A) or (VI-B): 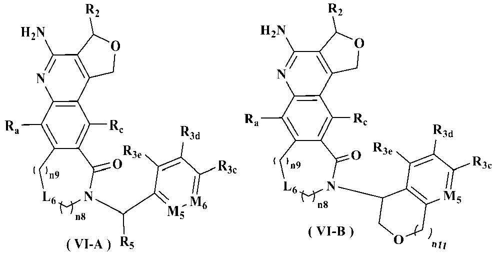
n11 is selected from 0, 1 or 2; M₅ is selected from N or CR₃ₐ; M₆ is selected from N or CR_{3b};
R₃ₐ, R_{3b}, R_{3d} and R₃ₑ are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, -C(O)NR_{g}Rₕ, -N=S(O)R_{g}Rₕ, -S(O)R_{g}(=NRₕ), -P(O)R_{g}Rₕ or - C(=NRᵢ)NR_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5-to 12-membered heteroaryl and =CRₑR_{f};
R_{3c} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, -C(O)NR_{g}Rₕ, -N=S(O)R_{g}Rₕ, - S(O)R_{g}(=NRₕ), -P(O)R_{g}Rₕ or -C(=NRᵢ)NR_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f}.

In a preferred embodiment of the present invention, L₆ is selected from -O-, - S-, -C(O), -NR_{N}-, C₁₋₃ alkylene or C₂₋₄ alkenylene, wherein the C₁₋₃ alkylene and C₂₋₄ alkenylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
in a preferred embodiment of the present invention, n8 is selected from 1, 2 or 3; n9 is selected from 0, 1 or 2.

In a preferred embodiment of the present invention, each R₂ or R_{2c} is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl, C₃₋₈ cycloalkyl or -C(O)NRₑₑR_{ff}, wherein the amino, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl and C₃₋₈ cycloalkyl are optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl and C₃₋₈ cycloalkyl;
Rₑₑ and R_{ff} are each independently selected from hydrogen, deuterium, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl or cyano-substituted C₁₋₃ alkyl;
preferably hydrogen, deuterium, fluorine, chlorine, methyl, deuterated methyl, difluoromethyl, trifluoromethyl,

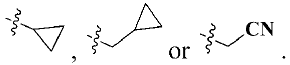

In a preferred embodiment of the present invention, Rₐ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl or cyano-substituted C₁₋₃ alkyl; preferably hydrogen, deuterium, fluorine, chlorine, methyl, deuterated methyl, difluoromethyl or trifluoromethyl.

In a preferred embodiment of the present invention, R_{c} is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl or cyano-substituted C₁₋₃ alkyl; preferably hydrogen, deuterium, fluorine, chlorine, methyl, deuterated methyl, difluoromethyl or trifluoromethyl.

In a preferred embodiment of the present invention, the compound, or the stereoisomer or the pharmaceutically acceptable salt thereof, is characterised in that R₃ and R₃ₑ are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl, 5-to 10-membered heteroaryl or -SF₅, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₃ₐ, R_{3b}, R_{3d} and R₃ₑ are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl.

The present invention further provides a compound represented by general formula (A-I), or a stereoisomer or a pharmaceutically acceptable salt thereof: 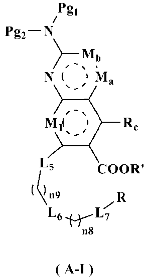
R' is selected from hydrogen, C₁₋₆ alkyl, C₁₋₆ deuteroalkyl, C₁₋₆ haloalkyl, C₁₋₆ alkoxy, halogenated C₁₋₆ alkoxy or C₁₋₆ hydroxyalkyl;
R is selected from halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl or 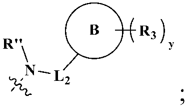
R" is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, - C(O)-C₁₋₆ alkyl, -C(O)O-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
preferably a compound represented by general formula (A-II): 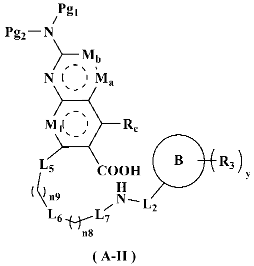
preferably, the compound represented by general formula (A-II) is further as represented by general formula (IV-E-I), general formula (IV-A-I) or general formula (IV-B-I): 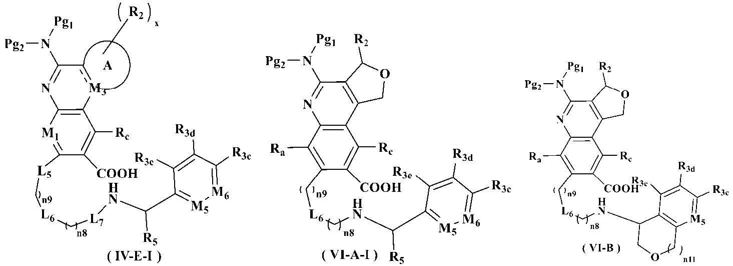
Pg₁ is selected from hydrogen, allyloxycarbonyl, trifluoroacetyl, tert-butylsulphinyl 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl or p-methoxyphenyl; preferably benzyl, p-methoxybenzyl or tert-butyloxycarbonyl;
Pg₂ is selected from hydrogen, allyloxycarbonyl, trifluoroacetyl, tert-butylsulphinyl 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl or p-methoxyphenyl; preferably hydrogen, benzyl, p-methoxybenzyl or tert-butyloxycarbonyl;
M₁, Mₐ, M_{b}, ring A, ring B, L₂, L₅, L₆, L₇, Rₐ, R_{b}, R_{c}, R₃, M₅, M₆, R₃ₐ, R_{3b}, R_{3c}, R_{3d}, R₃ₑ, R₅, n11, n8, n9, x and y are as defined in any one of the above embodiments.

The present invention further provides a method for preparing a compound represented by general formula (A), or a stereoisomer or a pharmaceutically acceptable salt thereof, wherein 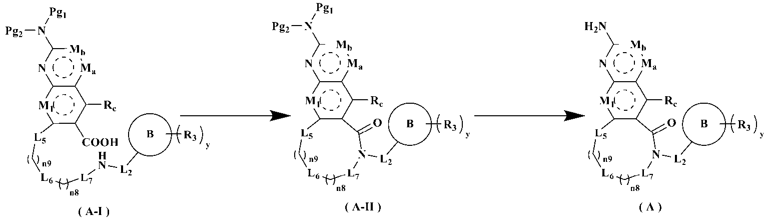
a compound represented by general formula (A-I) is reacted in the presence of a condensing agent and a base to obtain a compound represented by general formula (A-II), which is further deprotected to obtain the compound represented by general formula (A);
preferably, the method is a method for preparing a compound represented by general formula (IV-E), or a stereoisomer or a pharmaceutically acceptable salt thereof: 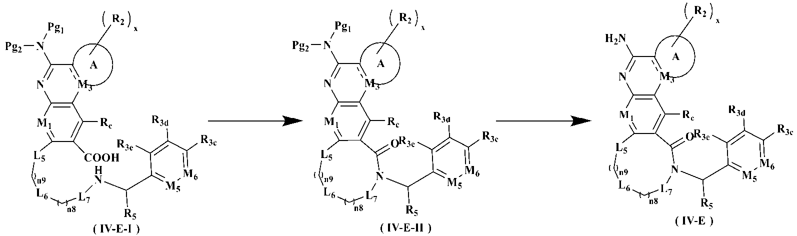
a compound represented by general formula (IV-E-I) is reacted in the presence of a condensing agent and a base to obtain a compound represented by general formula (IV-E-II), which is further deprotected to obtain the compound represented by general formula (IV-E);
alternatively, the method is a method for preparing a compound represented by general formula (VI-A), or a stereoisomer or a pharmaceutically acceptable salt thereof: 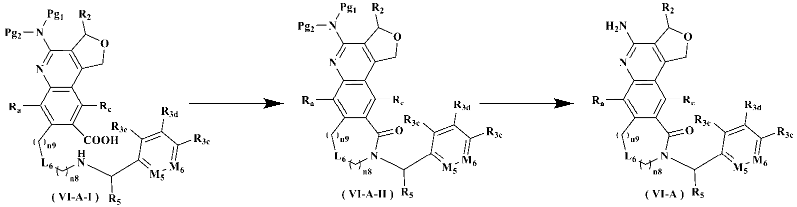
a compound represented by general formula (VI-A-I) is reacted in the presence of a condensing agent and a base to obtain a compound represented by general formula (VI-A-II), which is further deprotected to obtain the compound represented by general formula (VI-A);
alternatively, the method is a method for preparing a compound represented by general formula (VI-B), or a stereoisomer or a pharmaceutically acceptable salt thereof: 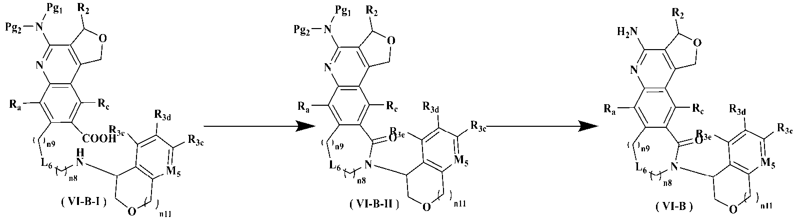
a compound represented by general formula (VI-B-I) is reacted in the presence of a condensing agent and a base to obtain a compound represented by general formula (VI-B-II), which is further deprotected to obtain the compound represented by general formula (VI-B);
Pg₁ is selected from hydrogen, allyloxycarbonyl, trifluoroacetyl, tert-butylsulphinyl 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl or p-methoxyphenyl; preferably benzyl, p-methoxybenzyl or tert-butyloxycarbonyl;
Pg₂ is selected from hydrogen, allyloxycarbonyl, trifluoroacetyl, tert-butylsulphinyl 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl or p-methoxyphenyl; preferably hydrogen, benzyl, p-methoxybenzyl or tert-butyloxycarbonyl;
M₁, Mₐ, M_{b}, ring A, ring B, L₂, L₅, L₆, L₇, Rₐ, R_{b}, R_{c}, R₃, M₅, M₆, R₃ₐ, R_{3b}, R_{3c}, R_{3d}, R₃ₑ, R₅, n11, n8, n9, x and y are as defined in any one of the above embodiments.

In a preferred embodiment of the present invention, the condensing agent in the preparation method is selected from thionyl chloride, phosphorus oxychloride, p-toluenesulphonyl chloride, methanesulphonyl chloride, dicyclohexylcarbodiimide, diisopropylcarbodiimide, 1-(3-dimethylaminopropyl)-3-ethylcarbodiimide, carbonyldiimidazole, ethyl chloroformate, 2-(7-azobenzotriazol-1-yl)-N,N,N',N'-tetramethyluronium hexafluorophosphate, O-(benzotriazol-1-yl)-tetramethyluronium hexafluorophosphate or tetramethylchloroformamidinium hexafluorophosphate; preferably phosphorus oxychloride, 2-(7-azobenzotriazol-1-yl)-N,N,N',N'-tetramethyluronium hexafluorophosphate, O-(benzotriazol-1-yl)-tetramethyluronium hexafluorophosphate or tetramethylchloroformamidinium hexafluorophosphate;
the base is selected from potassium carbonate, methylamine, triethylamine, diisopropylamine, pyridine, imidazole, N-methylimidazole or N-methylmorpholine.

The present invention further relates to a pharmaceutical composition comprising a therapeutically effective dose of the compound, or the stereoisomer or the pharmaceutically acceptable salt thereof as shown in any one of the above embodiments, and one or more pharmaceutically acceptable carriers, diluents, or excipients.

The present invention further relates to the use of the compound, or the stereoisomer or the pharmaceutically acceptable salt thereof as shown in any one of the above embodiments, or the pharmaceutical composition in the preparation of a PRMT5 inhibitor drug.

The present invention further relates to the use of the compound, or the stereoisomer or the pharmaceutically acceptable salt thereof as shown in any one of the above embodiments, or the pharmaceutical composition thereof in the preparation of a drug for the treatment of a cancer; preferably, the cancer is a cancer with MTAP gene deletion.

The present invention further relates to a method of using the compound, or the stereoisomer or the pharmaceutically acceptable salt thereof as shown in any one of the above embodiments, or the pharmaceutical composition thereof in the preparation of a drug for the treatment of a cancer; preferably, the cancer is a cancer with MTAP gene deletion.

In some embodiments, the cancer is selected from lung cancer, hepatocellular carcinoma, breast cancer, skin cancer, bladder cancer, liver cancer, pancreatic cancer, head and neck cancer, glioma, glioblastoma, oesophageal cancer, pancreatic cancer, mesothelioma, melanoma, astrocytoma, undifferentiated pleomorphic sarcoma, diffuse large B-cell lymphoma, leukaemia, gastric adenocarcinoma, myxofibrosarcoma, cholangiosarcoma, brain cancer, gastric cancer, renal cancer, endometrial cancer, ovarian tumour, prostate cancer, lymphoma, non-Hodgkin lymphoma, urinary tract cancer, soft tissue cancer, pleural cancer, large intestine cancer, colourectal cancer, biliary tract cancer or cholangiocarcinoma; the lung cancer is selected from non-small cell lung cancer, lung squamous cell carcinoma or lung adenocarcinoma; the oesophageal cancer is selected from oesophageal squamous cell carcinoma or oesophageal adenocarcinoma.

The present invention further relates to a method for preventing and/or treating a cancer, wherein the method comprises administering to a patient a therapeutically effective dose of the compound, or the stereoisomer or the pharmaceutically acceptable salt thereof as shown in any one of the above embodiments, or the pharmaceutical composition thereof.

The present invention further relates to a method for treating a cancer in a mammal, wherein the method comprises administering to the mammal a therapeutically effective amount of the compound of the present invention, or the pharmaceutically acceptable salt, the ester, the prodrug, the solvate, the hydrate or the derivative thereof.

In certain embodiments of the present invention, the weight percent of the compound, or the stereoisomer or the pharmaceutically acceptable salt thereof in the pharmaceutical composition is 0.1%-95%, preferably 90%, 85%, 80%, 75%, 70%, 60%, 50%, based on free base.

In certain embodiments of the present invention, the pharmaceutical composition is selected from a tablet, a capsule, a liquid preparation or an injection, preferably further comprises a filler, optionally further comprises a disintegrant, or further comprises one or more of a glidant or a lubricant.

In certain embodiments of the present invention, the pharmaceutical composition is an immediate-release preparation or a sustained-release preparation.

In certain embodiments of the present invention, the unit dose of the compound, or the stereoisomer or the pharmaceutically acceptable salt thereof in the pharmaceutical composition is 1-1000 mg, preferably 1-500 mg, or preferably 1 mg, 2 mg, 3 mg, 5 mg, 10 mg, 20 mg, 40 mg, 50 mg, 60 mg, 80 mg, 100 mg, 200 mg, 300 mg, 400 mg or 500 mg, based on free base.

In certain embodiments of the present invention, the compound, or the stereoisomer or the pharmaceutically acceptable salt thereof can be administered by any convenient method, for example, by oral, parenteral, buccal, sublingual, nasal, rectal, intrathecal or transdermal administration, and the pharmaceutical composition can be adjusted accordingly.

In certain embodiments of the present invention, the compound, or the stereoisomer or the pharmaceutically acceptable salt thereof can be formulated into a liquid or solid preparation, such as a syrup, a suspension, an emulsion, a tablet, a capsule, powder, a granule, or a lozenge.

In some embodiments, the method relates to the treatment of conditions such as lung cancer, hepatocellular carcinoma, breast cancer, skin cancer, bladder cancer, liver cancer, pancreatic cancer, head and neck cancer, glioma, glioblastoma, oesophageal cancer, pancreatic cancer, mesothelioma, melanoma, astrocytoma, undifferentiated pleomorphic sarcoma, diffuse large B-cell lymphoma, leukaemia, gastric adenocarcinoma, myxofibrosarcoma, cholangiosarcoma, brain cancer, gastric cancer, renal cancer, endometrial cancer, ovarian tumour, prostate cancer, lymphoma, non-Hodgkin lymphoma, urinary tract cancer, soft tissue cancer, pleural cancer, large intestine cancer, colourectal cancer, biliary tract cancer or cholangiocarcinoma.

In some embodiments, the lung cancer is selected from non-small cell lung cancer, lung squamous cell carcinoma or lung adenocarcinoma; the oesophageal cancer is selected from oesophageal squamous cell carcinoma or oesophageal adenocarcinoma.

### Detailed Description of the Invention

Unless stated to the contrary, all technical and scientific terms used herein generally have the same meanings as commonly understood by one of ordinary skill in the art. Specifically, the terms used in the description and claims have the following meanings.

The term "alkyl" refers to a linear or branched saturated aliphatic hydrocarbon group which may be optionally substituted with one or more substituents. In particular embodiments, the alkyl refers to a linear saturated hydrocarbon group having 1 to 20 (C₁₋₂₀), 1 to 15 (C₁₋₁₅), 1 to 12 (C₁₋₁₂), 1 to 10 (C₁₋₁₀), 1 to 8 (C₁₋₈), 1 to 6 (C₁₋₆), or 1 to 3 (C₁₋₃) carbon atoms, or a branched saturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. As used herein, linear C₁₋₆ alkyl and branched C₃₋₆ alkyl groups are also referred to as "lower alkyl". For example, C₁₋₆ alkyl refers to a linear saturated monovalent hydrocarbon group having 1 to 6 carbon atoms or a branched saturated monovalent hydrocarbon group having 3 to 6 carbon atoms. In one embodiment, the C₁₋₆ alkyl contains 1 to 6 (e.g., 1, 2, 3, 4, 5 or 6) carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched isomers thereof, etc. In one embodiment, the alkyl is optionally substituted alkyl as described elsewhere herein.

The term "alkylene" refers to alkyl in which one hydrogen atom is further substituted, wherein the "alkyl" is as defined above. Non-limiting examples of "alkylene" include: methylene (-CH₂-), ethylene (-(CH₂)₂-), propylene (-(CH₂)₃-) or butylene (-(CH₂)₄-). In one embodiment, the alkylene is optionally substituted alkyl as described elsewhere herein.

The term "alkenyl" refers to a linear or branched unsaturated aliphatic hydrocarbon group containing at least one carbon-carbon double bond which may be located at any position within the alkenyl, and the alkenyl may be optionally substituted with one or more substituents. In particular embodiments, the alkenyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆) or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. Unless otherwise specified, the term "alkenyl" as used herein includes both linear and branched alkenyl. For example, C₂₋₆ alkenyl refers to a linear unsaturated hydrocarbon group having 2 to 6 carbon atoms or a branched unsaturated hydrocarbon group having 3 to 6 carbon atoms. In one embodiment, the C₂₋₆ alkenyl contains 2 to 6 (e.g., 2, 3, 4, 5 or 6) carbon atoms. Non-limiting examples of alkenyl include: 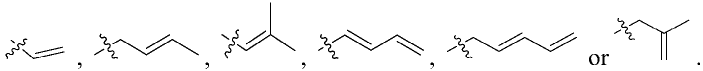 One of ordinary skill in the art can understand that the term "alkenyl" may also include groups having "cis" and "trans" configurations, or alternatively, groups having "E" and "Z" configurations. In one embodiment, the alkenyl is optionally substituted alkenyl as described elsewhere herein.

The term "alkenylene" refers to alkenyl in which one hydrogen atom is further substituted, wherein the "alkenyl" is as defined above. In one embodiment, the alkenylene is optionally substituted alkyl as described elsewhere herein.

The term "alkynyl" refers to a linear or branched unsaturated aliphatic hydrocarbon group containing at least one carbon-carbon triple bond which may be located at any position within the alkynyl, and the alkynyl may be optionally substituted with one or more substituents. In particular embodiments, the alkynyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. Unless otherwise specified, the term "alkynyl" as used herein includes both linear and branched alkynyl. For example, C₂₋₆ alkynyl refers to a linear unsaturated hydrocarbon group having 2 to 6 carbon atoms or a branched unsaturated hydrocarbon group having 3 to 6 carbon atoms. In one embodiment, the C₂₋₆ alkynyl contains 2 to 6 (e.g., 2, 3, 4, 5 or 6) carbon atoms. Non-limiting examples of alkynyl include: 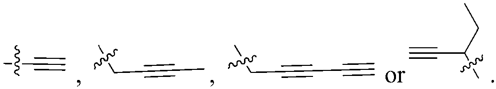 In one embodiment, the alkynyl is optionally substituted alkynyl as described elsewhere herein.

The term "alkynylene" refers to alkynyl in which one hydrogen atom is further substituted, wherein the "alkynyl" is as defined above. In one embodiment, the alkynylene is optionally substituted alkyl as described elsewhere herein.

The term "cycloalkyl" refers to a saturated or partially unsaturated aliphatic hydrocarbon cyclic group, which may be monocyclic or polycyclic (containing two or more rings), and which may be optionally substituted with one or more substituents. In particular embodiments, the cycloalkyl ring contains 3 to 20 (C₃₋₂₀), 3 to 14 (C₃₋₁₄), 3 to 12 (C₃₋₁₂), 3 to 8 (C₃₋₈) or 3 to 6 (C₃₋₆) carbon atoms. In one embodiment, the cycloalkyl ring contains 6 to 14 (C₆₋₁₄) or 7 to 10 (C₇₋₁₀) carbon atoms; it may contain one or more double bonds, but does not have a completely conjugated π electron system. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and polycyclic cycloalkyl includes spirocycloalkyl, fused cycloalkyl and bridged cycloalkyl in one embodiment. In one embodiment, the cycloalkyl is optionally substituted cycloalkyl as described elsewhere herein or cycloalkyl that is optionally fused to heterocyclyl, aryl or heteroaryl, and non-limiting examples include indanyl, tetrahydronaphthyl, benzocycloheptyl, etc.

The term "spirocycloalkyl" refers to an aliphatic hydrocarbon polycyclic group with monocyclic rings sharing one carbon atom (called a spiro atom); and it may contain one or more double bonds, but no ring has a completely conjugated π electron system. In particular embodiments, the spirocycloalkyl contains 5 to 20 (C₅₋₂₀), 6 to 14 (C₆₋₁₄) or 7 to 10 (C₇₋₁₀) (e.g., 7, 8, 9 or 10) carbon atoms. According to the number of shared spiro atoms between the rings, the spirocycloalkyl is divided into monospirocycloalkyl, bispirocycloalkyl or polyspirocycloalkyl. In one embodiment, the spirocycloalkyl is monospirocycloalkyl and bispirocycloalkyl. In one embodiment, the spirocycloalkyl is 4-membered/4-membered, 3-membered/5-membered, 4-membered/5-membered, 4-membered/6-membered, 5-membered/5-membered or 5-membered/6-membered monospirocycloalkyl. In one embodiment, the spirocycloalkyl is optionally substituted spirocycloalkyl as described elsewhere herein. Non-limiting examples of spirocycloalkyl include:

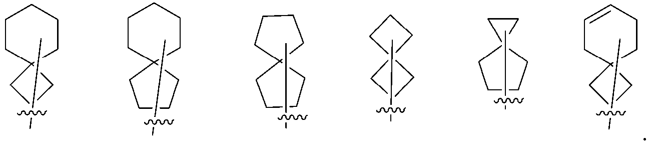

The term "fused cycloalkyl" refers to an all-carbon polycyclic group with each ring in the system sharing a pair of adjacent carbon atoms with another ring in the system, wherein one or more rings may contain one or more double bonds, but no ring has a completely conjugated π electron system. In particular embodiments, the fused cycloalkyl contains 5 to 20 (C₅₋₂₀), 6 to 14 (C₆₋₁₄) or 7 to 10 (C₇₋₁₀) (e.g., 7, 8, 9 or 10) carbon atoms. According to the number of constituent rings, the fused cycloalkyl can be divided into bicyclic, tricyclic, tetracyclic or polycyclic fused cycloalkyl. In one embodiment, the fused cycloalkyl is bicyclic or tricyclic, and in a further embodiment, the fused cycloalkyl is 3-membered/5-membered, 4-membered/5-membered, 5-membered/5-membered or 5-membered/6-membered bicyclic cycloalkyl. In one embodiment, the fused cycloalkyl is optionally substituted fused cycloalkyl as described elsewhere herein, or fused cycloalkyl that is optionally fused to heterocyclyl, aryl or heteroaryl. Non-limiting examples of fused cycloalkyl include: 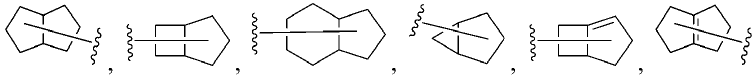 or

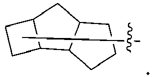

The term "bridged cycloalkyl" refers to an all-carbon polycyclic group in which any two rings share two carbon atoms that are not directly connected; and it may contain one or more double bonds, but no ring has a completely conjugated π electron system. In particular embodiments, the bridged cycloalkyl contains 5 to 20 (C₅₋₂₀), 6 to 14 (C₆₋₁₄) or 7 to 10 (C₇₋₁₀) (e.g., 7, 8, 9 or 10) carbon atoms. According to the number of constituent rings, the bridged cycloalkyl can be divided into bicyclic, tricyclic, tetracyclic or polycyclic bridged cycloalkyl, preferably bicyclic or tricyclic. In one embodiment, the bridged cycloalkyl is optionally substituted bridged cycloalkyl as described elsewhere herein. Non-limiting examples of bridged cycloalkyl include:

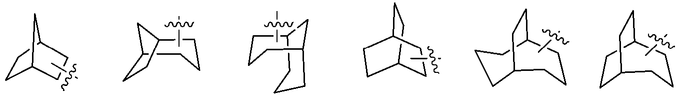

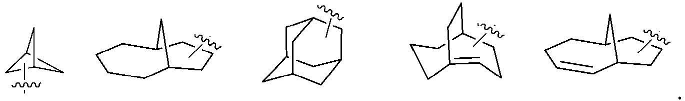

The term "cycloalkylene" refers to divalent cycloalkyl formed by further substituting one hydrogen atom on cycloalkyl, wherein the cycloalkylene is optionally substituted or unsubstituted, and the cycloalkyl is as defined above.

The term "heterocyclyl" refers to a saturated or partially unsaturated monocyclic or polycyclic cyclic hydrocarbon group, wherein one or more ring atoms are heteroatoms selected from nitrogen, oxygen, boron, phosphorus or sulphur, wherein the nitrogen, phosphorus or sulphur atom may be optionally oxidised, the nitrogen atom may be optionally quaternised, the ring carbon atoms may be optionally substituted with oxygen, but excluding ring moieties of -O-O- and -O-S-, and the remaining ring atoms are carbon atoms. The heterocyclyl may contain one or more double bonds but do not have a completely conjugated π electron system. In particular embodiments, the heterocyclyl contains 3 to 20, 3 to 14, 5 to 14, 7 to 14, 3 to 12, 5 to 12, 7 to 12, 7 to 10, 3 to 8 or 3 to 6 ring atoms, of which 1 to 4 are heteroatoms. In one embodiment, the heterocyclyl contains 3 to 6, 4 to 6, 3 to 8, 3 to 10, 6 to 10 or 7 to 11 ring atoms. In one embodiment, the heterocyclyl contains 3 to 8 (e.g., 3, 4, 5, 6, 7 or 8) or 7 to 12 (e.g., 7, 8, 9, 10, 11 or 12) ring atoms. Non-limiting examples of monocyclic heterocyclyl include tetrahydropyrrolyl, azetidinyl, oxetanyl, oxanyl, tetrahydrofuryl, tetrahydropyranyl, tetrahydrothienyl, dihydroimidazolyl, dihydrofuryl, dihydropyrazolyl, dihydropyrrolyl, piperidinyl, piperazinyl, morpholinyl, thiomorpholinyl, homopiperazinyl, pyranyl, etc. Polycyclic heterocyclyl includes spiroheterocyclyl, fused heterocyclyl and bridged heterocyclyl. In one embodiment, the heterocyclyl is optionally substituted heterocyclyl as described elsewhere herein, or heterocyclyl that is further fused to other cycloalkyl, heterocyclyl, aryl and heteroaryl through any two or more atoms on the ring.

The term "spiroheterocyclyl" refers to a polycyclic heterocyclic group in which the rings share one atom (called a spiro atom), wherein one or more ring atoms are heteroatoms selected from nitrogen, oxygen, boron, phosphorus or sulphur, and the remaining ring atoms are carbon atoms. The spiroheterocyclyl may contain one or more double bonds, but no ring has a completely conjugated π electron system. In particular embodiments, the spiroheterocyclyl contains 5 to 20 or 6 to 14 ring atoms. In one embodiment, the spiroheterocyclyl contains 7 to 11 (e.g., 7, 8, 9, 10 or 11) ring atoms. According to the number of shared spiro atoms between the rings, the spiroheterocyclyl is divided into monospiroheterocyclyl, bispiroheterocyclyl or polyspiroheterocyclyl, preferably monospiroheterocyclyl and bispiroheterocyclyl. In one embodiment, the spiroheterocyclyl is 4-membered/4-membered, 4-membered/5-membered, 4-membered/6-membered, 5-membered/5-membered or 5-membered/6-membered monospiroheterocyclyl. In one embodiment, the spiroheterocyclyl is optionally substituted spiroheterocyclyl as described elsewhere herein. Non-limiting examples of spiroheterocyclyl include:

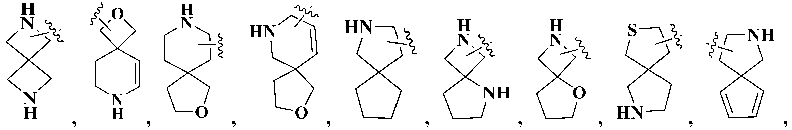

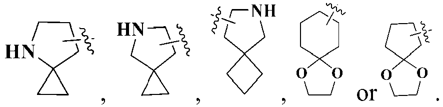

The term "fused heterocyclyl" refers to a polycyclic heterocyclic group with each ring in the system sharing a pair of adjacent atoms with another ring in the system, and one or more rings may contain one or more double bonds, but no ring has a completely conjugated π electron system, wherein one or more ring atoms are heteroatoms selected from nitrogen, oxygen, boron, phosphorus or sulphur, and the remaining ring atoms are carbon atoms. In particular embodiments, the fused heterocyclyl contains 5 to 20 or 6 to 14 ring atoms, and in one embodiment, the fused heterocyclyl contains 7 to 10 (e.g., 7, 8, 9 or 10) ring atoms. According to the number of constituent rings, the fused heterocyclyl can be divided into bicyclic, tricyclic, tetracyclic or polycyclic fused heterocyclyl, preferably bicyclic or tricyclic. In one embodiment, the fused heterocyclyl is 5-membered/5-membered or 5-membered/6-membered bicyclic fused heterocyclyl. In one embodiment, the fused heterocyclyl is optionally substituted fused heterocyclyl as described elsewhere herein, or fused heterocyclyl that can be fused to cycloalkyl, heterocyclyl, aryl or heteroaryl. Non-limiting examples of fused heterocyclyl include:

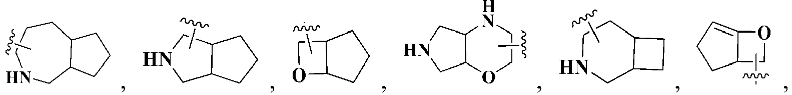

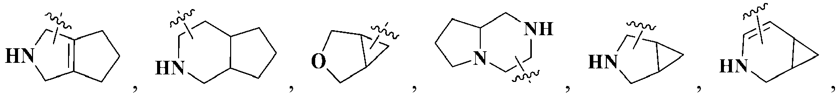

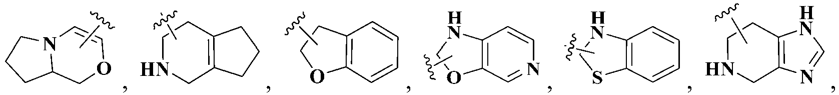

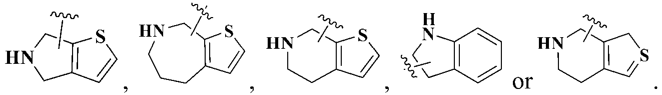

The term "bridged heterocyclyl" refers to a polycyclic heterocyclic group with any two rings sharing two atoms that are not directly connected. The bridged heterocyclyl may contain one or more double bonds, but no ring has a completely conjugated π electron system, wherein one or more ring atoms are heteroatoms selected from nitrogen, oxygen, boron, phosphorus or sulphur, and the remaining ring atoms are carbon atoms. In particular embodiments, the bridged heterocyclyl contains 5 to 20 or 6 to 14 ring atoms. In one embodiment, the bridged heterocyclyl contains 7 to 10 (e.g., 7, 8, 9 or 10) ring atoms. According to the number of constituent rings, the bridged heterocyclyl can be divided into bicyclic, tricyclic, tetracyclic or polycyclic bridged heterocyclyl, preferably bicyclic, tricyclic or tetracyclic. In one embodiment, the bridged heterocyclyl is bicyclic or tricyclic. In one embodiment, the bridged heterocyclyl is optionally substituted bridged heterocyclyl as described elsewhere herein. Non-limiting examples of bridged heterocyclyl include:

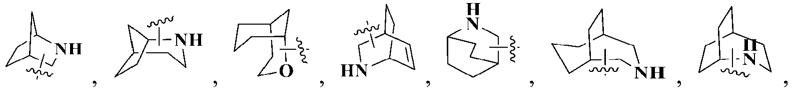

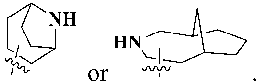

The term "tricyclic heterocyclyl" refers to a heterocyclyl group having three rings in the system, wherein the three rings may be a fused ring system, a spiro ring system, or a bridged ring system. In one embodiment, at least one of the three rings in the tricyclic heterocyclyl system is heterocyclyl, and the other two rings may be cycloalkyl, heterocyclyl, aryl or heteroaryl, wherein the cycloalkyl, heterocyclyl, aryl or heteroaryl is as defined above.Non-limiting examples thereof preferably include the following tricyclic heterocyclyl:

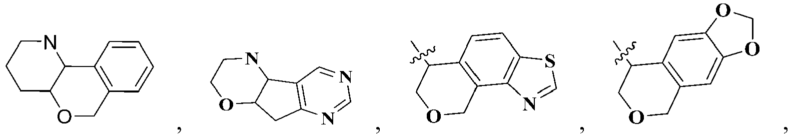

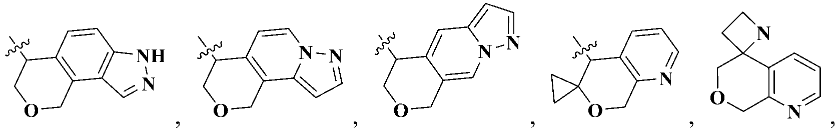

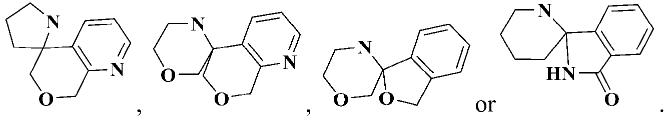

The term "heterocyclylene" refers to divalent heterocyclyl formed by further substituting one hydrogen atom on heterocyclyl, wherein the heterocyclylene is optionally substituted or unsubstituted, and the heterocyclyl is as defined above.

The term "aryl" refers to an all-carbon monocyclic or fused polycyclic (i.e., rings sharing a pair of adjacent carbon atoms) group containing at least one conjugated π electron system, which may be optionally substituted with one or more substituents. In particular embodiments, the aryl contains 6 to 20, 6 to 14, 6 to 12 or 6 to 10 ring atoms. In one embodiment, the aryl may further refer to a bicyclic, tricyclic or tetracyclic ring system, in which at least one ring is an aromatic ring and the other rings may be saturated or partially unsaturated carbocyclic rings or rings containing one or more heteroatoms independently selected from O, S and N. In one embodiment, the aryl is selected from benzo 5- to 10-membered heteroaryl, benzo 3- to 10-membered cycloalkyl or benzo 3- to 10-membered heterocyclyl. In one embodiment, the aryl is selected from benzo 5- or 6-membered heteroaryl, benzo 3-to 6-membered cycloalkyl or benzo 3- to 6-membered heterocyclyl, wherein the heterocyclyl is heterocyclyl containing 1 to 3 nitrogen atoms, oxygen atoms or sulphur atoms. Non-limiting examples thereof include phenyl, naphthyl, fluorenyl, azulenyl, anthryl, phenanthrenyl, pyrenyl, biphenyl, terphenyl, dihydronaphthyl, indenyl, tetrahydronaphthyl (tetralinyl), 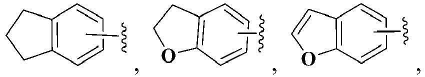 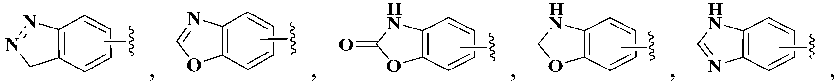 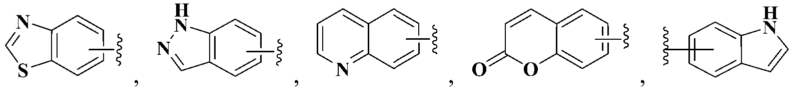 and

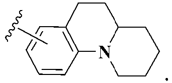

The term "arylene" refers to divalent aryl formed by further substituting one hydrogen atom on aryl, wherein the arylene is optionally substituted or unsubstituted, and the aryl is as defined above.

The term "heteroaryl" refers to an optionally substituted monocyclic or polycyclic group or ring system containing at least one aromatic ring, wherein the aromatic ring has one or more heteroatoms independently selected from O, S and N. In particular embodiments, the heteroaryl contains 5 to 20, 5 to 14, 5 to 12 or 5 to 10 ring atoms, of which 1 to 4 are heteroatoms. In one embodiment, the heteroaryl contains 5 or 6 ring atoms. In particular embodiments, the heteroaryl may further refer to a bicyclic, tricyclic or tetracyclic ring, in which at least one ring is an aromatic ring having one or more heteroatoms independently selected from O, S and N, and the other rings may be saturated or partially unsaturated carbocyclic rings or rings containing one or more heteroatoms independently selected from O, S and N. In one embodiment, the heteroaryl is selected from heteroaryl fused 6- to 10-membered aryl, heteroaryl fused 3- to 10-membered cycloalkyl, or heteroaryl fused 3- to 10-membered heterocyclyl. Further in one embodiment, the heteroaryl is selected from 5- or 6-membered heteroaryl fused 6- to 10-membered aryl, 5- or 6-membered heteroaryl fused 3- to 6-membered cycloalkyl, or 5- or 6-membered heteroaryl fused 3- to 6-membered heterocyclyl, wherein the heterocyclyl is heterocyclyl containing 1 to 3 nitrogen atoms, oxygen atoms or sulphur atoms. Non-limiting examples thereof include: furyl, imidazolyl, isothiazolyl, isoxazolyl, oxadiazolyl, oxazolyl, pyrazinyl, pyrazolyl, pyridazinyl, pyridyl, pyrimidyl, pyrrolyl, thiadiazolyl, thiazolyl, thienyl, tetrazolyl, triazinyl, triazolyl, benzofuryl, benzoimidazolyl, benzisoxazolyl, benzopyranyl, benzothiadiazolyl, benzothienyl, nenzophenylthio, benzothienyl, benzotriazolyl, imidazopyridyl, imidazothiazolyl, indolizinyl, indolyl, indazolyl, isobenzofuryl, isobenzothienyl, isoindolyl, isoquinolyl, naphthyridinyl, oxazolopyridyl, phthalazinyl, pteridyl, purinyl, pyridopyridyl, pyrrolopyridyl, quinolyl, quinoxalinyl, quinazolinyl, thiadiazolopyrimidyl, thienopyridyl, acridyl, benzoindolyl, carbazolyl, dibenzofuryl, phenanthrolinyl, phenanthridinyl, phenarsazinyl, phenazinyl, phenothiazinyl, phenoxazinyl, xanthenyl, 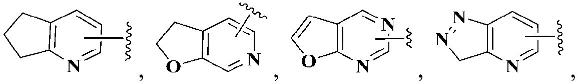 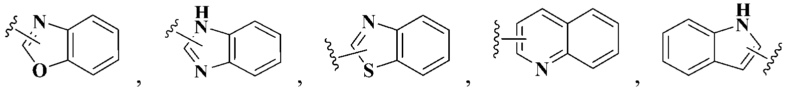 and

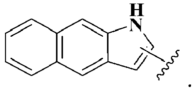

The term "heteroarylene" refers to divalent heteroaryl formed by further substituting one hydrogen atom on cycloalkyl, wherein the heteroarylene is optionally substituted or unsubstituted, and the heteroaryl is as defined above.

The term "heteroalkyl" refers to a stable linear or branched, or cyclic hydrocarbon group, or a combination thereof, and is composed of the indicated number of carbon atoms and one or more (in one embodiment, one to three) heteroatoms selected from O, N, Si and S, wherein the nitrogen and sulphur atoms are optionally oxidised and the nitrogen heteroatom may be optionally quaternised. In one embodiment, the heteroatoms O, N and S may be placed at any interior position of the heteroalkyl group. In one embodiment, the heteroatom Si may be placed at any position (e.g., interior or terminal position) of the heteroalkyl group, including the position where the alkyl group is connected to the rest of the molecule. Non-limiting examples thereof include: -CH₂-CH₂-O-CH₃, -CH₂-CH₂-NH-CH₃, - CH₂-CH₂-N (CH₃)-CH₃, -CH₂-S-CH₂-CH₃, -CH₂-CH₂-S(O)-CH₃, -CH₂-CH₂-S(O)₂-CH₃, -CH=CH-O-CH₃, -Si (CH₃)₃, -CH₂-CH=N-OCH₃ and-CH=CH-N (CH₃)-CH₃. Up to two heteroatoms can be consecutive, for example, -CH₂-NH-O-CH₃ and -CH₂-O-Si(CH₃)₃. In particular embodiments, the heteroalkyl is optionally substituted heteroalkyl as described elsewhere herein.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropoxy, cyclobutoxy, cyclopentyloxy or cyclohexyloxy. In one embodiment, the alkoxy is optionally substituted alkoxy as described elsewhere herein.

The term "alkylacyl" refers to -C(O)-alkyl, wherein the alkyl is as defined above.

The term "haloalkyl" refers to alkyl substituted with one or more halogen, wherein the alkyl is as defined above. Non-limiting examples of haloalkyl include: trifluoromethyl, -CH₂CF₃,

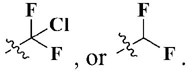

The term "haloalkoxy" refers to alkoxy substituted with one or more halogen, wherein the alkoxy is as defined above.

The term "hydroxyalkyl" refers to alkyl substituted with hydroxyl, wherein the alkyl is as defined above.

The term "alkylthio" refers to -S-(alkyl) and -S-(unsubstituted cycloalkyl), wherein the alkyl or cycloalkyl is as defined above. Non-limiting examples of alkylthio include: methylthio, ethylthio, propylthio, butylthio, cyclopropylthio, cyclobutylthio, cyclopentylthio or cyclohexylthio. In one embodiment, the alkylthio is optionally substituted alkylthio as described elsewhere herein.

The term "haloalkylthio" refers to alkylthio substituted with one or more halogen, wherein the alkylthio is as defined above.

The term "alkenylcarbonyl" refers to -C(O)-(alkenyl), wherein the alkenyl is as defined above. Non-limiting examples of alkenylcarbonyl include: ethenylcarbonyl, propenylcarbonyl, or butenylcarbonyl. In one embodiment, the alkenylcarbonyl is optionally substituted alkenylcarbonyl as described elsewhere herein.

The term "aminocarbonyl" refers to NH₂-C(O)-.

The term "alkylaminocarbonyl" refers to aminocarbonyl (NH₂-C(O)-) in which one or both of the hydrogenare substituted with alkyl, wherein the alkyl is as defined above.

The term "alkylamino" refers to amino in which one or both of the hydrogen are substituted with alkyl, wherein the alkyl is as defined above.

The term "carbonyl" refers to a -C(O)-, -(CO)- or -C(=O)- group. All the symbols are used interchangeably in the description.

The term "halogen" refers to fluorine, chlorine, bromine or iodine. The term "oxo" or "side oxygen" refers to =O.

The term "C(X)" or "C(=X)" refers to 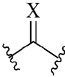 group. When X is O, it indicates that the group is carbonyl; when X is S, it indicates that the group ismercapto; when X is NR, it indicates that the group is 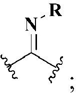 when X is CRR, it indicates that the group is

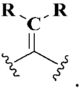

The term "hydrogen" includes proton (¹H), deuterium (²H), tritium (³H) and/or a mixture thereof. In particular embodiments, one or more hydrogen-occupied positions in the compound may be enriched with deuterium and/or tritium. Such isotopically enriched analogues may be prepared from suitable isotopically labelled starting materials obtained from commercial sources or by known literature procedures.

The alkyl, alkylene, alkenyl, alkynyl, cycloalkyl, heterocyclyl, aryl, arylene, heteroaryl, heteroarylene, heteroalkyl, alkoxy, alkylthio, hydroxyalkyl, alkenylcarbonyl, aminocarbonyl, alkylaminocarbonyl, alkylamino or alkylacyl may be substituted or unsubstituted. In one embodiment, the substituent is selected from one or more of the following groups: alkyl, alkenyl, alkynyl, alkoxy, alkylthio, alkylamino, alkylacyl, halogen, mercapto, hydroxyl, nitro, cyano, azidyl, oximido, phosphate group, oxo, thio, carboxyl, carboxylate group, cycloalkyl, heterocyclyl, aryl, heteroaryl, heterocycloalkoxy, cycloalkylthio or heterocycloalkylthio.

Different expressions such as "X is selected from A, B, or C", "X is selected from A, B and C", "X is A, B or C", and "X is A, B and C" all express the same meaning, that is, X can be any one or more of A, B, and C.

"Optional" or "optionally" means that the event or circumstance subsequently described may but not necessarily occur, and the description includes the occasion where the event or circumstance occurs or does not occur. For example, "heterocyclic group optionally substituted with alkyl" means the alkyl may but need not be present, the description includes the case where the heterocyclic group is substituted with alkyl and the case where the heterocyclic group is not substituted with alkyl.

In various parts of the present invention, linking substituents are described. When it is clear that a linking group is required for the structure, the markush variables listed for that group should be understood as referring to the linking group. For example, if a linking group is required for the structure and the markush group definition for that variable lists "alkyl" or "aryl," it should be understood that the "alkyl" or "aryl" respectively represents the attached alkylene group or arylene group.

The term "substituted" means that any one or more hydrogen atoms on the designated atom are substituted with a substituent, provided that the valence state of the designated atom is normal, and the substituted compound is stable in one embodiment. When the substituent is oxo (i.e., =O), it means that two hydrogen atoms are substituted. The term "optionally substituted" means that a group may or may not be substituted. Unless otherwise specified, the type and number of substituents may be arbitrary on the basis of chemical practicability. It goes without saying, the substituents may be only in possible chemical positions thereof, and those skilled in the art can determine the possible or impossible substitutions (by experiment or theory) without paying too much effort. For example, an amino or hydroxyl group with free hydrogen may be unstable when attached to a carbon atom with an unsaturated (e.g., olefinic) bond. The substituents can be selected from deuterium, halogen, amino, hydroxyl, cyano, nitro, alkyl, alkenyl, alkynyl, oxo, thio, deuteroalkyl, haloalkyl, alkoxy, haloalkoxy, hydroxyalkyl, cyano-substituted alkyl, cycloalkyl, heterocyclyl, aryl or heteroaryl.

The term "substituted or unsubstituted" means that a group may or may not be substituted. When a group may be substituted, the substituents are selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -SF₅, -C₃₋₁₂ cycloalkyl, 3- to 12-membered heterocyclyl, C₆₋₁₂ aryl, 5- to 12-membered heteroaryl, C(O)R, C(O)OR, C(O)NRR', N=S(O)RR', S(O)R(=NR'), P(O)RR' or =RR';
R and R' are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, - -C₃₋₁₂ cycloalkyl, 3- to 12-membered heterocyclyl, C₆₋₁₂ aryl or 5- to 12-membered heteroaryl.

Unless stated to the contrary, the indefinite articles "a" and "an" and the definite article "the" in the present description and claims include plural as well as singular forms.

"Pharmaceutical composition" denotes a mixture containing one or more compounds described herein or physiologically/pharmaceutically acceptable salts or prodrugs thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

The "pharmaceutically acceptable salt" refers to salts of the compound of the present invention, which are safe and effective when used in mammals, and have appropriate biological activity.

The "stereoisomer" comprises all enantiomerically/diastereomerically/stereoisomerically pure and enantiomerically/diastereomerically/stereoisomerically enriched compounds of the present invention.

"Stereoisomerically pure" means a composition comprising one stereoisomer of a compound and being substantially free of another stereoisomer of the compound. For example, a stereoisomerically pure composition of a compound having one chiral centre will be substantially free of the opposite enantiomer of the compound. A stereoisomerically pure composition of a compound having two chiral centres will be substantially free of other diastereomers of the compound. A typical stereoisomerically pure compound comprises greater than about 80% by weight of one stereoisomer of the compound and less than about 20% by weight of another stereoisomer of the compound, greater than about 90% by weight of one stereoisomer of the compound and less than about 10% by weight of another stereoisomer of the compound, greater than about 95% by weight of one stereoisomer of the compound and less than about 5% by weight of another stereoisomer of the compound, greater than about 97% by weight of one stereoisomer of the compound and less than about 3% by weight of another stereoisomer of the compound, or greater than about 99% by weight of one stereoisomer of the compound and less than about 1% by weight of another stereoisomer of the compound.

"Stereoisomerically enriched" means a composition comprising greater than about 55% by weight, greater than about 60% by weight, greater than about 70% by weight, or greater than about 80% by weight of one stereoisomer of a compound.

"Enantiomerically pure" means a stereoisomerically pure composition of a compound having one chiral centre. Similarly, the term "enantiomerically enriched" refers to a stereomerically enriched composition of a compound having one chiral centre.

"Optical activity" and "enantiomeric activity" mean a combination of molecules having an enantiomeric or diastereomeric excess of no less than about 50%, no less than about 70%, no less than about 80%, no less than about 90%, no less than about 91%, no less than about 92%, no less than about 93% , no less than about 94%, no less than about 95%, no less than about 96%, no less than about 97%, no less than about 98%, no less than about 99%, no less than about 99.5%, or no less than about 99.8%. In particular embodiments, the compound comprises about 95% or more of the desired enantiomer or diastereomer and about 5% or less of the less preferred enantiomer or diastereomer, based on the total weight of the racemate.

When describing optically active compounds, the prefixes R and S are used to indicate the absolute configuration of the molecule relative to its chiral centre. (+) and (-) are used to represent the optical rotation of the compound, that is, the direction of the plane of polarised light rotated by the optically active compound. The prefix (-) indicates that the compound is levorotatory, that is, the compound rotates the plane of polarised light to the left or counterclockwise. The prefix (+) indicates that the compound is dextrorotatory, that is, the compound rotates the plane of polarised light to the right or clockwise. However, the signs of optical rotation (+) and (-) are independent of the absolute configurations R and S of the molecule.

### Detailed Description of Embodiments

The present invention will be further described below in conjunction with examples, but these examples are not meant to limit the scope of the present invention.

### Example

The structures of the compounds of the present invention are determined by nuclear magnetic resonance (NMR) or/and mass spectrometry (MS). The NMR shift (δ) is given in 10⁻⁶ (ppm). NMR is determined with a Bruker AVANCE-400 nuclear magnetic instrument. The solvents for determination are deuterated dimethyl sulphoxide (DMSO-*d₆*), deuterated chloroform (CDCl₃), and deuterated methanol (CD₃OD), and the internal standard is tetramethylsilane (TMS).

MS is determined with a FINNIGAN LCQAd (ESI) mass spectrometer (manufacturer: Thermo, model: Finnigan LCQ advantage MAX).

HPLC is determined with Agilent 1200DAD high-pressure liquid chromatograph instrument (Sunfire C₁₈ 150 × 4.6 mm chromatographic column) and Waters 2695-2996 high-pressure liquid chromatograph instrument (Gimini C₁₈ 150 × 4.6 mm chromatographic column).

The average kinase inhibition rate and IC₅₀ value are determined with NovoStar microplate reader (BMG Company, Germany).

For thin layer chromatography (TLC), silica gel plates from Yantai Huanghai HSGF254 or Qingdao GF254 are used. Silica gel plates with a thickness of 0.15 mm-0.2 mm are employed for TLC, while plates with a thickness of 0.4 mm-0.5 mm are used for TLC separation and purification.

For column chromatography, Yantai Huanghai silica gel of 200-300 mesh is typically used as a carrier.

The known starting materials of the present invention can be synthesised by methods known in the art, or can be purchased from ABCR GmbH & Co. KG, Acros Organics, Aldrich Chemical Company, Accela ChemBio Inc., Chembee Chemicals Inc. and other companies.

Unless otherwise specially specified in examples, reactions can all be carried out under argon atmosphere or nitrogen atmosphere. Argon atmosphere or nitrogen atmosphere means that a reaction flask is connected to an argon or nitrogen balloon with a volume of about 1 L. Hydrogen atmosphere means that a reaction flask is connected to a hydrogen balloon with a volume of about 1 L.

Pressurised hydrogenation reactions are performed using either Parr 3916EKX hydrogenation apparatus with Qinglan QL-500 hydrogen generator or HC2-SS hydrogenation apparatus.

Hydrogenation reactions are typically performed by evacuating the system and refilling it with hydrogen gas, repeating this operation three times.

Microwave reactions are performed using CEM Discover-S 908860 microwave reactor.

Unless otherwise specially specified in examples, a solution refers to an aqueous solution.

Unless otherwise specially specified in examples, the reaction temperature is room temperature, which is 20°C to 30°C.

The progress of a reaction in the examples is monitored by thin layer chromatography (TLC). Developing agent systems used in the reaction include: A: dichloromethane and methanol system, B: n-hexane and ethyl acetate system, C: petroleum ether and ethyl acetate system, D: acetone, the volume ratio of the solvents is adjusted according to the polarity of the compounds.

Eluent systems for column chromatography and developing agent systems for thin layer chromatography, which are used to purify compounds, include: A: n-hexane and ethyl acetate system, B: n-hexane and tetrahydrofuran system, the volume ratio of the solvents is adjusted according to the polarity of the compounds, and a small amount of basic or acidic reagents such as triethylamine and acetic acid can also be added for adjustment.

The chiral preparative HPLC conditions in the examples are as follows, wherein t_{R} represents retention time:
Chiral preparative conditions:

| | |
|---|---|
| Instrument | WATERS 150 Mgm preparative SFC |
| Column type | ChiralCel AD, 250×30 mm I.D., 10 µm |
| Column pressure | 100 bar |
| Mobile phase | A for CO₂ and B for Ethanol, B 40% |
| Flow rate | 100 mL/min |
| Detection wavelength | UV 254 nm |
| Column temperature | 39°C |

Chiral analysis conditions:

| | |
|---|---|
| Instrument | Waters Acquity UPC2 |
| Column type | Chiralcel AD-3 50*4.6 mm I.D., 3 um |
| Column pressure | 120 bar |
| Mobile phase | A for CO2 and B for Ethanol (0.05% DEA), 40% |
| Flow rate | 2.5 mL/min |
| Detection wavelength | UV 254 nm |
| Column temperature | 25°C |

The compounds in the embodiments of the present invention are prepared with reference to the preparation steps of the following examples:

### Example 1

### 4-Amino-1-methyl-10-(2-trifluoromethyl-5,8-dihydro-6 H-pyrano [3,4-b]pyridin-5-yl)-7,8,9,10-tetrahydroazepino[3,4-g]pyrazolo[4,3-c]quinolin-11(1H)-one

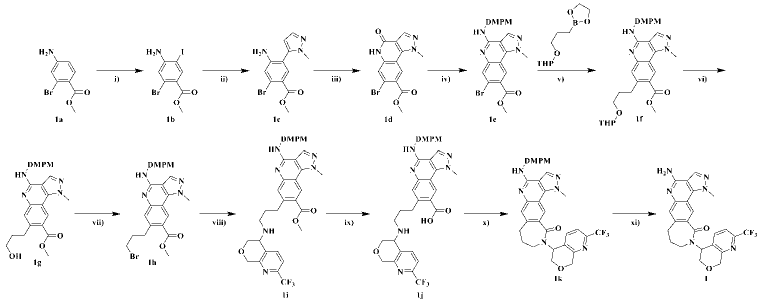

Step 1: Methyl 4-amino-2-bromo-carboxylate 1a (10.00 g, 43.67 mmol) and *N-*iodosuccinimide (10.13 g, 45.00 mmol) were dispersed in acetonitrile (100 mL). The mixture was stirred and reacted at 25°C for 16 hours. The reaction solution was filtered to remove insoluble materials. The filtrate was concentrated under reduced pressure to dryness, and the resulting residue was purified by silica gel column chromatography using eluent system A to obtain **1b** (14.00 g, yield: 90.3%). MS m/z (ESI): 356 [M+1]⁺
Step 2: **1b** (9.00 g, 25.35 mmol), 1-methyl-1*H*-pyrazole-5-boronic acid pinacol ester (10.55 g, 50.70 mmol), [1,1'-bis(diphenylphosphino)ferrocene]palladium(II) dichloride (1.86 g, 2.54 mmol), and potassium phosphate (10.75 g, 50.70 mmol) were dispersed in 1,4-dioxane (150 mL) and water (30 mL). The mixture was stirred and reacted at 80°C for 16 hours. The reaction solution was filtered to remove insoluble materials. The filtrate was concentrated under reduced pressure to dryness, and the resulting residue was purified by silica gel column chromatography using eluent system A to obtain **1c** (6.50 g, yield: 83.0%). MS m/z (ESI): 310 [M+1]⁺
Step 3: **1c** (6.50 g, 21.04 mmol) and *N*,*N*'-carbonyldiimidazole potassium phosphate (6.82 g, 42.08 mmol) were dispersed in *N*-methylpyrrolidone (65 mL). The mixture was stirred and reacted at 150°C for 1 hour. The reaction solution was poured into water (650 mL), and the precipitated solid was filtered to obtain **1d** (5.71 g, yield: 81.0%). MS m/z (ESI): 336 [M+1]⁺
Step 4: **1d** (5.71 g, 17.04 mmol), 2,4-dimethoxybenzylamine (5.69 g, 34.08 mmol), 1,8-diazabicyclo[5.4.0]undec-7-ene (5.18 g, 34.08 mmol) and benzotriazol-1-yloxytris(dimethylamino)phosphonium hexafluorophosphate (15.06 g, 34.08 mmol) were dispersed in dimethyl sulphoxide (50 mL). The mixture was stirred and reacted at 25°C for 1 hour. The reaction solution was poured into water (500 mL), and the precipitated solid was filtered to obtain **1e** (6.41 g, yield: 77.7%). MS m/z (ESI): 486 [M+1]⁺
Step 5: **1e** (5.2 g, 10.72 mmol), 4,4,5,5-tetramethyl-2-(3-(tetrahydro-2H-pyran-2-yl)oxy)propyl)-1,3,2-dioxaborolane (4.3 g, 13.19 mmol), [n-butyl-di(1-adamantyl)phosphine](2-amino-1,1'-biphenyl-2-yl)palladium(II) methanesulphonate (800 mg, 1.10 mmol), and caesium carbonate (5.37 g, 16.48 mmol) were dissolved in dioxane (60 mL) and water (15 mL), and the mixture was reacted under microwave irradiation at 110°C under nitrogen atmosphere for 2 hours. Water (100 mL) was added to the reaction solution, and the mixture was extracted with ethyl acetate (100 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **1f** (3.3 g, yield: 51.7%). MS m/z (ESI): 549 [M+1]⁺
Step 6: **1f** (3.3 g, 6.01 mmol) was dissolved in methanol (60 mL), trifluoroacetic acid (12 mL) was added, and the mixture was stirred and reacted at room temperature for 2 hours. The reaction solution was adjusted to pH=8-9 with a sodium bicarbonate aqueous solution, extracted with dichloromethane (containing 10% methanol, 100 mL×2), washed with a saturated sodium chloride aqueous solution, dried over anhydrous sodium sulphate, and filtered. The filtrate was concentrated under reduced pressure, and the resulting residue was purified by silica gel column chromatography using eluent system C to obtain **1g** (2.2 g, yield: 78.9%). MS m/z (ESI): 465 [M+1]⁺
Step 7: **1g** (2.2 g, 4.74 mmol) was dissolved in dichloromethane (120 mL), and the mixture was purged three times with nitrogen and cooled to 0°C. Triphenylphosphine (2.49 g, 9.48 mmol) and carbon tetrabromide (3.14g, 9.48 mmol) were then added, and the system was reacted at 25°C for 2 hours. The reaction solution was distilled under reduced pressure, and the resulting residue was purified by silica gel column chromatography using eluent system C to obtain **1h** (1.92 g, yield: 77.1%). MS m/z (ESI): 527 [M+1]⁺
Step 8: **1h** (543 mg, 1.03 mmol), 2-(trifluoromethyl)-5,8-dihydro-6H-pyrano[3,4-b]pyridin-5-amine (270 mg, 1.24 mmol), sodium iodide (309 mg, 2.06 mmol) and potassium carbonate (427 mg, 3.09 mmol) were dissolved in acetonitrile (60 mL), and the mixture was purged three times with nitrogen. The reaction system was reacted at 80°C for 15 hours. The reaction solution was quenched with water, extracted with ethyl acetate (50 mL × 2), and washed with a saturated sodium chloride aqueous solution (50 mL × 2). The organic phase was dried over anhydrous sodium sulphate, the filtrate was concentrated under reduced pressure, and the resulting residue was purified by silica gel column chromatography using eluent system C to obtain **1i** (351 mg, yield: 51.3%). MS m/z (ESI): 665 [M+1]⁺
Step 9: **1i** (351 mg, 0.53 mmol) was dissolved in tetrahydrofuran (5 mL), water (5 mL) and methanol (5 mL), lithium hydroxide (63 mg, 2.64 mmol) was added, and the mixture was purged three times with nitrogen. The reaction system was reacted at 25°C for 16 hours. The resulting reaction system was adjusted to pH 5-6 with 1 M dilute hydrochloric acid, extracted with ethyl acetate (50 mL × 3), and washed with a saturated sodium chloride aqueous solution (50 mL × 2). The organic phase was dried over anhydrous sodium sulphate, and the filtrate was concentrated under reduced pressure to obtain **1j** (237 mg, yield: 68.7%). MS m/z (ESI): 651 [M+1]⁺
Step 10: **1j** (234 mg, 0.36 mmol) and 2-(7-azobenzotriazol-1-yl)-N,N,N',N'-tetramethyluronium hexafluorophosphate (68 mg, 0.54 mmol) were dissolved in N,N-dimethylformamide (20 mL), N,N-diisopropylethylamine (93 mg, 0.72 mmol, 72 µL) was added, and the reaction system was reacted at 25°C for 48 hours. The resulting reaction system was quenched with water, extracted with ethyl acetate (80 mL × 3), washed with a saturated sodium chloride aqueous solution, and dried over anhydrous sodium sulphate, and the filtrate was concentrated under reduced pressure. The residue was purified by silica gel column chromatography using eluent system A to obtain **1k** (122 mg, yield: 53.9%). MS m/z (ESI): 633 [M+1]⁺
Step 11: **1k** (122 mg, 0.19 mmol) was dissolved in trifluoroacetic acid (2 mL), and the reaction system was reacted at 90°C for 0.5 hours. The resulting reaction system was concentrated, and the residue was purified by silica gel column chromatography using eluent system A to obtain **Example 1** (47 mg, yield: 50.5%). MS m/z (ESI): 483 [M+1]⁺
¹H NMR (400 MHz, DMSO-*d6*) δ 9.23 (s, 1H), 8.33 (dd, 1H), 8.22 (s, 1H), 8.08 (br s, 2H), 7.87 (dd, 1H), 7.44 (d, 1H), 6.71 (t, 1H), 5.63-5.36 (m, 1H), 4.89-4.70 (m, 2H), 4.47-4.13 (m, 2H), 3.94-3.64 (m, 2H), 3.09 (dd, 2H), 2.18-1.91 (m, 2H).

### Examples 1-P1 & 1-P2

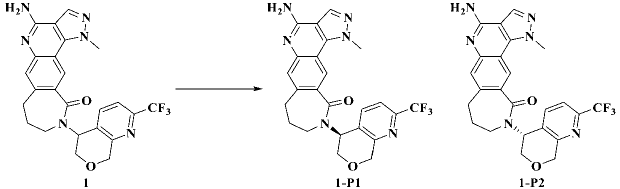

**Example 1** (45 mg, 0.093 mmol) was separated by chiral preparative HPLC to obtain rel-(S)-4-amino-1-methyl-10-(2-trifluoromethyl-5,8-dihydro-6H-pyrano[3,4-b]pyridin-5-yl)-7,8,9,10-tetrahydroazepino[3,4-g]pyrazolo[4,3-c]quinolin-11(1H)-one **1-P1** (21 mg, yield: 46.7%) and rel-(R)-4-amino-1-methyl-10-(2-trifluoromethyl-5,8-dihydro-6H-pyrano[3,4-b]pyridin-5-yl)-7,8,9,10-tetrahydroazepino[3,4-g]pyrazolo[4,3-c]quinolin-11(1H)-one **1-P2** (21 mg, yield: 46.7%).

**1-P1:** ¹H NMR (400 MHz, DMSO-*d6*) δ 9.23 (s, 1H), 8.33 (dd, 1H), 8.22 (s, 1H), 8.08 (br s, 2H), 7.87 (dd, 1H), 7.44 (d, 1H), 6.71 (t, 1H), 5.63-5.36 (m, 1H), 4.89-4.70 (m, 2H), 4.47-4.13 (m, 2H), 3.94-3.64 (m, 2H), 3.09 (dd, 2H), 2.18-1.91 (m, 2H).

**1-P2:** ¹H NMR (400 MHz, DMSO-*d6*) δ 9.23 (s, 1H), 8.33 (dd, 1H), 8.22 (s, 1H), 8.08 (br s, 2H), 7.87 (dd, 1H), 7.44 (d, 1H), 6.71 (t, 1H), 5.63-5.36 (m, 1H), 4.89-4.70 (m, 2H), 4.47-4.13 (m, 2H), 3.94-3.64 (m, 2H), 3.09 (dd, 2H), 2.18-1.91 (m, 2H).

The following examples were prepared by reference to **Example 1:**

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **2** | 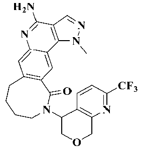 | 497 | **28** | 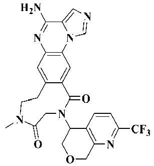 | 526 |
| **2-P1** | 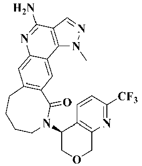 | 497 | **29** | 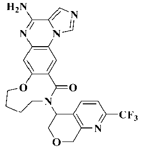 | 499 |
| **2-P2** | 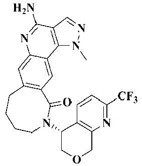 | 497 | **30** | 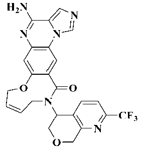 | 497 |
| **3** | 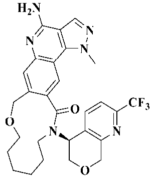 | 555 | **31** | 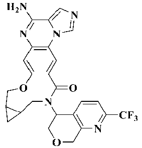 | 511 |
| **3-P1** | 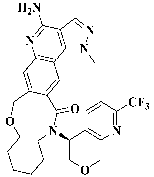 | 555 | **32** | 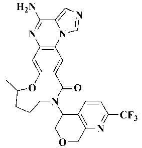 | 513 |
| **3-P2** | 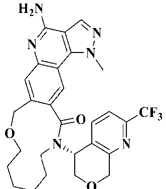 | 555 | **33** | 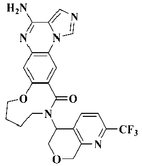 | 499 |
| **4** | 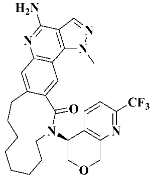 | 553 | **34** | 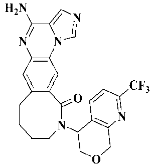 | 483 |
| **4-P1** | 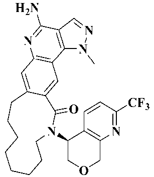 | 553 | **35** | 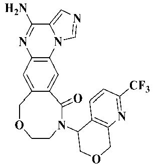 | 485 |
| **4-P2** | 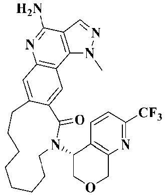 | 553 | **36** | 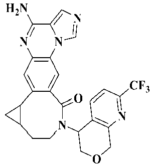 | 495 |
| **5** | 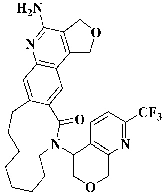 | 541 | **37** | 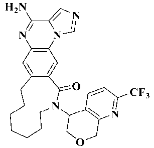 | 525 |
| **6** | 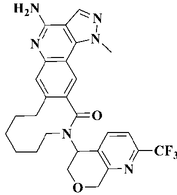 | 525 | **38** | 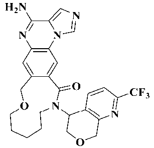 | 527 |
| **7** | 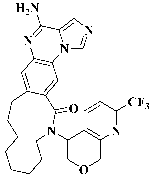 | 539 | **39** | 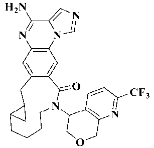 | 537 |
| **8** | 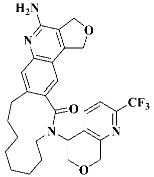 | 541 | **40** | 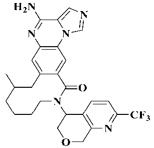 | 525 |
| **9** | 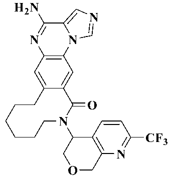 | 511 | **41** | 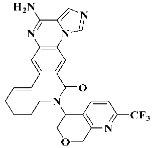 | 509 |
| **10** | 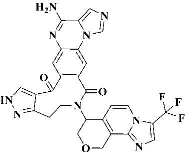 | 588 | **42** | 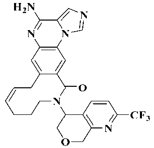 | 509 |
| **11** | 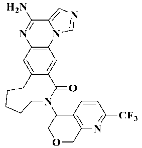 | 497 | **43** | 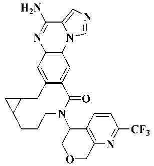 | 523 |
| **12** | 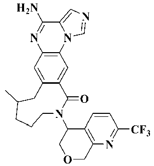 | 511 | **44** | 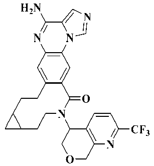 | 523 |
| **13** | 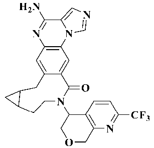 | 509 | **45** | 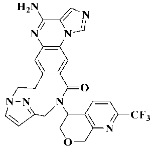 | 535 |
| **14** | 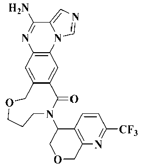 | 499 | **46** | 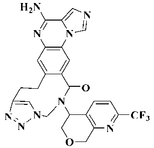 | 536 |
| **15** | 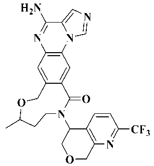 | 513 | **47** | 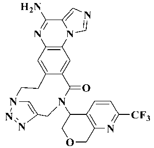 | 536 |
| **15-P1** | 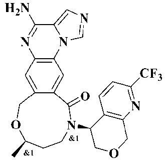 | 515 | **48** | 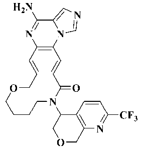 | 513 |
| **15-P2** | 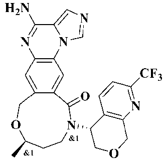 | 515 | **49** | 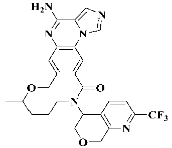 | 527 |
| **16** | 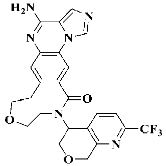 | 499 | **50** | 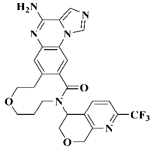 | 513 |
| **17** | 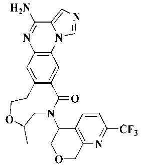 | 513 | **51** | 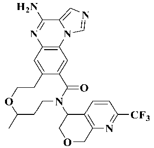 | 527 |
| **18** | 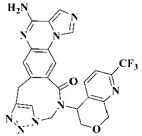 | 522 | **52** | 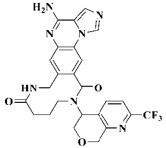 | 526 |
| **19** | 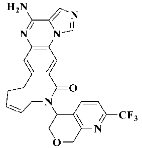 | 495 | **53** | 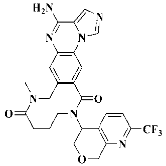 | 540 |
| **20** | 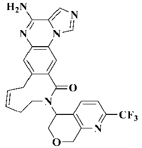 | 495 | **54** | 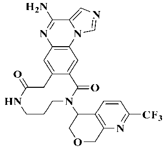 | 526 |
| **21** | 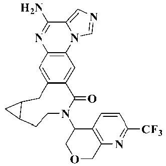 | 509 | **55** | 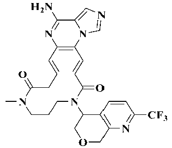 | 540 |
| **22** | 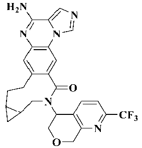 | 509 | **56** | 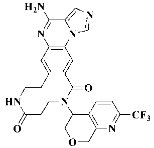 | 526 |
| **23** | 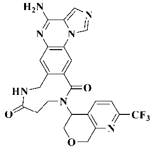 | 512 | **57** | 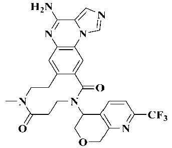 | 540 |
| **24** | 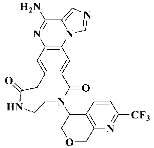 | 512 | **58** | 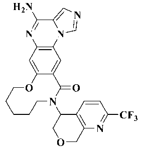 | 513 |
| **25** | 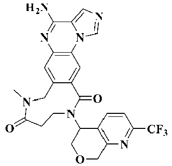 | 526 | **59** | 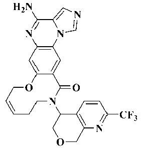 | 511 |
| **26** | 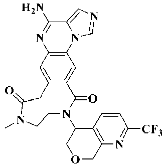 | 526 | **60** | 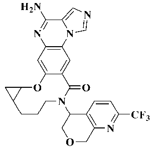 | 525 |
| **27** | 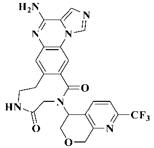 | 512 | **61** | 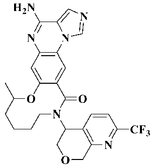 | 527 |

Alternatively, **Examples 14, 15,** and **48** were synthesised by reference to the following preparation method:

### Example 14

### 4-Amino-12-(2-(trifluoromethyl)-5,8-dihydro-6H-pyrano[3,4-b]pyridin-5-yl)-9,10,11,12-tetrahydroimidazo[1,5-a][1,5]oxazonino[8,7-g]quinoxalin-13(7H)-one

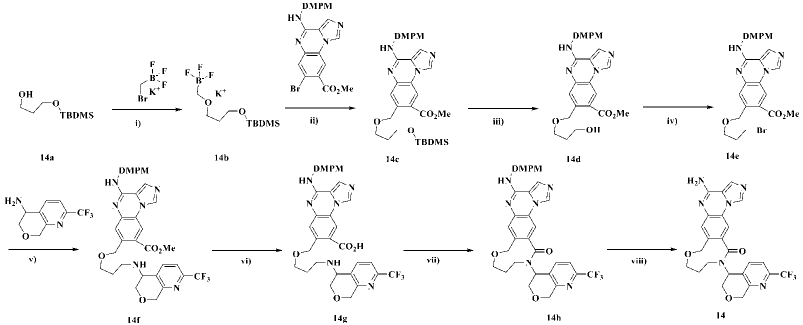

Step 1: 3-((tert-Butyldimethylsilyl)oxy)propan-1-ol **14a** (10 g, 52.53 mmol) was dissolved in tetrahydrofuran (100 mL), and sodium hydride (2.73 g, 68.29 mmol, 60% purity) was added in an ice bath. The mixture was stirred for 15 minutes, potassium (bromomethyl)trifluoroborate (10.02 g, 49.91 mmol) was then added, and the mixture was stirred and reacted at room temperature for about 16 hours. A potassium bifluoride solution (4.5 M) was added to the reaction solution, and the mixture was stirred at room temperature for 30 minutes. The reaction solution was subjected to rotary evaporation to dryness, and then hot acetone (1000 L) was added, and the resulting mixture was stirred at 80°C for about 15 minutes. After hot filtration to remove impurities, the acetone was distilled under reduced pressure until solids precipitated. Twice the volume of diethyl ether was added, and the mixture was filtered in an ice bath. The filter residue was dried to obtain **14b** (4.3 g, yield: 26.4%).
Step 2: Methyl 7-bromo-4-((3,4-dimethoxybenzyl)amino)imidazo[1,5-a]quinoxaline-8-carboxylate (5.2 g, 10.99 mmol), potassium (8,8,9,9-tetramethyl-3,7-dioxa-8-sila-1-boradecan-1-yl)trifluoroborate **14b** (4.3 g, 13.19 mmol), [n-butyl-di(1-adamantyl)phosphine](2-amino-1,1'-biphenyl-2-yl)palladium(II) methanesulphonate (800 mg, 1.10 mmol) and caesium carbonate (5.37 g, 16.48 mmol) were dissolved in dioxane (60 mL) and water (15 mL), and the mixture was reacted under microwave irradiation at 110°C under nitrogen atmosphere for 2 hours. Water (100 mL) was added to the reaction solution, and the mixture was extracted with ethyl acetate (100 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **14c** (3.3 g, yield: 50.5%). MS m/z (ESI): 595 [M+1]⁺
Step 3: **14c** (3.3 g, 5.56 mmol) was dissolved in tetrahydrofuran (60 mL), tetrabutylammonium fluoride (2.90 g, 11.10 mmol, 2M in THF) was added, and the mixture was stirred and reacted at room temperature for 2 hours. The reaction was quenched by adding a saturated ammonium chloride solution (100 mL) to the reaction solution, and extracted with ethyl acetate (100 mL × 2). After extraction, the organic phase was separated, dried over anhydrous sodium sulphate, and filtered. The filtrate was concentrated under reduced pressure, and the resulting residue was purified by silica gel column chromatography using eluent system C to obtain **14d** (1.5 g, yield: 56.3%). MS m/z (ESI): 481 [M+1]⁺
Step 4: **14d** (1.5 g, 3.12 mmol) was dissolved in dichloromethane (120 mL), and the mixture was purged three times with nitrogen and cooled to 0°C. Triphenylphosphine (1.64 g, 6.24 mmol) and carbon tetrabromide (2.07g, 6.24 mmol) were then added, and the system was reacted at 25°C for 2 hours. The reaction solution was distilled under reduced pressure, and the resulting residue was purified by silica gel column chromatography using eluent system C to obtain **14e** (630 mg, yield: 37.1%). MS m/z (ESI): 543 [M+1]⁺
Step 5: **14e** (560 mg, 1.03 mmol), 2-(trifluoromethyl)-5,8-dihydro-6H-pyrano[3,4-b]pyridin-5-amine (270 mg, 1.24 mmol), sodium iodide (309 mg, 2.06 mmol) and potassium carbonate (427 mg, 3.09 mmol) were dissolved in acetonitrile (60 mL), and the mixture was purged three times with nitrogen. The reaction system was reacted at 80°C for 15 hours. The reaction solution was quenched with water, extracted with ethyl acetate (50 mL × 2), and washed with a saturated sodium chloride aqueous solution (50 mL × 2). The organic phase was dried over anhydrous sodium sulphate, the filtrate was concentrated under reduced pressure, and the resulting residue was purified by silica gel column chromatography using eluent system C to obtain **14f** (360 mg, yield: 51.3%). MS m/z (ESI): 681 [M+1]⁺
Step 6: **14f** (350 mg, 0.51 mmol) was dissolved in tetrahydrofuran (5 mL), water (5 mL) and methanol (5 mL), lithium hydroxide (62 mg, 2.57 mmol) was added, and the mixture was purged three times with nitrogen. The reaction system was reacted at 25°C for 16 hours. The resulting reaction system was adjusted to pH 5-6 with 1 M dilute hydrochloric acid, extracted with ethyl acetate (50 mL × 3), and washed with a saturated sodium chloride aqueous solution (50 mL × 2). The organic phase was dried over anhydrous sodium sulphate, and the filtrate was concentrated under reduced pressure to obtain **14g** (125 mg, yield: 36.5%). MS m/z (ESI): 667 [M+1]⁺
Step 7: **14g** (125 mg, 0.18 µmol) and 2-(7-azobenzotriazol-1-yl)-N,N,N',N'-tetramethyluronium hexafluorophosphate (68 mg, 0.54 mmol) were dissolved in N,N-dimethylformamide (20 mL), N,N-diisopropylethylamine (93 mg, 0.72 mmol, 72 µL) was added, and the reaction system was reacted at 25°C for 48 hours. The resulting reaction system was quenched with water, extracted with ethyl acetate (80 mL × 3), washed with a saturated sodium chloride aqueous solution, and dried over anhydrous sodium sulphate, and the filtrate was concentrated under reduced pressure. The residue was purified by silica gel column chromatography using eluent system A to obtain **14h** (63 mg, yield: 53.9%). MS m/z (ESI): 649 [M+1]⁺
Step 8: **14h** (63 mg, 0.10 mmol) was dissolved in trifluoroacetic acid (2 mL), and the reaction system was reacted at 90°C for 0.5 hours. The resulting reaction system was concentrated, and the residue was purified by silica gel column chromatography using eluent system A to obtain **Example 14** (9.7 mg, yield: 19.4%). MS m/z (ESI): 499 [M+1]⁺
¹H NMR (400 MHz, DMSO-*d6*) δ 9.23 (d, 1H), 8.33 (dd, 1H), 8.22 (s, 1H), 8.08 (s, 2H), 7.87 (dd, 1H), 7.44 (d, 1H), 5.63 - 5.36 (m, 1H), 4.89 - 4.70 (m, 3H), 4.47 - 4.13 (m, 3H), 3.94 - 3.64 (m, 2H), 3.09 (dd, 2H), 2.18 - 1.91 (m, 1H), 1.15 (d, 1H).

### Example 15

### 4-Amino-9-methyl-12-(2-(trifluoromethyl)-5,8-dihydro-6H-pyrano[3,4-b]pyridin-5-yl)-9,10,11,12-tetrahydroimidazo [1,5-a] [1,5] oxazolo [8,7-g]quinoxalin-13(7H)-one

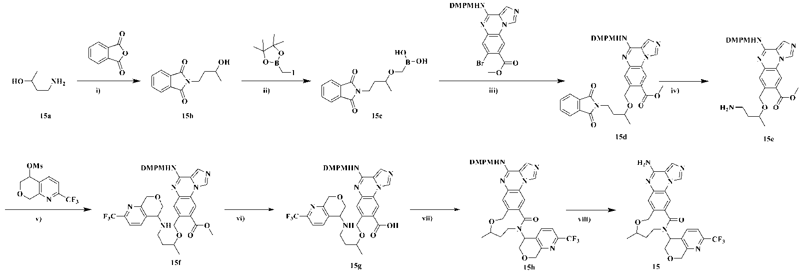

Step 1: 4-Aminobutan-2-ol **15a** (1.5 g, 16.83 mmol) and isobenzofuran-1,3-dione (2.99 g, 20.19 mmol) were dissolved in toluene (15 mL). The mixture was purged three times with nitrogen, and stirred and reacted at 120°C under nitrogen atmosphere for 16 hours. The reaction solution was concentrated under reduced pressure, and the concentrated residue was separated and purified by Pre-HPLC to obtain **15b** (2.5 g, yield: 67.8%). MS m/z (ESI): 220 [M+1]⁺
Step 2: NaH (729.81 mg, 18.25 mmol, 60% purity) was suspended in DMF (10 mL), and the reaction system was cooled to 0°C. **15b** (2 g, 9.12 mmol)/DMF (10 mL) was added, and the mixture was stirred at 0°C for 15 minutes. 2-(Iodomethyl)-4,4,5,5-tetramethyl-1,3,2-dioxaborolane (4.89 g, 18.25 mmol)/DMF (10 mL) was added, and the resulting mixture was purged three times with nitrogen, and stirred and reacted at 25°C under nitrogen atmosphere for 6 hours. Water (60 mL) was added to the reaction solution, and the mixture was extracted with ethyl acetate (60 mL × 3). The organic phases were combined, washed with water (60 mL × 3), dried over anhydrous sodium sulphate, filtered, and concentrated under reduced pressure, and the concentrated residue was separated and purified by Pre-HPLC to obtain **15c** (2 g, yield: 79.1%). MS m/z (ESI): 278 [M+1]⁺
Step 3: Methyl 7-bromo-4-[(2,4-dimethoxyphenyl)methylamino]imidazo[1,5-a]quinoxaline-8-carboxylate (1.5 g, 3.18 mmol) and [3-(1,3-dioxoisoindolin-2-yl)-1-methylpropoxy]methylboronic acid **15c** (1.06 g, 3.82 mmol) were dissolved in 1'4-dioxane (15 mL) and water (1.5 mL), and cataCXium A Pd G3 (347.68 mg, 477 µmol) and Cs₂CO₃ (3.11 g, 9.55 mmol) were sequentially added. The mixture was purged three times with nitrogen, and stirred and reacted under microwave irradiation at 110°C under nitrogen atmosphere for 1.5 hours. Water (60 mL) was added to the reaction solution, and the mixture was extracted with ethyl acetate (60 mL × 3). The organic phases were combined, washed with water (60 mL × 3), dried over anhydrous sodium sulphate, filtered, and concentrated under reduced pressure, and the concentrated residue was separated and purified using purification system C to obtain **15d** (3 g, yield: 25.2%). MS m/z (ESI): 624 [M+1]⁺
Step 4: **15d** (3 g, 2.41 mmol) was dissolved in EtOH (30 mL), and NH₂NH₂ (181.35 mg, 4.81 mmol, 85% purity) was added. The mixture was purged three times with nitrogen, and stirred and reacted at 90°C under nitrogen atmosphere for 3 hours. The reaction solution was concentrated under reduced pressure, and the concentrated residue was separated and purified by Pre-HPLC to obtain **15e** (470 mg, yield: 39.6%). MS m/z (ESI): 494 [M+1]⁺
Step 5: [2-(Trifluoromethyl)-6,8-dihydro-5H-pyrano[3,4-b]pyridin-5-yl]methanesulphonate (100 mg, 336 µmol) and **15e** (182.64 mg, 370 µmol) were dissolved in MeCN (10 mL), and Nal (100.85 mg, 673 µmol) and DIEA (86.96 mg, 673 µmol, 117 µL) were sequentially added. The mixture was purged three times with nitrogen, and stirred and reacted at 80°C under nitrogen atmosphere for 16 hours. The reaction solution was filtered, and the filtrate was concentrated under reduced pressure to obtain **15f** (155 mg, yield: 66.3%). MS m/z (ESI): 695 [M+1]⁺
Step 6: **15f** (155 mg, 223 µmol) was dissolved in THF (3 mL), MeOH (1 mL) and water (1 mL), and LiOH (21.37 mg, 892.47 µmol) was added. The mixture was purged three times with nitrogen, and stirred and reacted at 25°C under nitrogen atmosphere for 16 hours. The reaction solution was concentrated under reduced pressure, dissolved in water (10 mL), adjusted to pH = 4-5 with 2 M dilute hydrochloric acid, and extracted with ethyl acetate (20 mL × 3). The organic phases were combined, dried over anhydrous sodium sulphate, filtered, and concentrated under reduced pressure to obtain **15g** (52 mg, yield: 34.2%). MS m/z (ESI): 682 [M+1]⁺
Step 7: **15g** (52 mg, 76 µmol) was dissolved in DMF (5 mL), and DIEA (29.62 mg, 229 µmol, 39.92 µL) and HATU (43.23 mg, 115 µmol) were sequentially added. The mixture was purged three times with nitrogen, and stirred and reacted at 25°C under nitrogen atmosphere for 16 hours. The reaction solution was concentrated under reduced pressure to obtain **15h** (50 mg, crude).
Step 8: **15h** (50 mg, 60 µmol) was dissolved in TFA (2 mL), and the mixture was purged three times with nitrogen, and stirred and reacted at 80°C under nitrogen atmosphere for 0.5 hours. The reaction solution was concentrated under reduced pressure, and the concentrated residue was separated and purified by Pre-HPLC to obtain **Example 15** (17 mg). MS m/z (ESI): 513 [M+1]⁺
¹H NMR (400 MHz, DMSO-*d6*) δ 9.13 (s, 1H), 8.27 (d,1H), 7.99 (s, 1H), 7.90 (s, 1H), 7.85 (d, 1H), 7.37 (d, 3H), 5.38 (s, 1H), 4.92 - 4.71 (m, 3H), 4.50 - 4.35 (m, 2H), 4.26 (dd, 1H), 3.67 (d, 1H), 3.44 (dd, 1H), 1.32 - 1.03 (m, 3H), 0.94 (d, 3H).

### Examples 15-P1 & 15-P2

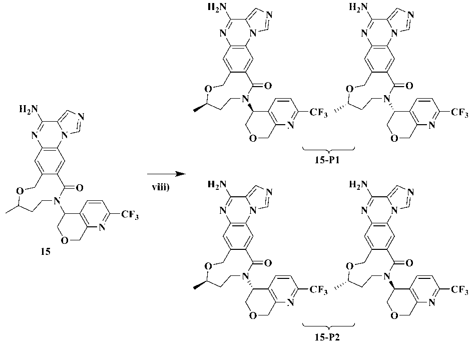

**Example 15** (17 mg, 0.33 mmol) was separated by preparative HPLC to obtain an enantiomeric mixture of (R)-4-amino-9-methyl-12-((S)-2-(trifluoromethyl)-5,8-dihydro-6H-pyrano[3,4-b]pyridin-5-yl)-9,10,11,12-tetrahydroimidazo[1,5-a][1,5]oxazolo[8,7-g]quinoxalin-13(7H)-one and (S)-4-amino-9-methyl-12-((R)-2-(trifluoromethyl)-5,8-dihydro-6H-pyrano[3,4-b]pyridin-5-yl)-9,10,11,12-tetrahydroimidazo[1,5-a][1,5]oxazolo[8,7-g]quinoxalin-13(7H)-one (**15-P1,** 7.6 mg, yield: 44.7%) and an enantiomeric mixture of (R)-4-amino-9-methyl-12-((R)-2-(trifluoromethyl)-5,8-dihydro-6H-pyrano[3,4-b]pyridin-5-yl)-9,10,11,12-tetrahydroimidazo[1,5-a][1,5]oxazolo[8,7-g]quinoxalin-13(7H)-one and (S)-4-amino-9-methyl-12-((S)-2-(trifluoromethyl)-5,8-dihydro-6H-pyrano[3,4-b]pyridin-5-yl)-9,10,11,12-tetrahydroimidazo[1,5-a][1,5]oxazolo[8,7-g]quinoxalin-13(7H)-one (**15-P2**, 7.9 mg, yield: 46.5%).

**15-P1:** ¹H NMR (400 MHz, DMSO-*d6*) δ 9.13 (s, 1H), 8.27 (d,1H), 7.99 (s, 1H), 7.90 (s, 1H), 7.85 (d, 1H), 7.37 (d, 3H), 5.38 (s, 1H), 4.92 - 4.71 (m, 3H), 4.50 - 4.35 (m, 2H), 4.26 (dd, 1H), 3.67 (d, 1H), 3.44 (dd, 1H), 1.32 - 1.03 (m, 3H), 0.94 (d, 3H).

**15-P2:** ¹H NMR (400 MHz, DMSO-*d6*) δ 9.13 (s, 1H), 8.27 (d,1H), 7.99 (s, 1H), 7.90 (s, 1H), 7.85 (d, 1H), 7.37 (d, 3H), 5.38 (s, 1H), 4.92 - 4.71 (m, 3H), 4.50 - 4.35 (m, 2H), 4.26 (dd, 1H), 3.67 (d, 1H), 3.44 (dd, 1H), 1.32 - 1.03 (m, 3H), 0.94 (d, 3H).

### Example 48

### 4-Amino-13-(2-(trifluoromethyl)-5,8-dihydro-6H-pyrano[3,4-b]pyridin-5-yl)-10,11,12,13-tetrahydro-7H-imidazo [1,5-a] [1,6] oxazino [3,4-g] quinoxalin-14(9H)-one

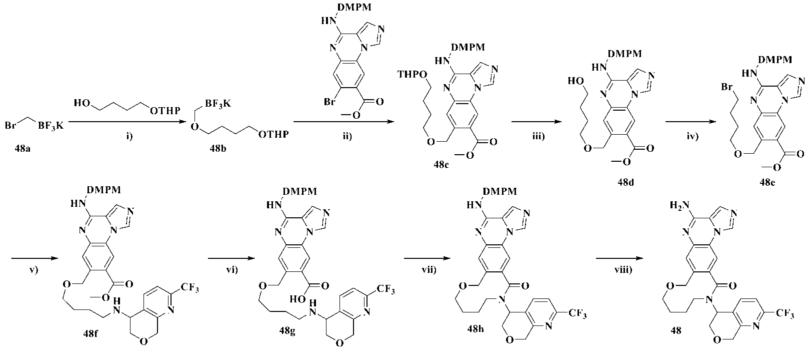

Step 1: 4-((Tetrahydro-2H-pyran-2-yl)oxy)butan-1-ol **48a** (6.6 g, 37.88 mmol) was dissolved in tetrahydrofuran (100 mL), and sodium hydride (1.52 g, 37.88 mmol, 60% purity) was added in an ice bath. The mixture was stirred for 15 minutes, potassium (bromomethyl)trifluoroborate (7.61 g, 37.88 mmol) was then added, and the mixture was stirred and reacted at room temperature for about 16 hours. A potassium bifluoride solution (4.5 M) was added to the reaction solution, and the mixture was stirred at room temperature for 30 minutes. The reaction solution was subjected to rotary evaporation to dryness, and then hot acetone (100 mL) was added, and the resulting mixture was stirred at 80°C for about 15 minutes. After hot filtration to remove impurities, the acetone was distilled under reduced pressure until solids precipitated. Twice the volume of diethyl ether was added, and the mixture was filtered in an ice bath. The filter residue was dried to obtain **48b** (6.7 g, 22.78 mmol, yield: 60.1%).
Step 2: **48b** (1.25 g, 4.24 mmol), [n-butyl-di(1-adamantyl)phosphine](2-amino-1,1'-biphenyl-2-yl)palladium(II) methanesulphonate (309.05 mg, 424.36 µmol) and caesium carbonate (4.15 g, 12.73 mmol) were dissolved in dioxane (40 mL) and water (10 mL), and the mixture was reacted at 100°C under nitrogen atmosphere for 16 hours. Water (100 mL) was added to the reaction solution, and the mixture was extracted with ethyl acetate (100 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **48c** (2.46 g, yield: 100%). MS m/z (ESI): 579 [M+1]⁺
Step 3: **48c** (2.46 g, 4.25 mmol) was added to a single-necked flask containing methanol (20 mL) and tetrahydrofuran (20 mL), p-toluenesulphonic acid (73.12 mg, 425.12 µmol) was added under nitrogen atmosphere, and the mixture was stirred at room temperature for 2 hours. The reaction was quenched by adding a saturated ammonium chloride solution (100 mL) to the reaction solution, and extracted with ethyl acetate (100 mL × 2). After extraction, the organic phase was separated, dried over anhydrous sodium sulphate, and filtered. The filtrate was concentrated under reduced pressure, and the resulting residue was purified by silica gel column chromatography using eluent system C to obtain **48d** (1.9 g, yield: 90.4%). MS m/z (ESI): 495 [M+1]⁺
Step 4: **48d** (2 g, 4.04 mmol) was dissolved in dichloromethane (20 mL), and the mixture was purged three times with nitrogen and cooled to 0°C. Triphenylphosphine (1.59 g, 6.07 mmol) and carbon tetrabromide (2.01 g, 6.07 mmol) were then added, and the system was reacted at 25°C for 2 hours. The reaction solution was distilled under reduced pressure, and the resulting residue was purified by silica gel column chromatography using eluent system C to obtain **48e** (1.5 g, yield: 66.5%). MS m/z (ESI): 558 [M+1]⁺
Step 5: **48e** (0.5 g, 896.97 µmol), 2-(trifluoromethyl)-5,8-dihydro-6H-pyrano[3,4-b]pyridin-5-amine (391.39 mg, 1.79 mmol), sodium iodide (269.09 mg, 1.79 mmol) and DIEA (352.50 mg, 2.73 mmol, 475.07 µL) were dissolved in acetonitrile (20 mL), and the mixture was purged three times with nitrogen. The reaction system was reacted at 80°C for 15 hours. The reaction solution was quenched with water, extracted with ethyl acetate (50 mL × 2), and washed with a saturated sodium chloride aqueous solution (50 mL × 2). The organic phase was dried over anhydrous sodium sulphate, the filtrate was concentrated under reduced pressure, and the resulting residue was purified by silica gel column chromatography using eluent system C to obtain **48f** (190 mg, yield: 30.5%). MS m/z (ESI): 695 [M+1]⁺
Step 6: **48f** (0.19 g, 273.50 µmol) was dissolved in tetrahydrofuran (5 mL), water (5 mL) and methanol (5 mL), lithium hydroxide (45.95 mg, 1.09 mmol) was added, and the mixture was purged three times with nitrogen. The reaction system was reacted at 25°C for 16 hours. The resulting reaction system was adjusted to pH 5-6 with 1 M dilute hydrochloric acid, extracted with ethyl acetate (50 mL × 3), and washed with a saturated sodium chloride aqueous solution (50 mL × 2). The organic phase was dried over anhydrous sodium sulphate, and the filtrate was concentrated under reduced pressure to obtain **48g** (130 mg, yield: 69.8%). MS m/z (ESI): 681 [M+1]⁺
Step 7: **48g** (65 mg, 95.49 µmol) and 2-(7-azobenzotriazol-1-yl)-N,N,N',N'-tetramethyluronium hexafluorophosphate (54.43 mg, 143.24 µmol) were dissolved in N,N-dimethylformamide (5 mL), N,N-diisopropylethylamine (37.03 mg, 286.48 µmol, 49.90 µL) was added, and the reaction system was reacted at 25°C for 48 hours. The resulting reaction system was quenched with water, extracted with ethyl acetate (80 mL × 3), washed with a saturated sodium chloride aqueous solution, and dried over anhydrous sodium sulphate, and the filtrate was concentrated under reduced pressure. The residue was purified by silica gel column chromatography using eluent system A to obtain **48h** (63.28 mg, 95.49 µmol, yield: 100%, crude), which was directly used in the next step. MS m/z (ESI): 663 [M+1]⁺
Step 8: **48h** (63.28 mg, 95.49 µmol) was dissolved in trifluoroacetic acid (5 mL), and the reaction system was reacted at 90°C for 0.5 hours. The resulting reaction system was concentrated, and the residue was purified by silica gel column chromatography using eluent system A to obtain **Example 48** (12 mg, yield: 24.5%). MS m/z (ESI): 513 [M+1]⁺
¹H NMR (400 MHz, DMSO-*d6*) δ 9.32 (d, 1H), 8.94 (s, 1H), 8.37 - 8.11 (m, 3H), 7.89 (t, 1H), 7.48 (d, 1H), 5.70 (s, 1H), 5.01 - 4.71 (m, 3H), 4.55 - 4.11 (m, 3H), 3.84 - 3.56 (m, 4H), 2.05 - 1.28 (m, 4H).

### Example 62

### (Z)-4-((2,4-Dimethoxybenzyl)amino)-13-(2-(trifluoromethyl)-5,8-dihydro-6H-pyrano[3,4-b]pyridin-5-yl)-10,11,12,13-tetrahydroazecino[4,3-g]imidazo[1,5-a]quinoxalin-14(9H)-one

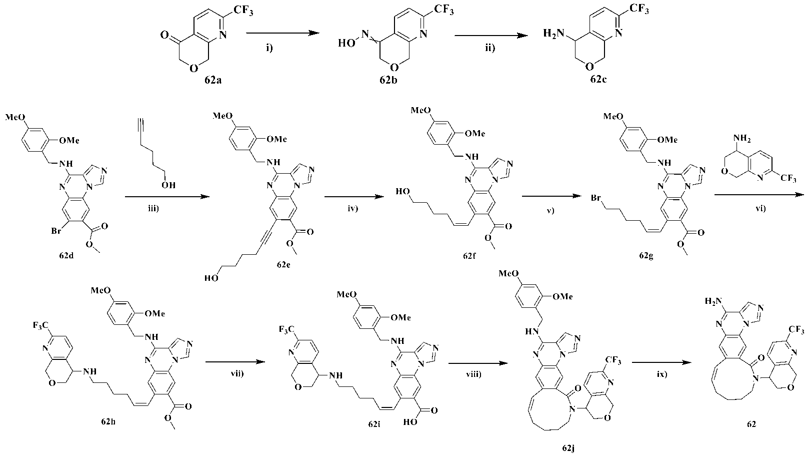

Step 1: 2-(Trifluoromethyl)-8H-pyrano[3,4-b]pyridin-5-one **62a** (3 g, 13.82 mmol) and hydroxylamine hydrochloride (960 mg, 13.82 mmol) were dissolved in ethanol (30 mL) and sodium acetate (3.40 g, 41.45 mmol), and the mixture was purged three times with nitrogen. The system was reacted at 80°C for 1 hour. The resulting reaction system was cooled to room temperature, concentrated, quenched with water, extracted with ethyl acetate (100 mL × 3), washed with a saturated sodium chloride aqueous solution, dried over anhydrous sodium sulphate, filtered, and concentrated. The residue was purified by silica gel column chromatography using eluent system B to obtain **62b** (3.10 g, yield: 96.7%). MS m/z (ESI): 233 [M+1]⁺
Step 2: **62b** (3.1 g, 13.35 mmol) and wet palladium on carbon (811 mg, 0.67 mmol, 10% purity) were dissolved in methanol (40 mL), and the mixture was purged three times with hydrogen, and reacted at 25°C under a hydrogen balloon for 4 hours. The resulting reaction system was filtered and concentrated to obtain **62c** (2.80 g, crude). MS m/z (ESI): 219 [M+1]⁺
Step 3: Methyl 7-bromo-4-((2,4-dimethoxybenzyl)amino)imidazo[1,5-a]quinoxaline-8-carboxylate **62d** (1 g, 2.12 mmol) and cuprous iodide (81 mg, 0.42 mmol) were dissolved in N,N-dimethylformamide (11 mL) and triethylamine (11 mL), and the mixture was purged three times with nitrogen. Hex-5-yn-1-ol (1.04 g, 10.61 mmol) and bis(triphenylphosphine) palladium(II) dichloride (149 mg, 0.21 mmol) were added, and the resulting mixture was purged three times with nitrogen. The system was reacted at 40°C for 16 hours. The resulting reaction system was cooled to room temperature, concentrated, quenched with water, extracted with ethyl acetate (60 mL × 3), washed with a saturated sodium chloride aqueous solution, dried over anhydrous sodium sulphate, filtered, and concentrated to obtain **62e** (0.95 g, crude). MS m/z (ESI): 489 [M+1]⁺
Step 4: **62e** (0.95 g, 1.94 mmol) and Raney nickel (100 mg) were dissolved in methanol (10 mL) and tetrahydrofuran, and the mixture was purged three times with hydrogen, and reacted at 25°C under a hydrogen balloon for 0.5 hours. The resulting reaction system was filtered and concentrated to obtain **62f** (0.90 g, crude). MS m/z (ESI): 491 [M+1]⁺
Step 5: **62f** (0.9 g, 1.83 mmol) was dissolved in dichloromethane (10 mL), and the mixture was purged three times with nitrogen and cooled to 0°C. Triphenylphosphine (962 mg, 3.67 mmol) and carbon tetrabromide (1.22g, 3.67 mmol) were then added, and the system was reacted at 25°C for 2 hours. The resulting reaction system was concentrated. The residue was purified by silica gel column chromatography using eluent system B to obtain **62g** (560 mg, yield: 55.2%). MS m/z (ESI): 553 [M+1]⁺
Step 6: **62g** (1 g, 1.81 mmol), 2-(trifluoromethyl)-5,8-dihydro-6H-pyrano[3,4-b]pyridin-5-amine (788 mg, 3.61 mmol), and sodium iodide (541.67 mg, 3.61 mmol) were dissolved in acetonitrile (60 mL), N,N-diisopropylethylamine (1.40 g, 10.84 mmol, 1.89 mL) was added, and the mixture was purged three times with nitrogen. The reaction system was reacted at 80°C for 15 hours. The resulting reaction system was quenched with water, extracted with ethyl acetate (80 mL × 3), washed with a saturated sodium chloride aqueous solution, dried over anhydrous sodium sulphate, filtered, and concentrated. The residue was purified by silica gel column chromatography using eluent system A to obtain **62h** (410 mg, yield: 32.9%). MS m/z (ESI): 691 [M+1]⁺
Step 7: **62h** (430 mg, 0.62 mmol) was dissolved in tetrahydrofuran (5 mL), water (5 mL) and methanol (5 mL), lithium hydroxide (60 mg, 2.49 mmol) was added, and the mixture was purged three times with nitrogen. The reaction system was reacted at 25°C for 16 hours. The resulting reaction system was adjusted to pH 5-6 with 1 M dilute hydrochloric acid, extracted with ethyl acetate (50 mL × 3), washed with a saturated sodium chloride aqueous solution (30 mL), dried over anhydrous sodium sulphate, filtered, and concentrated to obtain **62i** (410 mg, crude). MS m/z (ESI): 677 [M+1]⁺
Step 8: **62i** (56 mg, 83 µmol) and 2-(7-azobenzotriazol-1-yl)-N,N,N',N'-tetramethyluronium hexafluorophosphate (62 mg, 0.17 mmol) were dissolved in N,N-dimethylformamide (10 mL), N,N-diisopropylethylamine (54 mg, 0.41 mmol, 72 µL) was added, and the mixture was purged three times with nitrogen. The reaction system was reacted at 25°C for 48 hours. The resulting reaction system was quenched with water, extracted with ethyl acetate (80 mL × 3), washed with a saturated sodium chloride aqueous solution, dried over anhydrous sodium sulphate, filtered, and concentrated. The residue was purified by silica gel column chromatography using eluent system A to obtain **62j** (16 mg, yield: 29.4%). MS m/z (ESI): 659 [M+1]⁺
Step 9: **62j** (5 mg, 7.6 µmol) was dissolved in trifluoroacetic acid (2 mL), and the mixture was purged three times with nitrogen. The reaction system was reacted at 90°C for 0.5 hours. The resulting reaction system was concentrated, and the residue was purified by silica gel column chromatography using eluent system A to obtain **Example 62** (0.55 mg, yield: 14.2%). MS m/z (ESI): 509 [M+1]⁺
¹H NMR (400 MHz, MeOD) δ 9.07 (d, 1H), 8.27 (d, 1H), 8.18-8.06 (m, 1H), 7.99-7.92 (m, 1H), 7.79 (dd, 3.0 Hz, 1H), 7.25 (d, 1H), 6.65 (dd, 1H), 6.10-5.99 (m, 1H), 5.88-5.78 (m, 1H), 4.51 (dd, 1H), 4.34 (dd, 1H), 4.28-4.20 (m, 1H), 3.65-3.58 (m, 1H), 3.09-2.98 (m, 1H), 2.13 (s, 1H), 2.04-1.96 (m, 1H), 1.56 (s, 3H), 1.29 (s, 2H).

### Examples 62-P1 & 62-P2

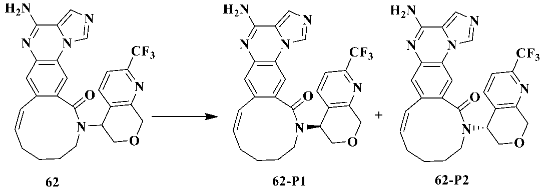

**Example 62** (55 mg, 4.24 mmol) was separated by chiral preparative HPLC to obtain rel-(R,Z)-4-amino-13-(2-(trifluoromethyl)-5,8-dihydro-6H-pyrano[3,4-b]pyridin-5-yl)-10,11,12,13-tetrahydroazecino[4,3-g]imidazo[1,5-a]quinoxalin-14(9H)-one **62-P1** (18 mg, yield: 32.7%) and rel-(S,Z)-4-amino-13-(2-(trifluoromethyl)-5,8-dihydro-6H-pyrano[3,4-b]pyridin-5-yl)-10,11,12,13-tetrahydroazecino[4,3-g]imidazo[1,5-a]quinoxalin-14(9H)-one **62-P2** (20 mg, yield: 36.4%).

**62-P1 (t_{R}: 1.624 min):** ¹H NMR (400 MHz, MeOD) δ 9.06 (dd, 1H), 8.21 (dd, 1H), 8.08-7.91 (m, 2H), 7.78 (dd, 1H), 7.25 (dd, 1H), 6.65 (dd, 1H), 6.09-5.77 (m, 1H), 5.52-5.26 (m, 1H), 4.60 (s, 1H), 4.51 (dd, 1H), 4.23 (ddd, 1H), 3.62 (t, 1H), 3.04 (dt, 1H), 2.23-2.07 (m, 1H), 2.03-1.95 (m, 1H), 1.77 (q, 1H), 1.60 (dt, 1H), 1.45-1.20 (m, 3H).

**62-P2 (t_{R}: 1.410 min):** ¹H NMR (400 MHz, MeOD) δ 8.97 (d, 1H), 8.12 (dd, 1H), 7.98-7.82 (m, 2H), 7.69 (dd, 1H), 7.15 (d, 1H), 6.55 (dd, 1H), 6.00-5.69 (m, 1H), 5.42-5.16 (m, 1H), 4.42 (dd, 1H), 4.28-4.08 (m, 2H), 3.52 (t, 1H), 2.95 (dt, 1H), 2.12-1.97 (m, 1H), 1.93-1.84 (m, 1H), 1.71-1.61 (m, 1H), 1.46 (d, 1H), 1.37-1.05 (m, 3H).

### Example 63

### 4-Amino-13-(2-(trifluoromethyl)-5,8-dihydro-6H-pyrano[3,4-b]pyridin-5-yl)-8,9,10,11,12,13-hexahydroazecino[4,3-g]imidazo[1,5-a]quinoxalin-14(7H)-one

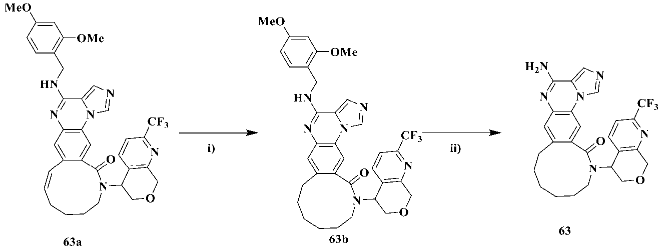

Step 1: (*Z*)-4-((2,4-Dimethoxybenzyl)amino)-13-(2-(trifluoromethyl)-5,8-dihydro-6H-pyrano[3,4-b]pyridin-5-yl)-10,11,12,13-tetrahydroazecino[4,3-g]imidazo[1,5-a]quinoxalin-14(9H)-one **63a** (15 mg, 23 µmol) and platinum dioxide (3 mg, 12 µmol) were dissolved in methanol (3 mL), and the mixture was purged three times with hydrogen. The reaction system was reacted at 25°C under a hydrogen balloon for 1 hour. The resulting reaction system was filtered and concentrated to obtain **63b** (12 mg, crude).

Referring to the synthesis method described in Step 8 of **Example 14, Example 63** (3 mg, yield: 32.4%) was obtained from **63b** (12 mg, 18 µmol). MS m/z (ESI): 511 [M+1]⁺

The following examples were prepared by reference to **Example 62:**

| **Example** | **Structure** | **MS m/z (ESI) [M+1]⁺** | **Example** | **Structure** | **MS m/z (ESI) [M+1]⁺** |
|---|---|---|---|---|---|
| **64** | 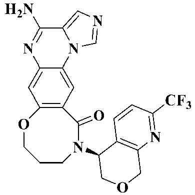 | 485 | **177-P2** | 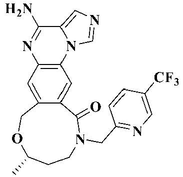 | 471 |
| **65** | 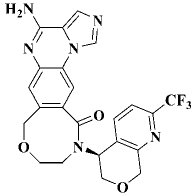 | 485 | **178** | 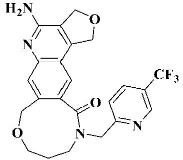 | 459 |
| **66** | 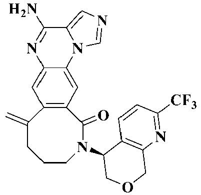 | 495 | **179** | 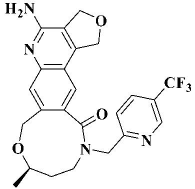 | 473 |
| **67** | 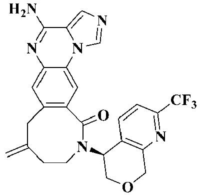 | 495 | **180** | 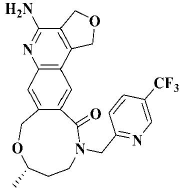 | 473 |
| **68** | 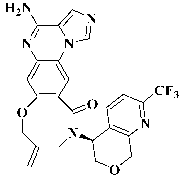 | 497 | **181** | 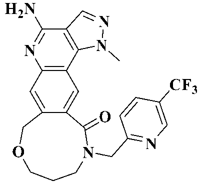 | 471 |
| **69** | 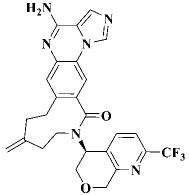 | 509 | **182** | 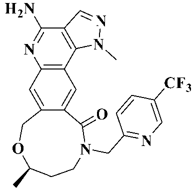 | 485 |
| **70** | 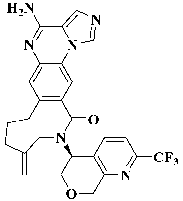 | 509 | **183** | 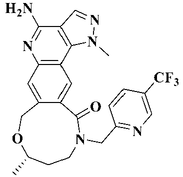 | 485 |
| **71** | 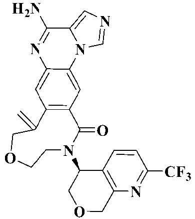 | 511 | **184** | 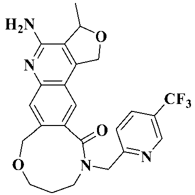 | 473 |
| **72** | 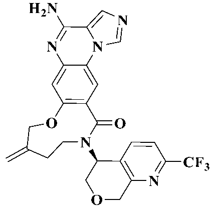 | 511 | **184-P1** | 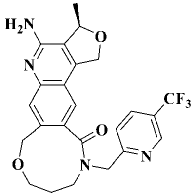 | 473 |
| **73** | 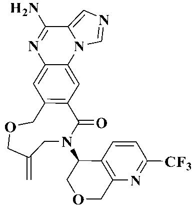 | 511 | **184-P2** | 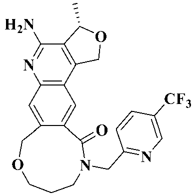 | 473 |
| **74** | 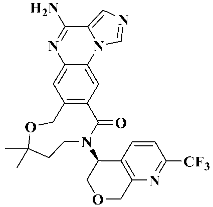 | 527 | **185** | 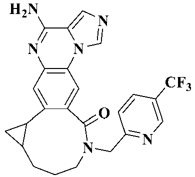 | 467 |
| **75** | 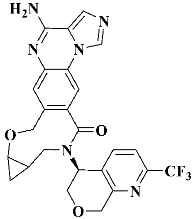 | 511 | **186** | 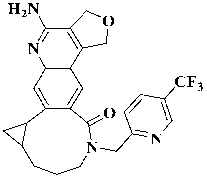 | 469 |
| **76** | 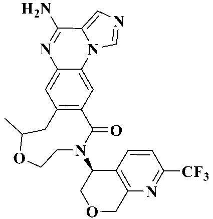 | 513 | **187** | 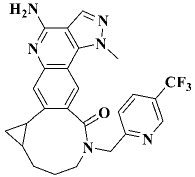 | 481 |
| **77** | 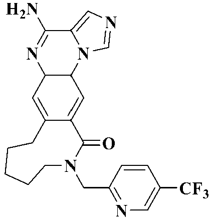 | 457 | **188** | 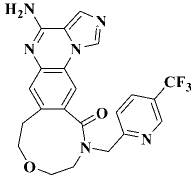 | 457 |
| **78** | 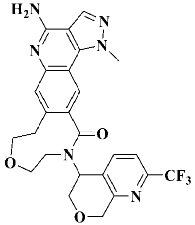 | 513 | **189** | 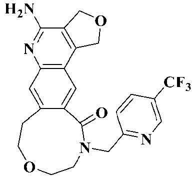 | 459 |
| **79** | 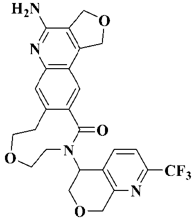 | 501 | **190** | 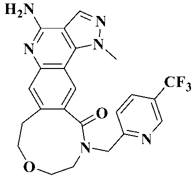 | 471 |
| **80** | 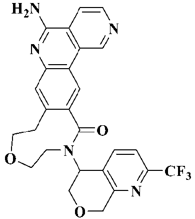 | 510 | **191** | 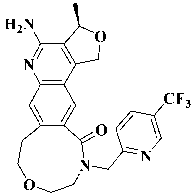 | 473 |
| **81** | 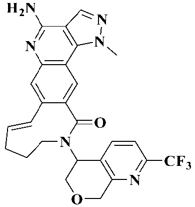 | 509 | **192** | 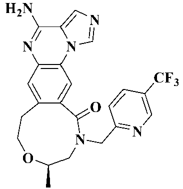 | 471 |
| **82** | 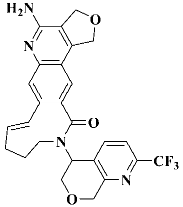 | 497 | **193** | 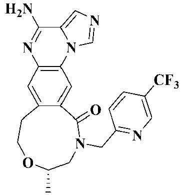 | 471 |
| **83** | 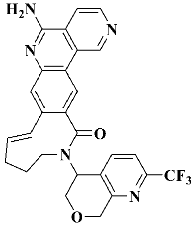 | 506 | **194** | 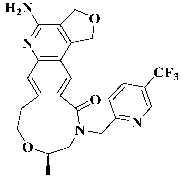 | 473 |
| **84** | 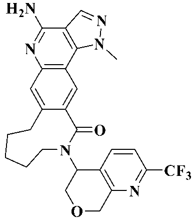 | 511 | **195** | 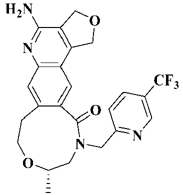 | 473 |
| **85** | 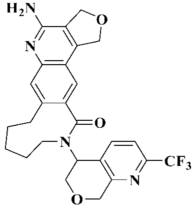 | 499 | **196** | 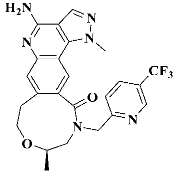 | 485 |
| **86** | 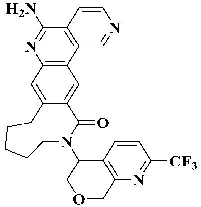 | 508 | **197** | 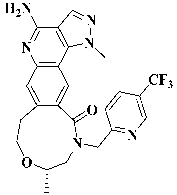 | 485 |
| **87** | 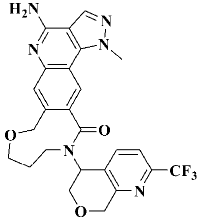 | 513 | **198** | 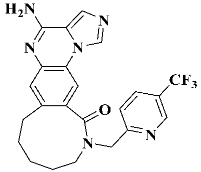 | 455 |
| **88** | 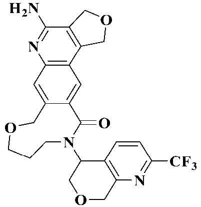 | 501 | **199** | 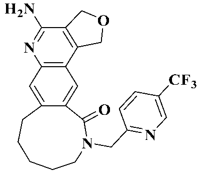 | 457 |
| **89** | 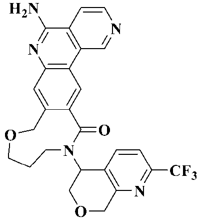 | 510 | **200** | 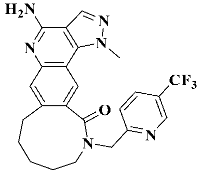 | 469 |
| **90** | 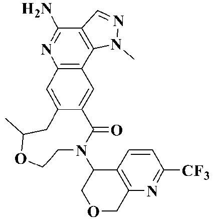 | 527 | **201** | 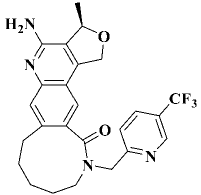 | 471 |
| **91** | 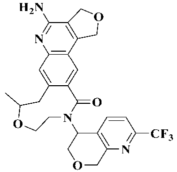 | 515 | **202** | 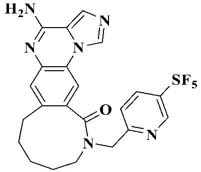 | 513 |
| **92** | 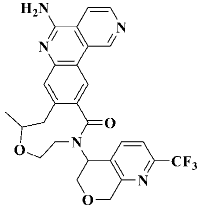 | 524 | **203** | 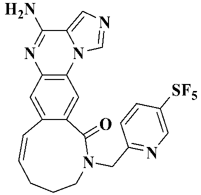 | 511 |
| **93** | 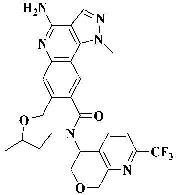 | 527 | **204** | 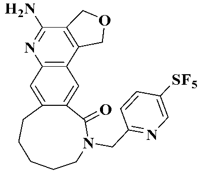 | 515 |
| **94** | 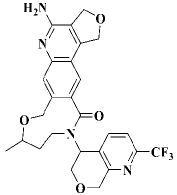 | 515 | **205** | 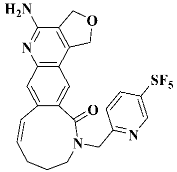 | 513 |
| **95** | 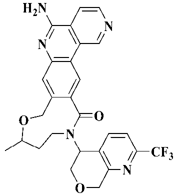 | 524 | **206** | 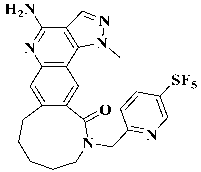 | 527 |
| **96** | 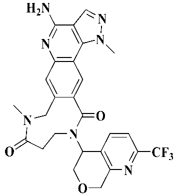 | 540 | **207** | 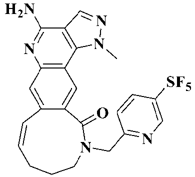 | 525 |
| **97** | 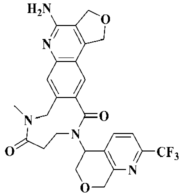 | 528 | **208** | 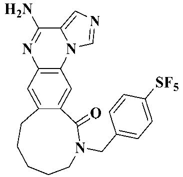 | 512 |
| **98** | 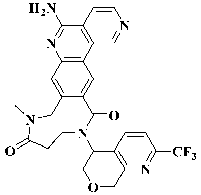 | 537 | **209** | 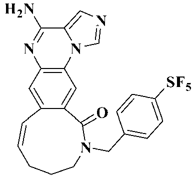 | 510 |
| **99** | 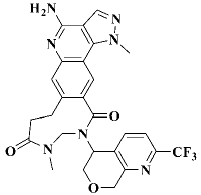 | 540 | **210** | 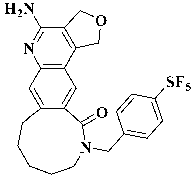 | 514 |
| **100** | 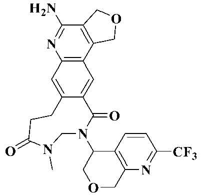 | 528 | **211** | 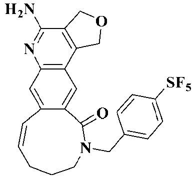 | 512 |
| **101** | 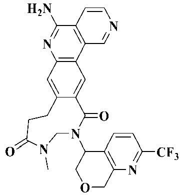 | 537 | **212** | 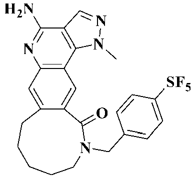 | 526 |
| **102** | 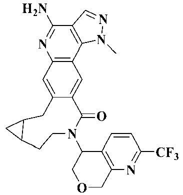 | 523 | **213** | 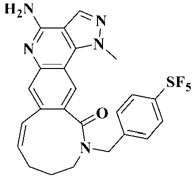 | 524 |
| **103** | 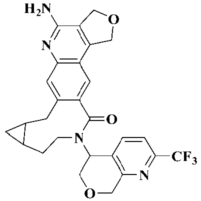 | 511 | **214** | 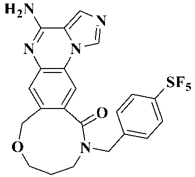 | 514 |
| **104** | 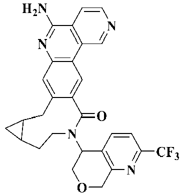 | 520 | **215** | 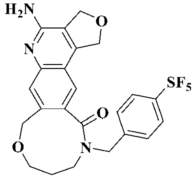 | 516 |
| **105** | 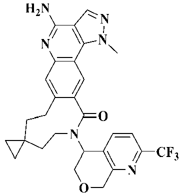 | 537 | **216** | 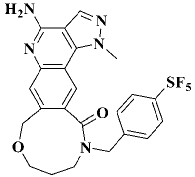 | 528 |
| **106** | 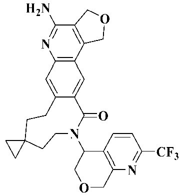 | 525 | **217** | 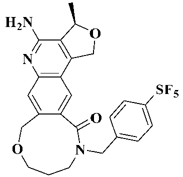 | 530 |
| **107** | 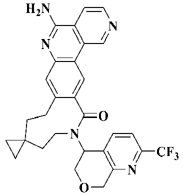 | 534 | **218** | 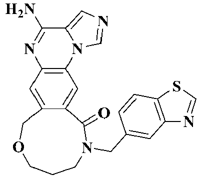 | 445 |
| **108** | 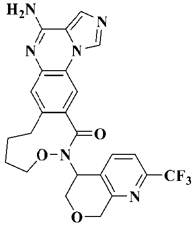 | 499 | **219** | 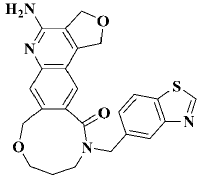 | 447 |
| **109** | 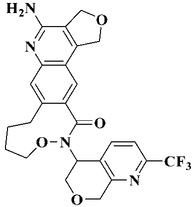 | 501 | **220** | 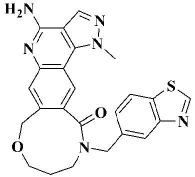 | 459 |
| **110** | 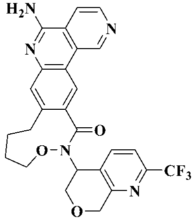 | 510 | **221** | 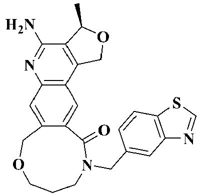 | 461 |
| **111** | 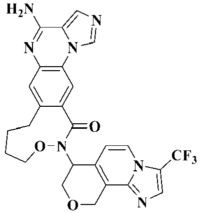 | 538 | **222** | 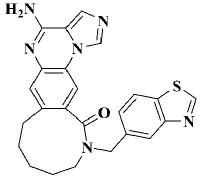 | 443 |
| **112** | 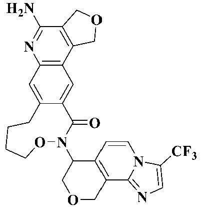 | 540 | **223** | 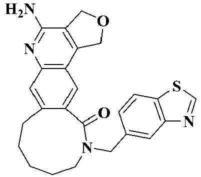 | 445 |
| **113** | 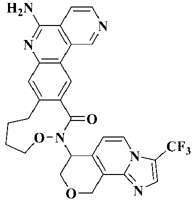 | 549 | **224** | 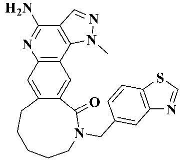 | 457 |
| **114** | 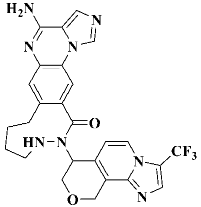 | 537 | **225** | 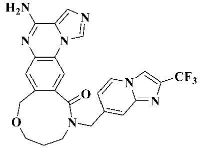 | 496 |
| **115** | 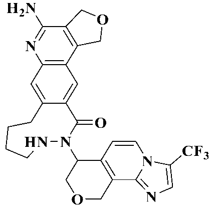 | 539 | **226** | 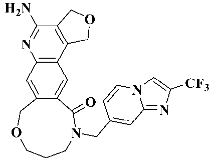 | 498 |
| **116** | 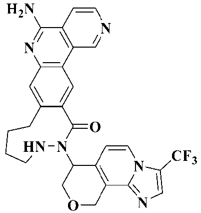 | 548 | **227** | 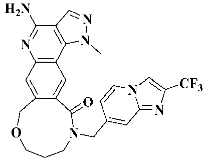 | 510 |
| **117** | 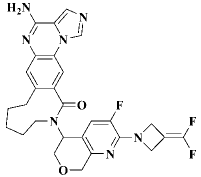 | 550 | **228** | 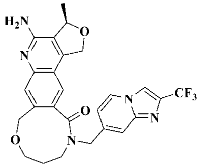 | 512 |
| **118** | 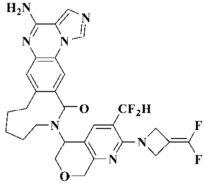 | 582 | **229** | 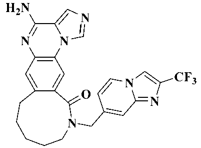 | 494 |
| **119** | 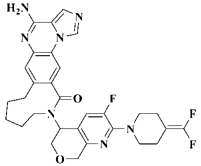 | 578 | **230** | 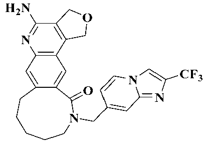 | 496 |
| **120** | 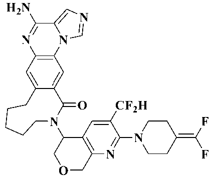 | 610 | **231** | 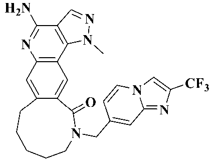 | 508 |
| **121** | 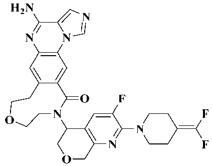 | 580 | **232** | 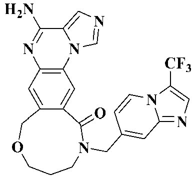 | 496 |
| **122** | 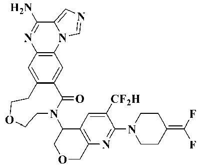 | 612 | **233** | 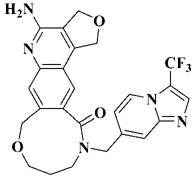 | 498 |
| **123** | 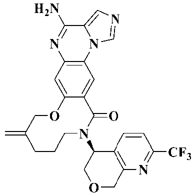 | 525 | **234** | 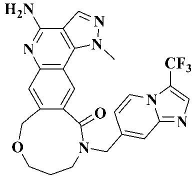 | 510 |
| **124** | 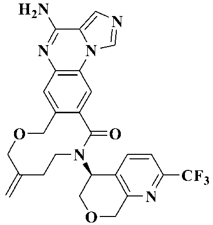 | 525 | **235** | 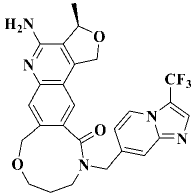 | 512 |
| **125** | 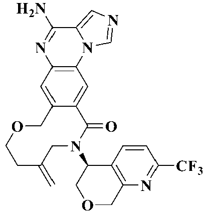 | 525 | **236** | 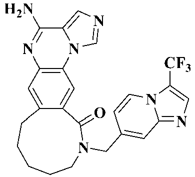 | 494 |
| **126** | 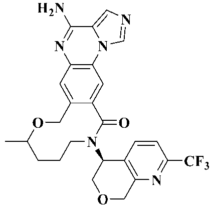 | 527 | **237** | 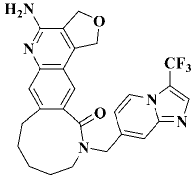 | 496 |
| **127** | 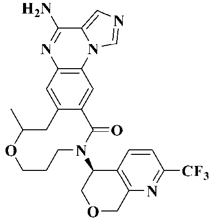 | 527 | **238** | 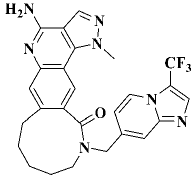 | 508 |
| **128** | 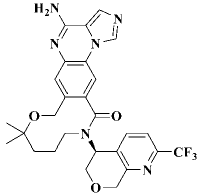 | 541 | **239** | 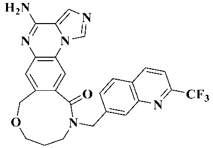 | 507 |
| **129** | 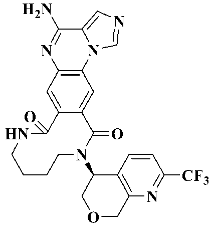 | 526 | **240** | 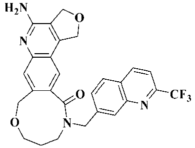 | 509 |
| **130** | 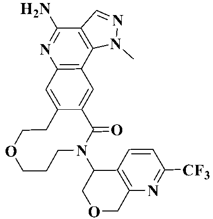 | 527 | **241** | 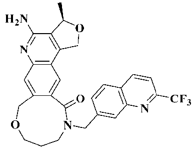 | 523 |
| **131** | 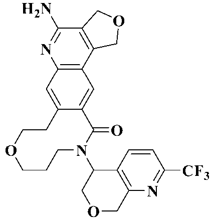 | 515 | **242** | 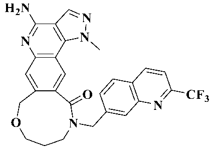 | 521 |
| **132** | 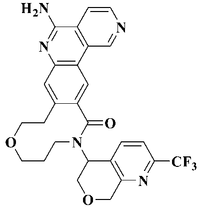 | 523 | **243** | 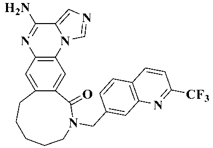 | 505 |
| **133** | 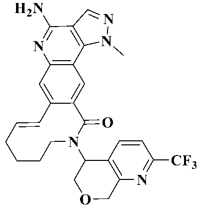 | 523 | **244** | 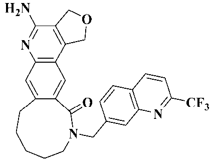 | 507 |
| **134** | 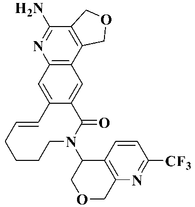 | 511 | **245** | 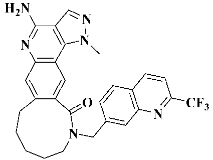 | 519 |
| **135** | 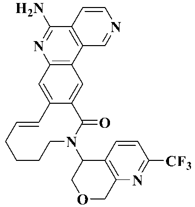 | 520 | **246** | 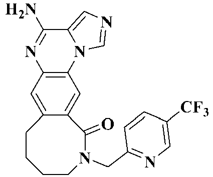 | 441 |
| **136** | 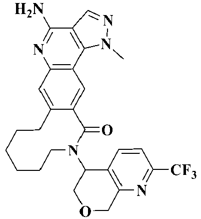 | 525 | **247** | 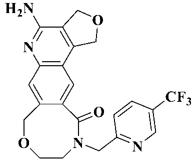 | 445 |
| **137** | 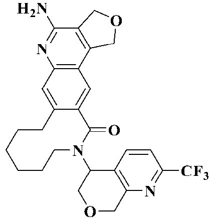 | 513 | **248** | 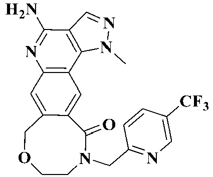 | 457 |
| **138** | 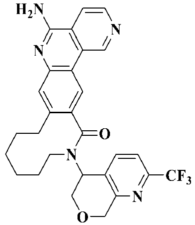 | 522 | **249** | 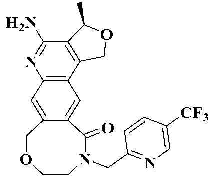 | 459 |
| **139** | 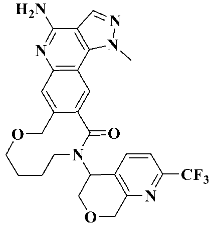 | 527 | **250** | 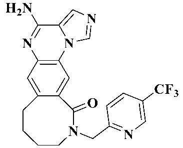 | 441 |
| **140** | 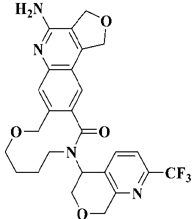 | 515 | **251** | 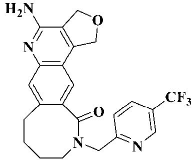 | 443 |
| **141** | 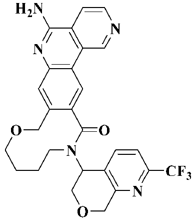 | 524 | **252** | 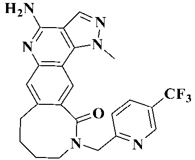 | 455 |
| **142** | 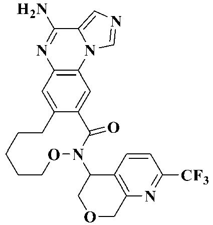 | 513 | **253** | 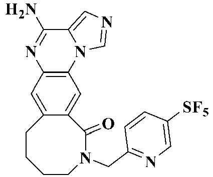 | 499 |
| **143** | 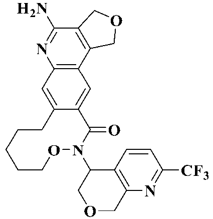 | 515 | **254** | 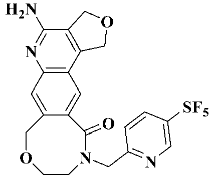 | 503 |
| **144** | 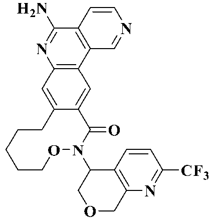 | 524 | **255** | 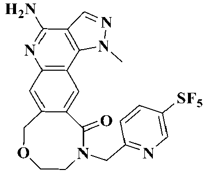 | 515 |
| **145** | 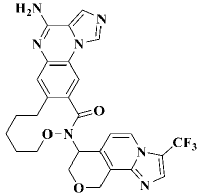 | 552 | **256** | 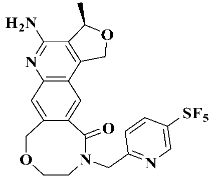 | 517 |
| **146** | 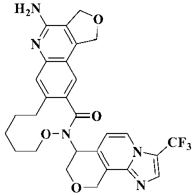 | 554 | **257** | 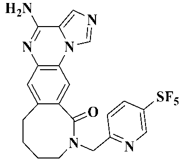 | 499 |
| **147** | 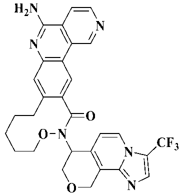 | 563 | **258** | 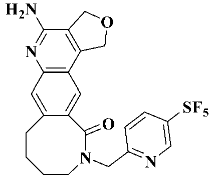 | 501 |
| **148** | 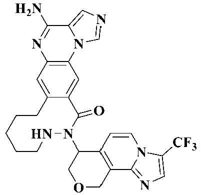 | 551 | **259** | 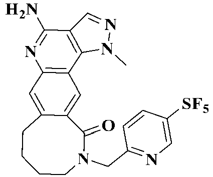 | 513 |
| **149** | 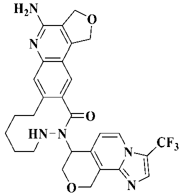 | 553 | **260** | 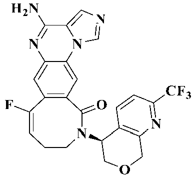 | 499 |
| **150** | 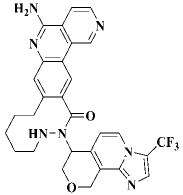 | 562 | **261** | 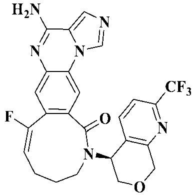 | 515 |
| **151** | 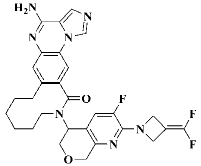 | 564 | **262** | 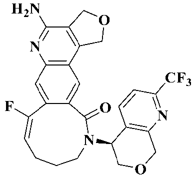 | 515 |
| **152** | 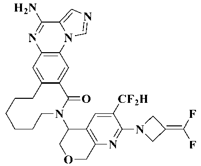 | 596 | **263** | 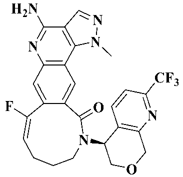 | 527 |
| **153** | 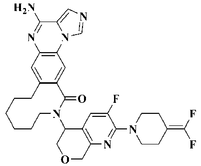 | 592 | **264** | 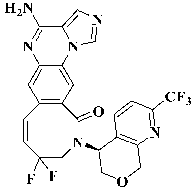 | 517 |
| **154** | 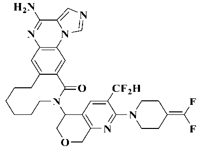 | 624 | **265** | 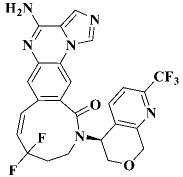 | 533 |
| **155** | 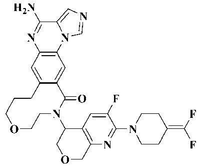 | 594 | **266** | 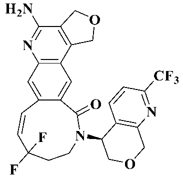 | 533 |
| **156** | 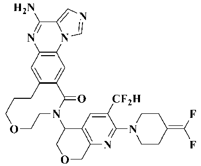 | 626 | **267** | 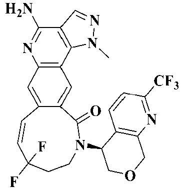 | 545 |
| **157** | 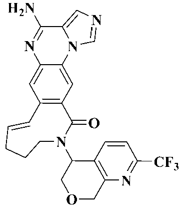 | 495 | **268-P1** | 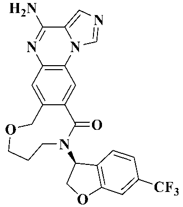 | 484 |
| **160** | 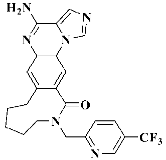 | 457 | **269** | 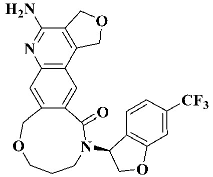 | 486 |
| **161** | 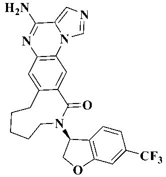 | 482 | **270** | 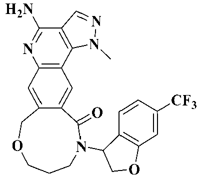 | 498 |
| **162** | 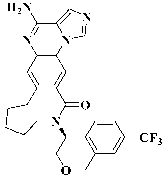 | 496 | **270-P1** | 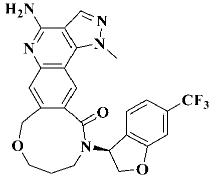 | 498 |
| **163** | 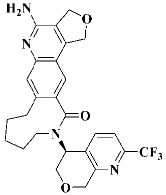 | 499 | **270-P2** | 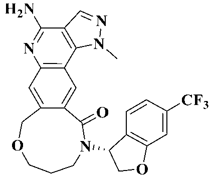 | 498 |
| **164-P1** | 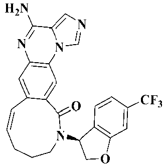 | 480 | **271** | 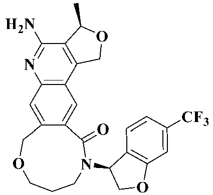 | 500 |
| **165-P1** | 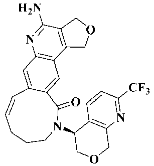 | 497 | **272** | 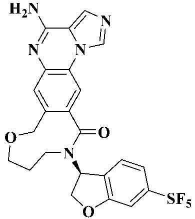 | 542 |
| **166** | 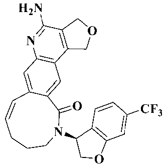 | 482 | **273** | 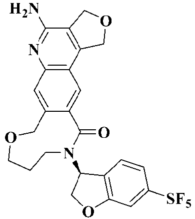 | 544 |
| **167** | 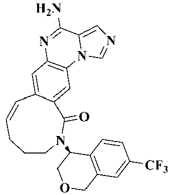 | 494 | **274** | 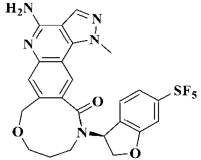 | 556 |
| **168** | 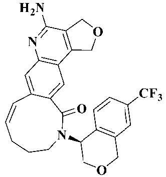 | 496 | **275** | 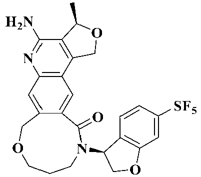 | 558 |
| **169** | 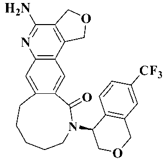 | 498 | **276** | 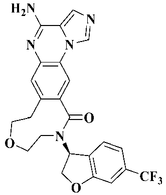 | 484 |
| **170** | 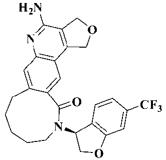 | 484 | **277** | 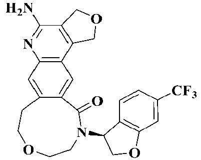 | 486 |
| **171** | 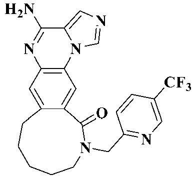 | 455 | **278** | 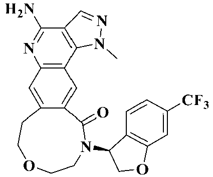 | 498 |
| **172** | 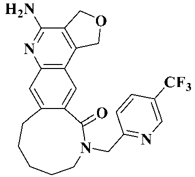 | 457 | **279** | 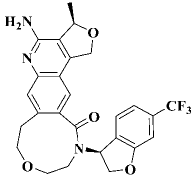 | 500 |
| **173** | 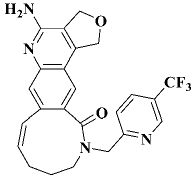 | 455 | **280** | 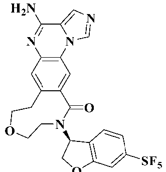 | 542 |
| **174** | 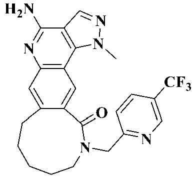 | 469 | **281** | 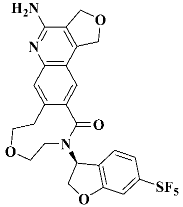 | 544 |
| **175** | 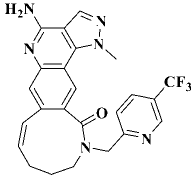 | 467 | **282** | 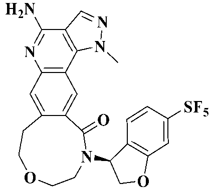 | 556 |
| **176** | 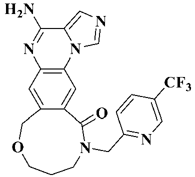 | 457 | **283** | 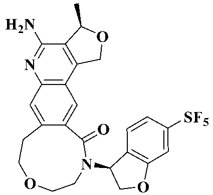 | 558 |
| **177-P1** | 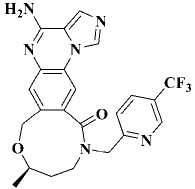 | 471 | | | |

Alternatively, **Examples 65, 77,** and **157** were prepared using the following method:

### Example 65

### 4-Amino-11-(2-(trifluoromethyl)-5,8-dihydro-6H-pyrano[3,4-b]pyridin-5-yl)-10,11-dihydro-7H-imidazo[1,5-a][1,4]oxazino[7,6-g]quinoxalin-12(9H)-one

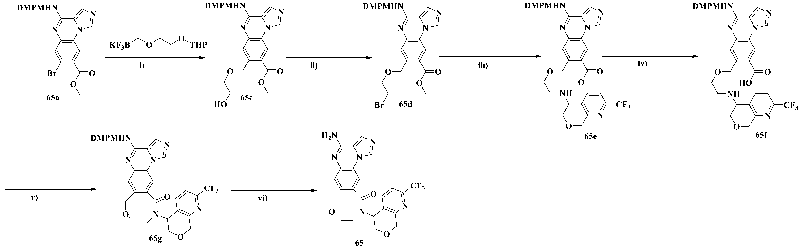

Step 1 : A solution of methyl 7-bromo-4-[(3,4-dimethoxyphenyl)methylamino]imidazo[1,5-a]quinoxaline-8-carboxylate **65a** (600 mg, 1.27 mmol), potassium trifluoro(2-tetrahydropyran-2-yloxyethoxymethyl)borate (677 mg, 2.55 mmol), caesium carbonate (827 mg, 2.55 mmol), and CataCxiumA-Pd-G3 (93 mg, 0.13 mmol) in 1,4-dioxane (15 mL) and water (3 mL) was purged with nitrogen, sealed in a microwave vial, and stirred under microwave irradiation at 110°C for 2.5 hours. The reaction solution was filtered to remove solids, and the filtrate was diluted with ethyl acetate (250 mL), and washed with water and a sodium chloride aqueous solution. The organic phase was concentrated, and the residue was purified by silica gel column chromatography to obtain **65c** (210 mg, yield: 35.6%) as a pale yellow oily liquid. MS m/z (ESI): 467 [M+1]⁺
Referring to the synthesis method of **Example 62, Example 65** (22 mg, yield: 3.7%) was obtained from 65c (620 mg, 1.33 mmol). MS m/z (ESI): 485 [M+1]⁺
¹H NMR (400 MHz, MeOD) δ 9.28 (d, 1H), 8.40 (m, 2H), 8.07, 7.78 (d, 1H), 7.75 (m, 1H), 7.44 (m, 1H), 6.03,5.83 (s, 1H), 5.00 (m, 2H), 4.78 (m, 2H), 4.42 (m, 1H), 4.18 (m, 2H), 3.95 (m, 1H), 3.70 (d, 1H), 3.42 (m, 1H), 3.14 (m, 1H).

### Example 77

### 4-Amino-12-((5-(trifluoromethyl)pyridin-2-yl)methyl)-5a,7,8,9,10,11,12,14a-octahydro-13H-azonino[4,3-g]imidazo[1,5-a]quinoxalin-13-one

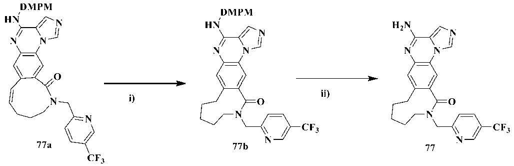

Referring to the synthesis method described in Step 1 to Step 2 of **Example 63, Example 77** (6 mg, yield: 13.3%) was obtained from *(Z)*-4-((3,5-dimethoxybenzyl)amino)-12-((5-(trifluoromethyl)pyridin-2-yl)methyl)-9,10,11,12-tetrahydro-13H-azonino[4,3-g]imidazo[1,5-a]quinoxalin-13-one **77a** (60 mg, 4.24 mmol). MS m/z (ESI): 455 [M+1]⁺

### Example 157

### (Z)-4-Amino-11-(2-(trifluoromethyl)-5,8-dihydro-6H-pyrano[3,4-b] pyridin-5-yl)-10,11-dihydroazocino[4,3-g]imidazo[1,5-a]quinoxalin-12(9H)-one

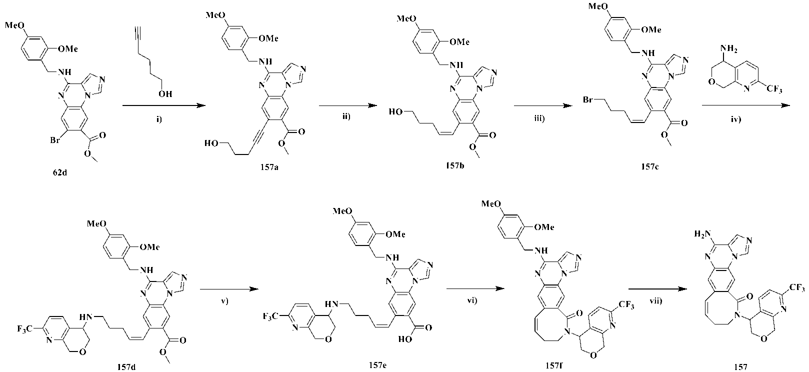

Referring to the method described in Step 3 to Step 9 of **Example 62, Example 157** (22 mg, yield: 1.1%) was obtained from 7-bromo-4-((3,4-dimethoxybenzyl)amino)imidazo[1,5-a]quinoxaline-8-carboxylate 62d (2 g, 4.25 mmol). MS m/z (ESI): 481 [M+1]⁺
¹H NMR (400 MHz, DMSO-d6) δ 9.18 (d, 1H), 8.22 (s, 1H), 8.15 - 8.05 (m, 1H), 8.01 - 7.89 (m, 1H), 7.84 (dd, 1H), 7.62 (s, 2H), 7.18 (d, 1H), 6.59 (ddd, 1H), 5.93 (m, 1H), 5.55 (d, 1H), 4.94 - 4.74 (m, 2H), 4.34 - 4.12 (m, 2H), 3.76 (dd, 2H), 3.16 (d, 1H), 2.76 (td, 1H).

### Example 158

### (Z)-4-Amino-12-(2-(trifluoromethyl)-5,8-dihydro-6H-pyrano[3,4-b]pyridin-5-yl)-9,10,11,12-tetrahydro-13H-azonino[4,3-g]imidazo[1,5-a]quinoxalin-13-one

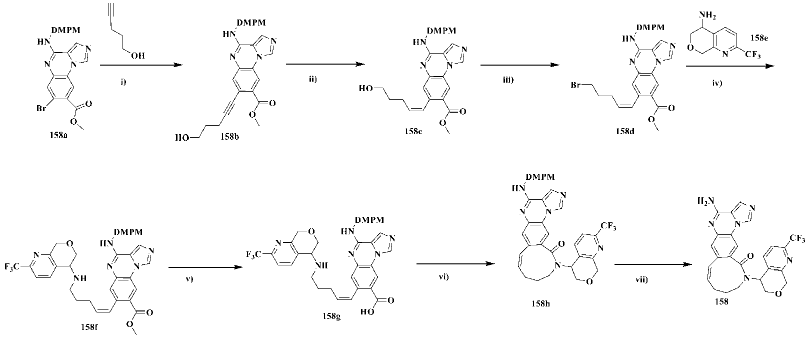

Referring to the synthesis method described in Step 3 to Step 9 of **Example 62, Example 158** (23 mg, yield: 1.1%) was obtained from methyl 7-bromo-4-((2,4-dimethoxybenzyl)amino)imidazo[1,5-a]quinoxaline-8-carboxylate **158a** (2 g, 4.24 mmol). MS m/z (ESI): 495 [M+1]⁺
¹H NMR (400 MHz, MeOD) δ 9.05 (s, 1H), 8.19-8.08 (m, 2H), 7.95 (s, 1H), 7.78 (dd, 1H), 7.31 (d, 1H), 6.63 (dd, , 1H), 6.28-5.93 (m, 1H), 5.53 (d, 1H), 4.43 (dd, 1H), 4.34-4.16 (m, 2H), 3.97-3.69 (m, 1H), 3.42 (d, 1H), 2.26-1.87 (m, 3H), 1.54-1.26 (m, 2H).

### Examples 158-P1 & 158-P2

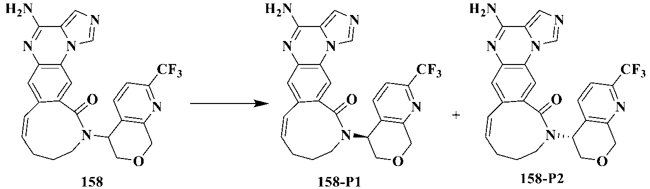

**Example 158** (140 mg, 0.28 mmol) was separated by chiral preparative HPLC to obtain (R, Z)-4-amino-12-(2-(trifluoromethyl)-5,8-dihydro-6H-pyrano[3,4-b]pyridin-5-yl)-9,10,11,12-tetrahydro-13H-aza[4,3-g]imidazo[1,5-a]quinoxalin-13-one **158-P1** (46 mg, yield: 32.9%) and rel-(R, Z)-4-amino-12-(2-(trifluoromethyl)-5,8-dihydro-6H-pyrano[3,4-b]pyridin-5-yl)-9,10,11,12-tetrahydro-13H-aza[4,3-g]imidazo[1,5-a]quinoxalin-13-one **158-P2** (45 mg, yield: 32.1%).

**158-P1 (t_{R}: 1.733 min):** ¹H NMR (400 MHz, MeOD) δ 9.05 (s, 1H), 8.19-8.07 (m, 2H), 7.93 (s, 1H), 7.78 (d, 1H), 7.30 (s, 1H), 6.63 (dd, 1H), 6.10 (dq, 1H), 5.53 (d, 1H), 4.37 (ddd, 1H), 4.22 (ddd, 1H), 3.92 (d, 1H), 3.72-3.60 (m, 1H), 3.42 (d, 1H), 2.21 (dd, 1H), 2.12-1.88 (m, 2H), 1.53-1.26 (m, 2H).

**158-P2 (t_{R}: 0.771 min):** ¹H NMR (400 MHz, MeOD) δ 9.04 (d, 1H), 8.20-8.08 (m, 2H), 7.93 (d, 1H), 7.78 (d, 1H), 7.30 (s, 1H), 6.63 (dd, 1H), 6.31-5.90 (m, 1H), 5.53 (d, 1H), 4.37 (ddd, 1H), 4.22 (ddd, 1H), 3.92 (d, 1H), 3.66 (t, 1H), 3.42 (d, 1H), 2.21 (dd, 1H), 2.13-1.86 (m, 2H), 1.53-1.26 (m, 2H).

### Example 159

### 4-Amino-12-(2-(trifluoromethyl)-5,8-dihydro-6H-pyrano[3,4-b]pyridin-5-yl)-7,8,9,10,11,12-hexahydro-13H-azonino[4,3-g]imidazo[1,5-a]quinoxalin-13-one

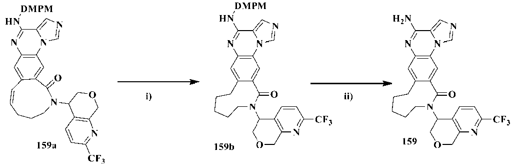

Referring to the synthesis method described in Step 1 to Step 2 of **Example 63, Example 159** (15 mg, yield: 17.7%) was obtained from (Z)-4-((3,5-dimethoxybenzyl)amino)-12-(2-(trifluoromethyl)-5,8-dihydro-6H-pyrano[3,4-b]pyridin-5-yl)-9,10,11,12-tetrahydro-13H-azonino[4,3-g]imidazo[1,5-a]quinoxalin-13-one **159a** (110 mg, 4.24 mmol). MS m/z (ESI): 497 [M+1]⁺
¹H NMR (400 MHz, MeOD) δ 9.06 (d, 1H), 8.25-8.10 (m, 2H), 7.96 (d, 1H), 7.80 (dd, 1H), 7.42 (d, 1H), 6.05 (s, 1H), 4.40-4.22 (m, 2H), 3.94-3.84 (m, 1H), 3.20 (dd, 1H), 3.00 (dd, 1H), 2.84-2.73 (m, 1H), 2.58 (ddd, 1H), 1.98 (d, 2H), 1.81-1.59 (m, 2H), 1.50 (s, 1H), 1.27-1.19 (m, 1H), 1.07 (dd, 1H).

Alternatively, **Examples 164, 165,** and **167** were prepared by reference to the following method:

### Example 164

### (Z)-4-Amino-12-(6-(trifluoromethyl)-2,3-dihydrobenzofuran-3-yl)-9,10,11,12-tetrahydro-13H-azo[4,3-g]imidazo[1,5-a]quinoxalin-13-one

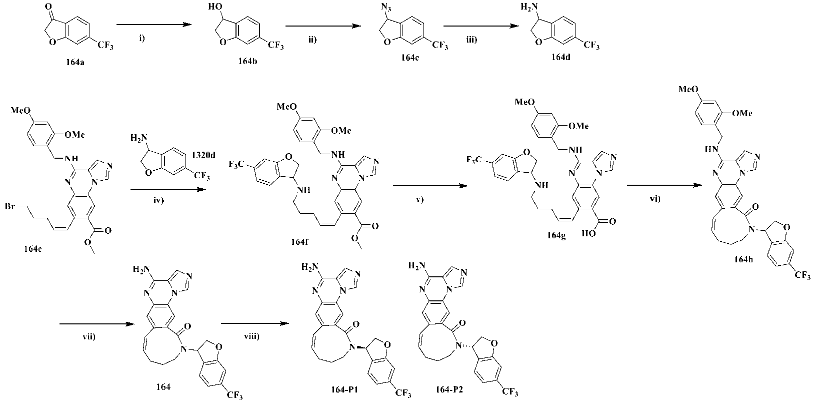

Step 1: 6-(Trifluoromethyl)benzofuran-3-one **164a** (2 g, 9.89 mmol) was dissolved in methanol (10 mL), and the mixture was purged three times with nitrogen. Sodium borohydride (449 mg, 11.87 mmol) was added, and the resulting mixture was reacted at 25°C for 1 hour. The resulting reaction system was quenched with a saturated sodium bicarbonate aqueous solution, and extracted with ethyl acetate (80 mL × 3). The organic phases were combined, washed with a saturated sodium chloride aqueous solution (30 mL), dried over anhydrous sodium sulphate, filtered, and concentrated. The residue was purified by silica gel column chromatography using eluent system A to obtain **164b** (16 mg, yield: 89.1%). MS m/z (ESI): 205 [M+1]⁺
Step 2: **164b** (0.15 g, 0.73 mmol) was dissolved in tetrahydrofuran (5 mL), and the mixture was purged three times with nitrogen and cooled to 0°C. Diphenylphosphoryl azide (214 mg, 0.88 mmol, 168 µL) and 1,8-diazabicyclo[5,4,0]undec-7-ene (134 mg, 0.88 mmol, 132 µL) were added, and the resulting mixture was stirred and reacted at 25°C for 16 hours. The resulting reaction system was quenched with water, extracted with ethyl acetate (50 mL × 3), washed with a saturated sodium chloride aqueous solution (30 mL), dried over anhydrous sodium sulphate, filtered, and concentrated. The residue was purified by silica gel column chromatography using eluent system A to obtain **164c** (130 mg, yield: 77.2%). MS m/z (ESI): 230 [M+1]⁺
Step 3: **164c** (0.13 g, 567.29 µmol) and triphenylphosphine (446 mg, 1.70 mmol) were dissolved in water (0.1 mL) and tetrahydrofuran (10 mL), and the mixture was purged three times with nitrogen, and reacted at 50°C for 15 hours. The reaction system was cooled to room temperature, and 0.5 M hydrochloric acid (30 mL) was added. The mixture was extracted three times with ethyl acetate (30 mL × 3), and the aqueous phase was subjected to rotary evaporation to dryness to obtain **164d** (60 mg, yield: 52.2%).
Referring to the synthesis method described in Step 6 to Step 9 of **Example 62, Example 164** (23 mg, yield: 5.2%) was obtained from methyl (Z)-7-(5-bromopent-1-en-1-yl)-4-(2,4-dimethoxybenzyl)amino)imidazo[1,5-a]quinoxaline-8-carboxylate **164e** (500 mg, 0.93 mmol). MS m/z (ESI): 480 [M+1]⁺
¹H NMR (400 MHz, DMSO-d6) δ 9.04 (s, 1H), 8.08 (d, 1H), 7.86 (s, 1H), 7.58-7.37 (m, 3H), 7.24 (t, 1H), 7.18-7.05 (m, 2H), 6.45 (dd, 1H), 6.11-5.75 (m, 2H), 4.84-4.72 (m, 1H), 2.04 (s, 1H), 1.92 (s, 1H), 1.69 (d, 1H), 1.37 (d, 1H), 1.22 (s, 1H), 1.08 (s, 1H).

Step 8: **Example 164** (50 mg, 0.10 mmol) was separated by chiral preparative HPLC to obtain rel-(R, Z)-4-amino-12-(6-(trifluoromethyl)-2,3-dihydrobenzofuran-3-yl)-9,10,11,12-tetrahydro-13H-azo[4,3-g]imidazo[1,5-a]quinoxalin-13-one **164-P1**(18 mg, yield: 36.0%, MS m/z (ESI): 480 [M+1]⁺) and rel-(R, Z)-4-amino-12-(6-(trifluoromethyl)-2,3-dihydrobenzofuran-3-yl)-9,10,11,12-tetrahydro-13H-azo[4,3-g]imidazo[1,5-a]quinoxalin-13-one **164-P2** (20 mg, yield: 40.0%, MS m/z (ESI): 480 [M+1]⁺).

**164-P1 (t_{R}: 3.213 min):** ¹H NMR (400 MHz, MeOD) δ 9.01 (s, 1H), 8.09 (d, 1H), 7.93 (d, 1H), 7.59 (t, 1H), 7.33-7.25 (m, 2H), 7.11 (d, 1H), 6.65-6.56 (m, 1H), 6.18 (d, 1H), 6.06-5.92 (m, 1H), 4.81-4.71 (m, 2H), 3.57 (s, 2H), 2.17 (s, 2H), 1.28 (s, 2H).

**164-P2 (t_{R}: 3.006 min):** ¹H NMR (400 MHz, MeOD) δ 9.01 (s, 1H), 8.09 (d1H), 7.93 (d, 1H), 7.62-7.56 (m, 1H), 7.32-7.24 (m, 2H), 7.12 (d, 1H), 6.64-6.57 (m, 1H), 6.19 (s, 1H), 6.01 (d, 2H), 4.81-4.72 (m, 2H), 3.56 (d, 2H), 2.17 (s, 2H), 1.28 (s, 2H).

### Example 165

### (Z)-4-Amino-12-(2-trifluoromethyl-5,8-dihydro-6H-pyrano[3,4-b]pyridin-5-yl)-1,3,9,10,11,12-hexahydro-13H-azo[3,4-g]furo[3,4-c]quinolin-13-one

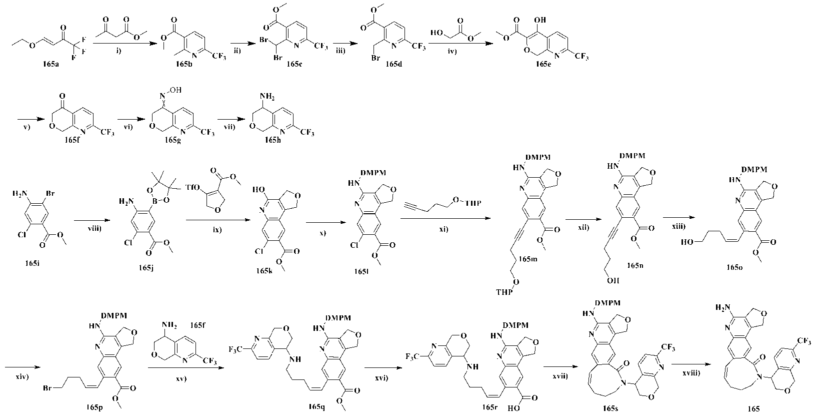

Step 1: Methyl acetoacetate (82.88 g, 713.80 mmol) was dissolved in glacial acetic acid (1 L), 4-ethoxy-1,1,1-trifluoro-3-buten-2-one **165a** (100 g, 594.84 mmol) and ammonium acetate (183.40 g, 2.38 mol) were sequentially added, and the mixture was stirred at 120°C for 16 hours. The resulting reaction system was cooled to room temperature, subjected to rotary evaporation to dryness to remove acetic acid, quenched with water, extracted with ethyl acetate (2 L × 3), washed with a saturated sodium chloride aqueous solution, dried over anhydrous sodium sulphate, filtered, and concentrated. The residue was purified by silica gel column chromatography using eluent system A to obtain **165b** (80 g, yield: 61.3%). MS m/z (ESI): 220 [M+1]⁺
Step 2: **165b** (80.5 g, 367.31 mmol) was dissolved in carbon tetrachloride (800 mL), N-bromosuccinimide (130.75 g, 734.62 mmol) and azobisisobutyronitrile (12.06 g, 73.46 mmol) were added under nitrogen atmosphere, and the mixture was stirred at 77°C for 16 hours. Upon completion of the reaction, the reaction system was subjected to rotary evaporation to dryness to remove the solvent, to obtain **165c** (crude, 80 g), which was used directly in the next step. MS m/z (ESI): 376 [M+1]⁺
Step 3: **165c** (80 g, 365.03 mmol) was dissolved in tetrahydrofuran (800 mL), diethyl phosphite (30.25 g, 219.02 mmol) and N,N-diisopropylethylamine (28.31 g, 219.02 mmol, 38.15 mL) were added, and the system was reacted at 25°C for 2 hours. Upon completion of the reaction, the reaction system was subjected to rotary evaporation to dryness, and the residue was purified by silica gel column chromatography using eluent system A to obtain **165d** (80 g, yield: 73.5%). MS m/z (ESI): 298 [M+1]⁺
Step 4: Sodium hydride (71.66 g, 1.79 mol) was added to a three-necked flask, followed by the addition of tetrahydrofuran (solvent, 1.7 L) and methyl glycolate (64.55 g, 716.64 mmol) in an ice bath under nitrogen atmosphere, and the mixture was stirred for half an hour. **165d** (178 g, 597.20 mmol) was then added to the system, and the reaction system was reacted at 25°C for 16 hours. Upon completion of the reaction, the reaction system was quenched with water, extracted with ethyl acetate (2 L × 3), washed with a saturated sodium chloride aqueous solution, dried over anhydrous sodium sulphate, filtered, and concentrated to obtain **165e** (crude, 187 g). MS m/z (ESI): 276 [M+1]⁺
Step 5: **165e** (187 g, 679.55 mmol) was dissolved in a mixed solvent of concentrated hydrochloric acid (500 mL) and 1,4-dioxane (500 mL), and the reaction system was reacted at 110°C for 0.5 hours. Upon completion of the reaction, the reaction system was subjected to rotary evaporation to dryness to remove the solvent, adjusted to pH 8 with a saturated sodium bicarbonate solution, extracted with ethyl acetate (2 L × 3), washed with a saturated sodium chloride aqueous solution, dried over anhydrous sodium sulphate, filtered, and concentrated. The residue was purified by silica gel column chromatography using eluent system A to obtain **165f** (37.9 g, yield: 25.6%). MS m/z (ESI): 218 [M+1]⁺
Step 6: 2-(Trifluoromethyl)-8H-pyrano[3,4-b]pyridin-5-one **165f** (21 g, 96.74 mmol) and hydroxylamine hydrochloride (6.72 g, 96.74 mmol) were dissolved in ethanol (210 mL) and sodium acetate (23.80 g, 290.15 mmol), and the mixture was purged three times with nitrogen. The system was reacted at 80°C for 1 hour. The resulting reaction system was cooled to room temperature, concentrated, quenched with water, extracted with ethyl acetate (500 mL × 3), washed with a saturated sodium chloride aqueous solution, dried over anhydrous sodium sulphate, filtered, and concentrated. The residue was purified by silica gel column chromatography using eluent system B to obtain **165g** (21.70 g, yield: 96.7%). MS m/z (ESI): 233 [M+1]⁺
Step 7: **165g** (20.98 g, 90.37 mmol), zinc powder (59.08 g, 903.69 mmol) and ammonium chloride (48.34 g, 903.69 mmol) were dissolved in ethanol (40 mL), and the mixture was reacted at 50°C for 4 hours. The resulting reaction system was filtered, concentrated, and slurried with dichloromethane, to obtain **165h** (12.6 g, yield: 63.9%). MS m/z (ESI): 219 [M+1]⁺
Step 8: Methyl 4-amino-5-bromo-2-chlorobenzoate **165i** (80 g, 302.45 mmol), bis(pinacolato)diboron (115.21 g, 453.68 mmol), 1,1'-bis(diphenylphosphino)ferrocene (16.77 g, 30.25 mmol), potassium acetate (59.37 g, 604.91 mmol), and palladium acetate (3.40 g, 15.12 mmol) were added to 1,4-dioxane (800 mL), and the mixture was purged three times with nitrogen. The reaction system was stirred at 80°C for 16 hours. The reaction solution was cooled to room temperature, and filtered to remove insoluble materials. The filtrate was concentrated, and the residue was purified by silica gel column chromatography using eluent system B to obtain **165j** (84 g, 269.60 mmol, yield: 89.1%). MS m/z (ESI): 312 [M+1]⁺
Step 9: **165j** (70.5 g, 226.27 mmol) was added to ethanol (1000 mL) and water (200 mL), followed by methyl 4-(((trifluoromethyl)sulphonyl)oxy)-2,5-dihydrofuran-3-carboxylate (62.49 g, 226.27 mmol), sodium carbonate (47.97 g, 452.55 mmol) and bis(triphenylphosphine)palladium(II) dichloride (15.88 g, 22.63 mmol), and the mixture was purged three times with nitrogen. The reaction system was reacted at 80°C for 4 hours. The reaction solution was directly concentrated, water was added, and the mixture was stirred for 10 min. The resulting system was filtered by suction, and the solid was slurried with ethyl acetate, to obtain **165k** (49 g, 175.20 mmol, yield: 77.4%). MS m/z (ESI): 280 [M+1]⁺
Step 10: **165k** (56.7 g, 202.74 mmol) was dissolved in dimethyl sulphoxide (25.23 mL), and 2,4-dimethoxybenzylamine (40.68 g, 243.28 mmol) and 1,8-diazabicyclo[5.4.0]undec-7-ene (92.59 g, 608.21 mmol, 90.78 mL) were added, followed by a BOP reagent (269.00 g, 608.21 mmol). The reaction system was reacted at room temperature for 1 hour. The reaction solution was added to ice water, and extracted with ethyl acetate. The organic phase was dried, concentrated, and slurried with methanol to obtain **165l** (43 g, 100.26 mmol, yield: 49.5%). MS m/z (ESI): 429 [M+1]⁺
Step 11: **165l** (5 g, 11.66 mmol) was dissolved in N,N-dimethylformamide (50 mL) and placed in a sealed tube. 2-(4-Pentynyloxy)tetrahydro-2H-pyran (2.94 g, 17.49 mmol), caesium carbonate (7.60 g, 23.32 mmol), dichlorobis(tricyclohexylphosphine)palladium(II) (860.62 mg, 1.17 mmol), and 2-dicyclohexylphosphino-2',4',6'-triisopropylbiphenyl (1.11 g, 2.33 mmol) were added, and the mixture was purged three times with nitrogen. The reaction system was reacted at 100°C for 2 hours. Water was added to the reaction solution, and the mixture was extracted with ethyl acetate, washed with a saturated sodium chloride aqueous solution, dried over anhydrous sodium sulphate, filtered, and concentrated. The residue was purified by silica gel column chromatography using eluent system B to obtain **165m** (6.05 g, 10.79 mmol, yield: 92.6%). MS m/z (ESI): 561 [M+1]⁺
Step 12: **165m** (6.05 g, 10.79 mmol) was dissolved in methanol (60 mL), trifluoroacetic acid (12 mL) was added, and the reaction system was reacted at room temperature for 1 hour. The reaction solution was adjusted to pH=8-9 with a sodium bicarbonate aqueous solution, extracted with dichloromethane (containing 10% methanol), washed with a saturated sodium chloride aqueous solution, dried over anhydrous sodium sulphate, filtered, and concentrated. The residue was purified by silica gel column chromatography using eluent system B to obtain **165n** (3.64 g, 7.64 mmol, yield: 70.8%). MS m/z (ESI): 477 [M+1]⁺
Step 13: **165n** (1 g, 2.10 mmol) was dissolved in dichloromethane (10 mL) and methanol (10 mL), and nickel acetate tetrahydrate (104.44 mg, 419.71 µmol) was added. Sodium borohydride (158.78 mg, 4.20 mmol) was slowly added in portions at room temperature, and the reaction system was reacted at room temperature for 3 minutes. Water was added to the reaction solution, and the mixture was extracted with ethyl acetate, washed with a saturated sodium chloride aqueous solution, dried over anhydrous sodium sulphate, filtered, and concentrated. The residue was purified by silica gel column chromatography using eluent system B to obtain **165o** (0.39 g, 0.81 mmol, yield: 38.8%). MS m/z (ESI): 479 [M+1]⁺
Step 14: **165o** (1.3 g, 2.72 mmol) was dissolved in dichloromethane (30 mL) to form a clear solution. Carbon tetrabromide (3.60 g, 10.87 mmol) and triphenylphosphine (2.85 g, 10.87 mmol) were added, and the reaction system was stirred at room temperature for 3 hours. The reaction solution was directly concentrated, and the residue was purified by silica gel column chromatography using eluent system A to obtain **165p** (1.27 g, 2.35 mmol, yield: 86.3%). MS m/z (ESI): 541 [M+1]⁺
Step 15: **165p** (1.4 g, 2.59 mmol) was dissolved in acetonitrile (30 mL). 2-(Trifluoromethyl)-5,8-dihydro-6H-pyrano[3,4-b]pyridin-5-amine (676.97 mg, 3.10 mmol), potassium carbonate (1.07 g, 7.76 mmol), and sodium iodide (775.15 mg, 5.17 mmol) were added, and the mixture was purged three times with nitrogen. The reaction system was reacted at 80°C for 16 hours. The resulting reaction system was quenched with water, extracted with ethyl acetate (150 mL × 3), washed with a saturated sodium chloride aqueous solution, dried over anhydrous sodium sulphate, filtered, and concentrated. The residue was purified by silica gel column chromatography using eluent system B to obtain **165q** (726 mg, 1.07 mmol, yield: 41.4%). MS m/z (ESI): 679 [M+1]⁺
Step 16: **165q** (762 mg, 1.12 mmol) was dissolved in tetrahydrofuran (10 mL), methanol (10 mL) and water (10 mL), lithium hydroxide monohydrate (141.33 mg, 3.37 mmol) was added, and the reaction system was reacted at 50°C for 3 hours. The reaction solution was concentrated to remove the organic phase, adjusted to neutral with 2N hydrochloric acid, extracted with dichloromethane (containing 10% methanol), washed with a saturated sodium chloride aqueous solution, dried over anhydrous sodium sulphate, filtered, and concentrated to obtain **165r** (553 mg, 831.99 µmol, yield: 74.1%). MS m/z (ESI): 665 [M+1]⁺
Step 17: **165r** (553 mg, 831.99 µmol) was dissolved in N,N-dimethylformamide (20 mL), N,N-diisopropylethylamine (215.05 mg, 1.66 mmol, 289.83 µL) and O-(7-azabenzotriazol-1-yl)-N,N,N',N'-tetramethyluronium hexafluorophosphate (470.83 mg, 1.25 mmol) were added, and the reaction system was stirred at room temperature for 30 minutes. The resulting reaction system was quenched with water, extracted with ethyl acetate (50 mL × 3), washed with a saturated sodium chloride aqueous solution, dried over anhydrous sodium sulphate, filtered, and concentrated. The residue was purified by silica gel column chromatography using eluent system A to obtain **165s** (436 mg, 674.24 µmol, yield: 81.0%). MS m/z (ESI): 647 [M+1]⁺
Step 18: **165s** (436 mg, 674.24 µmol) was added to trifluoroacetic acid (10 mL), and the reaction system was reacted at 80°C for 1 hour. The reaction solution was adjusted to pH=8-9 with a sodium bicarbonate aqueous solution, extracted with dichloromethane (containing 10% methanol), washed with a saturated sodium chloride aqueous solution, dried over anhydrous sodium sulphate, filtered, and concentrated. The residue was purified by silica gel column chromatography using eluent system A to obtain **Example 165** (121 mg, yield: 36.15%). MS m/z (ESI): 497 [M+1]⁺

### Examples 165-P1 & 165-P2

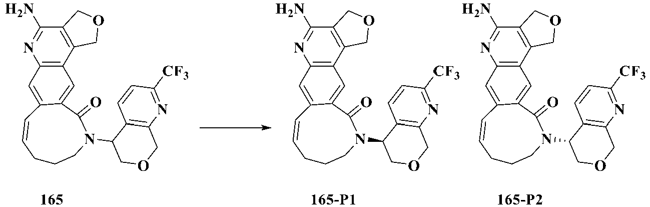

**Example 165** (388 mg, 0.78 mmol) was separated by chiral preparative HPLC to obtain rel-(S)-(Z)-4-amino-12-(2-trifluoromethyl-5,8-dihydro-6H-pyrano[3,4-b]pyridin-5-yl)-1,3,9,10,11,12-hexahydro-13H-azonino[3,4-g]furo[3,4-c]quinolin-13-one **165-P1** (181 mg, yield: 46.6%) and rel-(R)-(Z)-4-amino-12-(2-trifluoromethyl-5,8-dihydro-6H-pyrano[3,4-b]pyridin-5-yl)-1,3,9,10,11,12-hexahydro-13H-azonino[3,4-g]furo[3,4-c]quinolin-13-one **165-P2** (177 mg, yield: 45.6%).

**165-P1 (t_{R}: 3.029 min)**: ¹H NMR (400 MHz, DMSO-*d6*) δ 8.11 (dd, 1H), 7.86 (dd, 1H), 7.46 (d, 2H), 7.28 (d, 1H), 6.66 (s, 1H), 6.60 (q, 1H), 6.50-5.70 (m, 1H), 5.50-5.10 (m, 1H), 5.34 (s, 2H), 5.03 (s, 2H), 4.93-4.74 (m, 2H), 4.34-4.13 (m, 2H), 3.75-3.10 (m, 2H), 2.18-1.70 (m, 2H), 1.45-1.25 (m, 2H).

**165-P2 (t_{R}: 2.383 min)**: ¹H NMR (400 MHz, DMSO-*d6*) δ 8.11 (dd, 1H), 7.86 (dd, 1H), 7.46 (d, 2H), 7.28 (d, 1H), 6.66 (s, 1H), 6.60 (q, 1H), 6.50-5.70 (m, 1H), 5.50-5.10 (m, 1H), 5.34 (s, 2H), 5.03 (s, 2H), 4.93-4.74 (m, 2H), 4.34-4.13 (m, 2H), 3.75-3.10 (m, 2H), 2.18-1.70 (m, 2H), 1.45-1.25 (m, 2H).

### Example 167

### (Z)-4-Amino-12-(7-trifluoromethylisochroman-4-yl)-9,10,11,12-tetrahydro-13H-azonino[4,3-g]imidazo[1,5-a]quinoxalin-13-one

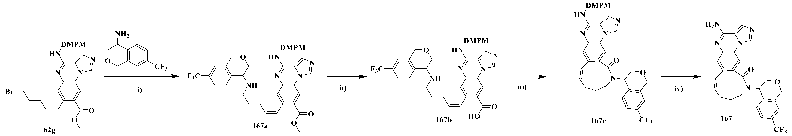

Referring to the synthesis method described in Step 6 to Step 9 of Example 62, **Example 167** (7 mg, yield: 0.7%) was obtained from methyl *(Z)-*7-(6-bromopent-1-en-1-yl)-4-(2,4-dimethoxybenzylamino)imidazo[1,5-a]quinoxaline-8-carboxylate **62g** (1.08 g, 2.00 mmol). MS m/z (ESI): 494 [M+1]⁺

¹H NMR (400 MHz, DMSO-*d*₆) δ 9.12 (d, 1H), 8.16 (dd, 1H), 7.90 (s, 1H), 7.66 (m, 2H), 7.57 (d, 1H), 7.38 (s, 2H), 7.16 (s, 1H), 6.60 (m, 1H), 6.15 (m, 1H), 5.77 (m, 1H), 4.90 (m, 1H), 4.75 (m, 1H), 4.45-4.10 (m, 2H), 3.85-2.95 (m, 2H), 2.15-1.60 (m, 2H), 1.42-1.10 (m, 2H).

### Example 171

### (Z)-4-Amino-12-((5-(trifluoromethyl)pyridin-2-yl)methyl)-9,10,11,12-tetrahydro-13H-azonino[4,3-g]imidazo[1,5-a]quinoxalin-13-one

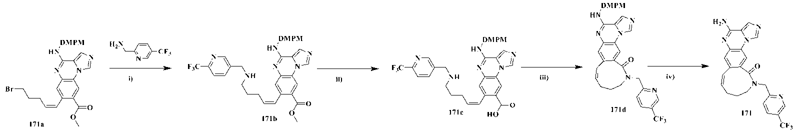

Referring to the synthesis method described in Step 6 to Step 9 of **Example 62, Example 171** (10 mg, yield: 2.9%) was obtained from methyl *(Z)*-7-(5-bromopent-1-en-1-yl)-4-((3,5-dimethoxybenzyl)amino)imidazo[1,5-a]quinoxaline-8-carboxylate 171a (500 mg, 4.24 mmol). MS m/z (ESI): 453 [M+1]⁺

¹H NMR (400 MHz, MeOD) δ 9.08 (s, 1H), 8.88 (s, 1H), 8.21-8.08 (m, 2H), 7.96 (s, 1H), 7.74 (d, 1H), 7.33 (s, 1H), 6.59 (d, 1H), 6.08 (td, 1H), 5.18 (d, 1H), 4.56 (d, 1H), 3.65-3.44 (m, 2H), 2.20 (dt, 1H), 1.90 (dq, 2H), 1.50 (d, 1H).

Alternatively, the following examples were prepared using the following method:

### Example 173

### (Z)-4-Amino-12-((5-(trifluoromethyl)pyridin-2-yl)methyl)-1,3,9,10,11,12-hexahydro-13H-azo[3,4-g]furo[3,4-c]quinolin-13-one

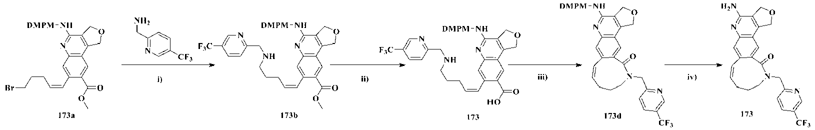

Step 1: Methyl 7-[(Z)-5-bromopent-1-enyl]-4-[(2,4-dimethoxyphenyl)methylamino]-1,3-dihydrofuro[3,4-c]quinoline-8-carboxylate **173a** (0.7 g, 1.29 mmol) was dissolved in acetonitrile (20 mL). [5-(Trifluoromethyl)-2-pyridyl]methanamine (273.27 mg, 1.55 mmol), potassium carbonate (536.06 mg, 3.88 mmol), and sodium iodide (387.58 mg, 2.59 mmol) were added, and the mixture was purged three times with nitrogen. The reaction system was reacted at 80°C for 16 hours. The resulting reaction system was quenched with water, extracted with ethyl acetate (100 mL × 3), washed with a saturated sodium chloride aqueous solution, dried over anhydrous sodium sulphate, filtered, and concentrated. The residue was purified by silica gel column chromatography using eluent system A to obtain **173b** (329 mg, 516.76 µmol, yield: 40.0%). MS m/z (ESI): 637 [M+1]⁺
Step 2: **173b** (329 mg, 516.76 µmol) was dissolved in tetrahydrofuran (5 mL), methanol (5 mL) and water (5 mL), lithium hydroxide monohydrate (65.05 mg, 1.55 mmol) was added, and the reaction system was reacted at 50°C for 3 hours. The reaction solution was concentrated to remove the organic phase, adjusted to neutral with 2N hydrochloric acid, extracted with dichloromethane (containing 10% methanol), washed with a saturated sodium chloride aqueous solution, dried over anhydrous sodium sulphate, filtered, and concentrated to obtain **173c** (220 mg, 353.34 µmol, yield: 68.4%). MS m/z (ESI): 623 [M+1]⁺
Step 3: **173c** (210 mg, 337.28 µmol) was dissolved in N,N-dimethylformamide (20 mL), N,N-diisopropylethylamine (65.38 mg, 505.92 µmol, 88.12 µL) and O-(7-azabenzotriazol-1-yl)-N,N,N',N'-tetramethyluronium hexafluorophosphate (190.87 mg, 505.92 µmol) were added, and the reaction system was stirred at room temperature for 30 minutes. The resulting reaction system was quenched with water, extracted with ethyl acetate (50 mL × 3), washed with a saturated sodium chloride aqueous solution, dried over anhydrous sodium sulphate, filtered, and concentrated. The residue was purified by silica gel column chromatography using eluent system A to obtain **173d** (188 mg, 310.94 µmol, yield: 92.2%). MS m/z (ESI): 604 [M+1]⁺
Step 4: **173d** (188 mg, 310.94 µmol) was added to trifluoroacetic acid (10 mL), and the reaction system was reacted at 80°C for 1 hour. The reaction solution was adjusted to pH=8-9 with a sodium bicarbonate aqueous solution, extracted with dichloromethane (containing 10% methanol), washed with a saturated sodium chloride aqueous solution, dried over anhydrous sodium sulphate, filtered, and concentrated. The residue was subjected to reversed-phase preparative chromatography to obtain **Example 173** (13.4 mg, 29.49 µmol, yield: 9.5%). MS m/z (ESI): 454 [M+1]⁺
¹H NMR (400 MHz, DMSO-*d6*) δ 8.59 (s, 1H), 8.59 (s, 2H), 8.24 (dd, 1H), 7.74 (s, 1H), 7.69 (d, 1H), 7.52 (s, 1H), 6.62 (d, 1H), 6.07 (d, 1H), 5.46 (dd, 2H), 5.13-4.99 (m, 3H), 4.53 (d, 1H), 2.21-2.08 (m, 1H), 2.05-1.87 (m, 1H), 1.70 (d, 2H), 1.40 (s, 1H), 1.27 (d, 1H)

### Example 176

### 4-Amino-12-((5-(trifluoromethyl)pyridin-2-yl)methyl)-9,10,11,12-tetrahydroimidazo[1,5-a][1,5]oxazonino[8,7-g]quinoxalin-13(7H)-one

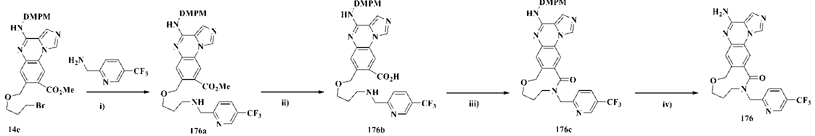

Referring to the method described in Step 5 to Step 8 of Example **14, Example 176** (10 mg, yield: 5.3%) was obtained from methyl 7-((3-bromopropoxy)methyl)-4-((3,4-dimethoxybenzyl)amino)imidazo[1,5-a]quinoxaline-8-carboxylate **14e** (220 mg, 0.41 mmol). MS m/z (ESI): 457 [M+1]⁺
¹H NMR (400 MHz, DMSO-*d6*) δ 9.20 (d, 1H), 9.02 - 8.97 (m, 1H), 8.24 (dd, 1H), 8.09 (s, 1H), 7.91 (d, 1H), 7.80 (d, 1H), 7.40 (d, 3H), 5.21 (d, 1H), 4.65 (d, 1H), 4.56 - 4.43 (m, 2H), 3.50 (t, 4H), 1.25 (d, 2H).

Alternatively, the following examples were prepared using the following method:

### Example 177

### 4-Amino-9-methyl-12-((5-(trifluoromethyl)pyridin-2-yl)methyl)-9,10,11,12-tetrahydroimidazo[1,5-a][1,5]oxazolo[8,7-g]quinoxalin-13(7H)-one

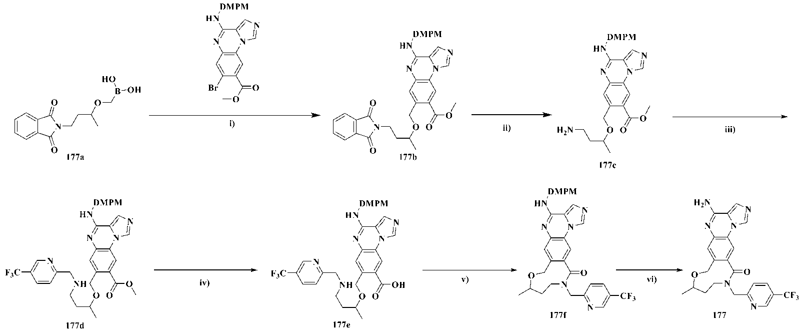

Step 1: 7.7-Dimethyl-4-[(2,4-dimethoxyphenyl)methylamino]imidazo[1,5-a]quinazoline-8-carboxylate **177a** (4 g, 8.49 mmol), [3-(1,3-dioxoisoindolin-2-yl)-1-methylpropoxy]methylboronic acid (3.53 g, 12.73 mmol), [n-butyl-di(1-adamantyl)phosphine](2-amino-1,1'-biphenyl-2-yl)palladium(II) methanesulphonate (1.85 g, 2.55 mmol), and potassium phosphate (5.40 g, 25.46 mmol) were dissolved in dioxane (50 mL) and water (10 mL), and the mixture was purged three times with nitrogen, and stirred at 110°C for 16 hours. The resulting reaction system was cooled to room temperature, quenched with water, extracted with ethyl acetate (60 mL × 3), washed with a saturated sodium chloride aqueous solution, dried over anhydrous sodium sulphate, filtered, and concentrated. The residue was purified by silica gel column chromatography using eluent system B to obtain **177b** (3.5 g, yield: 66.1%). MS m/z (ESI): 624 [M+1]⁺
Step 2: **177b** (500 mg, 801.73 µmol) was dissolved in ethanol (10 mL), hydrazine hydrate (141.65 mg, 2.41 mmol, 85% purity) was added, and the system was reacted at 80°C for 16 hours. The resulting reaction system was cooled to room temperature and filtered, and the solid was dried to obtain **177c** (210 mg, yield: 53.1%). MS m/z (ESI): 494 [M+1]⁺
Step 3: **177c** (1 g, 2.03 mmol) and 5-(trifluoromethyl)pyridine-2-carbaldehyde (425.75 mg, 2.43 mmol) were dissolved in methanol (50 mL), followed by the addition of acetic acid (243.35 mg, 4.05 mmol) and sodium cyanoborohydride (381.96 mg, 6.08 mmol), and the system was reacted at 25°C for 16 hours. The resulting reaction system was quenched with water, extracted with ethyl acetate (60 mL × 3), washed with a saturated sodium chloride aqueous solution, dried over anhydrous sodium sulphate, filtered, and concentrated. The residue was purified by silica gel column chromatography using eluent system B to obtain **177d** (400 mg, yield: 30.2%). MS m/z (ESI): 525 [M+1]⁺
Step 4: **177d** (32.6 mg, 49.95 µmol) was dissolved in tetrahydrofuran (1 mL), water (1 mL) and methanol (1 mL), lithium hydroxide (10.48 mg, 249.75 µmol) was added, and the reaction system was reacted at 25°C for 16 hours. The resulting reaction system was adjusted to pH 5-6 with 1 M dilute hydrochloric acid, extracted with ethyl acetate (5 mL × 3), washed with a saturated sodium chloride aqueous solution (30 mL), dried over anhydrous sodium sulphate, filtered, and concentrated to obtain **177e** (31 mg, yield: 97.1%). MS m/z (ESI): 639 [M+1]⁺
Step 5: **177e** (451.8 mg, 707.45 µmol) and N,N-diisopropylethylamine (548.58 mg, 4.24 mmol, 739.33 µL) were dissolved in N,N-dimethylformamide (10 mL), 2-(7-azobenzotriazol-1-yl)-N,N,N',N'-tetramethyluronium hexafluorophosphate (533.80 mg, 1.41 mmol) was added, and the reaction system was reacted at 25°C for 16 hours. The resulting reaction system was quenched with water, extracted with ethyl acetate (80 mL × 3), washed with a saturated sodium chloride aqueous solution, dried over anhydrous sodium sulphate, filtered, and concentrated. The residue was purified by silica gel column chromatography using eluent system B to obtain **177f** (430 mg, yield: 97.9%). MS m/z (ESI): 621 [M+1]⁺
Step 6: **177f** (400 mg, 644.52 µmol) was dissolved in trifluoroacetic acid (8 mL), and the reaction system was reacted at 80°C for 0.5 hours. Upon completion of the reaction as monitored by LCMS, the reaction solution was subjected to rotary evaporation to dryness to obtain a crude product, which was purified by preparative liquid chromatography to obtain **Example 177** (205.8 mg, yield: 67.8%). MS m/z (ESI): 471 [M+1]⁺
¹H NMR (400 MHz, DMSO-*d6*) δ 9.22 (s, 1H), 9.00 (d, 1H), 8.29 - 8.19 (m, 1H), 8.07 (s, 1H), 7.94 (s, 1H), 7.78 (dd, 1H), 7.48 (s, 2H), 7.39 (d, 1H), 5.22 (dd, J = 16.1, 7.2 Hz, 1H), 4.85 (dd, 1H), 4.52 (dd, 1H), 4.32 (dd, 1H), 3.43 (d, 2H), 3.23 (d, 1H), 1.68 (dd, 1H), 1.18 (s, 1H), 1.12 (t, 3H).

### Example 178

### 4-Amino-12-((5-(trifluoromethyl)pyridin-2-yl)methyl)-3,7,9,10,11,12-hexahydrofuro[3,4-c][1,5]oxazolino[7,8-g]quinolin-13(1H)-one

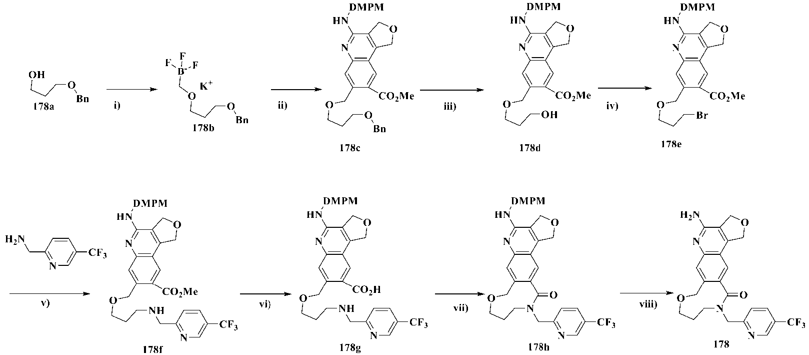

Referring to the synthesis method of **Example 14, Example 178** (25 mg, yield: 0.9%) was obtained from 3-(benzyloxy)propan-1-ol **178a** (1 g, 6 mmol). MS m/z (ESI): 459 [M+1]⁺
¹H NMR (400 MHz, DMSO-*d*₆) δ 8.97 (d, 1H), 8.24 (dd, 1H), 7.76 (d, 1H), 7.61 (s, 1H), 7.52 (s, 1H), 5.42 (s, 2H), 5.19 (d, 1H), 5.06 (s, 2H), 4.74 (d, 1H), 4.58 (d, 1H), 4.45 (d, 1H), 3.81 (d, 1H), 3.44 (m, 1H), 3.26 (m, 2H), 1.91 (m, 1H), 1.23 (d, 1H).

### Example 184

### 4-Amino-3-methyl-12-((5-(trifluoromethyl)pyridin-2-yl)methyl)-3,7,9,10,11,12-hexahydrofuro[3,4-c][1,5]oxazolo [7,8-g]quinoxalin-13(1H)-one

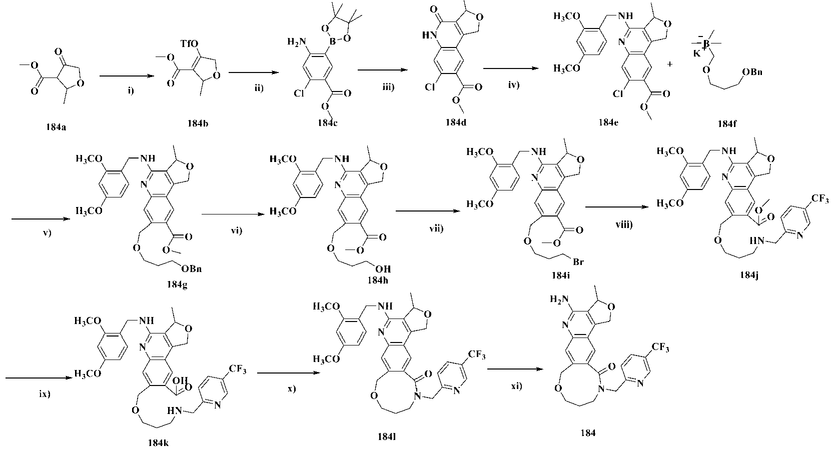

Referring to the synthesis of **Example 178, Example 184** (12.5 mg, yield: 0.6%) was obtained from methyl 2-methyl-4-oxotetrahydrofuran-3-carboxylate **184a** (500 mg, 3.16 mmol) through steps of coupling, deprotection, bromination, substitution, hydrolysis, cyclisation, and deprotection. MS m/z (ESI): 473[M+1]⁺

¹H NMR (400 MHz, DMSO-*d6*) δ 8.96 (d, 1H), 8.26 - 8.22 (m, 1H), 7.75 (m, 1H), 7.50 (s, 1H), 7.38 (s, 1H), 6.56 (s, 2H), 5.47 - 5.24 (m, 4H), 5.19 (m, 1H), 4.71 (m, 1H), 4.53 (m, 1H), 4.43 (d, 1H), 3.77 (d, 1H), 3.23 (d, 2H), 2.03 - 1.88 (m, 2H), 1.40 (d, 3H).

### Example 184-P1

### (R)-4-Amino-3-methyl-12-((5-(trifluoromethyl)pyridin-2-yl)methyl)-3,7,9,10,11,12-hexahydrofuro[3,4-c][1,5]oxazolo [7,8-g]quinoxalin-13(1H)-one

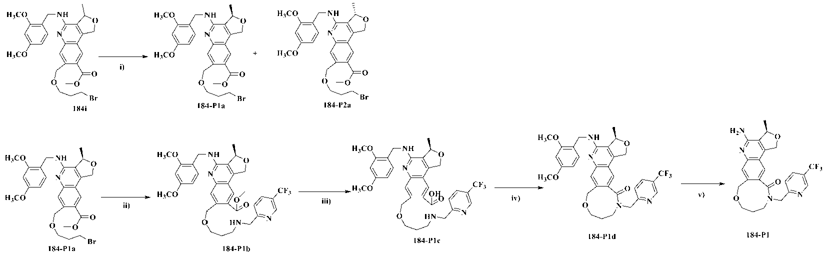

### Step 1

Compound **184i** was separated by chiral preparative HPLC to obtain **184-P1a** (200 mg) and **184-P2a** (220 mg). MS m/z (ESI): 561[M+1]⁺
Referring to Step 8 to Step 11 of **Example 14, Example 184-P1** (22 mg) was obtained from **184-P1a** (200 mg, 0.394 mmol) through steps of substitution, hydrolysis, cyclisation, and deprotection. MS m/z (ESI): 473 [M+1]⁺

¹HNMR (400 MHz, DMSO-*d6*) δ 8.96 (d, 1H), 8.26 - 8.22 (m, 1H), 7.75 (m, 1H), 7.50 (s, 1H), 7.38 (s, 1H), 6.56 (s, 2H), 5.47 - 5.24 (m, 4H), 5.19 (m, 1H), 4.71 (m, 1H), 4.53 (m, 1H), 4.43 (d, 1H), 3.77 (d, 1H), 3.23 (d, 2H), 2.03 - 1.88 (m, 2H), 1.40 (d, 3H).

### Example 184-P2

### (S)-4-Amino-3-methyl-12-((5-(trifluoromethyl)pyridin-2-yl)methyl)-3,7,9,10,11,12-hexahydrofuro [3,4-c][1,5]oxazolo [7,8-g]quinoxalin-13(1H)-one

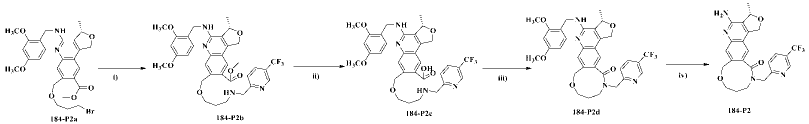

Referring to the preparation of **Example 184-P1, Example 184-P2** (18 mg) was obtained from **184-P2a** (220 mg, 0.394 mmol) through steps of substitution, hydrolysis, cyclisation, and deprotection. MS m/z (ESI): 473 [M+1]⁺

¹HNMR (400 MHz, DMSO-*d6*) δ 8.96 (d, 1H), 8.26 - 8.22 (m, 1H), 7.75 (m, 1H), 7.50 (s, 1H), 7.38 (s, 1H), 6.56 (s, 2H), 5.47 - 5.24 (m, 4H), 5.19 (m, 1H), 4.71 (m, 1H), 4.53 (m, 1H), 4.43 (d, 1H), 3.77 (d, 1H), 3.23 (d, 2H), 2.03 - 1.88 (m, 2H), 1.40 (d, 3H).

### Example 190

### 4-Amino-1-methyl-12-((5-(trifluoromethyl)pyridin-2-yl)methyl)-1,7,9,10,11,12-hexahydro-13H-[1,5]oxazolo[7,8-g]pyrazolo[4,3-c]quinolin-13-one

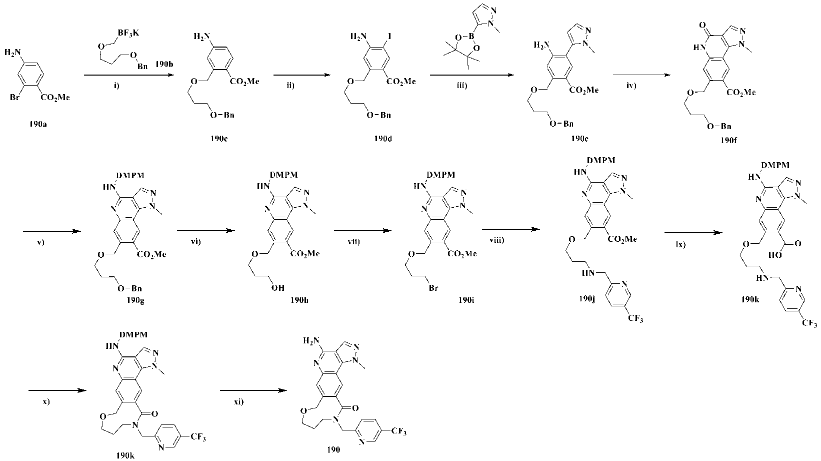

Referring to the preparation method of Example 165, Example 190 (17 mg, yield: 2.6%) was obtained from methyl 4-amino-2-bromobenzoate 190a (5 g, 21.73 mmol). MS m/z (ESI): 471 [M+1]⁺
¹H NMR (400 MHz, DMSO-*d6*) δ 8.97 (d, 1H), 8.38 (s, 1H), 8.26 (dd, 1H), 8.07 (s, 1H), 7.80 (d, 1H), 7.59 (s, 2H), 5.20 (d, 1H), 4.75 (d, 1H), 4.55 (dd, 2H), 3.81 (dt, 1H), 3.54 - 3.36 (m, 3H), 3.26 (d, 3H), 1.90 (q, 1H), 1.25 (d, 1H).

### Example 214

### 4-Amino-12-(4-(pentafluoro-λ⁶-sulphanyl)benzyl)-9,10,11,12-tetrahydroimidazo[1,5-a][1,5]oxazonino[8,7-g]quinoxalin-13(7H)-one

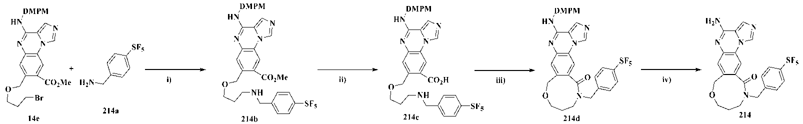

Referring to the synthesis of Example **14, Example 214** (10 mg, yield: 5.3%) was obtained from methyl 7-((3-bromopropoxy)methyl)-4-((3,4-dimethoxybenzyl)amino)imidazo[1,5-a]quinoxaline-8-carboxylate **14e** (220 mg, 0.41 mmol). MS m/z (ESI): 514 [M+1]⁺

¹H NMR (400 MHz, DMSO-*d6*) δ 9.21 (d, 1H), 8.10 (s, 1H), 7.93 (d, 3H), 7.69 (d, 2H), 7.40 (d, 3H), 5.24 (d, 1H), 4.57 (dd, 2H), 4.31 (d, 1H), 3.75 (d, 1H), 3.46 (t, 1H), 3.29 (s, 1H), 3.10 (d, 1H), 1.92 (dd, 1H), 1.20 (d, 1H).

The following examples were prepared by reference to **Example 62:**

| **Example** | **Structure** | **MS m/z (ESI) [M+1]⁺** | **Example** | **Structure** | **MS m/z (ESI) [M+1]⁺** |
|---|---|---|---|---|---|
| **284** | 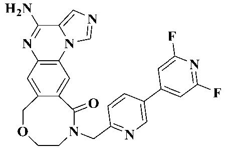 | 488 | **350** | 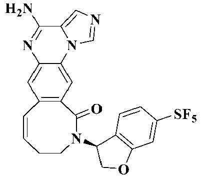 | 524 |
| **285** | 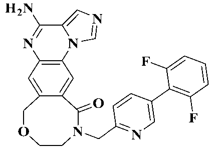 | 487 | **351** | 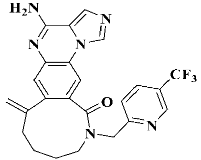 | 467 |
| **286** | 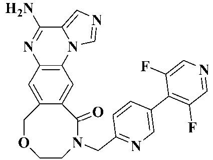 | 488 | **352** | 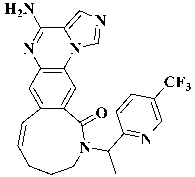 | 467 |
| **287** | 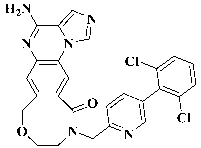 | 519 | **352-P1** | 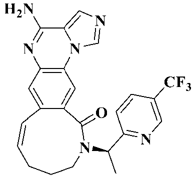 | 467 |
| **288** | 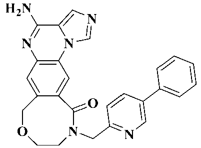 | 451 | **352-P2** | 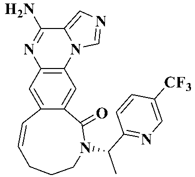 | 467 |
| **289** | 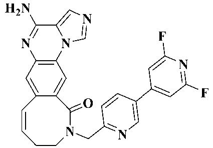 | 484 | **353** | 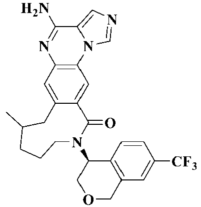 | 510 |
| **290** | 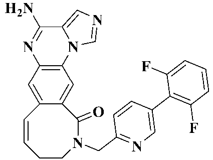 | 483 | **354** | 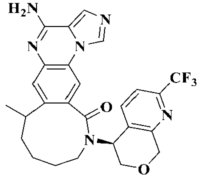 | 511 |
| **291** | 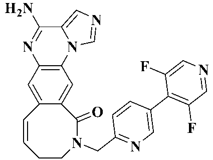 | 484 | **355** | 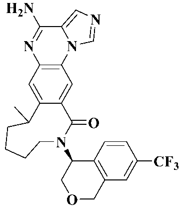 | 510 |
| **292** | 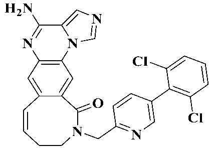 | 515 | **356** | 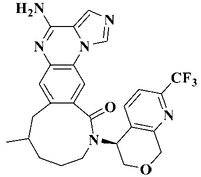 | 511 |
| **293** | 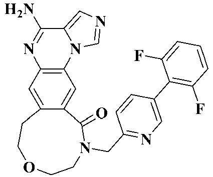 | 501 | **357** | 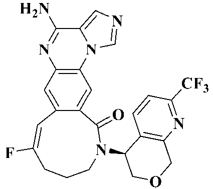 | 513 |
| **294** | 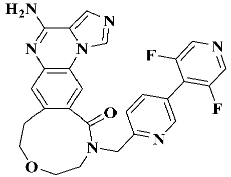 | 502 | **358** | 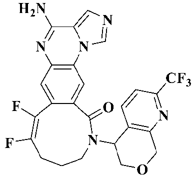 | 531 |
| **295** | 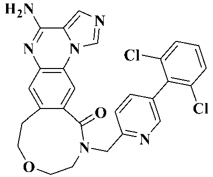 | 533 | **359** | 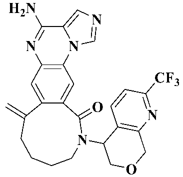 | 509 |
| **296** | 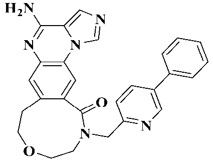 | 465 | **360** | 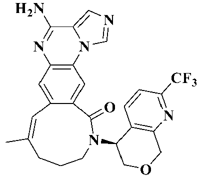 | 509 |
| **297** | 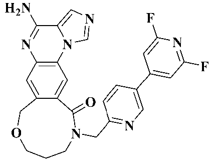 | 502 | **361** | 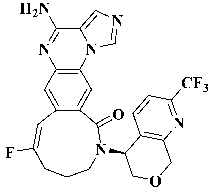 | 513 |
| **298** | 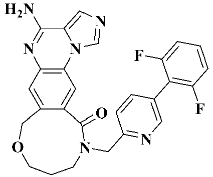 | 501 | **362-P1** | 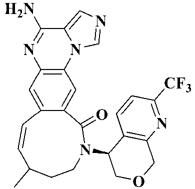 | 509 |
| **299** | 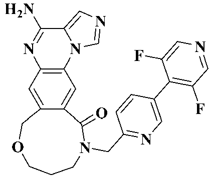 | 502 | **363** | 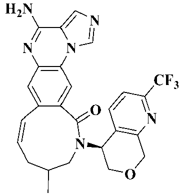 | 509 |
| **300** | 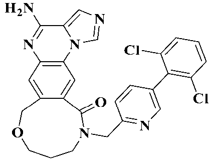 | 533 | **364** | 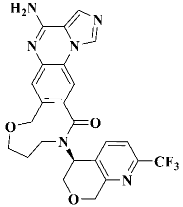 | 499 |
| **301** | 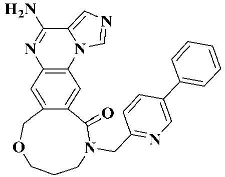 | 465 | **365** | 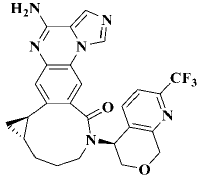 | 509 |
| **302** | 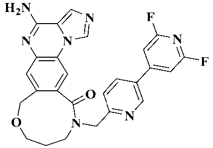 | 502 | **366** | 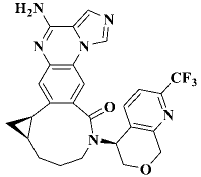 | 509 |
| **303** | 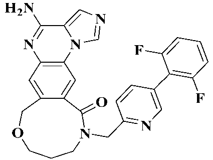 | 501 | **367** | 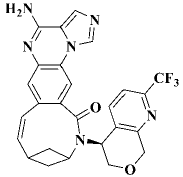 | 507 |
| **304** | 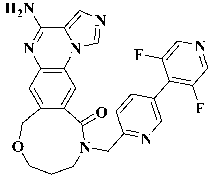 | 502 | **368** | 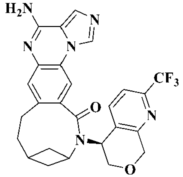 | 509 |
| **305** | 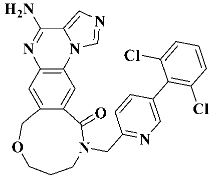 | 533 | **369** | 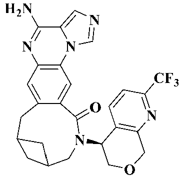 | 509 |
| **306** | 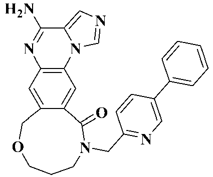 | 465 | **370** | 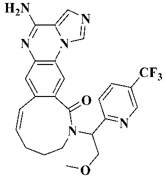 | 497 |
| **307** | 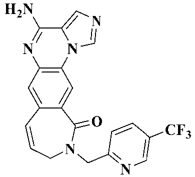 | 424 | **371** | 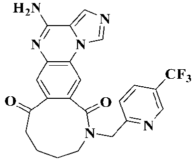 | 469 |
| **308** | 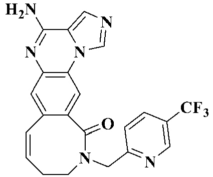 | 439 | **372** | 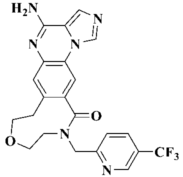 | 457 |
| **309** | 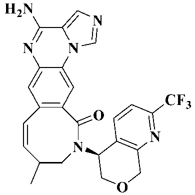 | 495 | **373** | 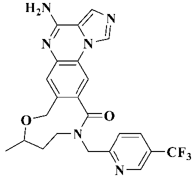 | 471 |
| **310** | 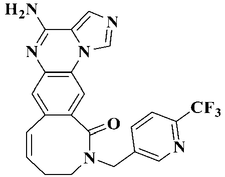 | 439 | **374** | 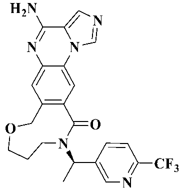 | 471 |
| **311-P1** | 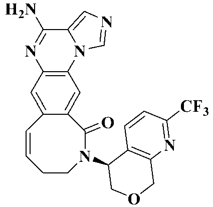 | 481 | **375** | 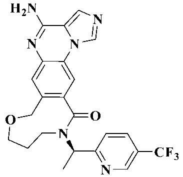 | 471 |
| **312** | 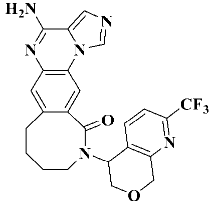 | 482 | **376** | 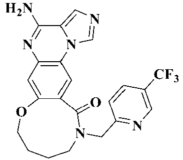 | 457 |
| **313** | 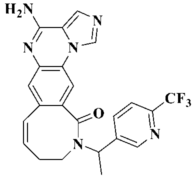 | 453 | **377** | 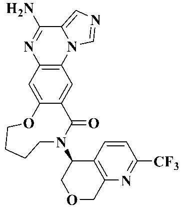 | 499 |
| **313-P1** | 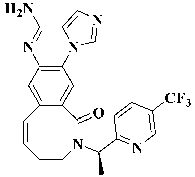 | 453 | **378** | 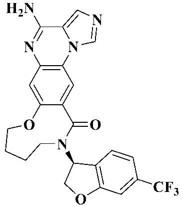 | 484 |
| **313-P2** | 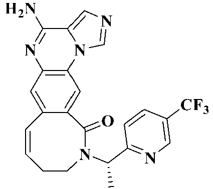 | 453 | **379** | 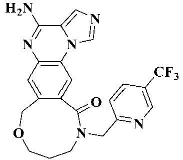 | 457 |
| **314** | 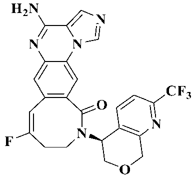 | 499 | **380** | 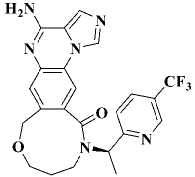 | 471 |
| **315** | 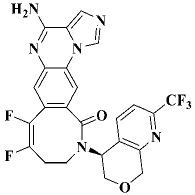 | 517 | **381** | 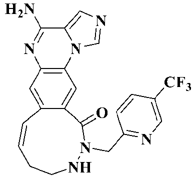 | 454 |
| **316** | 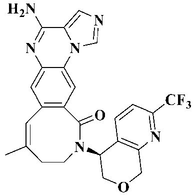 | 495 | **382** | 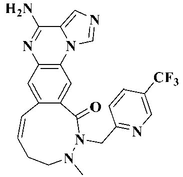 | 468 |
| **317** | 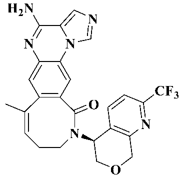 | 495 | **383** | 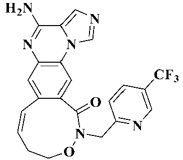 | 455 |
| **318** | 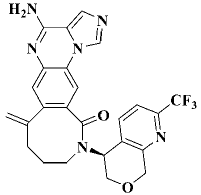 | 495 | **384** | 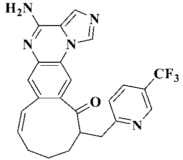 | 451 |
| **319** | 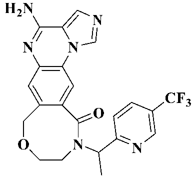 | 457 | **385** | 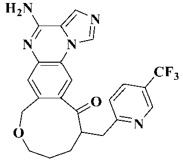 | 456 |
| **320** | 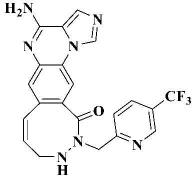 | 440 | **386** | 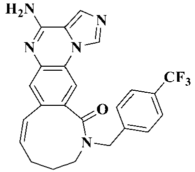 | 451 |
| **321** | 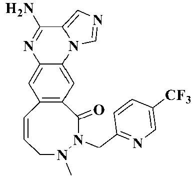 | 454 | **387** | 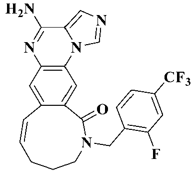 | 470 |
| **322** | 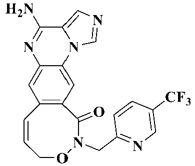 | 441 | **388** | 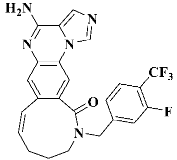 | 470 |
| **323** | 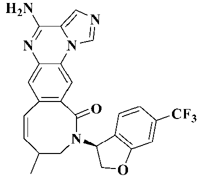 | 480 | **389** | 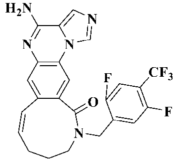 | 488 |
| **324** | 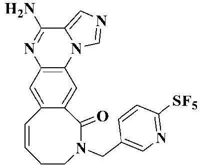 | 497 | **390** | 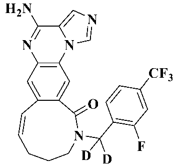 | 472 |
| **325-P1** | 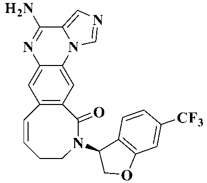 | 466 | **391** | 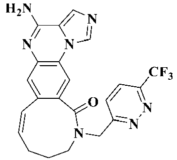 | 454 |
| **326** | 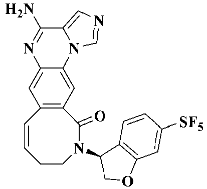 | 524 | **392** | 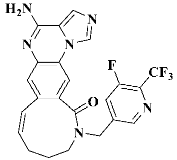 | 471 |
| **327** | 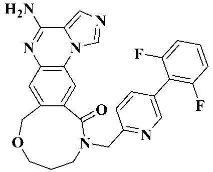 | 501 | **393** | 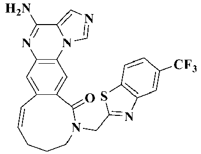 | 509 |
| **328** | 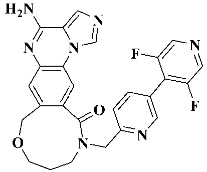 | 502 | **394** | 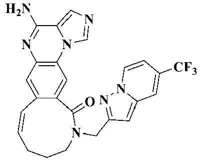 | 492 |
| **329** | 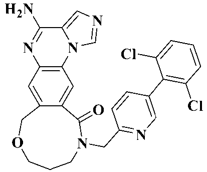 | 533 | **395** | 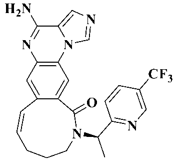 | 467 |
| **330** | 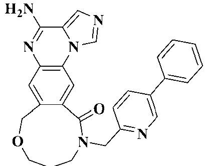 | 465 | **396** | 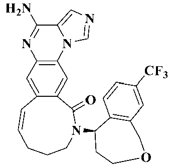 | 508 |
| **331** | 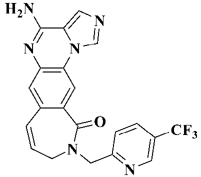 | 425 | **397** | 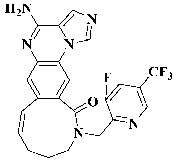 | 471 |
| **332** | 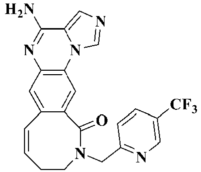 | 439 | **398** | 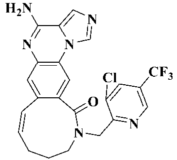 | 487 |
| **333** | 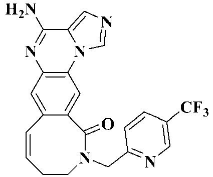 | 439 | **399** | 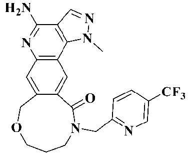 | 471 |
| **334** | 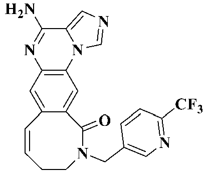 | 439 | **400** | 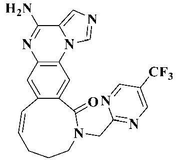 | 454 |
| **335** | 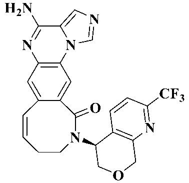 | 481 | **401** | 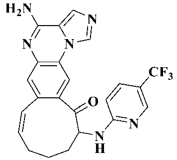 | 453 |
| **336** | 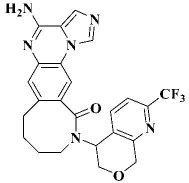 | 483 | **402** | 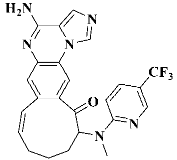 | 467 |
| **337** | 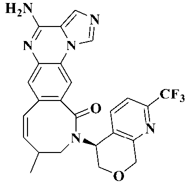 | 495 | **403** | 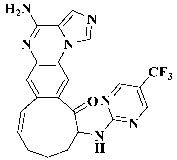 | 454 |
| **338** | 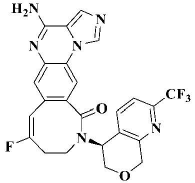 | 499 | **404** | 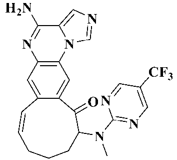 | 468 |
| **339** | 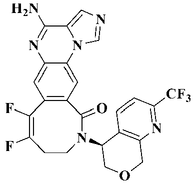 | 517 | **405** | 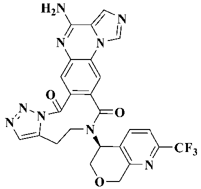 | 550 |
| **340** | 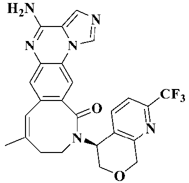 | 495 | **406** | 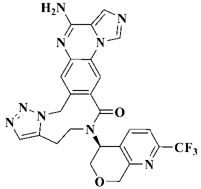 | 536 |
| **341** | 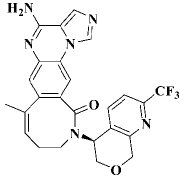 | 495 | **407** | 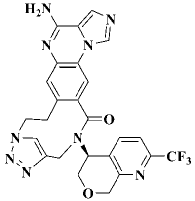 | 536 |
| **342** | 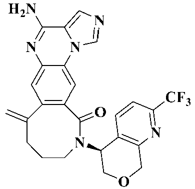 | 495 | **408** | 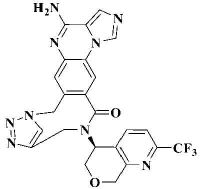 | 522 |
| **343** | 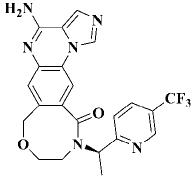 | 457 | **409** | 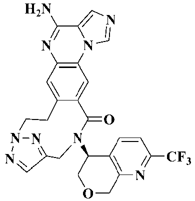 | 536 |
| **344** | 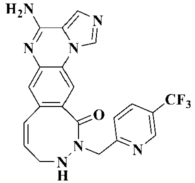 | 440 | **410** | 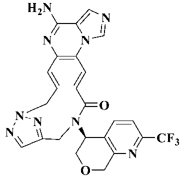 | 522 |
| **345** | 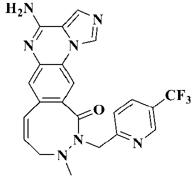 | 454 | **411** | 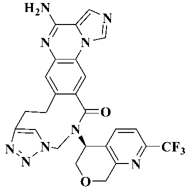 | 536 |
| **346** | 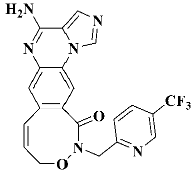 | 441 | **412** | 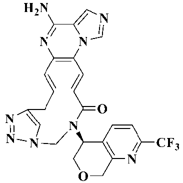 | 522 |
| **347** | 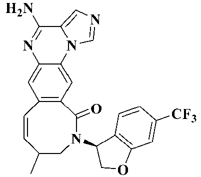 | 480 | **413** | 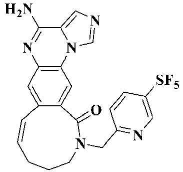 | 511 |
| **348** | 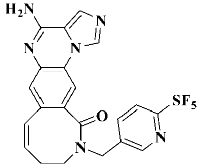 | 497 | **414** | 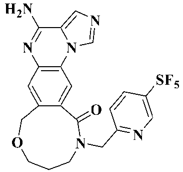 | 515 |
| **349** | 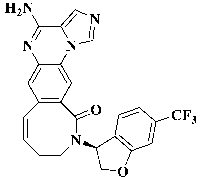 | 466 | | | |

Alternatively, the following examples were prepared using the following method:

### Example 250

### 4-Amino-11-(5-(trifluoromethyl)pyridin-2-yl)methyl-8,9,10,11-tetrahydroazocino[4,3-g]imidazo[1,5-a]quinoxalin-12(7H)-one

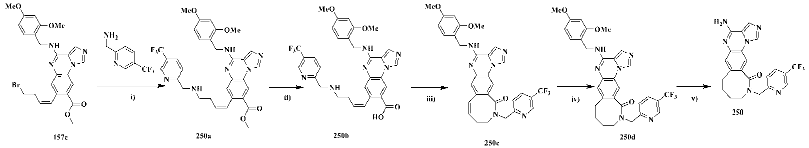

Referring to the synthesis method described in Step 4 to Step 9 of **Example 62, Example 250** (15 mg, yield: 7.4%) was obtained from methyl (Z)-7-(5-bromobut-1-en-1-yl)-4-((3,5-dimethoxybenzyl)amino)imidazo[1,5-a]quinoxaline-8-carboxylate **157c.** MS m/z (ESI): 441 [M+1]⁺

¹H NMR (400 MHz, DMSO-*d6*) δ 9.40 (s, 1H), 8.97 (d, 1H), 8.32 (s, 1H), 8.28 (s, 1H), 8.24 (dd, 1H), 7.66 (d, 1H), 7.42 (s, 1H), 5.17 (d, 1H), 4.65 (d, 1H), 3.24 (d, 2H), 2.95 (dd, 1H), 2.76 (t, 1H), 2.19 - 2.08 (m, 1H), 1.84 (d, 1H), 1.67 (d, 1H), 1.46 (d, 1H).

Alternatively, the following examples were synthesised by reference to the following preparation method:

### Example 268

### 4-Amino-12-(6-trifluoromethyl-2,3-dihydrobenzofuran-3-yl)-9,10,11,12-tetrahydroimidazo[1,5-a][1,5]oxazonino[8,7-g]quinoxalin-13(7H)-one

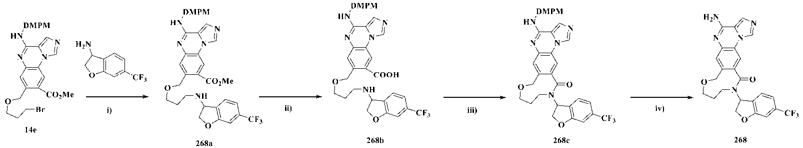

Referring to the synthesis method of Example **14, Example 268** (20 mg, yield: 7.5%) was obtained from methyl 7-((3-bromopropoxy)methyl)-4-((3,4-dimethoxybenzyl)amino)imidazo[1,5-a]quinoxaline-8-carboxylate **14e** (300 mg, 0.55 mmol) and 6-(trifluoromethyl)-2,3-dihydrobenzofuran-3-amine (168 mg, 0.83 mmol). MS m/z (ESI): 484 [M+1]⁺

¹H NMR (400 MHz, DMSO-*d6*) δ 8.39 (dd, 2H), 8.05 (s, 1H), 7.52 (d, 1H), 7.49 - 7.36 (m, 2H), 7.29 (dq, 1H), 5.15 (td, 1H), 4.84 (d, 2H), 4.57 (dd, 1H), 4.32 (m, 1H), 3.71 (m, 1H), 3.55 (m, 3H), 1.90 (m, 2H).

### Example 270

### 4-Amino-1-methyl-12-(6-(trifluoromethyl)-2,3-dihydrobenzofuran-3-yl)-1,7,9,10,11,12-hexahydro-13H-[1,5]oxazonino[7,8-g]pyrazolo[4,3-c]quinolin-13-one

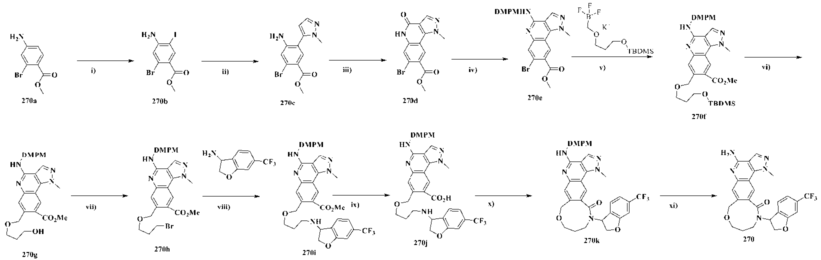

Step 1: Methyl 4-amino-2-bromo-carboxylate **270a** (10.00 g, 43.67 mmol) and N-iodosuccinimide (10.13 g, 45.00 mmol) were dispersed in acetonitrile (100 mL). The mixture was stirred and reacted at 25°C for 16 hours. The reaction solution was filtered to remove insoluble materials. The filtrate was concentrated under reduced pressure to dryness, and the resulting residue was purified by silica gel column chromatography using eluent system A to obtain **270b** (14.00 g, yield: 90.3%). MS m/z (ESI): 356 [M+1]⁺
Step 2: **270b** (9.00 g, 25.35 mmol), 1-methyl-1H-pyrazole-5-boronic acid pinacol ester (10.55 g, 50.70 mmol), [1,1'-bis(diphenylphosphino)ferrocene]palladium(II) dichloride (1.86 g, 2.54 mmol), and potassium phosphate (10.75 g, 50.70 mmol) were dispersed in 1,4-dioxane (150 mL) and water (30 mL). The mixture was stirred and reacted at 80°C for 16 hours. The reaction solution was filtered to remove insoluble materials. The filtrate was concentrated under reduced pressure to dryness, and the resulting residue was purified by silica gel column chromatography using eluent system A to obtain **270c** (6.50 g, yield: 83.0%). MS m/z (ESI): 310 [M+1]⁺
Step 3: **270c** (6.50 g, 21.04 mmol) and N,N-carbonyldiimidazole potassium phosphate (6.82 g, 42.08 mmol) were dispersed in N-methylpyrrolidone (65 mL). The mixture was stirred and reacted at 150°C for 1 hour. The reaction solution was poured into water (650 mL), and the precipitated solid was filtered to obtain **270d** (5.71 g, yield: 81.0%). MS m/z (ESI): 336 [M+1]⁺
Step 4: **270d** (5.71 g, 17.04 mmol), 2,4-dimethoxybenzylamine (5.69 g, 34.08 mmol), 1,8-diazabicyclo[5.4.0]undec-7-ene (5.18 g, 34.08 mmol) and benzotriazol-1-yloxytris(dimethylamino)phosphonium hexafluorophosphate (15.06 g, 34.08 mmol) were dispersed in dimethyl sulphoxide (50 mL). The mixture was stirred and reacted at 25°C for 1 hour. The reaction solution was poured into water (500 mL), and the precipitated solid was filtered to obtain **270e** (6.41 g, yield: 77.7%). MS m/z (ESI): 485 [M+1]⁺

Referring to the synthesis method of **Example 14, Example 270** (35 mg, yield: 6.8%) was obtained from **270e** (500 mg, 0.41 mmol). MS m/z (ESI): 498 [M+1]⁺
¹H NMR (400 MHz, DMSO-*d6*) δ 8.52 (s, 2H), 8.25 - 7.93 (m, 1H), 7.77 - 7.58 (m, 2H), 7.29 (dd, 2H), 6.06 - 5.79 (m, 1H), 4.99 - 4.77 (m, 3H), 4.54 - 4.44 (m, 4H), 3.82 (dd, 1H), 3.42 - 3.39 (m, 2H), 3.30 - 3.10 (m, 2H), 1.67 - 1.24 (m, 1H), 1.09 (dd, 1H).

### Example 298

### 4-Amino-12-((5-(2,6-difluorophenyl)pyridin-2-yl)methyl)-9,10,11,12-tetrahydroimidazo[1,5-a][1,5]oxazolo[8,7-g]quinoxalin-13(7H)-one

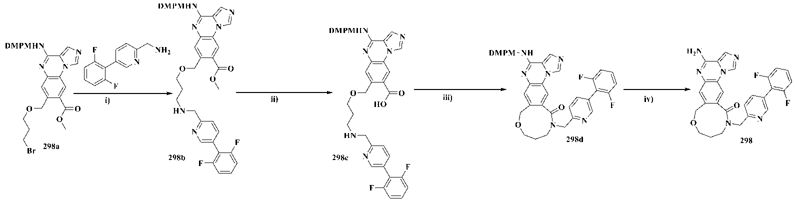

Referring to the synthesis of **Example 62, Example 298** (25 mg, yield: 13.1%) was obtained from methyl 7-((3-bromopropoxy)methyl)-4-(3,4-dimethoxybenzyl)amino)imidazo[1,5-a]quinoxaline-8-carboxylate **298a** (200 mg, 0.37 mmol). MS m/z (ESI): 501 [M+1]⁺
¹H NMR (400 MHz, MeOD) δ 9.32 (s, 1H), 8.68 (s, 1H), 8.37 (d, 2H), 8.06 (m, 1H), 7.82 (m, 1H), 7.57 (s, 1H), 7.54-7.46 (m, 1H), 7.19-7.15 (m, 2H), 5.42 (d, 1H), 4.90-4.86 (m, 1H), 4.59 (d, 1H), 4.49 (d, 1H), 3.97 (d, 1H), 3.58-3.39 (m, 3H), 2.23-2.02 (m, 2H).

### Example 311

### (Z)-4-((2,4-Dimethoxybenzyl)amino)-13-(2-(trifluoromethyl)-5,8-dihydro-6H-pyrano[3,4-b]pyridin-5-yl)-10,11,12,13-tetrahydroazocino[4,3-g]imidazo[1,5-a]quinoxalin-14(9H)-one

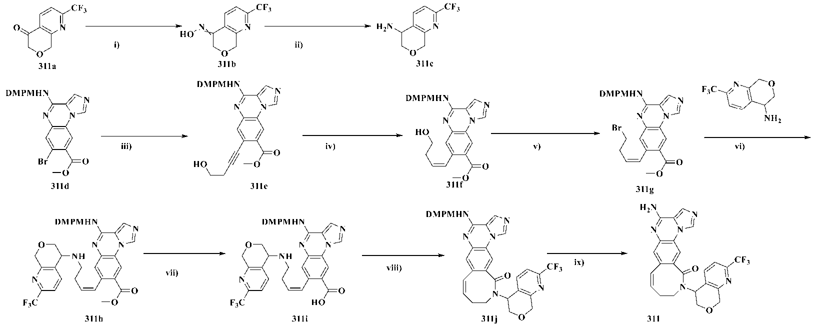

Step 1: 2-(Trifluoromethyl)-8H-pyrano[3,4-b]pyridin-5-one **311a** (3 g, 13.82 mmol) and hydroxylamine hydrochloride (960 mg, 13.82 mmol) were dissolved in ethanol (30 mL) and sodium acetate (3.40 g, 41.45 mmol), and the mixture was purged three times with nitrogen. The system was reacted at 80°C for 1 hour. The resulting reaction system was cooled to room temperature, concentrated, quenched with water, extracted with ethyl acetate (100 mL × 3), washed with a saturated sodium chloride aqueous solution, dried over anhydrous sodium sulphate, filtered, and concentrated. The residue was purified by silica gel column chromatography using eluent system B to obtain **311b** (3.10 g, yield: 96.7%). MS m/z (ESI): 233 [M+1]⁺
Step 2: **311b** (20.98 g, 90.37 mmol), zinc powder (59.08 g, 903.69 mmol) and ammonium chloride (48.34 g, 903.69 mmol) were dissolved in ethanol (40 mL), and the mixture was reacted at 50°C for 4 hours. The resulting reaction system was filtered, concentrated, and slurried with dichloromethane, to obtain **311c** (12.6 g, yield: 63.9%). MS m/z (ESI): 219 [M+1]⁺
Step 3: **311d** (20 g, 42.44 mmol) and cuprous iodide (1.62 g, 8.49 mmol) were dissolved in N,N-dimethylformamide (200 mL) and triethylamine (14.17 g, 140.04 mmol, 19.53 mL), and the mixture was purged three times with nitrogen. 3-Butyn-1-ol (14.87 g, 212.18 mmol) and bis(triphenylphosphine)palladium(II) dichloride (2.98 g, 4.24 mmol) were added, and the resulting mixture was purged three times with nitrogen. The system was reacted at 40°C for 16 hours. The resulting reaction system was cooled to room temperature, concentrated, quenched with water, extracted with ethyl acetate (60 mL × 3), washed with a saturated sodium chloride aqueous solution, dried over anhydrous sodium sulphate, filtered, and concentrated to obtain **311e** (19 g, crude). MS m/z (ESI): 461 [M+1]⁺
Step 4: **311e** (1.8 g, 3.91 mmol) and Raney nickel (4.59 g, 78.18 mmol) were dissolved in tetrahydrofuran (250 mL) and methanol (50 mL), and the mixture was purged three times with hydrogen, and reacted at 25°C under a hydrogen balloon for 2 hours. The resulting reaction system was filtered and concentrated to obtain **311f** (1.6 g, crude). MS m/z (ESI): 463 [M+1]⁺
Step 5: **311f** (4.8 g, 10.38 mmol) was dissolved in dichloromethane (50 mL), and the mixture was purged three times with nitrogen and cooled to 0°C. Triphenylphosphine (10.89 g, 41.51 mmol) and carbon tetrabromide (13.77 g, 41.51 mmol) were then added, and the system was reacted at 25°C for 2 hours. The resulting reaction system was concentrated. The residue was purified by silica gel column chromatography using eluent system B to obtain methyl (Z)-7-(4-bromobut-1-en-1-yl)-4-((2,4-dimethoxybenzyl)amino)imidazo[1,5-a]quinoxaline-8-carboxylate **311g** (3 g, yield: 55.2%). MS m/z (ESI): 525 [M+1]⁺
Step 6: **311g** (1.5 g, 2.86 mmol), 2-(trifluoromethyl)-5,8-dihydro-6H-pyrano[3,4-b]pyridin-5-amine (1.25 g, 5.71 mmol), and sodium iodide (855.87 mg, 5.71 mmol) were dissolved in acetonitrile (40 mL), ground potassium carbonate (1.18 g, 8.57 mmol) was added, and the mixture was purged three times with nitrogen. The reaction system was reacted at 80°C for 15 hours. The resulting reaction system was quenched with water, extracted with ethyl acetate (80 mL × 3), washed with a saturated sodium chloride aqueous solution, dried over anhydrous sodium sulphate, filtered, and concentrated. The residue was purified by silica gel column chromatography using eluent system A to obtain **311h** (252 mg, yield: 13.5%). MS m/z (ESI): 662 [M+1]⁺
Step 7: **311h** (252 mg, 380.86 µmol) was dissolved in tetrahydrofuran (2 mL), water (2 mL) and methanol (2 L), lithium hydroxide (63.92 mg, 1.52 mmol) was added, and the mixture was purged three times with nitrogen. The reaction system was reacted at 25°C for 16 hours. The resulting reaction system was adjusted to pH 5-6 with 1 M dilute hydrochloric acid, extracted with ethyl acetate (50 mL × 3), washed with a saturated sodium chloride aqueous solution (30 mL), dried over anhydrous sodium sulphate, filtered, and concentrated to obtain **311i** (242 mg, crude). MS m/z (ESI): 648 [M+1]⁺
Step 8: **311i** (240 mg, 370.57 µmol) and 2-(7-azobenzotriazol-1-yl)-N,N,N',N'-tetramethyluronium hexafluorophosphate (279.61 mg, 741.15 µmol) were dissolved in N,N-dimethylformamide (3 mL), N,N-diisopropylethylamine (287.36 mg, 2.22 mmol, 387.28 µL) was added, and the mixture was purged three times with nitrogen. The reaction system was reacted at 25°C for 16 hours. The resulting reaction system was quenched with water, extracted with ethyl acetate (80 mL × 3), washed with a saturated sodium chloride aqueous solution, dried over anhydrous sodium sulphate, filtered, and concentrated. The residue was purified by silica gel column chromatography using eluent system A to obtain **311j** (195 mg, yield: 84%). MS m/z (ESI): 630 [M+1]⁺
Step 9: **311j** (190 mg, 301.29 µmol) was dissolved in trifluoroacetic acid (5 mL), and the mixture was purged three times with nitrogen. The reaction system was reacted at 90°C for 0.5 hours. Upon completion of the reaction as monitored by LCMS, the reaction solution was subjected to rotary evaporation to dryness to obtain a crude product, which was purified by preparative liquid chromatography to obtain **Example 311** (76 mg, yield: 52.5%). MS m/z (ESI): 481 [M+1]⁺
¹H NMR (400 MHz, DMSO-*d6*) δ 9.18 (d, 1H), 8.22 (s, 1H), 8.15-8.05 (m, 1H), 8.01-7.89 (m, 1H), 7.84 (dd, 1H), 7.62 (s, 2H), 7.18 (d, 1H), 6.59 (ddd, 1H), 5.91 (m, 1H), 5.55 (d, 1H), 4.94-4.74 (m, 2H), 4.34-4.12 (m, 2H), 3.80-3.62 (m, 2H), 3.16 (d, 1H), 2.76 (td, 1H).

### Examples 311-P1 & 311-P2

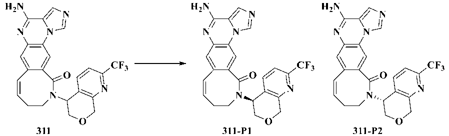

**Example 311** (76 mg, 0.16 mmol) was separated by chiral preparative HPLC to obtain rel-(S)-(Z)-4-((2,4-dimethoxybenzyl)amino)-13-(2-(trifluoromethyl)-5,8-dihydro-6H-pyrano[3,4-b]pyridin-5-yl)-10,11,12,13-tetrahydroazocino[4,3-g]imidazo[1,5-a]quinoxalin-14(9H)-one **311-P1** (35 mg, yield: 46.1%) and rel-(R)-(Z)-4-((2,4-dimethoxybenzyl)amino)-13-(2-(trifluoromethyl)-5,8-dihydro-6H-pyrano[3,4-b]pyridin-5-yl)-10,11,12,13-tetrahydroazocino[4,3-g]imidazo[1,5-a]quinoxalin-14(9H)-one **311-P2** (36 mg, yield: 47.3%).

**311-P1 (t_{R}: 5.346 min)**: ¹H NMR (400 MHz, DMSO-*d6*) δ 9.40 (d, 1H), 9.21 (br s, 2H), 8.38 (s, 1H), 8.30 (d, 1H), 8.14-7.75 (m, 2H), 7.29 (d, 1H), 6.62 (dd, 1H), 6.01 (m, 1H), 5.56 (s, 1H), 4.85 (qd, 2H), 4.26 (m, 2H), 3.77 (m, 1H), 2.80 (m, 1H), 2.50-1.78 (m, 2H).

**311-P2 (t_{R}: 4.447 min):** ¹H NMR (400 MHz, DMSO-*d6*) δ 9.40 (d, 1H), 9.21 (br s, 2H), 8.38 (s, 1H), 8.30 (d, 1H), 8.14-7.75 (m, 2H), 7.29 (d, 1H), 6.62 (dd, 1H), 6.01 (m, 1H), 5.56 (s, 1H), 4.85 (qd, 2H), 4.26 (m, 2H), 3.77 (m, 1H), 2.80 (m, 1H), 2.50-1.78 (m, 2H).

### Example 313

### 4-Amino-11-(1-(5-(trifluoromethyl)pyridin-2-yl)ethyl)-10,11-dihydroazocino[4,3-g]imidazo[1,5-a]quinoxalin-12(9H)-one

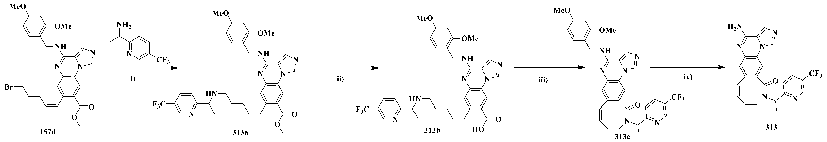

Referring to the synthesis of **Example 62, Example 313** (102 mg, yield: 25.1%) was obtained from methyl Z-7-(6-bromopent-1-en-1-yl)-4-((2,4-dimethoxybenzyl)amino)imidazo[1,5-a]quinoxaline-8-carboxylate **157d** (500 mg, 0.55 mmol). MS m/z (ESI): 453 [M+1]⁺

### Examples 313-P1 & 313-P2

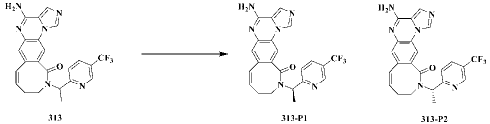

**Example 313** (102 mg, 0.23 mmol) was separated by chiral preparative HPLC to obtain rel-(R,Z)-4-amino-11-(1-(5-(trifluoromethyl)pyridin-2-yl)ethyl)-10,11-dihydroazocino[4,3-g]imidazo[1,5-a]quinoxalin-12(9H)-one **313-P1** (38 mg, yield: 37.2%) and rel-(S,Z)-4-amino-11-(1-(5-(trifluoromethyl)pyridin-2-yl)ethyl)-10,11-dihydroazocino[4,3-g]imidazo[1,5-a]quinoxalin-12(9H)-one **313-P2** (42 mg, yield: 41.2%).

**313-P1 (t_{R}: 1.100 min):** ¹H NMR (400 MHz, DMSO-d6) δ 9.17 (s, 1H), 8.97 (s, 1H), 8.21 (d, 1H), 8.10 (s, 1H), 7.89 (s, 1H), 7.69 (dd, 1H), 7.44 (s, 2H), 7.15 (d, 1H), 6.57 (dd, 1H), 5.90 - 5.47 (m, 2H), 3.78 (dt, 1H), 3.56 - 3.38 (m, 1H), 2.47 - 2.21 (m, 2H), 1.73 (dd, 3H).

**313-P2 (t_{R}: 1.490 min):** ¹H NMR (400 MHz, DMSO-d6) δ 8.97 (s, 1H), 8.21 (d, 1H), 8.10 (s, 1H), 7.89 (s, 1H), 7.69 (dd, 1H), 7.43 (s, 2H), 7.15 (d, 1H), 6.57 (dd, 1H), 5.94 - 5.47 (m, 2H), 3.91 - 3.44 (m, 2H), 2.45 - 1.98 (m, 2H), 1.73 (t, 3H).

### Example 325

### (Z)-4-Amino-11-(6-(trifluoromethyl)-2,3-dihydrobenzofuran-3-yl)-10,11-dihydroazo[4,3-g]imidazo[1,5-a]quinoxalin-12(9H)-one

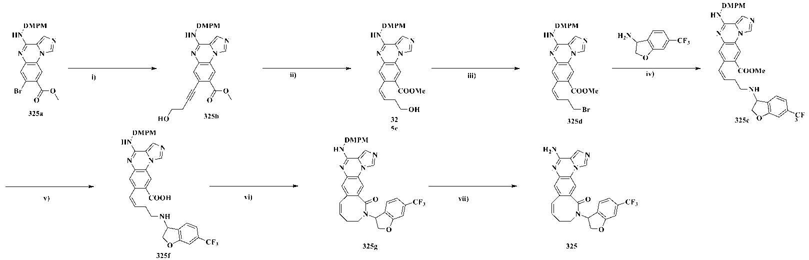

Step 1: Methyl 7-bromo-4-((2,4-dimethoxybenzyl)amino)imidazo[1,5-a]quinoxaline-8-carboxylate **325a** (20 g, 42.44 mmol) and cuprous iodide (1.62 g, 8.49 mmol) were dissolved in N,N-dimethylformamide (200 mL) and triethylamine (14.17 g, 140.04 mmol, 19.53 mL), and the mixture was purged three times with nitrogen. 3-Butyn-1-ol (14.87 g, 212.18 mmol) and bis(triphenylphosphine)palladium(II) dichloride (2.98 g, 4.24 mmol) were added, and the resulting mixture was purged three times with nitrogen. The system was reacted at 40°C for 16 hours. The resulting reaction system was cooled to room temperature, concentrated, quenched with water, extracted with ethyl acetate (60 mL × 3), washed with a saturated sodium chloride aqueous solution, dried over anhydrous sodium sulphate, filtered, and concentrated to obtain **325b** (19 g, crude). MS m/z (ESI): 461 [M+1]⁺
Step 2: **325b** (2.2 g, 4.78 mmol) was dissolved in tetrahydrofuran (100 mL), and methanol (100 mL) and Raney Ni (4.4 g) were added. The mixture was purged three times with hydrogen, and reacted at room temperature for 3 h. The reaction solution was filtered to remove insoluble materials. The filtrate was concentrated, and the residue was purified by silica gel column chromatography using eluent system B to obtain **325c** (1.7 g, yield: 76.9%). MS m/z (ESI): 463 [M+1]⁺
Step 3: **325c** (1.6 g, 3.46 mmol) was dissolved in dichloromethane (20 mL), triphenylphosphine (2.27 g, 8.65 mmol) was added, and the mixture was purged three times with nitrogen. Carbon tetrabromide (2.87 g, 8.65 mmol) was added under nitrogen atmosphere, and the resulting mixture was reacted at room temperature for 16 h. The reaction solution was concentrated to remove the solvent, and the residue was purified by silica gel column chromatography using eluent system B to obtain **325d** (1.6 g, yield: 87.9%). MS m/z (ESI): 526 [M+1]⁺
Step 4: **325d** (1.6 g, 2.86mmol) was dissolved in tetrahydrofuran (20 mL), N, N-diisopropylethylamine (738 mg, 5.71 mmol) and 6-(trifluoromethyl)-2,3-dihydrobenzofuran-3-amine (1.16 g, 5.71 mmol) were added, and the mixture was heated to 65°C and reacted for 48 h. The reaction solution was concentrated to remove the solvent, and the residue was purified by silica gel column chromatography using eluent B to obtain **325e** (1.1 g, yield: 59.5%). MS m/z (ESI): 648.5 [M+1]⁺
Step 5: **325e** (1.1 g, 1.70 mmol) was dissolved in tetrahydrofuran (10 mL), water (2 mL) and lithium hydroxide monohydrate (163 mg, 6.79 mmol) were added, and the mixture was reacted at room temperature for 16 h. The reaction solution was concentrated to remove the solvent, and the residual solution was adjusted to pH 5-6 with 1N HCl, extracted three times with ethyl acetate, and concentrated. The residue was purified by silica gel column chromatography using eluent B to obtain **325f** (1.0 g, yield: 92.9%). MS m/z (ESI): 634 [M+1]⁺
Step 6: **325f** (105 mg, 0.165 mmol) was dissolved in N, N-dimethylformamide (5 mL), N, N-diisopropylethylamine (107 mg, 0.828 mmol) and 2-(7-azabenzotriazol-1-yl)-N, N, N', N'-tetramethyluronium hexafluorophosphate (126 mg, 331 mmol) were added, and the mixture was reacted at room temperature for 16 h. The reaction solution was slowly added to water for quenching, extracted three times with ethyl acetate, and concentrated to obtain **325g** (100 mg, yield: 98.0%). MS m/z (ESI): 616 [M+1]⁺
Step 7: **325g** (100 mg, 0.162 mmol) was dissolved in trifluoroacetic acid (5 mL), and the mixture was heated to 80°C and reacted for 0.5 h. The reaction solution was concentrated, and the residue was purified by chromatography using eluent C to obtain **Example 325** (38 mg, yield: 50.2%). MS m/z (ESI): 466 [M+1]⁺
¹H NMR (400 MHz, DMSO-d6) δ 9.44 (d, 3H), 8.58-8.16 (m, 2H), 7.58 (d, 0.5H), 7.45 (d, 0.5H), 7.36-7.21 (m, 2H), 6.60 (t, 1H), 6.09 (d, 0.5H), 6.02-5.89 (m, 1H), 5.68 (dd, 0.5H), 4.88 (dt, 1H), 4.65 (m, 1H), 3.70 (d, 1H), 3.41 (dd, 0.5H), 3.12 (d,0.5H), 2.43-2.21 (m,1H).

### Example 331

### 4-Amino-10-((5-(trifluoromethyl)pyridin-2-yl)methyl)-9,10-dihydro-11H-azecino[4,3-g]imidazolo[1,5-a]quinoxalin-11-one

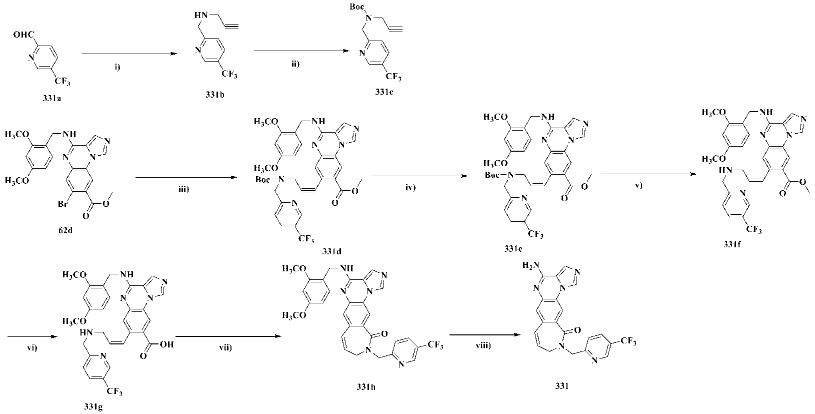

Step 1: 5-(Trifluoromethyl)pyridine-2-carbaldehyde **331a** (1.5 g, 8.57 mmol), 2-propyn-1-amine (471.81 mg, 8.57 mmol), sodium cyanoborohydride (1.61 g, 25.70 mmol), and acetic acid (102.88 mg, 1.71 mmol) were dissolved in methanol (20 mL), and the mixture was reacted at 20°C for 1 hour. The resulting reaction system was cooled to room temperature, concentrated, quenched with water, extracted with ethyl acetate (80 mL × 3), washed with a saturated sodium chloride aqueous solution, dried over anhydrous sodium sulphate, filtered, and concentrated, to obtain crude **331b** (1.69 g), which was used directly in the next step without purification. MS m/z (ESI): 215 [M+1]⁺
Step 2: **331b** (1.69 g, 7.89 mmol), di-tert-butyl dicarbonate (3.44 g, 15.78 mmol), 4-dimethylaminopyridine (192.79 mg, 1.58 mmol), and triethylamine (2.40 g, 23.67 mmol) were dissolved in dichloromethane (20 mL), and the mixture was concentrated. The residue was purified by silica gel column chromatography using eluent system B to obtain **331c** (1.05 g, yield: 42.2%). MS m/z (ESI): 315 [M+1]⁺
Step 3: **62d** (320 mg, 0.68 mmol), N-((5-(trifluoromethyl)pyridin-2-yl)methyl)prop-2-yn-1-yl tert-butyloxycarbonyl carbonate (640.20 mg, 2.04 mmol), dichlorobis(tricyclohexylphosphine)palladium(II) (50.12 mg, 67.90 µmol), 2-dicyclohexylphosphino-2',4',6'-triisopropylbiphenyl (64.73 mg, 135.79 µmol), and caesium carbonate (663.66 mg, 2.04 mmol) were dissolved in dioxane (6 mL), and the system was reacted at 100°C for 3 hours. The resulting reaction system was cooled to room temperature, concentrated, quenched with water, extracted with ethyl acetate (60 mL × 3), washed with a saturated sodium chloride aqueous solution, dried over anhydrous sodium sulphate, filtered, and concentrated to obtain **331d** (180 mg, yield: 37.5%). MS m/z (ESI): 705 [M+1]⁺
Step 4: **331d** (100 mg, 0.14 mmol) and Raney nickel (100 mg) were dissolved in methanol (10 mL) and tetrahydrofuran, and the mixture was purged three times with hydrogen, and reacted at 25°C under a hydrogen balloon for 0.5 hours. The resulting reaction system was filtered and concentrated to obtain **331e** (0.1 g, crude). MS m/z (ESI): 707 [M+1]⁺
Step 5: **331e** (0.1 g, 0.14 mmol) was dissolved in dichloromethane (2 mL) and trifluoroacetic acid (0.4 mL), and the system was reacted at 25°C for 0.5 hours, and concentrated to obtain **331f** (100 mg, crude). MS m/z (ESI): 607 [M+1]⁺
Step 6: **331f** (crude, 100 mg) and LiOH (10 mg, 0.42 mmol) were dissolved in methanol (2 mL), tetrahydrofuran (2 mL) and water (2 mL), and the reaction system was reacted at 50°C for 2 hours. The resulting reaction system was concentrated. The residue was purified by reversed-phase column chromatography using eluent system A to obtain **331g** (22 mg, yield: 26.5%). MS m/z (ESI): 593 [M+1]⁺
Step 7: **331g** (22 mg, 0.037 mmol) and 2-(7-azobenzotriazol-1-yl)-N,N,N',N'-tetramethyluronium hexafluorophosphate (28 mg, 0.074 mmol) were dissolved in N,N-dimethylformamide (3 mL), N,N-diisopropylethylamine (14.3 mg, 8.76 mmol, 19 µL) was added, and the mixture was reacted at 25°C for 16 hours. The system was concentrated to dryness, and the residue was purified by silica gel column chromatography using eluent system C to obtain **331h** (18 mg, yield: 84.6%). MS m/z (ESI): 575 [M+1]⁺
Step 8: **331h** (18 mg, 0.031 mmol) was dissolved in trifluoroacetic acid (2 mL), and the mixture was purged three times with nitrogen. The reaction system was reacted at 90°C for 0.5 hours. The resulting reaction system was concentrated, and the residue was purified by silica gel column chromatography using eluent system A to obtain **Example 331** (4.2 mg, yield: 31.9%). MS m/z (ESI): 425 [M+1]⁺
¹H NMR (400 MHz, DMSO-*d6*) δ 9.24 (s, 1H), 8.96 - 8.94 (m, 1H), 8.62 (s, 1H), 8.22 (m, 1H), 7.93 (s, 1H), 7.59 - 7.54 (m, 3H), 7.37 (s, 1H), 6.97 (m, 1H), 6.41 (m, 1H), 4.96 (s, 2H), 3.85 (m, 2H).

### Example 352

### (Z)-4-Amino-12-(1-(5-(trifluoromethyl)pyridin-2-yl)ethyl)-9,10,11,12-tetrahydro-13H-azo[4,3-g]imidazo[1,5-a]quinoxalin-13-one

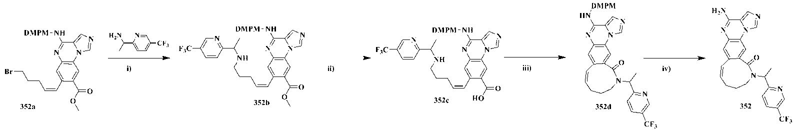

Referring to the synthesis method described in Step 6 to Step 9 of **Example 62, Example 352** (32 mg, yield: 7.4%) was obtained from methyl (*Z*)-7-(5-bromopent-1-en-1-yl)-4-(3,5-dimethoxybenzyl)amino)imidazo[1,5-a]quinoxaline-8-carboxylate **352a** (500 mg, 0.93 mmol). MS m/z (ESI): 467 [M+1]⁺
¹H NMR (400 MHz, MeOD) δ 9.11-9.06 (m, 1H), 8.91-8.87 (m, 1H), 8.13 (dd, 1H), 8.07 (d, 1H), 8.01 (s, 1H), 7.79 (d, 1H), 7.30 (d, 1H), 6.56 (dd, 1H), 6.09 (dtd, 1H), 5.42 (dq, 1H), 3.76-3.68 (m, 1H), 3.62-3.49 (m, 1H), 2.16 (ddd, 1H), 1.86 (dd, 4H), 1.49 (dt, 2H).

### Example 362

### (Z)-4-Amino-9-methyl-12-(2-(trifluoromethyl)-5,8-dihydro-6H-pyrano[3,4-b]pyridin-5-yl)-9,10,11,12-tetrahydro-13H-azo[4,3-g]imidazo[1,5-a]quinoxalin-13-one

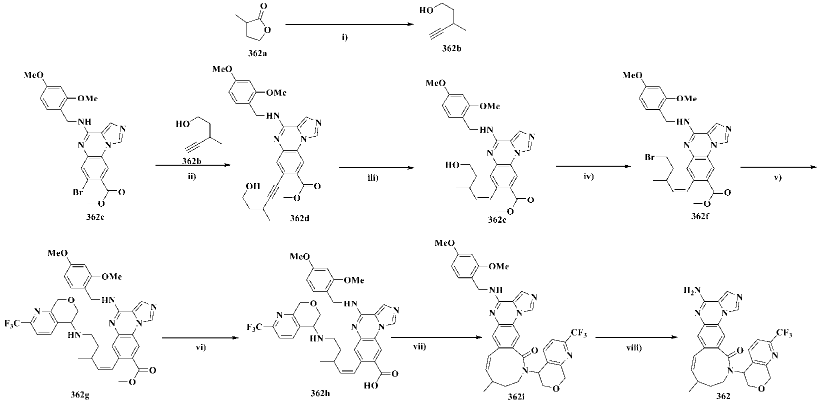

Step 1: 3-Methyltetrahydrofuran-2-one **362a** (7 g, 69.92 mmol) was dissolved in dichloromethane (70 mL), and the mixture was purged three times with nitrogen. Diisobutylaluminium hydride (12.84 g, 90.90 mmol, 16.08 mL) was added dropwise at -78°C, and the reaction solution was stirred at -78°C for 1 hour. The reaction solution was added to a sodium tartrate solution, and the mixture was stirred for 2 hours. Water (500 ml) was added for phase separation, and the aqueous phase was extracted with tert-butyl methyl ether (500 ml). The organic phase was dried over anhydrous sodium sulphate, and concentrated at 20°C, and the intermediate was used directly in the next step. The intermediate was dissolved in methanol (70 mL), and dimethyl (1-diazo-2-oxopropyl)phosphonate (14.78 g, 76.91 mmol) and potassium carbonate (19.33 g, 139.84 mmol) were added at room temperature. The mixture was purged three times with nitrogen, and stirred overnight at room temperature. The reaction solution was filtered, the filtrate was mixed with silica gel powder, and the mixture was concentrated at 20°C for sample loading. The residue was subjected to column chromatography (PE : EA=62 : 38) to obtain **362b** (3.8 g, 38.72 mmol, yield: 55.4%).

Step 2: Methyl 7-bromo-4-[(2,4-dimethoxyphenyl)methylamino]imidazo[1,5-a]quinoxaline-8-carboxylate **362c** (10 g, 21.22 mmol) and **362b** (3.8 g, 38.72 mmol) were dissolved in DMF (100 mL), and bis(triphenylphosphine)palladium(II) dichloride (1.49 g, 2.12 mmol), cuprous iodide (808.18 mg, 4.24 mmol) and triethylamine (7.09 g, 70.02 mmol, 9.77 mL) were sequentially added at room temperature. The reaction solution was stirred overnight at 40°C. LCMS showed consumption of the starting material and formation of the product. Water (1 L) was added to the reaction solution, and the mixture was extracted three times with EA (300 mL). The organic phase was dried over anhydrous sodium sulphate, and concentrated, and the crude product was subjected to column chromatography (DCM : MeOH = 94 : 6) to obtain **362d** (10 g, 20.47 mmol, yield: 96.5%). MS m/z (ESI): 489 [M+1]⁺
Step 3: **362d** (10 g, 20.47 mmol) was dissolved in tetrahydrofuran (50 mL) and methanol (50 mL), Raney nickel (1.27 g, 21.57 mmol) was added at room temperature, and the mixture was purged three times with hydrogen. The reaction solution was stirred overnight at room temperature. LCMS showed consumption of the starting material and formation of the product. The reaction solution was directly filtered by suction, and the filtrate was concentrated to obtain crude **362e,** which was used directly in the next step. MS m/z (ESI): 491 [M+1]⁺
Step 4: **362e** was dissolved in dichloromethane (10 mL), carbon tetrabromide (2.89 g, 8.72 mmol) and triphenylphosphine (2.29 g, 8.72 mmol) were sequentially added at room temperature, and the reaction solution was stirred for 2 hours. LCMS showed consumption of the starting material and formation of the product. Water (100 ml) was added to the reaction solution, and the mixture was extracted three times with ethyl acetate (30 ml). The organic phase was dried over anhydrous sodium sulphate, and concentrated, and the crude product was subjected to column chromatography (DCM : MeOH = 94 : 6) to obtain **362f** (1.1 g, 2.11 mmol, two-step yield: 96.7%). MS m/z (ESI): 553 [M+1]⁺
Step 5: **362f** (3 g, 5.75 mmol) was dissolved in acetonitrile (10 mL), potassium carbonate (2.39 g, 17.26 mmol) and sodium iodide (862.37 mg, 5.75 mmol) were added at room temperature, and the mixture was purged three times with nitrogen. The reaction solution was stirred overnight at 80°C. LCMS showed consumption of the starting material and formation of the product. Water (100 ml) was added to the reaction solution, and the mixture was extracted three times with ethyl acetate (30 ml). The organic phase was dried over anhydrous sodium sulphate, and concentrated, and the crude product was subjected to column chromatography (DCM : MeOH = 95 : 5) to obtain **362g** (2 g, 3.03 mmol, yield: 52.8%). MS m/z (ESI): 691 [M+1]⁺
Step 6: **362g** (500 mg, 759.06 µmol) was dissolved in water (2 mL), tetrahydrofuran (2 mL) and methanol (2 mL), and lithium hydroxide (18.18 mg, 759.06 µmol) was added at room temperature. The reaction solution was stirred overnight at room temperature. LCMS showed consumption of the starting material and formation of the product. The reaction solution was adjusted to pH 5-6 with dilute hydrochloric acid, and extracted with ethyl acetate. The organic phase was dried and concentrated to obtain crude **362h** (460 mg), which was used directly in the next step. MS m/z (ESI): 677 [M+1]⁺
Step 7: **362h** (600 mg, 930.69 µmol) was dissolved in N,N-dimethylformamide (10 mL), (7-azobenzotriazol-1-yl)-N,N,N',N'-tetramethyluronium hexafluorophosphate (702.24 mg, 1.86 mmol) was added at room temperature, and the mixture was purged three times with nitrogen. The reaction solution was stirred at room temperature for 24 hours. LCMS showed consumption of the starting material and formation of the product. Water (200 ml) was added to the reaction solution, and the mixture was extracted three times with ethyl acetate (100 ml). The organic phase was washed with a sodium chloride aqueous solution, dried over anhydrous sodium sulphate, and concentrated, and the crude product was subjected to column chromatography (DCM : MeOH = 95 : 5) to obtain **362i** (320 mg, 510.64 µmol, yield: 54.9%). MS m/z (ESI): 659 [M+1]⁺
Step 8: **362i** (300 mg, 478.72 µmol) was dissolved in trifluoroacetic acid (8 mL), and the mixture was purged three times with nitrogen. The reaction solution was stirred at 90°C for 0.5 hours. LCMS showed consumption of the starting material and formation of the product. The reaction solution was added to ice water, adjusted to weakly alkaline pH with sodium bicarbonate, and extracted with ethyl acetate, and the organic phase was concentrated. The crude product was subjected to basic preparative chromatography to obtain **Example 362** (30 mg, yield: 12.3%). MS m/z (ESI): 509 [M+1]⁺
¹H NMR (400 MHz, DMSO-*d6*) δ 9.17 (s, 1H), 8.13 (m, 2H), 8.04 (br s, 1H), 7.85 (t, 2H), 7.18 (m, 2H), 6.48 (dd, 1H), 5.53 (m, 1H), 4.82 (m, 2H), 4.22 (m, 2H), 3.77 (m, 1H), 3.55 (m, 2H), 1.23 (m, 3H), 0.97 (d, 1H), 0.85 (d, 2H).

### Example 374

### 4-Amino-12-(1-(5-(trifluoromethyl)pyridin-2-yl)ethyl)-9,10,11,12-tetrahydroimidazo[1,5-a][1,5]oxazonino[8,7-g]quinoxalin-13(7H)-one

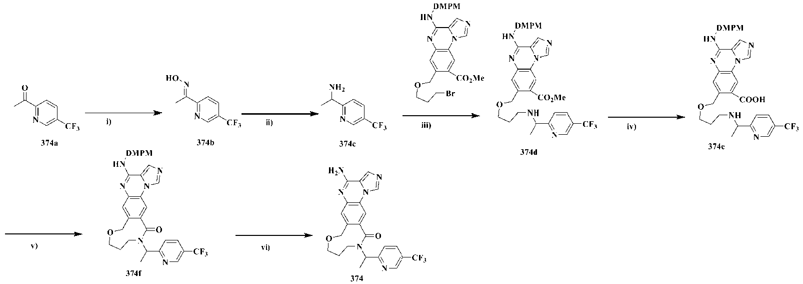

Step 1: 2-Acetyl-5-(trifluoromethyl)pyridine **374a** (5 g, 26.44 mmol) was dissolved in ethanol (100 mL), hydroxylamine hydrochloride (3.67 g, 52.87 mmol) and sodium acetate (8.67 g, 405.74 mmol) were added, and the mixture was stirred and reacted at 80°C for about 3 hours. Water (200 mL) was added to the reaction solution, and the mixture was extracted with ethyl acetate (200 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **374b** (4.8 g, yield: 88.9%). MS m/z (ESI): 205 [M+1]⁺
Step 2: **374b** (4.7 g, 23.02 mmol) was dissolved in trifluoroacetic acid (60 mL), zinc powder (7.5 g, 115.11 mmol) was added, and the mixture was heated and reacted at 80°C for 3 hours. The reaction solution was filtered, distilled under reduced pressure, adjusted to alkaline pH with a saturated sodium hydroxide solution, and extracted with dichloromethane (2 ×100 ml). After extraction, the organic phase was separated, dried over anhydrous sodium sulphate, and filtered. The filtrate was concentrated under reduced pressure, and the resulting residue was purified by silica gel column chromatography using eluent system C to obtain 374c (1.9 g, yield: 43.4%). MS m/z (ESI): 595 [M+1]⁺
Referring to the method described in Step 5 to Step 8 of Example **14, Example 374** (2 mg, yield: 1.2%) was obtained from **374c** (70 mg, 0.36 mmol). MS m/z (ESI): 471 [M+1]⁺
¹H NMR (400 MHz, DMSO-d6) δ 9.37 (s, 1H), 8.96 - 8.92 (m, 1H), 8.23 (d, 2H), 8.17 (d, 1H), 7.94 (dd, 1H), 7.48 (d, 1H), 5.55 (d, 1H), 4.75 (dd, 1H), 4.52 - 4.46 (m,1H), 3.85 - 3.69 (m, 1H), 3.22 - 3.06 (m,4H), 1.87 (d, 2H), 1.77 (d, 2H).

### Example 391

### (Z)-4-Amino-12-(6-(trifluoromethyl)pyridazin-3-yl)methyl-9,10,11,12-tetrahydro-13H-azonino[4,3-g]imidazo[1,5-a]quinoxalin-13-one

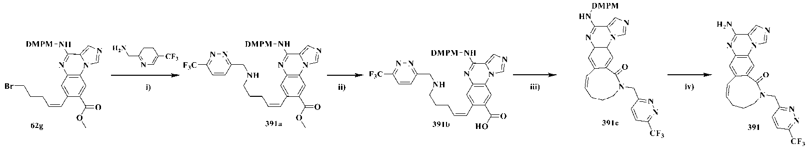

Referring to the synthesis method described in Step 6 to Step 9 of Example 62, Example 391 (9 mg, yield: 2.5%) was obtained from methyl *(Z)-*7-(6-bromopent-1-en-1-yl)-4-(2,4-dimethoxybenzylamino)imidazo[1,5-a]quinoxaline-8-carboxylate 62g (422 mg, 0.78 mmol). MS m/z (ESI): 454 [M+1]⁺
¹H NMR (400 MHz, DMSO-*d*₆) δ 9.22 (s, 1H), 8.31 (d, 1H), 8.25 (s, 1H), 8.10 (s, 1H), 8.05 (d, 1H), 7.26 (s, 1H), 6.53 (d, 1H), 5.98 (td, 1H), 5.15 (d, 1H), 4.87 (d, 1H), 3.48 (m, 2H), 2.13 (dt, 1H), 1.75 (m, 2H), 1.43 (d, 1H).

### Example 414

### 4-Amino-12-((5-(pentafluoro-λ⁶-sulphanyl)pyridin-2-yl)methyl)-9,10,11,12-tetrahydroimidazo[1,5-a][1,5]oxazonino[8,7-g]quinoxalin-13(7H)-one

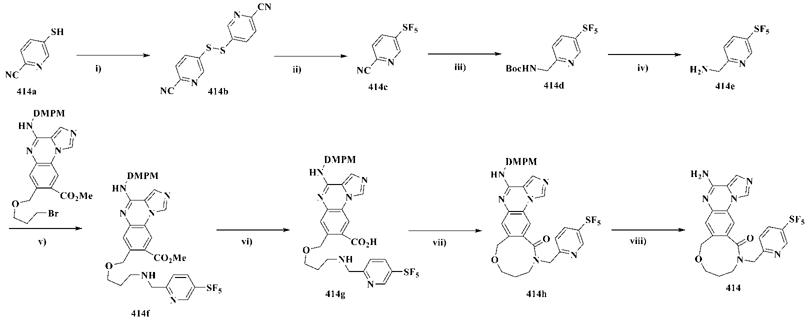

Step 1: 5-Sulphanyl-2-cyanopyridine **414a** (10.00 g, 73.53 mmol) and sodium iodide (4.47 g, 30.00 mmol) were dispersed in acetonitrile (200 mL), ferric chloride (4.83 g, 30.00 mmol) was added at 25°C, and the mixture was stirred and reacted at 25°C for 16 hours. The reaction solution was diluted with dichloromethane (200 mL), and filtered to remove insoluble materials. The filtrate was concentrated under reduced pressure to dryness, and the resulting residue was purified by silica gel column chromatography using eluent system A to obtain **414b** (6.15 g, yield: 62.0%). MS m/z (ESI): 271 [M+1]⁺
Step 2: **414b** (6.15 g, 22.78 mmol) and tetraethylammonium chloride (7.52 g, 45.56 mmol) were dispersed in acetonitrile (150 mL), and silver difluoride (52.85 g, 364.48 mmol) was added. The mixture was stirred and reacted at 25°C for 16 hours. The reaction solution was filtered to remove insoluble materials. The filtrate was concentrated under reduced pressure to dryness, and the resulting residue was purified by silica gel column chromatography using eluent system A to obtain **414c** (1.57 g, yield: 15.0%). MS m/z (ESI): 231 [M+1]⁺
Step 3: **414c** (1.57 g, 6.83 mmol), di-tert-butyl dicarbonate (2.83 g, 13.00 mmol) and cobalt chloride (1.07 g, 8.20 mmol) were dispersed in methanol (30 mL), sodium borohydride (1.82 g, 47.81 mmol) was added in portions at 0°C, and the mixture was stirred and reacted at 25°C for 1 hour. Water (2 mL) and dichloromethane (50 mL) were added to the reaction solution, and the mixture was filtered to remove insoluble materials. The filtrate was concentrated under reduced pressure to dryness, and the resulting residue was purified by silica gel column chromatography using eluent system A to obtain **414d** (1.65 g, yield: 72.5%). MS m/z (ESI): 335 [M+1]⁺
Step 4: **414d** (1.65 g, 4.94 mmol) was dispersed in hydrochloric acid (20 mL, 4 M, 1,4-dioxane solution). The mixture was stirred and reacted at 25°C for 2 hours. The reaction solution was concentrated under reduced pressure to dryness to obtain **414e** (1.10 g, yield: 95.5%). MS m/z (ESI): 235 [M+1]⁺
Referring to the method described in Step 5 to Step 8 of **Example 14, Example 414** (29 mg, yield: 3.8%) was obtained from **414e** (350 mg, 1.50 mmol). MS m/z (ESI): 515 [M+1]⁺
¹H NMR (400 MHz, DMSO-d6) δ 9.22 (s, 1H), 9.13 (s, 1H), 8.39 (dd, 1H), 8.10 (s, 1H), 7.99 - 7.38 (m, 5H), 5.18 (d, 1H), 4.71 - 4.40 (m, 3H), 3.80 (d, 1H), 3.48 (dd, 3H), 1.92 (d, 1H), 1.26 (d, 1H).

### Example 415

### (Z)-4-Amino-12-(1-(6-(trifluoromethyl)pyridin-3-yl)ethyl)-9,10,11,12-tetrahydro-13H-azo[4,3-g]imidazo[1,5-a]quinoxalin-13-one

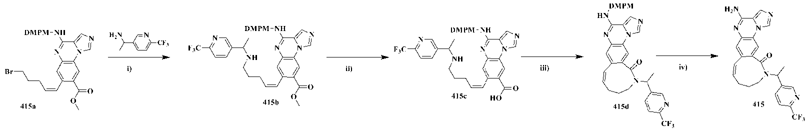

Referring to the synthesis method described in Step 6 to Step 9 of **Example 62, Example 415** (28 mg, yield: 6.5%) was obtained from methyl (*Z*)-7-(5-bromopent-1-en-1-yl)-4-(3,5-dimethoxybenzyl)amino)imidazo[1,5-a]quinoxaline-8-carboxylate **415a** (500 mg, 0.93 mmol). MS m/z (ESI): 467 [M+1]⁺
¹H NMR (400 MHz, DMSO-d6) δ 9.24 (d, 1H), 8.86 (s, 1H), 8.21-8.12 (m, 2H), 8.09-7.89 (m, 3H), 7.21 (s, 1H), 6.54 (t, 1H), 6.18-5.92 (m, 1H), 5.37 (dd, 1H), 3.55 (d, 2H), 2.14 (t, 1H), 2.03 (d, 1H), 1.77 (dd, 4H), 1.48 (s, 1H), 1.34 (s, 1H).

### Example 416

### (Z)-4-Amino-11-(5-(trifluoromethyl)pyridin-2-yl)methyl-10,11-dihydroazocino[4,3-g]imidazo[1,5-a]quinoxalin-12(9H)-one

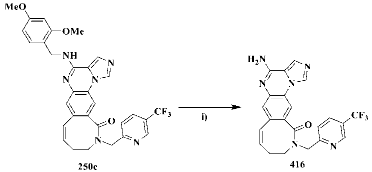

Referring to the method described in Step 9 of **Example 62, Example 416** (28 mg, yield: 63.8%) was obtained from (Z)-4-(2,4-dimethoxybenzylamino)-1-(5-(trifluoromethyl)pyridin-2-yl)methyl-10,11-dihydroazocino[4,3-*g*]imidazo[1,5-*a*]quinoxalin-12(9*H*)-one **250c** (60 mg, 0.10 mmol). MS m/z (ESI): 439 [M+1]⁺
¹H NMR (400 MHz, DMSO-d6) δ 9.31 (s, 1H), 8.96 (d, 1H), 8.27 (s, 1H), 8.26 (broad s, 2H), 8.24 (dd, 1H), 8.11 (s, 1H), 7.64 (d, 1H), 7.26 (s, 1H), 6.59 (d, 1H), 5.86 - 5.77 (m, 1H), 5.11 (d, 1H), 4.65 (d, 1H), 3.87 (t, 1H), 2.70 (td, 1H), 2.38 (dt, 1H)

### Example 417

### 4-Amino-13-((5-(trifluoromethyl)pyridin-2-yl)methyl)-10,11,12,13-tetrahydro-7H-imidazo [1,5-a][1,6] oxazino [3,4-g] quinoxalin-14(9H)-one

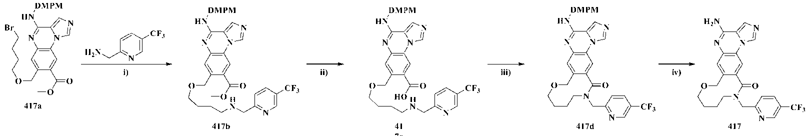

Referring to the method described in Step 6 to Step 9 of **Example 62, Example 417** (7 mg, yield: 1.6%) was obtained from methyl 7-((3-bromopropoxy)methyl)-4-((2,4-dimethoxybenzyl)amino)imidazo[1,5-a]quinoxaline-8-carboxylate **417a** (500 mg, 0.89 mmol). MS m/z (ESI): 471 [M+1]⁺
¹H NMR (400 MHz, DMSO-d6) δ 9.39 (s, 1H), 9.01 (s, 1H), 8.90 (broad s, 2H), 8.33 - 8.22 (m, 2H), 8.19 (s, 1H), 7.81 (d, J = 8.3 Hz, 1H), 7.52 (s, 1H), 5.26 (d, 1H), 4.75 (d, 1H), 4.48 (t, 1H), 4.40 (d, 1H), 3.66 (t, 2H), 3.22 (d, 2H), 2.04 - 1.61 (m, 4H)

### Example 418

### (Z)-4-Amino-12-(6-(trifluoromethyl)pyridazin-3-yl)methyl-9,10,11,12-tetrahydro-13H-azocino[4,3-g]imidazo[1,5-a]quinoxalin-13-one

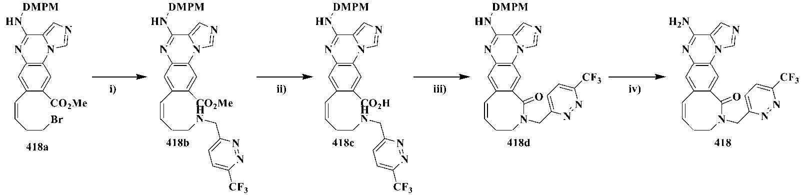

Referring to the synthesis method described in Step 6 to Step 9 of **Example 62, Example 418** (3.7 mg, yield: 1.5%) was obtained from methyl (*Z*)-7-(6-bromobut-1-en-1-yl)-4-(2,4-dimethoxybenzylamino)imidazo[1,5-a]quinoxaline-8-carboxylate **418a** (300 mg, 0.57 mmol). MS m/z (ESI): 440 [M+1]⁺
¹H NMR (400 MHz, MeOD) δ 9.09 (s, 1H), 8.19 (s, 1H), 8.15 (d, 1H), 8.06 (m, 2H), 7.32 (s, 1H), 6.61 (d, 1H), 5.90 (dd, 1H), 5.37 (d, 1H), 4.93 (d, 1H), 4.07 (t, 1H), 3.50 (s, 1H), 2.85 (dd, 1H), 2.50 (d, 1H).

### Example 419

### (Z)-4-Amino-12-(3',5'-difluoro-[3,4'-bipyridin]-6-yl)methyl-9,10,11,12-tetrahydro-13H-azonino[4,3-g]imidazo[1,5-a]quinoxalin-13-one

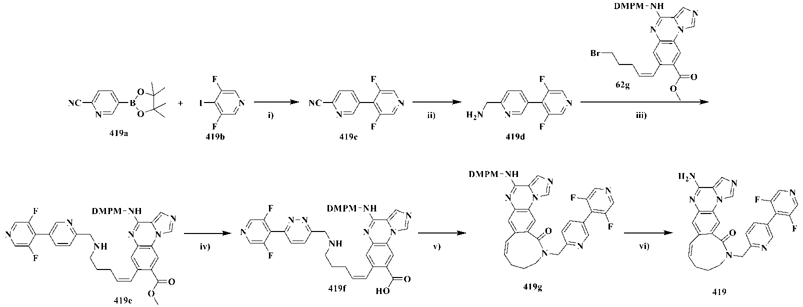

Step 1: 5-(4,4,5,5-Tetramethyl-1,3,2-dioxaborolan-2-yl)pyridine-2-carbonitrile **419a** (5.73 g, 24.90 mmol), 3,5-difluoro-4-iodopyridine **419b** (5 g, 20.75 mmol), Pd(dppf)Cl₂ (1.52 g, 2.08 mmol), and K₂CO₃ (5.74 g, 41.50 mmol) were dissolved in water (50 mL) and 1,4-dioxane (10 mL), and the mixture was stirred and reacted at 100°C for 12 hours. The system was cooled, quenched under atmospheric pressure, and concentrated under reduced pressure. The resulting residue was purified by silica gel column chromatography using eluent system A to obtain **419c** (3 g, yield: 77.7%). MS m/z (ESI): 218 [M+1]⁺
Step 2: **419c** (2.91 g, 13.40 mmol) was dissolved in THF (40 mL), 2M LiAlH₄/THF solution (8 mL) was added at 0°C, and the mixture was stirred and reacted at 20°C for 3 hours. The system was quenched with a saturated ammonium chloride solution, and concentrated under reduced pressure, and the resulting residue was purified by reversed-phase chromatography using eluent system D to obtain **419d** (2.15 g, yield: 72.5%). MS m/z (ESI): 222 [M+1]⁺
Referring to the synthesis method described in Step 6 to Step 9 of **Example 62, Example 419** (5.6 mg, yield: 2.5%) was obtained from **419d** and methyl (Z)-7-(6-bromohex-1-en-1-yl)-4-(2,4-dimethoxybenzylamino)imidazo[1,5-a]quinoxaline-8-carboxylate **62g** (270 mg, 0.50 mmol). MS m/z (ESI): 498 [M+1]⁺
¹H NMR (400 MHz, DMSO-d6) δ 9.17 (s, 1H), 8.77 (d, 1H), 8.73 (s, 2H), 8.22 (s, 1H), 8.11 (m, 1H), 7.90 (s, 1H), 7.66 (d, 1H), 7.39 (s, 2H), 7.19 (s, 1H), 6.54 (d, 1H), 6.00 (m, 1H), 5.06 (d, 1H), 4.47 (d, 1H), 3.43 (d, 2H), 2.12 (m, 1H), 1.79 (dq, 2H), 1.41 (d, 1H).

The following examples were prepared by reference to **Example 14:**

| **Example** | **Structure** | **MS m/z (ESI)** [M+1]⁺ | **Example** | **Structure** | **MS m/z (ESI)** [M+1]⁺ |
|---|---|---|---|---|---|
| 420 | 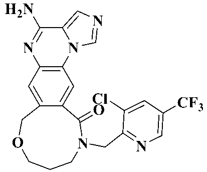 | 491 | 520-P2 | 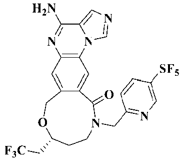 | 597 |
| 421 | 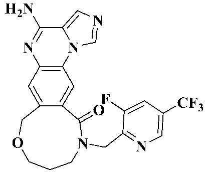 | 475 | 521 | 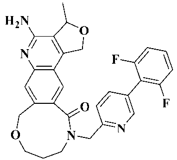 | 517 |
| 422 | 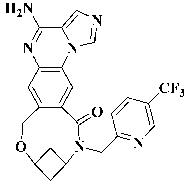 | 469 | 521-P1 | 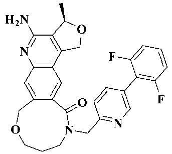 | 517 |
| 423 | 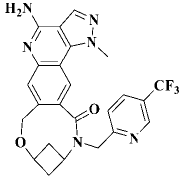 | 483 | 521-P2 | 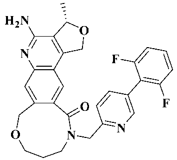 | 517 |
| **424** | 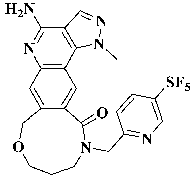 | 529 | **522** | 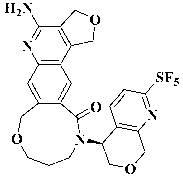 | 559 |
| **425** | 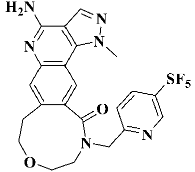 | 529 | **523** | 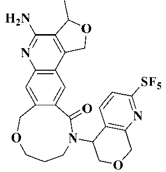 | 573 |
| **426** | 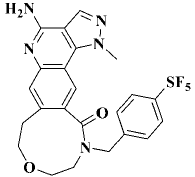 | 528 | **523-P1** | 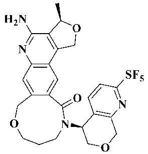 | 573 |
| **427** | 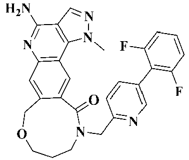 | 515 | **523-P2** | 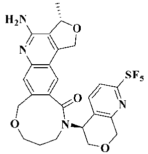 | 573 |
| **428** | 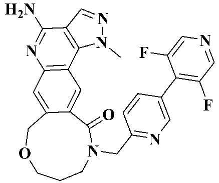 | 516 | **524** | 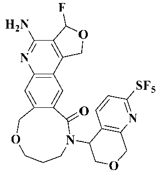 | 577 |
| **429** | 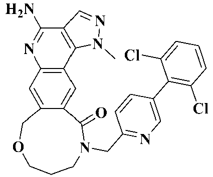 | 547 | **524-P1** | 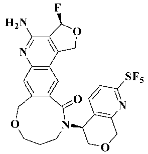 | 577 |
| **430** | 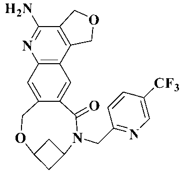 | 471 | **524-P2** | 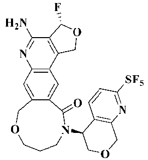 | 577 |
| **431** | 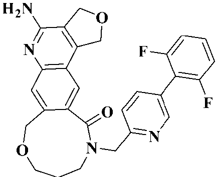 | 503 | **525** | 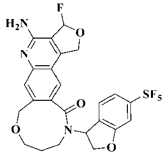 | 562 |
| **432** | 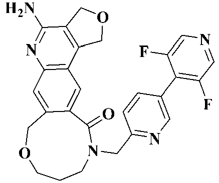 | 504 | **525-P1** | 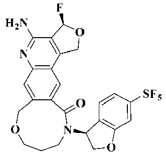 | 562 |
| **433** | 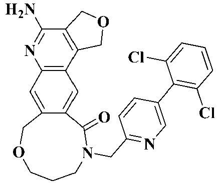 | 535 | **525-P2** | 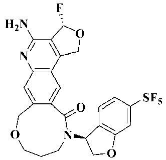 | 562 |
| **434** | 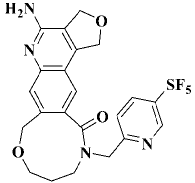 | 517 | **526** | 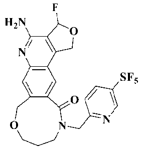 | 535 |
| **435-P1** | 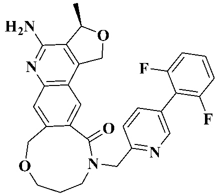 | 517 | **526-P1** | 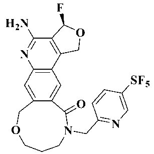 | 535 |
| **436** | 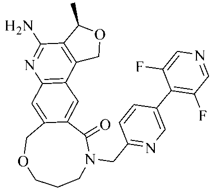 | 518 | **526-P2** | 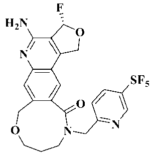 | 535 |
| **437-P1** | 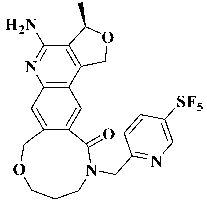 | 531 | **527** | 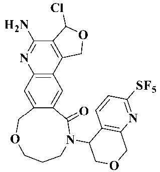 | 593 |
| **438** | 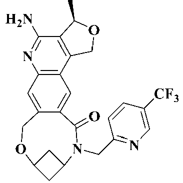 | 485 | **527-P1** | 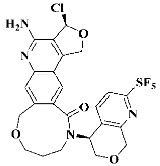 | 593 |
| **439** | 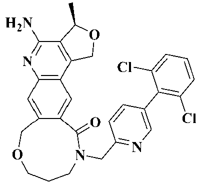 | 549 | **527-P2** | 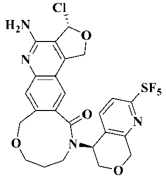 | 593 |
| **440** | 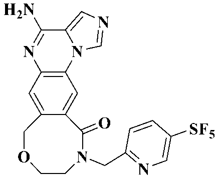 | 501 | **528** | 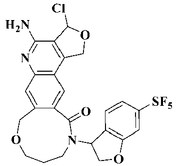 | 578 |
| **441** | 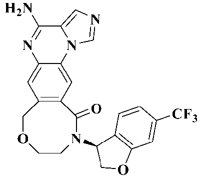 | 470 | **528-P1** | 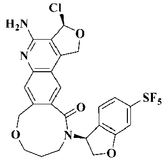 | 578 |
| **442** | 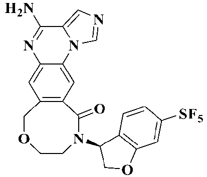 | 528 | **528-P2** | 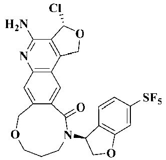 | 578 |
| **443** | 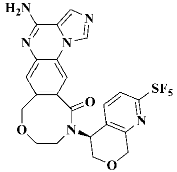 | 543 | **529** | 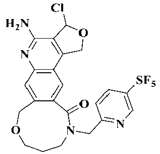 | 551 |
| **444** | 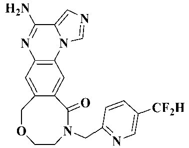 | 425 | **529-P1** | 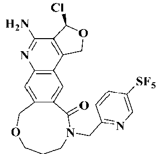 | 551 |
| **445** | 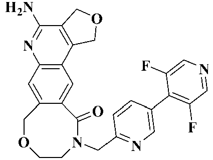 | 490 | **529-P2** | 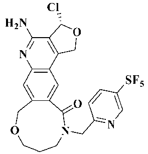 | 551 |
| **446** | 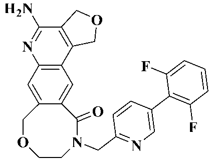 | 489 | **530** | 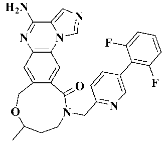 | 515 |
| **447** | 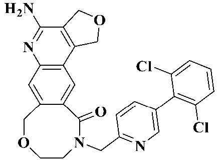 | 521 | **530-P1** | 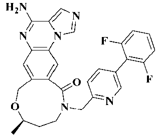 | 515 |
| **448** | 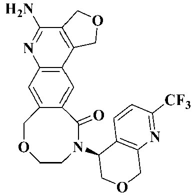 | 487 | **530-P2** | 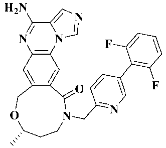 | 515 |
| **449** | 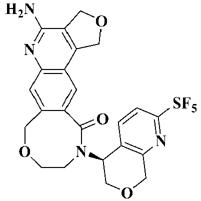 | 545 | **531** | 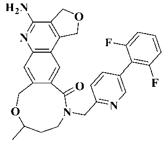 | 517 |
| **450** | 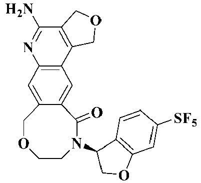 | 530 | **531-P1** | 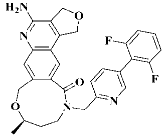 | 517 |
| **451** | 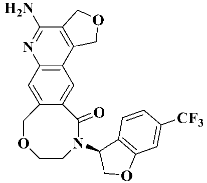 | 472 | **531-P2** | 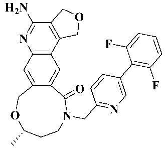 | 517 |
| **452** | 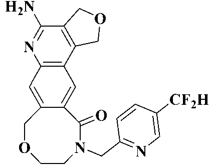 | 427 | **532** | 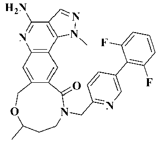 | 529 |
| **453** | 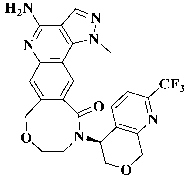 | 499 | **532-P1** | 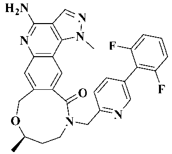 | 529 |
| **454** | 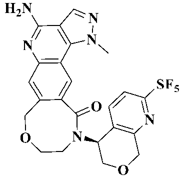 | 557 | **532-P2** | 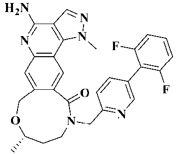 | 529 |
| **455** | 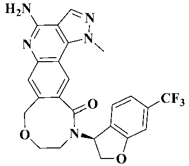 | 484 | **533** | 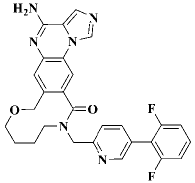 | 515 |
| **456** | 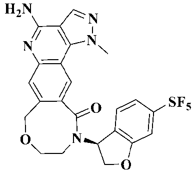 | 542 | **534** | 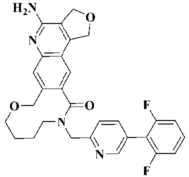 | 517 |
| **457** | 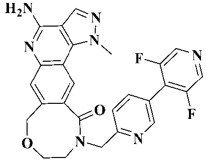 | 502 | **535** | 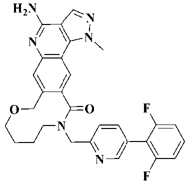 | 529 |
| **458** | 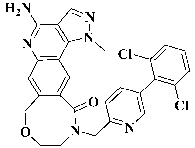 | 533 | **536** | 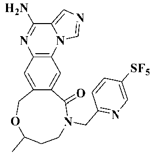 | 529 |
| **459** | 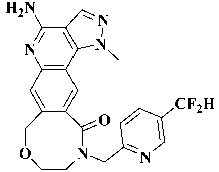 | 439 | **536-P1** | 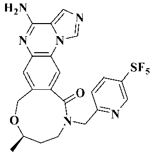 | 529 |
| **460** | 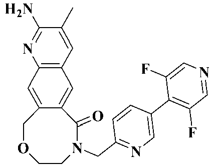 | 462 | **536-P2** | 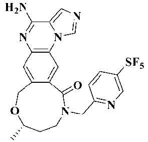 | 529 |
| **461** | 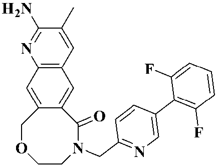 | 461 | **537** | 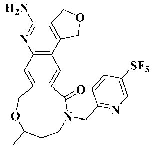 | 531 |
| **462** | 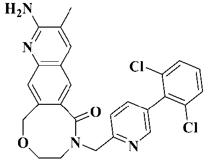 | 493 | **537-P1** | 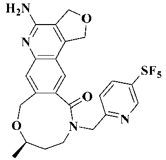 | 531 |
| **463** | 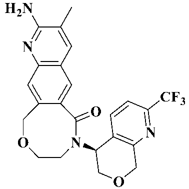 | 459 | **537-P2** | 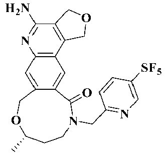 | 531 |
| **464** | 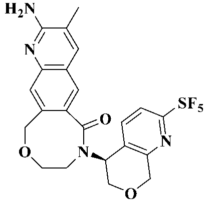 | 517 | **538** | 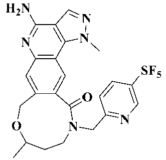 | 543 |
| **465** | 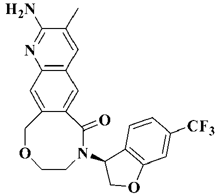 | 444 | **538-P1** | 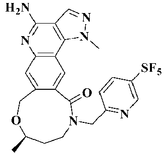 | 543 |
| **466** | 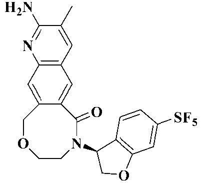 | 502 | **538-P2** | 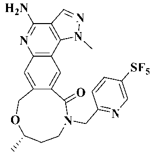 | 543 |
| **467** | 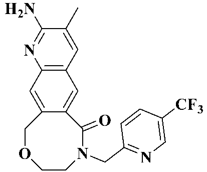 | 417 | **539** | 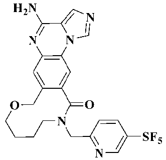 | 529 |
| **468** | 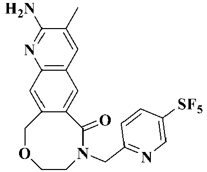 | 475 | **540** | 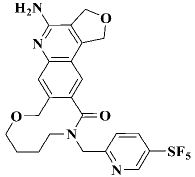 | 531 |
| **469** | 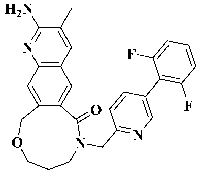 | 475 | **541** | 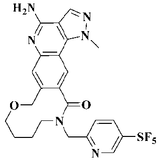 | 543 |
| **470** | 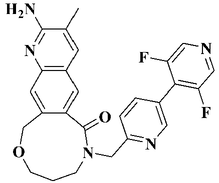 | 476 | **542** | 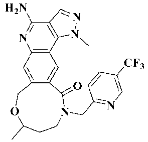 | 485 |
| **471** | 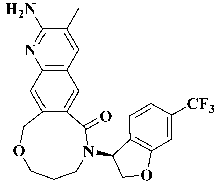 | 458 | **542-P1** | 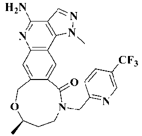 | 485 |
| **472** | 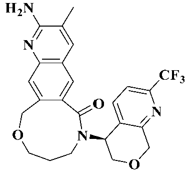 | 473 | **542-P2** | 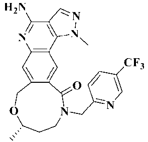 | 485 |
| **473** | 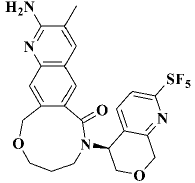 | 531 | **543** | 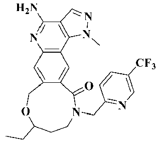 | 499 |
| **474** | 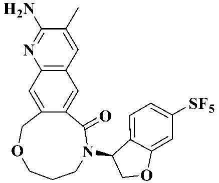 | 516 | **543-P1** | 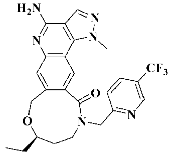 | 499 |
| **475** | 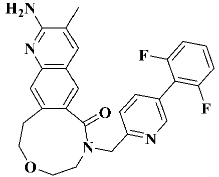 | 475 | **543-P2** | 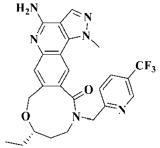 | 499 |
| **476** | 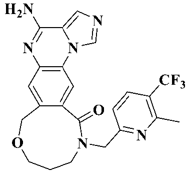 | 471 | **544** | 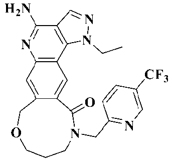 | 485 |
| **477** | 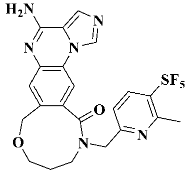 | 529 | **545** | 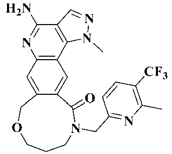 | 485 |
| **478** | 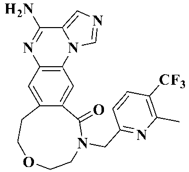 | 471 | **546** | 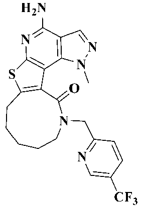 | 475 |
| **479** | 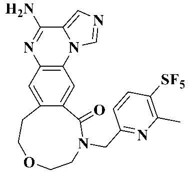 | 529 | **547** | 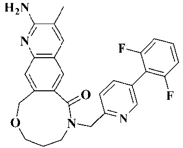 | 475 |
| **480** | 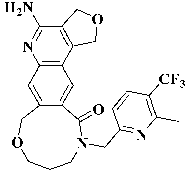 | 473 | **548** | 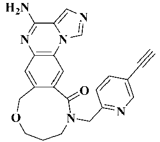 | 413 |
| **481** | 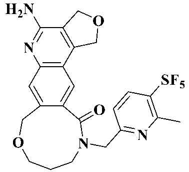 | 531 | **549** | 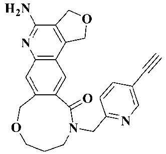 | 415 |
| **482** | 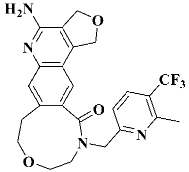 | 473 | **550** | 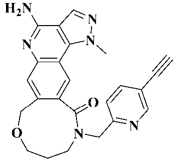 | 427 |
| **483** | 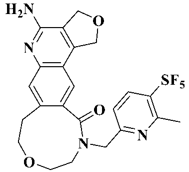 | 531 | **551** | 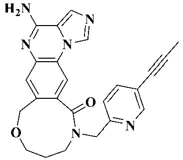 | 427 |
| **484** | 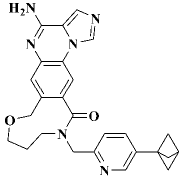 | 455 | **552** | 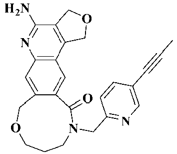 | 429 |
| **485** | 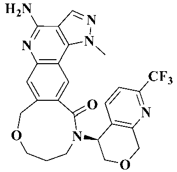 | 513 | **553** | 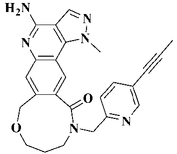 | 441 |
| **486** | 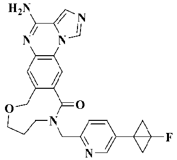 | 459 | **554** | 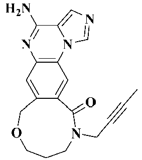 | 350 |
| **487** | 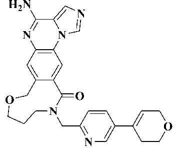 | 471 | **555** | 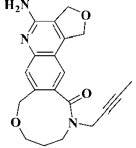 | 352 |
| **488** | 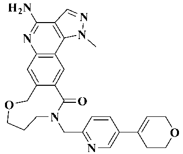 | 485 | **556** | 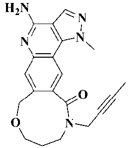 | 364 |
| **489** | 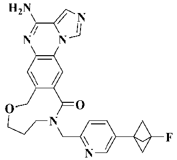 | 473 | **557** | 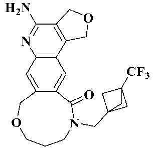 | 448 |
| **490** | 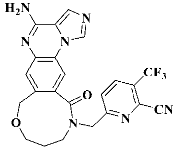 | 482 | **558** | 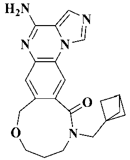 | 378 |
| **491** | 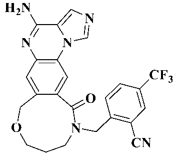 | 481 | **559** | 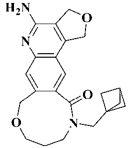 | 380 |
| **492** | 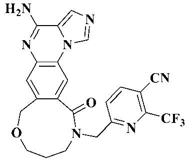 | 482 | **560** | 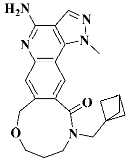 | 392 |
| **493** | 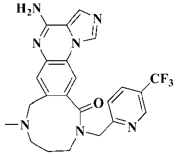 | 470 | **561** | 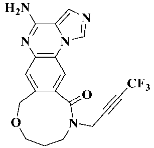 | 404 |
| **494** | 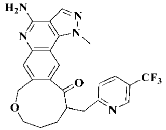 | 470 | **562** | 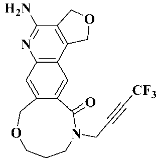 | 406 |
| **494-P1** | 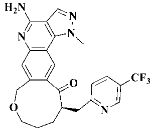 | 470 | **563** | 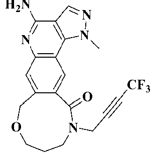 | 418 |
| **494-P2** | 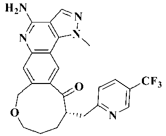 | 470 | **564** | 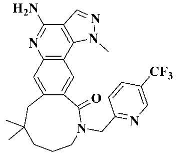 | 497 |
| **495** | 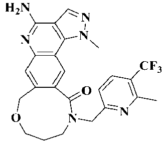 | 485 | **565** | 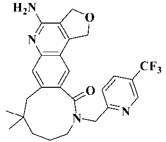 | 485 |
| **496** | 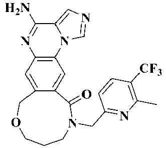 | 471 | **566** | 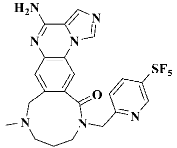 | 528 |
| **497** | 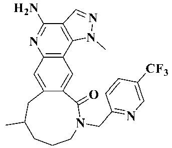 | 483 | **567** | 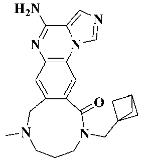 | 391 |
| **497-P1** | 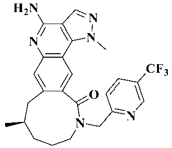 | 483 | **568** | 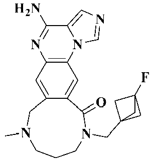 | 409 |
| **497-P2** | 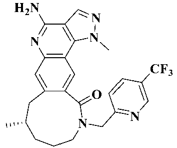 | 483 | **569** | 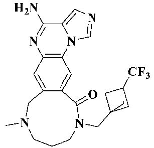 | 459 |
| **498** | 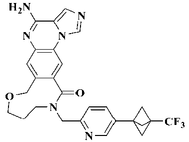 | 523 | **570** | 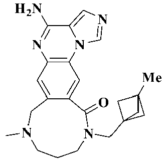 | 405 |
| **499** | 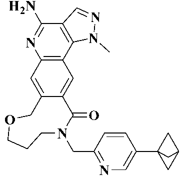 | 469 | **571** | 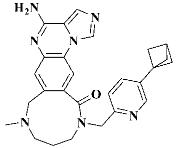 | 468 |
| **500** | 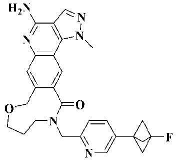 | 487 | **572** | 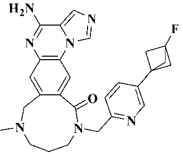 | 486 |
| **501** | 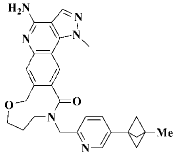 | 483 | **573** | 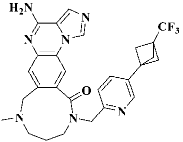 | 536 |
| **502** | 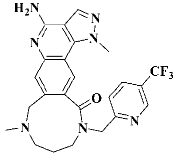 | 483 | **574** | 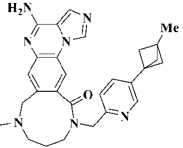 | 482 |
| **503** | 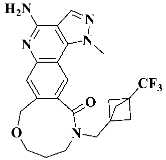 | 460 | **575** | 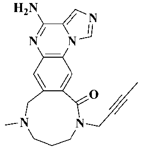 | 363 |
| **504** | 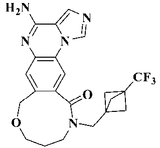 | 446 | **576** | 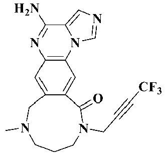 | 417 |
| **505** | 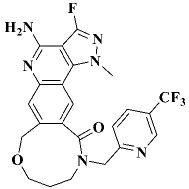 | 489 | **577** | 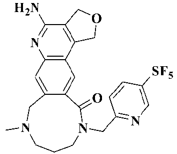 | 530 |
| **506** | 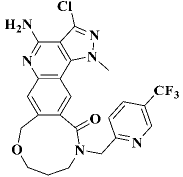 | 505 | **578** | 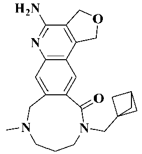 | 393 |
| **507** | 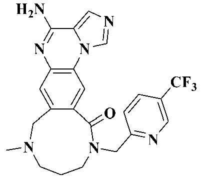 | 470 | **579** | 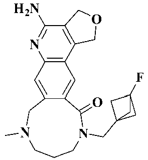 | 411 |
| **508** | 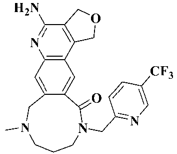 | 471 | **580** | 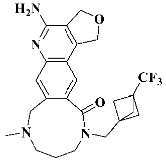 | 461 |
| **509** | 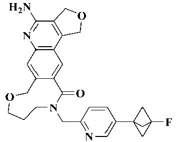 | 475 | **581** | 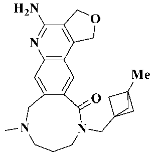 | 407 |
| **510** | 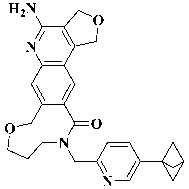 | 457 | **582** | 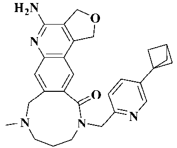 | 470 |
| **511** | 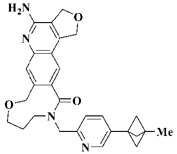 | 471 | **583** | 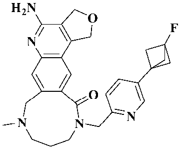 | 488 |
| **512** | 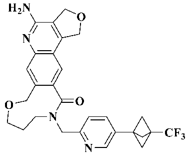 | 525 | **584** | 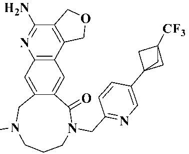 | 538 |
| **513** | 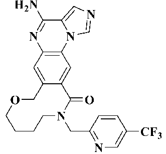 | 471 | **585** | 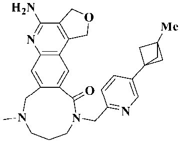 | 484 |
| **514** | 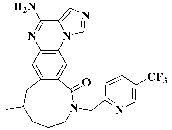 | 469 | **586** | 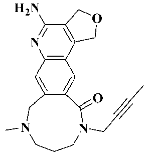 | 365 |
| **514-P1** | 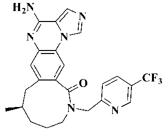 | 469 | **587** | 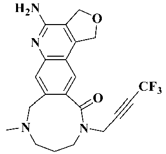 | 419 |
| **514-P2** | 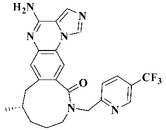 | 469 | **588** | 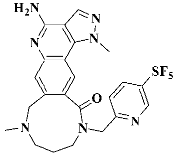 | 542 |
| **515** | 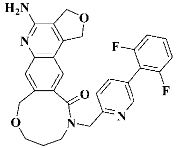 | 503 | **589** | 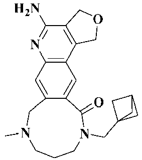 | 393 |
| **516** | 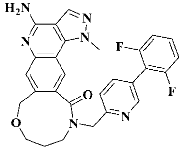 | 515 | **590** | 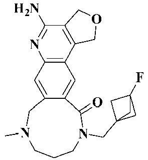 | 411 |
| **517** | 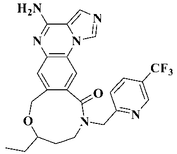 | 485 | **591** | 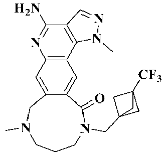 | 473 |
| **517-P1** | 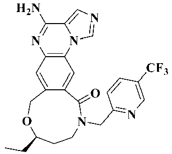 | 485 | **592** | 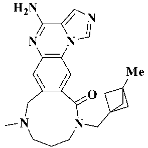 | 405 |
| **517-P2** | 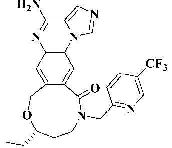 | 485 | **593** | 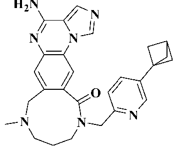 | 468 |
| **518** | 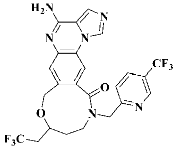 | 539 | **594** | 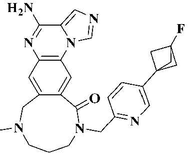 | 486 |
| **518-P1** | 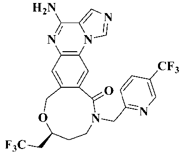 | 539 | **595** | 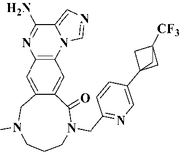 | 536 |
| **518-P2** | 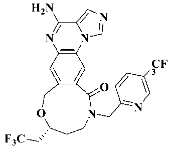 | 539 | **596** | 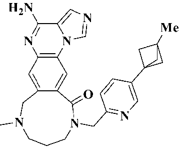 | 482 |
| **519** | 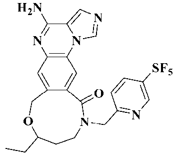 | 543 | **597** | 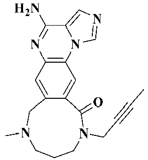 | 363 |
| **519-P1** | 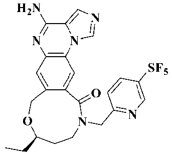 | 543 | **598** | 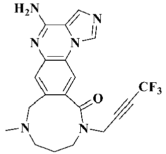 | 417 |
| **519-P2** | 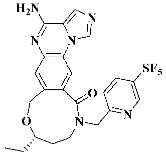 | 543 | **599** | 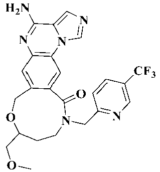 | 501 |
| **520** | 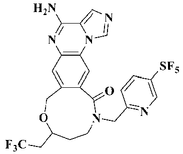 | 597 | **599-P1** | 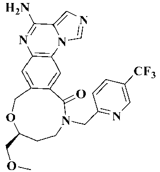 | 501 |
| **520-P1** | 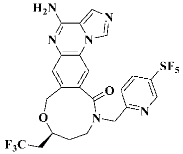 | 597 | **599-P2** | 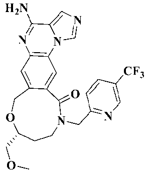 | 501 |

The following examples were prepared by reference to Example 62:

| **Example** | **Structure** | **MS m/z (ESI) [M+1]⁺** | **Example** | **Structure** | **MS m/z (ESI) [M+1]⁺** |
|---|---|---|---|---|---|
| **600** | 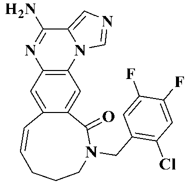 | 454 | **664** | 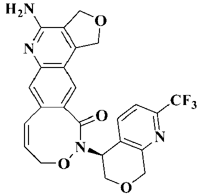 | 485 |
| **601** | 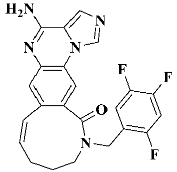 | 438 | **665** | 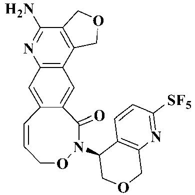 | 543 |
| **602** | 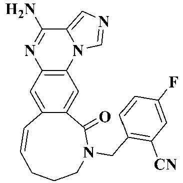 | 427 | **666** | 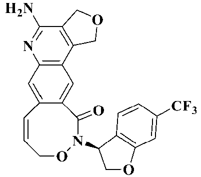 | 470 |
| **603** | 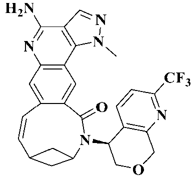 | 521 | **667** | 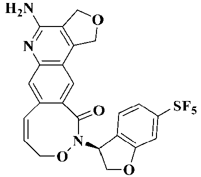 | 528 |
| **604** | 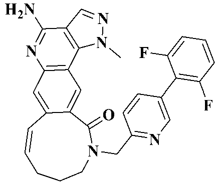 | 511 | **668** | 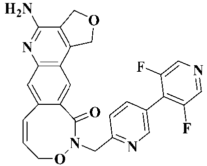 | 488 |
| **605** | 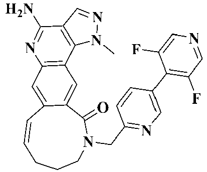 | 512 | **669** | 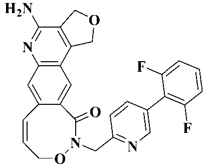 | 487 |
| **606** | 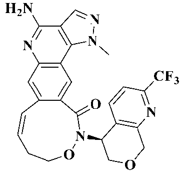 | 511 | **670** | 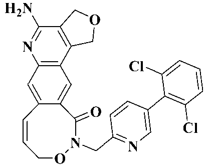 | 519 |
| **607** | 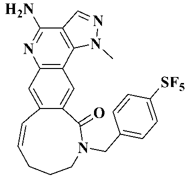 | 524 | **671** | 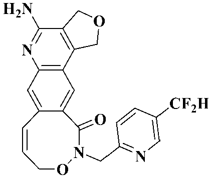 | 425 |
| **608** | 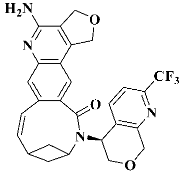 | 509 | **672** | 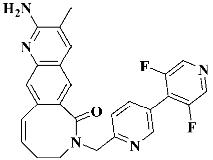 | 458 |
| **609** | 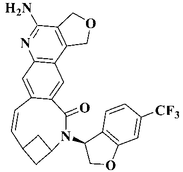 | 494 | **673** | 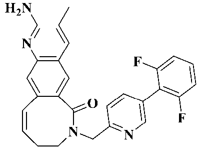 | 457 |
| **610** | 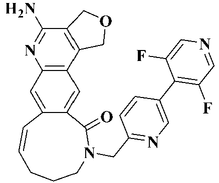 | 500 | **674** | 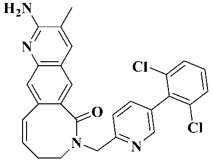 | 489 |
| **611** | 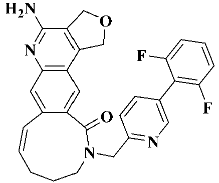 | 499 | **675** | 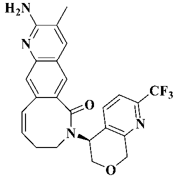 | 455 |
| **612** | 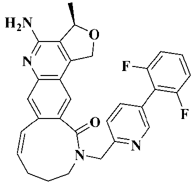 | 513 | **676** | 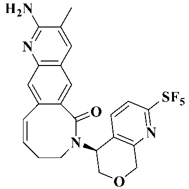 | 513 |
| **613** | 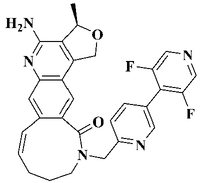 | 514 | **677** | 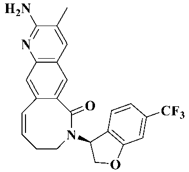 | 440 |
| **614** | 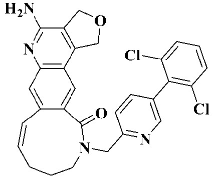 | 531 | **678** | 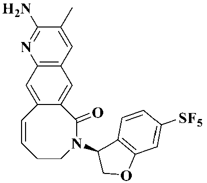 | 498 |
| **615** | 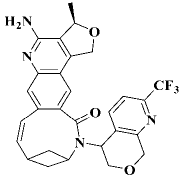 | 523 | **679** | 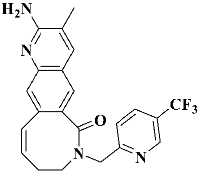 | 413 |
| **616** | 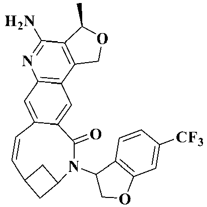 | 508 | **680** | 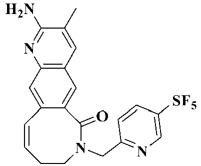 | 471 |
| **617** | 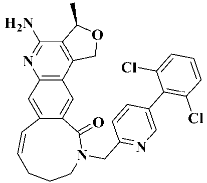 | 545 | **681** | 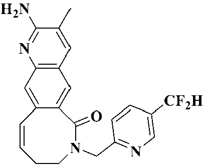 | 395 |
| **618** | 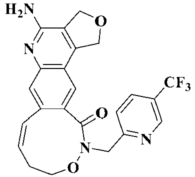 | 457 | **682** | 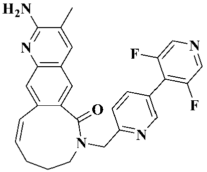 | 472 |
| **619** | 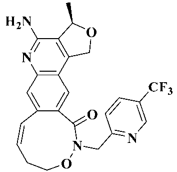 | 471 | **683** | 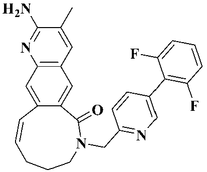 | 471 |
| **620** | 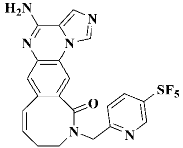 | 497 | **684** | 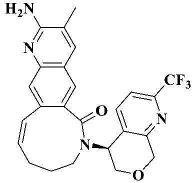 | 469 |
| **621** | 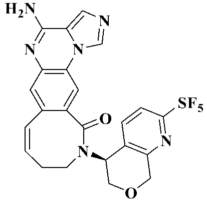 | 539 | **685** | 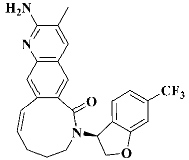 | 454 |
| **622** | 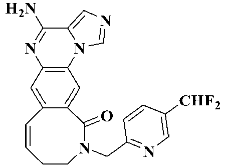 | 421 | **686** | 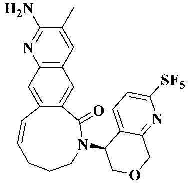 | 527 |
| **623** | 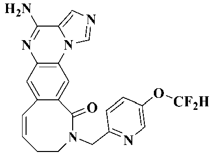 | 437 | **687** | 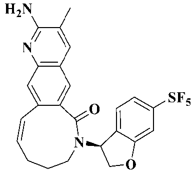 | 512 |
| **624** | 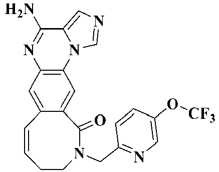 | 455 | **688** | 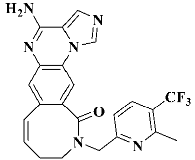 | 453 |
| **625** | 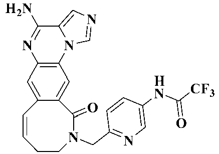 | 482 | **689** | 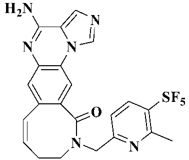 | 511 |
| **626** | 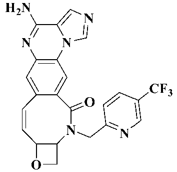 | 467 | **690** | 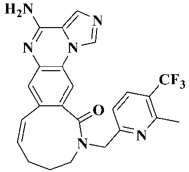 | 467 |
| **627** | 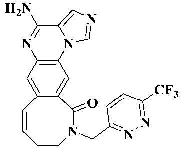 | 440 | **691** | 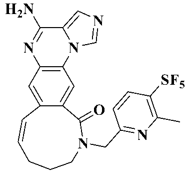 | 525 |
| **628** | 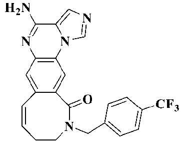 | 438 | **692** | 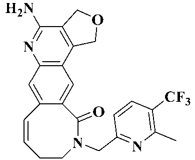 | 455 |
| **629** | 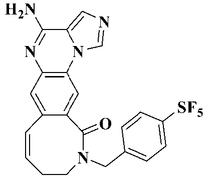 | 496 | **693** | 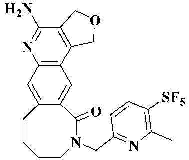 | 513 |
| **630** | 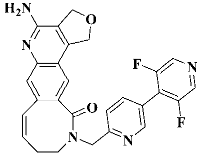 | 486 | **694** | 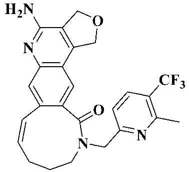 | 469 |
| **631** | 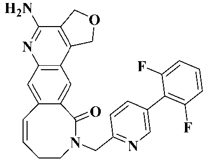 | 485 | **695** | 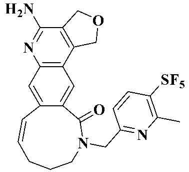 | 527 |
| **632** | 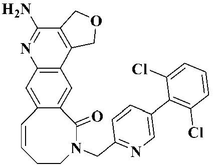 | 517 | **696** | 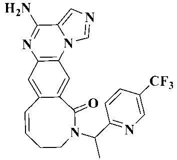 | 453 |
| **633** | 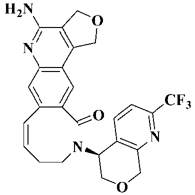 | 483 | **696-P1** | 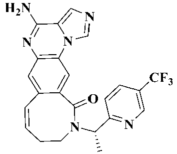 | 453 |
| **634** | 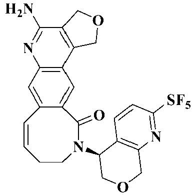 | 541 | **696-P2** | 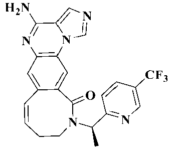 | 453 |
| **635** | 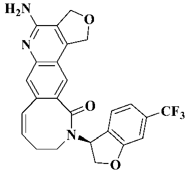 | 468 | **697** | 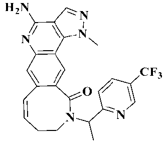 | 467 |
| **636** | 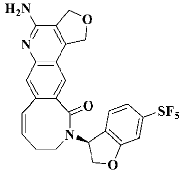 | 526 | **697-P1** | 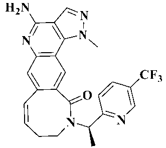 | 467 |
| **637** | 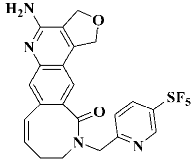 | 499 | **697-P2** | 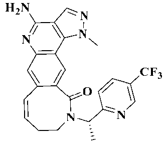 | 467 |
| **638** | 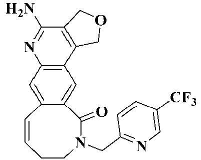 | 441 | **698** | 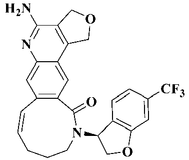 | 482 |
| **639** | 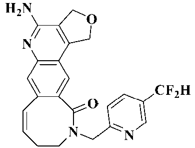 | 423 | **699** | 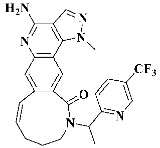 | 481 |
| **640** | 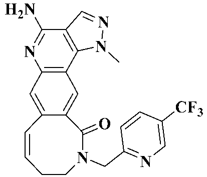 | 453 | **699-P1** | 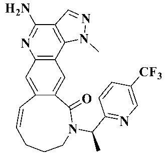 | 481 |
| **641** | 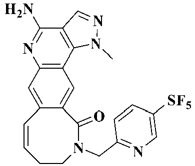 | 511 | **699-P2** | 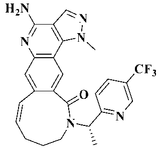 | 481 |
| **642** | 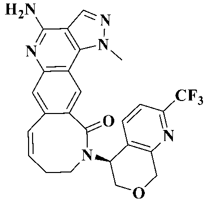 | 495 | **700** | 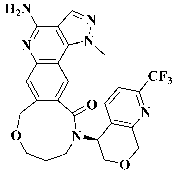 | 513 |
| **643** | 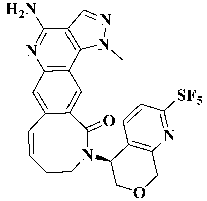 | 553 | **701** | 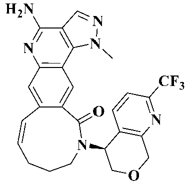 | 509 |
| **644** | 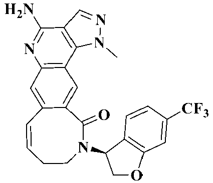 | 480 | **702** | 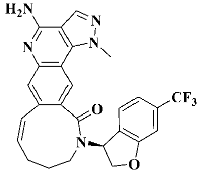 | 494 |
| **645** | 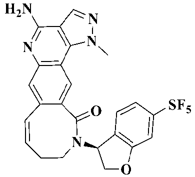 | 538 | **703** | 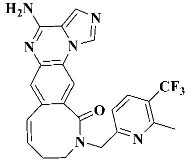 | 453 |
| **646** | 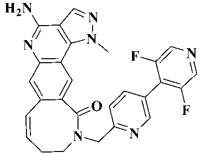 | 498 | **704** | 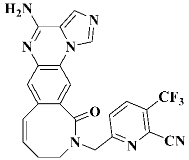 | 464 |
| **647** | 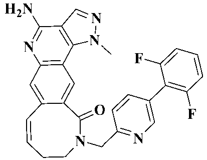 | 497 | 705 | 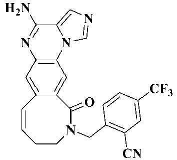 | 463 |
| **648** | 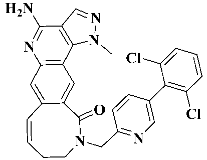 | 529 | **706** | 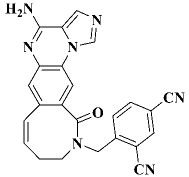 | 420 |
| **649** | 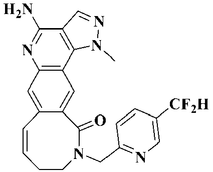 | 435 | **707** | 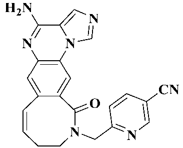 | 396 |
| **650** | 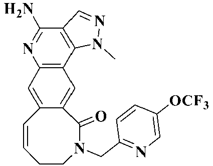 | 469 | **708** | 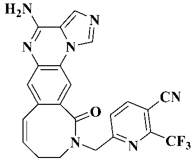 | 464 |
| **651** | 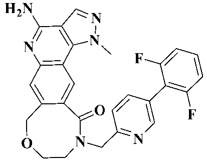 | 501 | **709** | 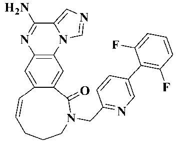 | 497 |
| **652** | 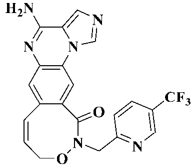 | 441 | **710** | 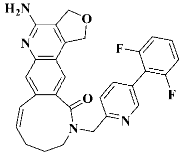 | 499 |
| **653** | 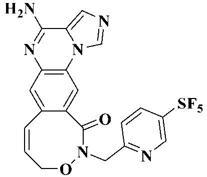 | 499 | **711** | 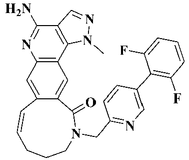 | 511 |
| **654** | 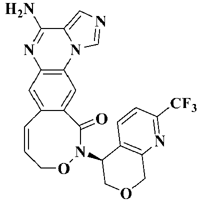 | 483 | **712** | 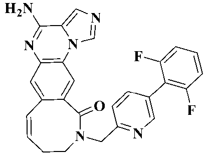 | 483 |
| **655** | 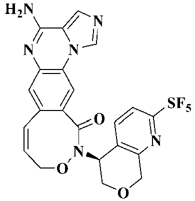 | 541 | **713** | 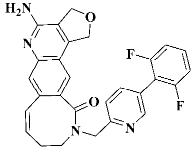 | 485 |
| **656** | 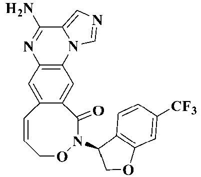 | 468 | **714** | 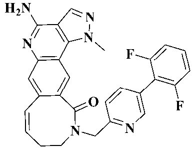 | 497 |
| **657** | 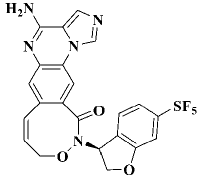 | 526 | **715** | 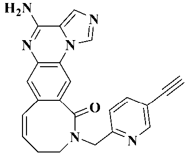 | 395 |
| **658** | 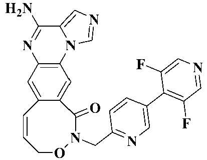 | 486 | **716** | 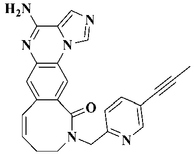 | 409 |
| **659** | 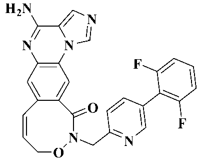 | 485 | **717** | 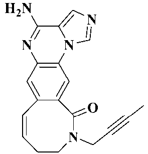 | 332 |
| **660** | 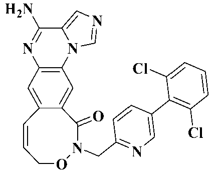 | 517 | **718** | 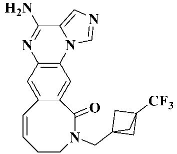 | 428 |
| **661** | 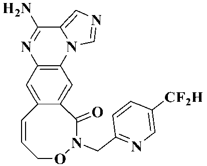 | 423 | **719** | 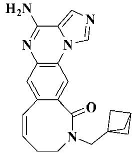 | 360 |
| **662** | 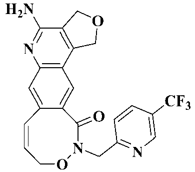 | 443 | **720** | 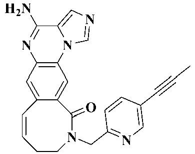 | 409 |
| **663** | 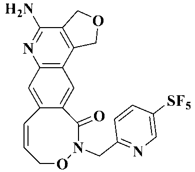 | 501 | **721** | 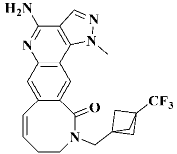 | 442 |

Alternatively, the following examples were prepared using the following method:

### Example 427

### 4-Amino-12-((5-(2,6-difluorophenyl)pyridin-2-yl)methyl)-1-methyl-1,7,9,10,11,12-hexahydro-13H-[1,5]oxazolo[7,8-g]pyrazolo[4,3-c]quinolin-13-one

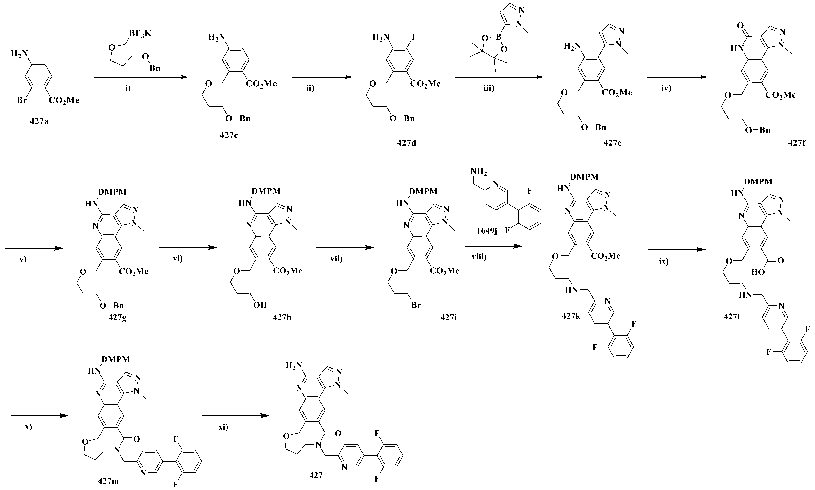

Referring to the preparation method of **Example 1, Example 427** (5.2 mg, yield: 2.3%) was obtained from methyl 4-amino-2-bromobenzoate **427a** (100 mg, 454 µmol). MS m/z (ESI): 516 [M+1]⁺
¹H NMR (400 MHz, DMSO-*d6*) δ 8.66 (s, 1H), 8.36 (d, 1H), 8.16 - 8.05 (m, 1H), 7.98 (d, 1H), 7.70 (d, 1H), 7.64 - 7.48 (m, 2H), 7.30 (t, 2H), 5.25 (d, 1H), 4.75 (d, 1H), 4.59 (d, 1H), 4.52 - 4.34 (m, 3H), 3.87 - 3.75 (m, 1H), 3.54 - 3.36 (m, 2H), 3.26 (s, 2H), 2.03 - 1.87 (m, 1H), 1.32 - 1.19 (m, 1H).

### Example 431

### 4-Amino-12-(5-(2,6-difluorophenyl)pyridin-2-yl)methyl-3,7,9,10,11,12-hexahydrofuro[3,4-c][1,5]oxazolino[7,8-g]quinolin-13(1H)-one

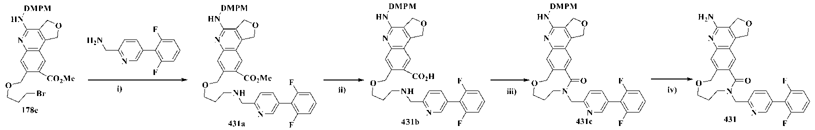

Referring to the synthesis method described in Step 5 to Step 8 of **Example 14, Example 431** (14 mg, yield: 2.8%) was obtained from methyl 7-(3-bromopropyl)methyl-4-(3,4-dimethoxybenzyl)amino-1,3-dihydrofuro[3,4-c]quinoline-8-carboxylate **178e** (545 mg, 1 mmol) and (5-(2,6-difluorophenyl)pyridin-2-yl)methanamine (242 mg, 1.2 mmol). MS m/z (ESI): 503 [M+1]⁺
¹H NMR (400 MHz, DMSO-*d*₆) δ 8.66 (s, 1H), 7.97 (dd, 1H), 7.66 (d, 1H), 7.57 (s, 1H), 7.55 (m, 2H), 7.30 (m, 2H), 5.43 (s, 1H), 5.25 (d, 1H), 5.06 (br s, 2H), 4.75 (d, 1H), 4.60 (d, 1H), 4.35 (d, 1H), 3.82 (d, 1H), 3.45 (m, 2H), 3.26 (m, 2H), 1.91 (m, 1H), 1.23 (d, 1H).

### Example 434

### 4-Amino-3-methyl-12-((5-(pentafluorosulphanyl)pyridin-2-yl)methyl)-3,7,9,10,11,12-hexahydrofuro[3,4-c][1,5]oxazolo[7,8-g]quinolin-13(1H)-one

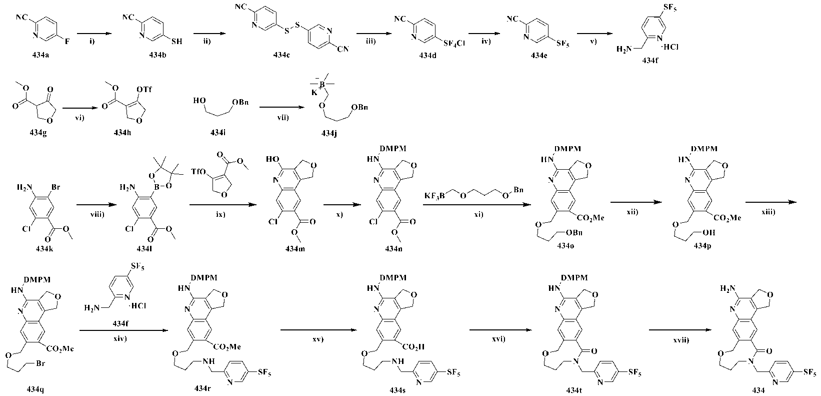

Step 1: 2-Cyano-5-fluoropyridine **434a** (50 g, 409.5 mmol) was dissolved in DMF (250 mL), and the mixture was cooled in an ice bath under nitrogen atmosphere. Na₂S (38.35 g, 491.4 mmol) was added in portions, and the reaction system was stirred at 20°C for 1 hour. The reaction solution was slowly added to 1N NaOH solution (2 L), and the mixture was extracted with dichloromethane (2 L). The phases were separated, and the aqueous phase was collected. The pH was adjusted to 2, resulting in the precipitation of a large amount of solid. After filtration, the filtrate was slurried with isopropanol and water, and the solid was collected to obtain **434b** (50 g, yield: 89.0%). MS m/z (ESI): 137 [M+1]⁺
Step 2: **434b** (50 g, 367.1 mmol) was dissolved in acetonitrile (250 mL), sodium iodide (27.5 g, 183.59 mmol) and ferric chloride (30 g, 183.6 mmol) were added under nitrogen atmosphere, and the reaction system was stirred at 20°C for 3 hours. The reaction solution was filtered to remove insoluble materials, and the filtrate was concentrated and dissolved in ethyl acetate (2 L). The mixture was extracted with water (2 L), and the phases were separated. The organic phase was washed with a saturated sodium chloride aqueous solution, dried, and concentrated under reduced pressure, and the resulting residue was purified by silica gel column chromatography using eluent A to obtain a brown solid. The solid was purified by slurring with ethyl acetate to obtain **434c** (10.5 g, yield: 21.1%). MS m/z (ESI): 271 [M+1]⁺
Step 3: Under nitrogen atmosphere, trichloroisocyanuric acid (83.82 g, 360.67 mmol) and super-dry potassium fluoride (27.94 g, 480.89 mmol) were added to a reaction flask, followed by anhydrous acetonitrile (130 mL). During stirring, 5,5'-dithiobis(2-cyanopyridine) **434c** (6.50 g, 24.04 mmol) was added, and the mixture was stirred at 80°C for 5 h. Upon completion of the reaction, the reaction system was cooled to 20°C, and filtered, and the filter cake was washed with anhydrous acetonitrile (20 mL). The filtrate was concentrated under reduced pressure to remove the organic solvent to obtain **434d** (crude, 14.80 g).
¹⁹F NMR (400 MHz) δ 136.3 ppm.

Step 4: Silver tetrafluoroborate (28.08 g, 144.27 mmol) was added to a solution of **434d** (14.80 g, crude) in anhydrous dichloromethane (130 mL). Under nitrogen atmosphere, the reaction suspension was heated to 40°C and stirred for 22 h. Upon completion of the reaction, the reaction system was concentrated under reduced pressure to remove the solvent dichloromethane, and ethyl acetate was added. The mixture was sonicated and filtered, and the filter cake was washed with ethyl acetate, and concentrated. The residue was purified by silica gel column chromatography using eluent system C to obtain **434e** (5.5 g, 23.90 mmol, two-step yield: 49.7%).

¹H NMR (400 MHz, CDCl₃) δ 9.09 (d, 1H), 8.24 (dd, 1H), 7.87 (d, 1H) ppm;
¹⁹F NMR (400 MHz, CDCl₃) δ 79.6 ppm.

Step 5: **434e** (5.7 g, 24.77 mmol) and 5% palladium on carbon (2.64 g) were dissolved in ethanol (100 mL), 4 M hydrogen chloride/methanol (37.15 mL, 148.59 mmol) was added, and the mixture was stirred at 20°C for 16 hours (15 psi hydrogen atmosphere). The reaction solution was filtered through Celite, and washed with methanol. The filtrate was concentrated to obtain a crude product, which was dispersed in ethyl acetate (50 mL), and filtered to obtain **434f** (5.80 g, 21.43 mmol, yield: 86.5%). MS m/z (ESI): 271 [M+1]⁺
Step 6: Methyl 4-oxotetrahydrofuran-3-carboxylate **434g** (45.6 g, 316.46 mmol) was dissolved in dichloromethane (500 mL), and N,N-diisopropylethylamine (64.50 g, 500.00 mmol) and trifluoromethanesulphonic anhydride (116.80 g, 400.00 mmol) were sequentially added in an ice bath. The mixture was stirred and reacted at room temperature for about 16 hours. Water (500 mL) was added to the reaction solution, and the mixture was extracted with ethyl acetate (400 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **434h** (75.3 g, yield: 86.1%).

Step 7: 3-(Benzyloxy)propan-1-ol **434i** (20 g, 120.48 mmol) was dissolved in tetrahydrofuran (400 mL), and sodium hydride (5.78 g, 144.57 mmol, 60% purity) was added in an ice bath. The mixture was stirred for 15 minutes, potassium (bromomethyl)trifluoroborate (24.09 g, 120.00 mmol) was then added, and the mixture was stirred and reacted at room temperature for about 16 hours. A potassium bifluoride solution (4.5 M) was added to the reaction solution, and the mixture was stirred at room temperature for 30 minutes. The reaction solution was subjected to rotary evaporation to dryness, and then hot acetone (2000 mL) was added, and the resulting mixture was stirred at 80°C for about 15 minutes. After hot filtration to remove impurities, the acetone was distilled under reduced pressure until solids precipitated. Twice the volume of diethyl ether was added, and the mixture was filtered in an ice bath. The filter residue was dried to obtain **437j** (12.5 g, yield: 37.9%).

Step 8: Methyl 4-amino-5-bromo-2-chlorobenzoate **434k** (80 g, 302.45 mmol), bis(pinacolato)diboron (115.21 g, 453.68 mmol), 1,1'-bis(diphenylphosphino)ferrocene (16.77 g, 30.25 mmol), potassium acetate (59.37 g, 604.91 mmol), and palladium acetate (3.40 g, 15.12 mmol) were added to 1,4-dioxane (800 mL), and the reaction system was stirred at 80°C under nitrogen atmosphere for 16 hours. The reaction solution was cooled to room temperature, and filtered to remove insoluble materials. The filtrate was concentrated, and the residue was purified by silica gel column chromatography using eluent system B to obtain methyl 4-amino-2-chloro-5-(4,4,5,5-tetramethyl-1,3,2-dioxaborolan-2-yl)benzoate **434l** (84 g, 269.60 mmol, yield: 89.1%). MS m/z (ESI): 312 [M+1]⁺
Step 9: **434l** (70.5 g, 226.27 mmol) was added to ethanol (1000 mL) and water (200 mL), followed by methyl 4-(((trifluoromethyl)sulphonyl)oxy)-2,5-dihydrofuran-3-carboxylate (62.49 g, 226.27 mmol), sodium carbonate (47.97 g, 452.55 mmol) and bis(triphenylphosphine)palladium(II) dichloride (15.88 g, 22.63 mmol), and the reaction system was reacted at 80°C under nitrogen atmosphere for 4 hours. The reaction solution was directly concentrated, water was added, and the mixture was stirred for 10 min. The resulting system was filtered by suction, and the solid was slurried with ethyl acetate, to obtain **434m** (49 g, 175.20 mmol, yield: 77.4%). MS m/z (ESI): 280 [M+1]⁺
Step 10: **434m** (56.7 g, 202.74 mmol) was dissolved in dimethyl sulphoxide (25.23 mL), and 2,4-dimethoxybenzylamine (40.68 g, 243.28 mmol) and 1,8-diazabicyclo[5.4.0]undec-7-ene (92.59 g, 608.21 mmol, 90.78 mL) were added, followed by a BOP reagent (269.00 g, 608.21 mmol). The reaction system was reacted at room temperature for 1 hour. The reaction solution was poured into ice water, and extracted with ethyl acetate. The organic phase was separated, dried, concentrated, and slurried with methanol to obtain **434n** (43 g, 100.26 mmol, yield: 49.5%). MS m/z (ESI): 429 [M+1]⁺
Step 11: **434n** (2.5 g, 5.83 mmol), potassium ((3-(benzyloxy)propoxy)methyl)trifluoro-L4-borate (2.50 g, 8.74 mmol), [n-butyl-di(1-adamantyl)phosphine](2-amino-1,1'-biphenyl-2-yl)palladium(II) methanesulphonate (424.53 mg, 582.93 µmol), and caesium carbonate (5.70 g, 17.49 mmol) were placed in a sealed tube, 1,4-dioxane (20 mL) and water (2 mL) were added, and the reaction system was reacted at 90°C under nitrogen atmosphere for 16 hours. The reaction solution was added to a saturated sodium chloride aqueous solution, and extracted with ethyl acetate (100 mL × 3). The organic phase was washed with a saturated sodium chloride aqueous solution, dried over anhydrous sodium sulphate, filtered, and concentrated under reduced pressure to remove the organic solvent. The residue was purified by silica gel column chromatography using eluent system B to obtain **434o** (1.76 g, 3.07 mmol, yield: 52.7%). MS m/z (ESI): 573 [M+1]⁺
Step 12: **434o** (17.1 g, 29.86 mmol) was dissolved in tetrahydrofuran (170 mL) and methanol (170 mL), acetic acid (10 mL), 10% wt palladium on carbon (8.55 g, 80.34 mmol) and 10% wt palladium hydroxide on carbon (8.55 g, 60.88 mmol) were added, and the reaction system was reacted at 50°C under hydrogen atmosphere at 60 psi pressure for 20 hours. The reaction solution was filtered by suction, and the filtrate was concentrated to obtain **434p** (9.64 g, 19.98 mmol, yield: 66.9%). MS m/z (ESI): 483 [M+1]⁺
Step 13: **434p** (9.64 g, 19.98 mmol) was dissolved in dichloromethane (100 mL), carbon tetrabromide (6.63 g, 19.98 mmol) and triphenylphosphine (5.24 g, 19.98 mmol) were added, and the reaction system was reacted at room temperature under nitrogen atmosphere for 3 hours. The reaction solution was directly concentrated, and the residue was purified by silica gel column chromatography using eluent system A to obtain **434q** (12.64 g, 16.45 mmol, yield: 82.4%). MS m/z (ESI): 545 [M+1]⁺
Step 14: A solution of **434f** (4.47 g, 16.50 mmol), **434q** (5.00 g, 9.17 mmol), sodium iodide (2.75 g, 18.33 mmol) and caesium carbonate (5.97 g, 18.33 mmol) in acetonitrile (50 mL) was reacted at 80°C for 15 hours. The reaction system was quenched with water, and extracted with ethyl acetate (100 mL × 3). The organic phase was dried over anhydrous sodium sulphate, filtered, and concentrated to remove the organic solvent. The residue was purified by silica gel column chromatography using eluent system C to obtain **434r** (3.10 g, 4.44 mmol, yield: 48.4%). MS m/z (ESI): 699 [M+1]⁺
Step 15: **434r** (3.30 g, 4.72 mmol) was dissolved in tetrahydrofuran (20 mL) and methanol (20 mL), 4M lithium hydroxide solution (5.90 mL, 23.62 mmol) was added, and the mixture was reacted at 50°C for 4 hours. The reaction system was adjusted to pH 2-3 with 1 M dilute hydrochloric acid, and extracted with ethyl acetate (50 mL × 3). The organic phase was washed with a saturated sodium chloride aqueous solution (30 mL), dried over anhydrous sodium sulphate, filtered, and concentrated under reduced pressure to obtain 4-((2,4-dimethoxybenzyl)amino)-7-((3-(((5-(pentafluoro-λ⁶-sulphanyl)pyridin-2-yl)methyl)amino)propoxy)methyl)-1,3-dihydrofuro[3,4-c]quinoline-8-carboxylic acid **434s** (3.00 g, crude). MS m/z (ESI): 685 [M+1]⁺
Step 16: **434s** (3.00 g, 4.38 mmol) and 2-(7-azobenzotriazol-1-yl)-N,N,N',N'-tetramethyluronium hexafluorophosphate (2.48 g, 6.57 mmol) were dissolved in N,N-dimethylformamide (15 mL), N,N-diisopropylethylamine (1.13 g, 8.76 mmol, 1.53 mL) was added, and the mixture was reacted at 25°C for 16 hours. The reaction system was quenched with water, and extracted with ethyl acetate (80 mL × 3). The organic phase was washed with a saturated sodium chloride aqueous solution, dried over anhydrous sodium sulphate, filtered, and concentrated to remove the organic solvent. The residue was purified by silica gel column chromatography using eluent system C to obtain **434t** (2.00 g, 3.00 mmol, yield: 47.9%). MS m/z (ESI): 667 [M+1]⁺
Step 17: **434t** (2.00 g, 3.00 mmol) was dissolved in trifluoroacetic acid (10 mL), and the reaction system was reacted at 90°C under nitrogen atmosphere for 1 hour. The reaction solution was concentrated, and the residue was purified by preparative HPLC chromatography to obtain **Example 434** (893 mg, 0.51 mmol, yield: 57.6%). MS m/z (ESI): 517 [M+1]⁺
¹H NMR (400 MHz, DMSO-*d6*) δ 9.11 (d, 1H), 8.40 (dd, 1H), 7.76 (d, 1H), 7.51 (s, 1H), 7.39 (s, 1H), 6.65 (s, 2H), 5.35 (t, 2H), 5.16 (d, 1H), 5.01 (t, 2H), 4.72 (d, 1H), 4.53 (d, 1H), 4.43 (d, 1H), 3.78 (dt, 1H), 3.41 (m, 2H), 3.25 (m, 1H), 1.91 (q, 1H), 1.25 (m, 1H)

### Example 435

### 4-Amino-12-((5-(2,6-difluorophenyl)pyridin-2-yl)methyl)-3-methyl-1,3,7,8,9,10,11,12-octahydro-13H-[1,5]diamine[7,8-g]furo[3,4-c]quinolin-13-one

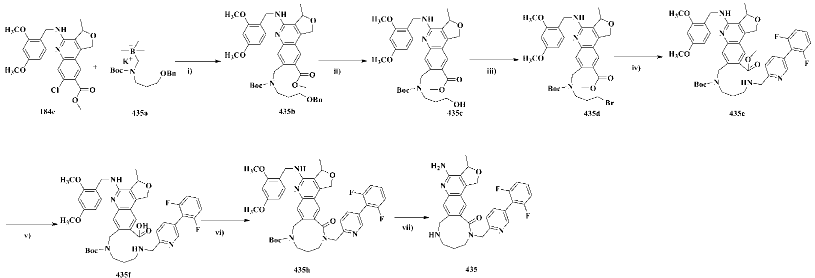

Referring to Step 1 to Step 11 of **Example 178, Example 435** (21 mg, yield: 0.45%) was obtained from methyl 2-methyl-4-oxotetrahydrofuran-3-carboxylate **184e** (4 g, 9.03 mmol) through steps of coupling, deprotection, bromination, substitution, hydrolysis, cyclisation, and deprotection. MS m/z (ESI): 515 [M+1]⁺
¹HNMR (400 MHz, DMSO-d6) δ 8.96 (d, 1H), 8.26 - 8.22 (m, 1H), 7.75 (m, 1H), 7.56(s, 1H), 7.50 (s, 1H), 7.38 (s, 1H), 7.15 (m, 2H), 6.56 (s, 2H), 5.47 - 5.24 (m, 4H), 5.19 (m, 1H), 4.71 (m, 1H), 4.53 (m, 1H), 4.43 (d, 1H), 3.77 (d, 1H), 3.23 (d, 2H), 2.03 - 1.88 (m, 2H), 1.40 (d, 3H).

### Example 437

### 4-Amino-3-methyl-12-((5-(pentafluorosulphanyl)pyridin-2-yl)methyl)-3,7,9,10,11,12-hexahydrofuro[3,4-c][1,5]oxazolo[7,8-g]quinolin-13(1H)-one

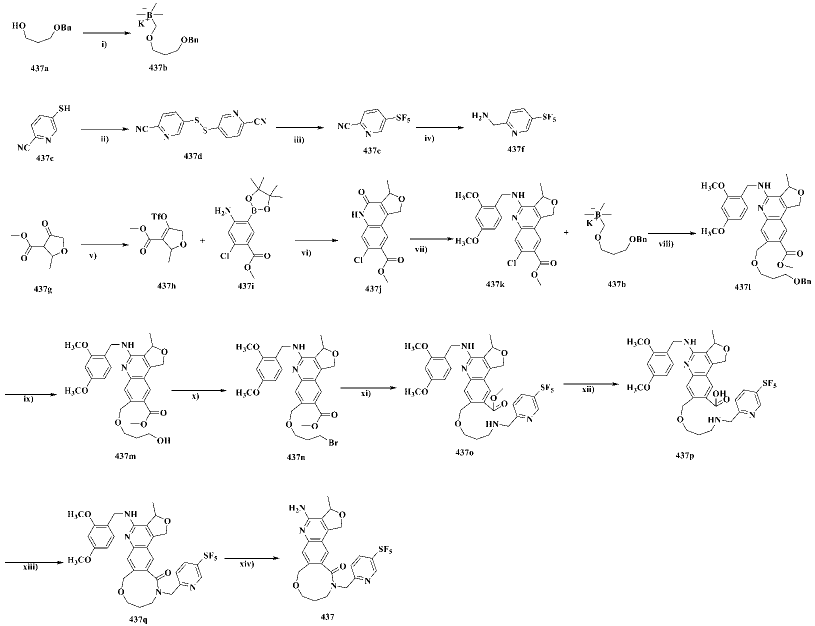

Step 1: 3-(Benzyloxy)propan-1-ol **437a** (20 g, 120.48 mmol) was dissolved in tetrahydrofuran (400 mL), and sodium hydride (5.78 g, 144.57 mmol, 60% purity) was added in an ice bath. The mixture was stirred for 15 minutes, potassium (bromomethyl)trifluoroborate (24.09 g, 120.00 mmol) was then added, and the mixture was stirred and reacted at room temperature for about 16 hours. A potassium bifluoride solution (4.5 M) was added to the reaction solution, and the mixture was stirred at room temperature for 30 minutes. The reaction solution was subjected to rotary evaporation to dryness, and then hot acetone (2000 mL) was added, and the resulting mixture was stirred at 80°C for about 15 minutes. After hot filtration to remove impurities, the acetone was distilled under reduced pressure until solids precipitated. Twice the volume of diethyl ether was added, and the mixture was filtered in an ice bath. The filter residue was dried to obtain **437b** (12.5 g, yield: 37.9%).

Step 2: 2-Cyano-5-mercaptopyridine **437c** (20 g, 147.06 mmol) was dissolved in tetrahydrofuran (400 mL), and iodine (11.12 g, 44.12 mmol) was added in an ice bath. The mixture was stirred and reacted at room temperature for about 16 hours. A saturated sodium thiosulphate solution was added to the reaction solution, and the mixture was stirred at room temperature for 30 minutes, and extracted with ethyl acetate (400 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **437d** (15.7 g, yield: 79.1%). MS m/z (ESI): 271 [M+1]⁺
Step 3: **437d** (15.7 g, 58.15 mmol) was dispersed in acetonitrile (400 mL), and tetraethylammonium chloride (19.19 g, 116.30 mmol) and silver difluoride (134.91 g, 930.40 mmol) were added in an ice bath. The mixture was stirred and reacted at room temperature for about 16 hours. The reaction solution was filtered, the filtrate was concentrated under reduced pressure, and the resulting residue was purified by silica gel column chromatography using eluent system B to obtain **437e** (5.62 g, yield: 21.0%). MS m/z (ESI): 231 [M+1]⁺
Step 4: **437e** (5.62 g, 24.43 mmol) was dispersed in methanol (50 mL), and palladium on carbon (5 g, 10%) was added. The mixture was stirred and reacted at room temperature under hydrogen atmosphere for about 16 hours. The reaction solution was filtered, and the filtrate was concentrated under reduced pressure to obtain **437f** (4.61 g, yield: 80.1%). MS m/z (ESI): 235 [M+1]⁺
Step 5: Methyl 2-methyl-4-oxotetrahydrofuran-3-carboxylate **437g** (50 g, 316.46 mmol) was dissolved in dichloromethane (500 mL), and *N,N-*diisopropylethylamine (64.50 g, 500.00 mmol) and trifluoromethanesulphonic anhydride (116.80 g, 400.00 mmol) were sequentially added in an ice bath. The mixture was stirred and reacted at room temperature for about 16 hours. Water (500 mL) was added to the reaction solution, and the mixture was extracted with ethyl acetate (400 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **437h** (82.5 g, yield: 89.9%).

Step 6: **437h** (40.51 g, 139.69 mmol) and **437i** (40.43 g, 130.00 mmol) were dissolved in dioxane (400 mL) and water (100 mL), and (1,1'-bis(diphenylphosphino)ferrocene)palladium(II) dichloride (4.75 g, 6.50 mmol) and potassium carbonate (53.82 g, 390.00 mmol) were added. The mixture was stirred and reacted at 100°C under nitrogen atmosphere for about 16 hours. The reaction solution was filtered, and the filtrate was extracted with ethyl acetate (200 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **437j** (24.80 g, yield: 65.1%). MS m/z (ESI): 294 [M+1]⁺
Step 7: **437j** (24.80 g, 84.64 mmol) was dissolved in dimethyl sulphoxide (200 mL), and 2,4-dimethoxybenzylamine (26.72 g, 160.00 mmol), 1,8-diazabicyclo[5.4.0]undec-7-ene (36.48 g, 240.00 mmol) and a BOP reagent (70.72 g, 160.00 mmol) were sequentially added. The mixture was stirred and reacted at room temperature under nitrogen atmosphere for about 2 hours. The reaction solution was poured into water (2000 mL) and ethyl acetate (300 mL), and the precipitated solid was filtered to obtain **437k** (26.97 g, yield: 72.1%). MS m/z (ESI): 443 [M+1]⁺
Step 8: **437k** (18.33 g, 41.47 mmol), **437b** (12.5 g, 45.62 mol), [n-butyl-di(1-adamantyl)phosphine](2-amino-1,1'-biphenyl-2-yl)palladium(II) methanesulphonate (4.44 g, 6.10 mol) and sodium carbonate (8.80 g, 83.00 mmol) were dissolved in toluene (200 mL) and water (40 mL), and the mixture was reacted at 100°C under nitrogen atmosphere for 16 hours. The reaction solution was extracted with ethyl acetate (200 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **437l** (16.45 g, yield: 67.7%). MS m/z (ESI): 587 [M+1]⁺
Step 9: **437l** (16.45 g, 28.07 mmol) was dissolved in tetrahydrofuran (200 mL), methanol (100 mL) and acetic acid (3 mL). Palladium on carbon (5 g, 10%) and palladium hydroxide on carbon (5 g, 10%) were added, and the mixture was reacted at 50°C under hydrogen atmosphere for 16 hours. The reaction solution was filtered, the filtrate was concentrated under reduced pressure, and the resulting residue was purified by silica gel column chromatography using eluent system B to obtain **437m** (10.15 g, yield: 72.9%). MS m/z (ESI): 497 [M+1]⁺
Step 10: **437m** (10.15 g, 20.46 mmol) and triphenylphosphine (13.40 g, 51.15 mmol) were dissolved in dichloromethane (200 mL). Carbon tetrabromide (16.93 g, 51.15 mmol) was added, and the mixture was reacted at room temperature under nitrogen atmosphere for 1 hour. The reaction solution was concentrated under reduced pressure, and the resulting residue was purified by silica gel column chromatography using eluent system B to obtain **437n** (9.87 g, yield: 86.4%). MS m/z (ESI): 559 [M+1]⁺
Step 11: **437n** (4.00 g, 7.17 mmol), **437f** (2.01 g, 8.60 mmol), potassium carbonate (4.95 g, 35.85 mmol) and sodium iodide (2.15 g, 14.34 mmol) were dispersed in acetonitrile (40 mL). The mixture was reacted at 80°C under nitrogen atmosphere for 16 hours. The reaction solution was filtered, the filtrate was concentrated under reduced pressure, and the resulting residue was purified by silica gel column chromatography using eluent system B to obtain **437o** (2.21 g, yield: 43.3%). MS m/z (ESI): 713 [M+1]⁺
Step 12: **437o** (2.21 g, 3.10 mmol) was dissolved in methanol (30 mL) and water (10 mL), and lithium hydroxide (372 mg, 15.50 mmol) was added. The mixture was reacted at room temperature under nitrogen atmosphere for 16 hours. The reaction solution was concentrated under reduced pressure to remove most of the solvent, and water (20 mL) was added to the residue. The mixture was adjusted to pH 6 with 6 N hydrochloric acid, and the precipitated solid was filtered to obtain **437p** (1.75 g, yield: 80.8%). MS m/z (ESI): 699 [M+1]⁺
Step 13: **437p** (300 mg, 0.43 mmol) and N,N-diisopropylethylamine (277 mg, 2.15 mmol) were dissolved in N,N-dimethylformamide (20 mL), and O-(7-azabenzotriazol-1-yl)*-N,N,N',N*'-tetramethyluronium hexafluorophosphate (327 mg, 0.86 mmol) was added. The mixture was reacted at room temperature under nitrogen atmosphere for 1 hour. Water (80 mL) was added to the reaction solution, and the mixture was extracted with ethyl acetate (40 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **437q** (210 mg, yield: 71.9%). MS m/z (ESI): 681 [M+1]⁺
Step 14: **437q** (100 mg, 0.15 mmol) was dissolved in trifluoroacetic acid (5 mL). The mixture was reacted at 80°C under nitrogen atmosphere for 1 hour. The reaction solution was concentrated under reduced pressure, and the residue was purified by silica gel column chromatography using eluent system A to obtain **437** (41 mg, yield: 52.6%). MS m/z (ESI): 531 [M+1]⁺
¹H NMR (400 MHz, DMSO-d6) δ 9.11 (d, 1H), 8.40 (dd, 1H), 8.08 (d, 2H), 7.78 (d, 1H), 7.61 (d, 2H), 5.55 - 5.34 (m, 3H), 5.17 (dd, 1H), 4.68 (ddd, 2H), 4.46 (d, 1H), 3.83 (d, 1H), 3.53 - 3.21 (m, 3H), 1.91 (s, 1H), 1.43 (d, 3H), 1.24 (d, 1H).

### Example 437-P1

### (R)-4-Amino-3-methyl-12-((5-(pentafluorosulphanyl)pyridin-2-yl)methyl)-3,7,9,10,11,12-hexahydrofuro[3,4-c][1,5]oxazolo[7,8-g]quinolin-13(1H)-one

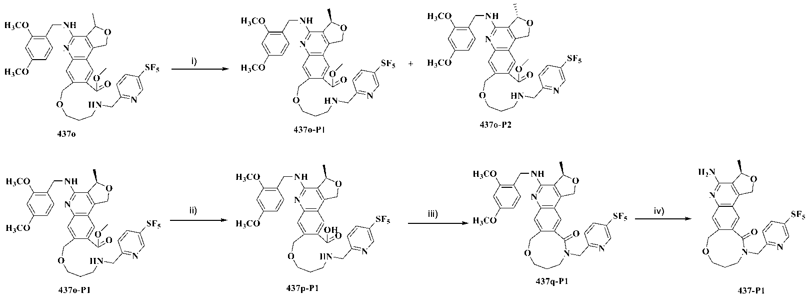

Step 1: Methyl 4-((2,4-dimethoxybenzyl)amino)-3-methyl-7-((3-(((5-(pentafluorosulphanyl)pyridin-2-yl)methyl)amino)propoxy)methyl)-1,3-dihydrofuro[3,4-c]quinoline-8-carboxylate **437o** (1.91 g, 2.68 mmol) was separated by chiral preparative HPLC to obtain methyl (R)-4-((2,4-dimethoxybenzyl)amino)-3-methyl-7-((3-(((5-(pentafluorosulphanyl)pyridin-2-yl)methyl)amino)propoxy)methyl)-1,3-dihydrofuro[3,4-c]quinoline-8-carboxylate **437o-P1** (810 mg, t_{R} = 2.504 min, yield: 42.4%) and methyl (S)-4-((2,4-dimethoxybenzyl)amino)-3-methyl-7-((3-(((5-(pentafluorosulphanyl)pyridin-2-yl)methyl)amino)propoxy)methyl)-1,3-dihydrofuro[3,4-c]quinoline-8-carboxylate **437o-P2** (760 mg, t_{R} = 3.825 min, yield: 39.8%).

Referring to Step 12 to Step 14 of **Example 437,** compound (R)-4-amino-3-methyl-12-((5-(pentafluorosulphanyl)pyridin-2-yl)methyl)-3,7,9,10,11,12-hexahydrofuro[3,4-c][1,5]oxazolo[7,8-g]quinolin-13(1H)-one **437-P1** (129 mg, yield: 21.4%) was obtained from compound methyl (R)-4-((2,4-dimethoxybenzyl)amino)-3-methyl-7-((3-(((5-(pentafluorosulphanyl)pyridin-2-yl)methyl)amino)propoxy)methyl)-1,3-dihydrofuro[3,4-c]quinoline-8-carboxylate **437o-P1** (810 mg, 1.14 mmol). MS m/z (ESI): 531 [M+1]⁺

¹HNMR (400 MHz, DMSO-d6) δ 9.11 (d, 1H), 8.40 (dd, 1H), 8.08 (d, 2H), 7.78 (d, 1H), 7.61 (d, 2H), 5.55 - 5.34 (m, 3H), 5.17 (dd, 1H), 4.68 (ddd, 2H), 4.46 (d, 1H), 3.83 (d, 1H), 3.53 - 3.21 (m, 3H), 1.91 (s, 1H), 1.43 (d, 3H), 1.24 (d, 1H).

### Example 437-P2

### (S)-4-Amino-3-methyl-12-((5-(pentafluorosulphanyl)pyridin-2-yl)methyl)-3,7,9,10,11,12-hexahydrofuro[3,4-c][1,5]oxazolo[7,8-g]quinolin-13(1H)-one

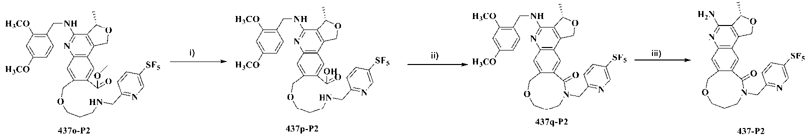

Referring to Step 12 to Step 14 of **Example 437,** compound (S)-4-amino-3-methyl-12-((5-(pentafluorosulphanyl)pyridin-2-yl)methyl)-3,7,9,10,11,12-hexahydrofuro[3,4-c][1,5]oxazolo[7,8-g]quinolin-13(1*H*)-one **437-P2** (115 mg, yield: 20.2%) was obtained from compound methyl (S)-4-((2,4-dimethoxybenzyl)amino)-3-methyl-7-((3-(((5-(pentafluorosulphanyl)pyridin-2-yl)methyl)amino)propoxy)methyl)-1,3-dihydrofuro[3,4-c]quinoline-8-carboxylate **437o-P2** (760 mg, 1.07 mmol). MS m/z (ESI): 531[M+1]

¹HNMR (400 MHz, DMSO-d6) δ 9.11 (d, 1H), 8.40 (dd, 1H), 8.08 (d, 2H), 7.78 (d, 1H), 7.61 (d, 2H), 5.55 - 5.34 (m, 3H), 5.17 (dd, 1H), 4.68 (ddd, 2H), 4.46 (d, 1H), 3.83 (d, 1H), 3.53 - 3.21 (m, 3H), 1.91 (s, 1H), 1.43 (d, 3H), 1.24 (d, 1H).

### Example 484

### 4-Amino-12-((5-(bicyclo[1.1.1]pentan-1-yl)pyridin-2-yl)methyl)-9,10,11,12-tetrahydroimidazo[1,5-a][1,5]oxazolo[8,7-g]quinoxalin-13(7H)-one

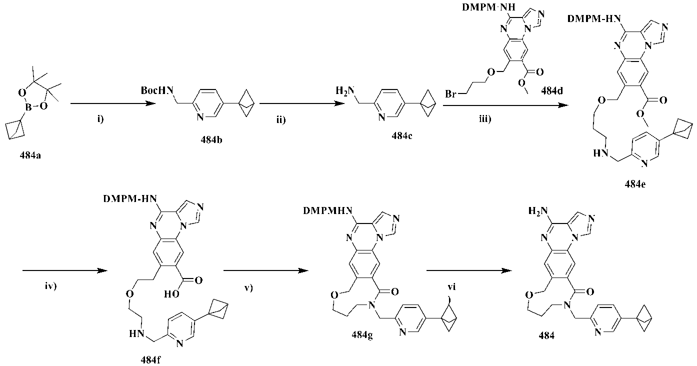

Step 1: tert-Butyl N-[(5-bromo-2-pyridyl)methyl]carbamate (1 g, 3.48 mmol), 2-(bicyclo[1.1.1]pentan-1-yl)-4,4,5,5-tetramethyl-1,3,2-dioxaborolane **484a** (1.01 g, 5.22 mmol, prepared according to a known method: "Science, 2017, vol. 357, p. 283 - 286"), bis[2-(2,4-difluorophenyl)-5-trifluoromethylpyridine][2-2'-bipyridine]iridium(III) hexafluorophosphate (70 mg, 70 µmol) and [4,4'-bis(1,1-dimethylethyl)-2,2'-bipyridine]nickel(II) dichloride (277 mg, 0.7 mmol) were dissolved in N,N-dimethylformamide (20 mL), and the mixture was purged three times with nitrogen. Morpholine (455 mg, 5.22 mmol, 0.47 mL) was added, and the system was irradiated in a blue light reactor (18 w, 456 nm) for 15 hours. The resulting reaction system was quenched with water, and extracted with ethyl acetate (50 mL × 3). The organic phases were combined, washed sequentially with a saturated sodium chloride aqueous solution (30 mL), dried over anhydrous sodium sulphate, filtered, and concentrated. The residue was purified by silica gel column chromatography using eluent system B to obtain **484b** (200 mg, yield: 21.0%). MS m/z (ESI): 275 [M+1]⁺
Step 2: **484b** (0.45 g, 1.64 mmol) and hydrogen chloride in dioxane (5 mL) were dissolved in 1'4-dioxane (5 mL), and the system was reacted at 25°C for 1 hour. The resulting reaction system was concentrated to obtain **484c** (0.28 g, crude). MS m/z (ESI): 175 [M+1]⁺
Referring to the synthesis method described in Step 6 to Step 9 of **Example 1304, Example 484** (10 mg, yield: 4.0%) was obtained from **484c** (106 mg, 0.61 mmol) and methyl 7-((3-bromopropoxy)methyl)-4-(3,4-dimethoxybenzyl)amino)imidazo[1,5-a]quinoxaline-8-carboxylate **484d** (300 mg, 0.55 mmol). MS m/z (ESI): 455 [M+1]⁺
¹H NMR (400 MHz, DMSO-d6) δ 9.21 (s, 1H), 8.41 (d, 1H), 8.07 (s, 1H), 7.91 (s, 1H), 7.67 (dd, 1H), 7.49 (d, 1H), 7.39 (d, 3H), 5.19 (d, 1H), 4.61 (d, 1H), 4.51 (d, 1H), 4.26 (d, 1H), 3.73 (d, 1H), 3.51 (d, 2H), 3.15 (d, 2H), 2.59 (s, 1H), 2.12 (s, 5H), 1.89 (d, 1H), 1.22 (d, 1H).

### Example 493

### 4-Amino-8-methyl-12-((5-(trifluoromethyl)pyridin-2-yl)methyl)-7,8,9,10,11,12-hexahydro-13H-[1,5]diazo[7,8-g]imidazo[1,5-a]quinoxalin-13-one

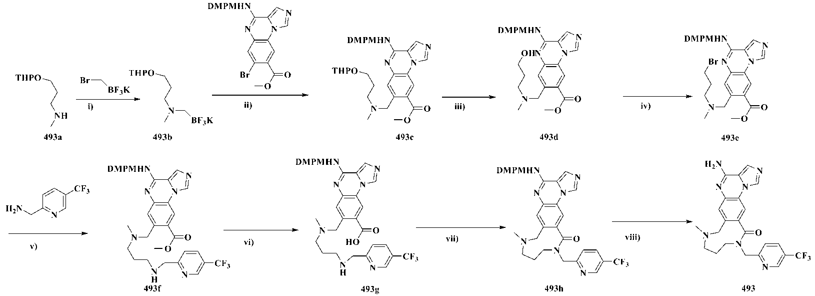

Step 1: **493a** (18 g, 103.89 mmol) was dissolved in tetrahydrofuran (200 mL), and sodium hydride (3.59 g, 89.63 mmol, 60% purity) was added in an ice bath. The mixture was stirred for 15 minutes, potassium (bromomethyl)trifluoroborate (18 g, 89.63 mmol) was then added, and the mixture was stirred and reacted at room temperature for about 16 hours. A potassium bifluoride solution (4.5 M) was added to the reaction solution, and the mixture was stirred at room temperature for 30 minutes. The reaction solution was subjected to rotary evaporation to dryness, and then hot acetone (100 mL) was added, and the resulting mixture was stirred at 80°C for about 15 minutes. After hot filtration to remove impurities, the acetone was distilled under reduced pressure until solids precipitated. Twice the volume of diethyl ether was added, and the mixture was filtered in an ice bath. The filter residue was dried to obtain **493b** (27 g, crude, yield: 100%).

Step 2: Methyl 7-bromo-4-((3,4-dimethoxybenzyl)amino)imidazo[1,5-a]quinoxaline-8-carboxylate (4.5 g, 9.55 mmol), **493b** (4.48 g, 15.28 mmol), [n-butyl-di(1-adamantyl)phosphine](2-amino-1,1'-biphenyl-2-yl)palladium(II) methanesulphonate (347.68 mg, 477.40 µmol) and caesium carbonate (9.34 g, 28.64 mmol) were dissolved in dioxane (50 mL) and water (10 mL), and the mixture was reacted at 100°C under nitrogen atmosphere for 16 hours. Water (100 mL) was added to the reaction solution, and the mixture was extracted with ethyl acetate (100 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **493c** (5.52 g, crude), which was used directly in the next step. MS m/z (ESI): 578 [M+1]⁺
Step 3: **493c** (5.4 g, 9.35 mmol) was added to a single-necked flask containing methanol (20 mL) and tetrahydrofuran (20 mL), and the mixture was adjusted to pH=7 with HCl (2 M, 46.74 mL) under nitrogen atmosphere, and stirred at room temperature for 2 hours. The reaction was quenched by adding a saturated ammonium chloride solution (100 mL) to the reaction solution, and extracted with ethyl acetate (100 mL × 2). After extraction, the organic phase was separated, dried over anhydrous sodium sulphate, and filtered. The filtrate was concentrated under reduced pressure, and the resulting residue was purified by silica gel column chromatography using eluent system C to obtain **493d** (1.1 g, 2.23 mmol, yield: 23.8%). MS m/z (ESI): 494 [M+1]⁺
Step 4: **493d** (1.1 g, 2.23 mmol) was dissolved in dichloromethane (20 mL), and the mixture was purged three times with nitrogen and cooled to 0°C. Triphenylphosphine (876.85 mg, 3.34 mmol) and carbon tetrabromide (1.11g, 3.34 mmol) were then added, and the system was reacted at 25°C for 2 hours. The reaction solution was distilled under reduced pressure, and the resulting residue was purified by silica gel column chromatography using eluent system C to obtain **493e** (0.24 g, 431.31 µmol, yield: 19.4%). MS m/z (ESI): 556/558 [M+1]⁺
Step 5: **493e** (0.24 g, 431.31 µmol), (5-(trifluoromethyl)pyridin-2-yl)methanamine (151.94 mg, 862.61 µmol), sodium iodide (129.30 mg, 862.61 µmol) and potassium carbonate (119.04 mg, 862.61 µmol) were dissolved in acetonitrile (10 mL), and the mixture was purged three times with nitrogen. The reaction system was reacted at 80°C for 15 hours. The reaction solution was quenched with water, extracted with ethyl acetate (50 mL × 2), and washed with a saturated sodium chloride aqueous solution (50 mL × 2). The organic phase was dried over anhydrous sodium sulphate, the filtrate was concentrated under reduced pressure, and the resulting residue was purified by silica gel column chromatography using eluent system C to obtain **493f** (0.13 g, 199.49 µmol, yield: 46.3%). MS m/z (ESI): 652 [M+1]⁺
Step 6: **493f** (0.13 g, 199.49 µmol) was dissolved in tetrahydrofuran (5 mL), water (5 mL) and methanol (5 mL), lithium hydroxide (33.51 mg, 797.94 µmol) was added, and the mixture was purged three times with nitrogen. The reaction system was reacted at 25°C for 16 hours. The resulting reaction system was adjusted to pH 5-6 with 1 M dilute hydrochloric acid, extracted with ethyl acetate (50 mL × 3), and washed with a saturated sodium chloride aqueous solution (50 mL × 2). The organic phase was dried over anhydrous sodium sulphate, and the filtrate was concentrated under reduced pressure to obtain **493g** (0.08 g, 125.46 µmol, yield: 62.9%). MS m/z (ESI): 638 [M+1]⁺
Step 7: **493g** (0.08 g, 125.46 µmol) and 2-(7-azobenzotriazol-1-yl)-N,N,N',N'-tetramethyluronium hexafluorophosphate (95.35 mg, 250.92 µmol) were dissolved in N,N-dimethylformamide (5 mL), N,N-diisopropylethylamine (97.29 mg, 752.76 µmol, 131.12 µL) was added, and the reaction system was reacted at 25°C for 2 hours. The resulting reaction system was quenched with water, extracted with ethyl acetate (80 mL × 3), washed with a saturated sodium chloride aqueous solution, and dried over anhydrous sodium sulphate, and the filtrate was concentrated under reduced pressure. The residue was purified by silica gel column chromatography using eluent system A to obtain **493h** (0.076 g, 122.65 µmol, crude), which was directly used in the next step. MS m/z (ESI): 620 [M+1]⁺
Step 8: **493h** (76.07 mg, 122.76 µmol) was dissolved in trifluoroacetic acid (5 mL), and the reaction system was reacted at 90°C for 0.5 hours. The resulting reaction system was concentrated, and the residue was purified by Pre-HPLC to obtain **Example 493** (3 mg, 6.39 µmol, yield: 5.2%). MS m/z (ESI): 470 [M+1]⁺
¹H NMR (400 MHz, DMSO-d6) δ 9.19 (s, 1H), 8.97 (s, 1H), 8.22 (dd, 1H), 8.01 (s, 1H), 7.90 (s, 1H), 7.84 (d, 1H), 7.36 (s, 2H), 7.32 (s, 1H), 5.16 (d, 1H), 4.41 (d, 1H), 3.80 (d, 1H), 3.55 (s, 1H), 3.25 (d, 4H), 2.33 (s, 3H), 1.36 - 0.93 (m, 2H).

### Example 502

### 4-Amino-1,8-dimethyl-12-((5-(trifluoromethyl)pyridin-2-yl)methyl)-7,8,9,10,11,12-hexahydro-[1,5]diazonino[7,8-g]pyrazolo[4,3-c]quinolin-13(1H)-one

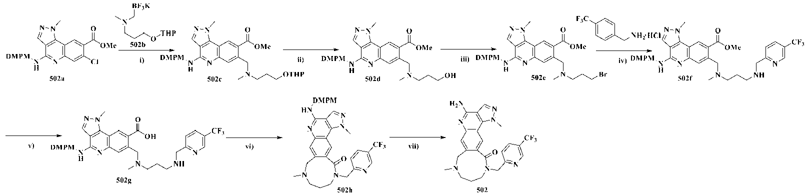

Step 1: A solution of methyl 7-chloro-4-((2,4-dimethoxybenzyl)amino)-1-methyl-1H-pyrazolo[4,3-c]quinoline-8-carboxylate **502a** (800 mg, 1.65 mmol), potassium trifluoro((methyl(3-((tetrahydro-2H-pyran-2-yl)oxy)propyl)amino) methyl)borate **502b** (725 mg, 2.47 mmol), caesium carbonate (1.61 g, 4.95 mmol), and CataCxiumA-Pd-G3 (120.05 mg, 0.16 mmol) in 1,4-dioxane (15 mL) and water (3 mL) was stirred at 100°C under nitrogen atmosphere for 4 hours. The reaction solution was filtered to remove solids, and the filtrate was diluted with ethyl acetate (250 mL), and washed with water and a sodium chloride aqueous solution. The organic phase was concentrated, and the residue was purified by silica gel column chromatography to obtain **502c** (602 mg, yield: 61.7%). MS m/z (ESI): 592 [M+1]⁺
Step 2: 4 N hydrochloric acid (0.20 mL, 5.00 mmol) was added to a solution of **502c** (602 mg, 1.02 mmol) in methanol (15 mL), and the mixture was stirred at 20°C for 2 hours. Upon completion of the reaction, the reaction system was quenched with a sodium bicarbonate solution, extracted with DCM, dried, and subjected to rotary evaporation to dryness and column chromatography to obtain **502d** (515 mg, yield: 99.7%). MS m/z (ESI): 508 [M+1]⁺
Referring to the synthesis method described in Step 5 to Step 9 of **Example 62, Example 502** (6.5 mg, yield: 1.3%) was obtained from **502d** (515 mg, 1.01 mmol). MS m/z (ESI): 484 [M+1]⁺

¹H NMR (400 MHz, DMSO-d6) δ 8.89 (s, 1H), 8.63 - 8.36 (m, 3 H), 8.22 - 8.11 (m, 1 H), 8.01 - 7.54 (m, 2 H), 7.31 - 7.11 (m, 1 H), 6.30 - 5.91 (m, 1 H), 5.71 - 5.44 (m, 1 H), 5.41 - 5.22 (m, 1 H), 5.14 - 4.85 (m, 2 H), 4.57 - 4.41 (m, 3 H), 4.34 - 4.06 (m, 2 H), 3.52 - 3.17 (m, 3 H), 2.58 - 2.52 (m, 2 H), 2.29 - 2.15 (m, 1 H).

### Example 503

### 4-Amino-1-methyl-12-((3-(trifluoromethyl)bicyclo[1.1.1]pentan-1-yl)methyl)-1,7,9,10,11,12-hexahydro-13H-[1,5]oxazolo[7,8-g]pyrazolo[4,3-c]quinolin-13-one

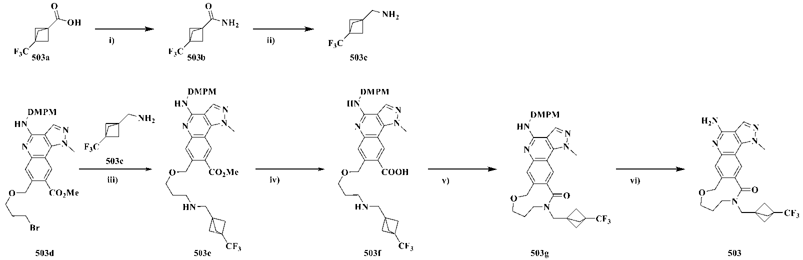

Step 1: 3-(Trifluoromethyl)bicyclo[1.1.1]pentane-1-carboxylic acid **503a** (5 g, 27.76 mmol) was dissolved in DCM (50 mL), oxaloyl chloride (4.58 g, 36.09 mmol, 3 mL) was added, and the mixture was stirred at 25°C for 2 hours. A solution of ammonia in methanol (7 M, 11.90 mL) was added, and the resulting mixture was purged three times with nitrogen, and stirred and reacted at 25°C under nitrogen atmosphere for 16 hours. The reaction solution was concentrated under reduced pressure, and water (100 mL) was added to the concentrated residue. The mixture was stirred for 15 minutes and filtered, and the filter cake was azeotroped with ethanol (60 mL) and dried under reduced pressure to obtain **503b** (3.5 g, yield: 70.4%).

¹H NMR (400 MHz, DMSO-d6) δ 7.45 (s, 1H), 7.15 (s, 1H), 2.12 (s, 6H).

Step 2: **503b** (2.2 g, 12.28 mmol) was dissolved in THF (30 mL), and the reaction system was cooled to 0°C. LiAlH₄ (2.5 M, 14.7 mL) was slowly added, and the mixture was purged three times with nitrogen, and stirred and reacted at 25°C under nitrogen atmosphere for 16 hours. A sodium hydroxide aqueous solution (6 M, 10 mL) was slowly added to the reaction solution, and the mixture was filtered. The filter cake was rinsed with diethyl ether (30 mL), and the filtrate was collected, filtered, and concentrated under reduced pressure (without being subjected to rotary evaporation to dryness). Diethyl ether (10 mL) was added to the concentrated residue, followed by hydrogen chloride in ethyl acetate (7 M, 6 mL), and the resulting mixture was stirred at 25°C for 15 minutes and filtered. The filter cake was collected and dried under reduced pressure to obtain **503c** (900 mg, yield: 44.4%).

¹H NMR (400 MHz, DMSO-d6) δ 3.45 (d, 2H), 2.99 (d, 2H),1.99 (s, 6H).

Step 3: Compound methyl 7-((3-bromopropoxy)methyl)-4-(3,4-dimethoxybenzyl)amino)-1-methyl-1H-pyrazolo[4,3-c]quinoline-8-carboxylate **503d** (200 mg, 359 µmol) and **503c** (108.51 mg, 538 µmol) were dissolved in MeCN (2 mL), and NaI (107.56 mg, 718 µmol) and K₂CO₃ (198.34 mg, 1.44 mmol) were sequentially added. The mixture was purged three times with nitrogen, and stirred and reacted at 80°C under nitrogen atmosphere for 16 hours. The reaction solution was filtered, the filtrate was concentrated under reduced pressure, and the concentrated residue was separated and purified by Pre-HPLC to obtain **503e** (90 mg, yield: 29.0%). MS m/z (ESI): 642 [M+1]⁺
Step 4: **503e** (80 mg, 125 µmol) was dissolved in THF (0.6 mL)/MeOH (0.2 mL)/water (0.2 mL), and LiOH (11.94 mg, 499 µmol) was added. The mixture was purged three times with nitrogen, and stirred and reacted at 25°C under nitrogen atmosphere for 16 hours. The reaction solution was concentrated under reduced pressure, and water (6 mL) was added to the concentrated residue. The mixture was adjusted to pH 4-5 with 2 M hydrochloric acid and filtered, and the filter cake was dried under reduced pressure to obtain **503f** (90 mg, crude), which was used directly in the next step. MS m/z (ESI): 629 [M+1]⁺
Step 5: **503f** (85 mg, 135 µmol) was dissolved in DMF (456 µL), and DIEA (35.01 mg, 271 µmol, 47 µL) and HATU (76.64 mg, 203 µmol) were sequentially added. The mixture was purged three times with nitrogen, and stirred and reacted at 25°C under nitrogen atmosphere for 5 hours. The reaction solution was concentrated under reduced pressure to obtain **503g** (90 mg, crude).
Step 6: **503g** (85 mg, 139 µmol) was dissolved in TFA (1 mL), and the mixture was purged three times with nitrogen, and stirred and reacted at 80°C under nitrogen atmosphere for 0.5 hours. The reaction solution was concentrated under reduced pressure, and the concentrated residue was separated and purified by Pre-HPLC to obtain **Example 503** (10 mg). MS m/z (ESI): 460 [M+1]⁺
¹H NMR (400 MHz, DMSO-*d6*)δ 13.34 (s, 2H), 8.53 (s, 1H), 7.98 (s, 1H), 7.64 (s, 1H), 4.70 (d, 1H), 4.54 (d, 1H), 4.46 (s, 2H), 4.10 (d, 1H), 3.84 - 3.74 (m, 1H), 3.37 (dd, 3H), 3.31 - 3.09 (m, 4H), 2.05 - 1.98 (m, 4H), 1.86 (q, 1H), 1.22 (d, 1H).

### Example 508

### 4-Amino-8-methyl-12-((5-(trifluoromethyl)pyridin-2-yl)methyl)-1,3,7,8,9,10,11,12-octahydro-13H-[1,5]diazonino[7,8-glfuro[3,4-c]quinolin-13-one

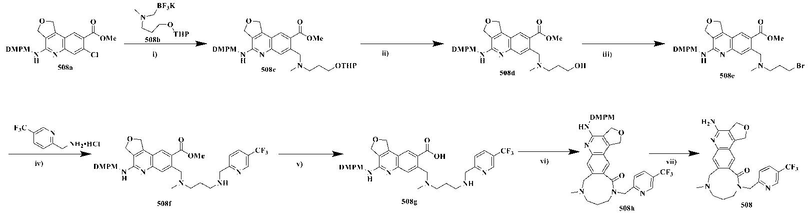

Step 1: A solution of methyl 7-chloro-4-((2,4-dimethoxybenzyl)amino)-1,3-dihydrofuro[3,4-c]quinoline-8-carboxylate **508a** (1.0 g, 2.33 mmol), potassium trifluoro((methyl(3-((tetrahydro-2H-pyran-2-yl)oxy)propyl)amino)methyl)borate **508b** (1.03 g, 3.50 mmol), caesium carbonate (2.28 g, 7.00 mmol), and CataCxiumA-Pd-G3 (169.81 mg, 0.23 mmol) in 1,4-dioxane (15 mL) and water (3 mL) was stirred at 100°C under nitrogen atmosphere for 4 hours. The reaction solution was filtered to remove solids, and the filtrate was diluted with ethyl acetate (250 mL), and washed with water and a sodium chloride aqueous solution. The organic phase was concentrated, and the residue was purified by silica gel column chromatography to obtain **508c** (289 mg, yield: 21.4%). MS m/z (ESI): 580 [M+1]⁺
Step 2: 4 N hydrochloric acid (0.20 mL, 5.00 mmol) was added to a solution of **508c** (550 mg, 0.95 mmol) in methanol (15 mL), and the mixture was stirred at 20°C for 2 hours. Upon completion of the reaction, the reaction system was quenched with a sodium bicarbonate solution, extracted with DCM, dried, and subjected to rotary evaporation to dryness and column chromatography to obtain **508d** (460 mg, yield: 97.8%). MS m/z (ESI): 496 [M+1]⁺
Referring to the synthesis method described in Step 5 to Step 9 of **Example 62, Example 508** (35.2 mg, yield: 8.0%) was obtained from **508d** (460 mg, 0.93 mmol). MS m/z (ESI): 472 [M+1]⁺
¹H NMR (400 MHz, DMSO-d6) δ 9.01 (br s, 1H), 8.27 (br d, 1 H), 8.05 - 7.56 (m, 3 H), 5.50 - 5.37 (m, 2 H), 5.21 - 4.86 (m, 4 H), 4.85 - 4.31 (m, 2 H), 3.25 - 2.96 (m, 4 H), 2.93 - 2.78 (m, 1 H), 2.74 - 2.63 (m, 4 H) ppm.

### Example 510

### 4-Amino-12-((5-(bicyclo[1.1.1]pentan-1-yl)pyridin-2-yl)methyl)-3,7,9,10,11,12-hexahydrofuro[3,4-c][1,5]oxazolo[7,8-g]quinolin-13(1H)-one

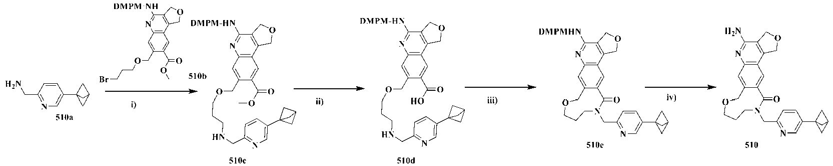

Referring to the synthesis method described in Step 5 to Step 8 of **Example 177, Example 510** (5 mg, yield: 1.2%) was obtained from (5-(bicyclo[1.1.1]pentan-1-yl)pyridin-2-yl)methanamine **510a** (240 mg, 1.38 mmol) and methyl 7-((3-bromopropoxy)methyl)-4-(3,4-dimethoxybenzyl)amino)-1,3-dihydrofuro[3,4-c]quinoline-8-carboxylate **510b** (500 mg, 0.92 mmol). MS m/z (ESI): 457 [M+1]⁺
¹H NMR (400 MHz, DMSO-d6)δ 8.40 (d, 1H), 7.66 (dd, 1H), 7.50 (s, 1H), 7.44 (d, 1H), 7.35 (s, 1H), 6.63 (s, 2H), 5.34 (s, 2H), 5.18 (d, 1H), 5.01 (s, 2H), 4.68 (d, 1H), 4.55-4.45 (m, 2H), 4.21 (d, 1H), 3.73 (d, 1H), 3.13 (d, 3H), 2.57 (d, 1H), 2.15-2.04 (m, 7H).

### Example 514

### 4-Amino-8-methyl-12-((5-(trifluoromethyl)pyridin-2-yl)methyl)-7,8,9,10,11,12-hexahydro-13H-azo[4,3-g]imidazo[1,5-a]quinoxalin-13-one

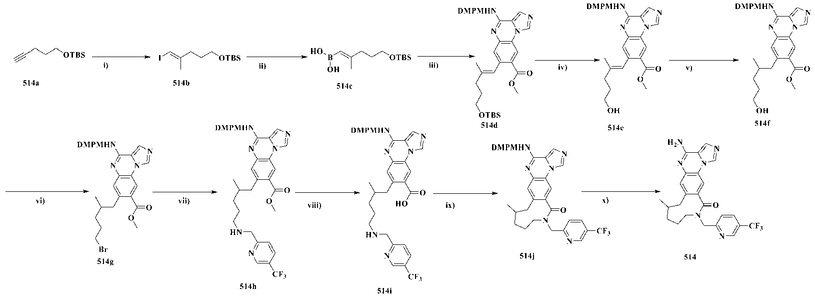

Step 1: Trimethylaluminium (42.7 mL, 68.3 mmol, 1.6 M) was added dropwise to a solution of zirconocene dichloride (1.20 g, 4.11 mmol) in dichloromethane (34 mL) at -70°C under nitrogen atmosphere, and then deionised water (185 mg, 10.3 mmol) was added dropwise to the reaction solution. The reaction solution was stirred at -70°C for 0.5 hours, and then heated to room temperature and stirred for another 0.5 hours. The reaction solution was cooled to -70°C, and tert-butyl-dimethyl-4-ynyloxysilane (4.06 g, 20.47 mmol) was added dropwise to the reaction solution. The mixture was then heated to room temperature and stirred for 1.5 hours. The reaction solution was cooled to -70°C, and a solution of iodine (10.4 g, 40.98 mmol) in tetrahydrofuran (28 mL) was added dropwise to the reaction solution. The mixture was stirred at -70°C for 0.5 hours, and then heated to room temperature and stirred for another 1.5 hours. The reaction solution was slowly quenched with a sodium thiosulphate solution, and extracted with diethyl ether (100 mL × 3). The organic phases were combined and concentrated. The residue was purified by silica gel column chromatography to obtain **514b** (5.30 g, yield: 76.1%).

Step 2: n-Butyllithium (5.82 mL, 2.5 M) was added dropwise to a solution of **514b** (3.30 g, 9.70 mmol) and 2,2'-bipyridine (76 mg, 0.48 mmol) in tetrahydrofuran (32 mL) at -70°C under nitrogen atmosphere. The reaction solution was stirred at - 70°C for 0.5 hours. Triisopropyl borate (2.74 g, 14.55 mmol) was added dropwise to the reaction solution, and the mixture was heated to 0°C and stirred for 1 hour. The reaction solution was quenched with a saturated ammonium chloride aqueous solution, extracted with ethyl acetate (100 mL × 3), washed with a saturated sodium chloride aqueous solution (30 mL), dried over anhydrous sodium sulphate, filtered, and concentrated. The residue was purified by silica gel column chromatography to obtain **514c** (1.50 g, yield: 59.9%). MS m/z (ESI): 259 [M+1]⁺

Step 3: Methyl 7-bromo-4-[(3,4-dimethoxyphenyl)methylamino]imidazo[1,5-a]quinoxaline-8-carboxylate (2.11 g, 4.47 mmol), **514c** (1.50 g, 5.81 mmol), sodium carbonate (947 mg, 8.94 mmol) and 1,1'-bis(diphenylphosphino)ferrocene palladium(II) dichloride (327 mg, 0.45 mmol) were dissolved in water (3 mL) and dioxane (20 mL), and the mixture was purged three times with nitrogen, and stirred at 90°C for 2 hours. The reaction solution was concentrated, and the residue was purified by silica gel column chromatography to obtain **514d** (2.10 g, yield: 77.7%). MS m/z (ESI): 605 [M+1]⁺

Step 4: **514d** (2.00 g, 3.31 mmol) and tetrabutylammonium fluoride (6.61 mL, 1 M) were dissolved in tetrahydrofuran (10 mL). The reaction solution was stirred at room temperature for 16 hours. Upon completion of the reaction, the reaction solution was concentrated, and the residue was purified by silica gel column chromatography to obtain **514e** (1.20 g, yield: 73.9%). MS m/z (ESI): 491 [M+1]⁺

Step 5: **514e** (1.00 g, 2.04 mmol) and platinum dioxide (93 mg, 0.41 mmol) were dissolved in methanol (20 mL) and acetic acid (2.5 mL). The reaction solution was stirred at room temperature for 48 hours. Upon completion of the reaction, the reaction solution was filtered to remove solids and concentrated, and the residue was purified by silica gel column chromatography to obtain **514f** (860 mg, yield: 85.6%). MS m/z (ESI): 493 [M+1]⁺

Step 6: A solution of carbon tetrabromide (1.02 g, 3.09 mmol) in dichloromethane (3 mL) was added dropwise to a solution of **514f** (760 mg, 1.54 mmol) and triphenylphosphine (809 mg, 3.09 mmol) in dichloromethane (8 mL) at room temperature under nitrogen atmosphere. The reaction solution was stirred at room temperature for 16 hours. Upon completion of the reaction, the reaction solution was concentrated, and the residue was purified by silica gel column chromatography to obtain **514g** (660 mg, yield: 77.0%). MS m/z (ESI): 555 [M+1]⁺

Referring to the synthesis method of **Example 510, Example 514** (20 mg, yield:11.8%) was obtained from **514g** (200 mg, 0.36 mmol). MS m/z (ESI): 469 [M+1]⁺
¹H NMR (400 MHz, MeOD) δ 9.25 (s, 1H), 8.89 (s, 1H), 8.35 (s, 1H), 8.22 (d, 1H), 8.16 (dd, 1H), 7.74 (d, 1H), 7.53 (s, 1H), 5.22 (d, 1H), 4.71 (d, 1H), 3.51 (dd, 1H), 3.20 - 3.12 (m, 1H), 2.87 (dd, 1H), 2.57 (dd, 1H), 2.19 (t, 1H), 1.90 - 1.83 (m, 1H), 1.56 (dd, 3H), 1.16 (d, 3H).

### Examples 514-P1 & 514-P2

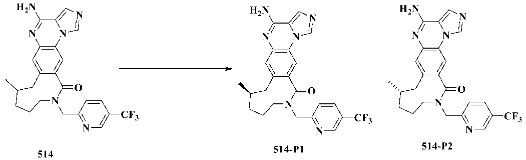

**Example 514** (42 mg, 0.09 mmol) was separated by chiral preparative HPLC to obtain (R)-4-amino-8-methyl-12-((5-(trifluoromethyl)pyridin-2-yl)methyl)-7,8,9,10,11,12-hexahydro-13H-azo[4,3-g]imidazo[1,5-a]quinoxalin-13-one **514-P1** (15 mg, yield: 35.7%) and (S)-4-amino-8-methyl-12-((5-(trifluoromethyl)pyridin-2-yl)methyl)-7,8,9,10,11,12-hexahydro-13H-azo[4,3-g]imidazo[1,5-a]quinoxalin-13-one **514-P2** (15 mg, yield: 35.7%).

**514-P1 (t_{R}: 1.902 min):** ¹H NMR (400 MHz, MeOD) δ 9.25 (s, 1H), 8.89 (s, 1H), 8.35 (s, 1H), 8.22 (d, 1H), 8.16 (dd, 1H), 7.74 (d, 1H), 7.53 (s, 1H), 5.22 (d, 1H), 4.71 (d, 1H), 3.51 (dd, 1H), 3.20 - 3.12 (m, 1H), 2.87 (dd, 1H), 2.57 (dd, 1H), 2.19 (t, 1H), 1.86 (m, 1H), 1.56 (dd, 3H), 1.16 (d, 3H).

**514-P2 (t_{R}: 3.117 min):** ¹H NMR (400 MHz, MeOD) δ 9.25 (s, 1H), 8.89 (s, 1H), 8.35 (s, 1H), 8.22 (d, 1H), 8.16 (dd, 1H), 7.74 (d, 1H), 7.53 (s, 1H), 5.22 (d, 1H), 4.71 (d, 1H), 3.51 (dd, 1H), 3.20 - 3.12 (m, 1H), 2.87 (dd, 1H), 2.57 (dd, 1H), 2.19 (t, 1H), 1.86 (m, 1H), 1.56 (dd, 3H), 1.16 (d, 3H).

### Example 555

### 4-Amino-12-(but-2-yn-1-yl)-3,7,9,10,11,12-hexahydrofuro[3,4-c] [1,5] oxazolo [7,8-g] quinolin-13(1H)-one

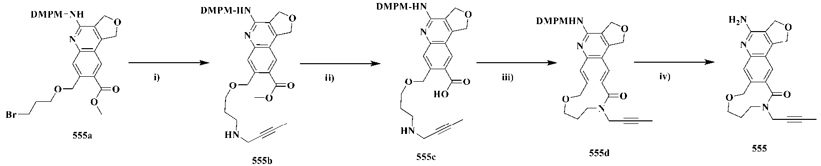

Referring to the synthesis method of **Example 173, Example 555** (38 mg, yield: 11.8%) was obtained from methyl 7-((3-bromopropoxy)methyl-4-(3,4-dimethoxybenzyl)amino)-1,3-dihydrofuro[3,4-c]quinoline-8-carboxylate **555a** (500 mg, 0.92 mmol) and prop-1-yn-1-amine (101 mg, 1.83 mmol). MS m/z (ESI): 352 [M+1]⁺
¹H NMR (400 MHz, DMSO-*d6*)δ 7.51 (s, 1H), 7.38 (s, 1H), 6.99 (s, 2H), 5.35 (t, 2H), 5.02 (d, 2H), 4.72 (dt, 2.6 Hz, 1H), 4.62-4.50 (m, 2H), 3.83 (dq, 1H), 3.73 (d, 1H), 3.47-3.38 (m, 2H), 3.22 (t, 1H), 2.00-1.78 (m, 4H), 1.23 (d, 1H).

### Example 556

### 4-Amino-1-methyl-12-((3-(trifluoromethyl)bicyclo[1.1.1]pentan-1-yl)methyl)-1,7,9,10,11,12-hexahydro-13H-[1,5]oxazolo[7,8-g]pyrazolo[4,3-c]quinolin-13-one

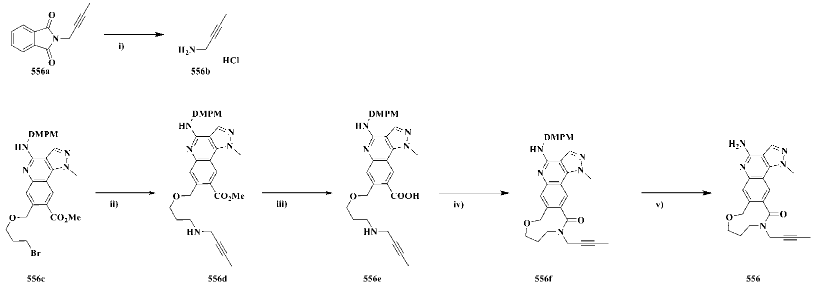

Step 1: 2-But-2-ynyl-octadiene-1,3-dione **556a** (10 g, 50.20 mmol) was dissolved in DCM (100 mL), and NH₂NH₂ (2.41 g, 75.30 mmol, 2.5 mL) was added. The mixture was purged three times with nitrogen, and stirred and reacted at 25°C under nitrogen atmosphere for 16 hours. The reaction solution was filtered, the filter cake was rinsed with diethyl ether (60 mL), and the filtrate was concentrated under reduced pressure (without being subjected to rotary evaporation to dryness). Diethyl ether (20 mL) was added to the concentrated residue, followed by a solution of hydrogen chloride in ethyl acetate (7 M) (20 mL). The mixture was filtered, and the filter cake was dried under reduced pressure to obtain **556b** (1.5 g, yield: 28.3%).

¹H NMR (400 MHz, DMSO-d6) δ 8.58 (s, 3H), 3.59 (dq, 2H), 1.82 (t, 3H).

Step 2: Methyl 7-(3-bromopropoxymethyl)-4-[(2,4-dimethoxyanilino)methyl]-1-methylpyrazolo[4,3-c]quinoline-8-carboxylate **556c** (500 mg, 781 µmol) and **556b** (123.57 mg, 1.17 mmol) were dissolved in MeCN (2 mL), and K₂CO₃ (431.40 mg, 3.12 mmol) and NaI (233.94 mg, 1.56 mmol) were sequentially added. The mixture was purged three times with nitrogen, and stirred and reacted at 80°C under nitrogen atmosphere for 16 hours. The reaction solution was filtered, the filtrate was concentrated under reduced pressure, and the concentrated residue was separated and purified by Pre-HPLC to obtain **556d** (120 mg, yield: 25.6%). MS m/z (ESI): 546 [M+1]⁺

Step 3: **556d** (120 mg, 220 µmol) was dissolved in THF (3 mL)/MeOH (1 mL)/water (1 mL), and LiOH (21.07 mg, 880 µmol) was added. The mixture was purged three times with nitrogen, and stirred and reacted at 25°C under nitrogen atmosphere for 16 hours. The reaction solution was concentrated under reduced pressure, and water (6 mL) was added to the concentrated residue. The mixture was adjusted to pH 4-5 with 2 M hydrochloric acid, and extracted with ethyl acetate (10 mL × 3). The organic phases were combined, dried over anhydrous sodium sulphate, filtered, and concentrated under reduced pressure to obtain **556e** (120 mg, crude). MS m/z (ESI): 533 [M+1]⁺

Step 4: **556e** (110 mg, 207 µmol) was dissolved in DMF (2 mL), and DIEA (80.23 mg, 621 µmol, 108 µL) and HATU (156.13 mg, 414 µmol) were sequentially added. The mixture was purged three times with nitrogen, and stirred and reacted at 25°C under nitrogen atmosphere for 5 hours. The reaction solution was concentrated under reduced pressure to obtain **556f** (120 mg, crude). MS m/z (ESI): 514 [M+1]⁺

Step 5: **556f** (110 mg, 214 µmol) was dissolved in TFA (1 mL), and the mixture was stirred and reacted at 80°C for 0.5 hours. The reaction solution was concentrated under reduced pressure, and the concentrated residue was separated and purified by Pre-HPLC to obtain **Example 556** (20 mg, yield: 23.6%). MS m/z (ESI): 364 [M+1]⁺

¹H NMR (400 MHz, DMSO-d6) δ 9.11 (d, 1H), 8.40 (dd, 1H), 7.78 (d, 1H), 7.67 (s, 1H), 7.61 (s, 1H), 5.46 (t, 2H), 5.22 - 5.03 (m, 3H), 4.75 (d, 1H), 4.61 (d, 1H), 4.46 (d, 1H), 3.83 (dd, 1H), 3.31 (s, 3H), 2.03 - 1.85 (m, 1H), 1.25 (d, 2H).

### Example 557

### 4-Amino-12-((3-(trifluoromethyl)bicyclo[1.1.1]pentan-1-yl)methyl)-3,7,9,10,11,12-hexahydrofuro[3,4-c][1,5]oxazolo[7,8-g]quinolin-13(1H)-one

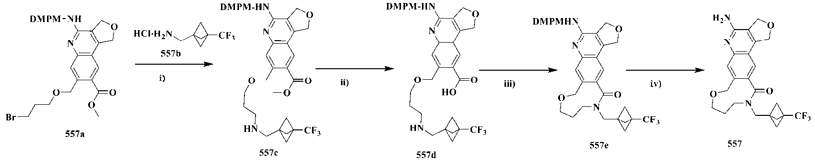

Referring to the synthesis method of **Example 173, Example 557** (35 mg, yield: 8.5%) was obtained from methyl 7-((3-bromopropoxy)methyl-4-(3,4-dimethoxybenzyl)amino)-1,3-dihydrofuro[3,4-c]quinoline-8-carboxylate **557a** (500 mg, 0.92 mmol) and (3-(trifluoromethyl)bicyclo[1.1.1]pentan-1-yl)methanamine hydrochloride **557b** (182 mg, 1.10 mmol). MS m/z (ESI): 448 [M+1]⁺

¹H NMR (400 MHz, DMSO-d6) δ 7.98 (s, 2H), 7.60 (s, 1H), 7.47 (s, 1H), 5.45 (s, 2H), 5.08 (d, 2H), 4.66 (d, 1H), 4.53 (d, 1H), 4.06 (d, 1H), 3.75 (d, 1H), 3.20-2.93 (m, 5H), 2.01 (q, 5H), 1.85 (d, 1H), 1.20 (d, 1H).

### Example 638

### (Z)-4-Amino-11-((5-(trifluoromethyl)pyridin-2-yl)methyl)-3,9,10,11-tetrahydronaphthyrido[3,4-g]furo[3,4-e]quinoxalin-12(1H)-one

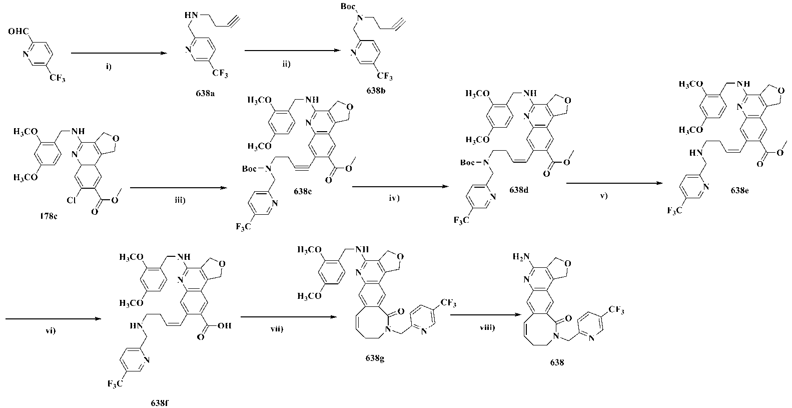

Referring to Step 1 to Step 5 of **Example 331, 638e** (250 mg, yield: 30.6%) was obtained from methyl 7-chloro-4-((2,4-dimethoxyphenyl)amino)-1,3-dihydrofuro[3,4-c]quinoxaline-8-carboxylate **178c** (560 mg, 1.31 mmol) and tert-butyl 3-yn-1-yl((5-(trifluoromethyl)pyridin-2-yl)methyl)carbamate **638b** (428 mg, 1.31 mmol) through steps of hydrogenation and deprotection. MS m/z (ESI): 623 [M+1]⁺
Step 6: **638e** (280 mg, 0.38 mmol) and lithium hydroxide (28 mg, 1.16 mmol) were dissolved in methanol (2 mL), tetrahydrofuran (2 mL), and water (2 mL), and the reaction system was reacted at 50°C for 4 hours. The resulting reaction system was concentrated. The residue was purified by reversed-phase column chromatography using eluent system A to obtain **638f** (170 mg, yield: 73.4%). MS m/z (ESI): 609 [M+1]⁺
Step 7: **638f** (150 mg, 0.25 mmol), 2-(7-azobenzotriazol-1-yl)-N,N,N',N'-tetramethyluronium hexafluorophosphate (186 mg, 0.49 mmol), and N,N-diisopropylethylamine (159 mg, 1.23 mmol) were dissolved in N,N-dimethylformamide (10 mL), and the reaction system was reacted at 20°C for 2 hours. The reaction solution was first quenched with ammonium chloride, and water (20 mL) and ethyl acetate (200 mL) were then added to the mixed solution. The organic phase was washed with a saturated sodium chloride aqueous solution (20 mL × 3), then dried and subjected to rotary evaporation to dryness. The residue was purified by reversed-phase column chromatography using eluent system A to obtain **638g** (100 mg, yield: 67.7%). MS m/z (ESI): 591 [M+1]⁺
Step 8: **638g** (40 mg, 0.067 mmol) was dissolved in trifluoroacetic acid (2 mL), and the mixture was purged three times with nitrogen. The reaction system was reacted at 90°C for 0.5 hours. The resulting reaction system was concentrated, and the residue was purified by silica gel column chromatography using eluent system A to obtain **Example 638** (16.3 mg, yield: 55.2%). MS m/z (ESI): 441 [M+1]⁺
¹H NMR (400 MHz, DMSO-d6) δ 8.95 (m, 1H), 8.23 (m, 1H), 7.69 (s, 1H), 7.62 (m, 1H), 7.47 (s, 1H), 6.68 (m, 1H), 5.90 (m, 1H), 5.42 (m, 2H), 5.13-5.05 (m, 3H), 4.62 (m, 1H), 3.75 (m, 1H), 3.36 (m, 2H), 2.72 (m, 1H), 2.38 (m, 1H).

### Example 688

### (Z)-4-Amino-11-((6-methyl-5-(trifluoromethyl)pyridin-2-yl)methyl)-10,11-dihydroazo[4,3-g]imidazo[1,5-a]quinoxalin-12(9H)-one

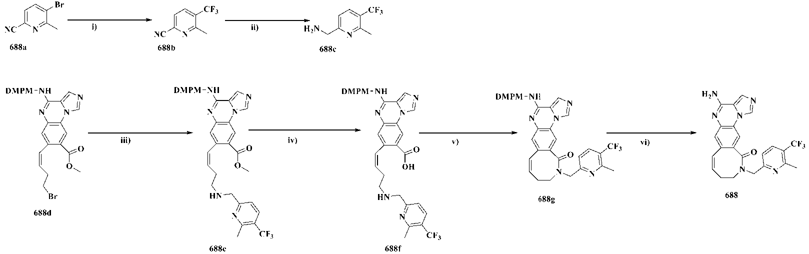

Step 1: 5-Bromo-6-methylpyridine-2-carbonitrile **688a** (8.00 g, 40.60 mmol), cuprous iodide (11.60 g, 60.90 mmol) and methyl 2,2-difluoro-2-(fluorosulphonyl)acetate (23.40 g, 121.81 mmol) were dissolved in N,N-dimethylformamide (50 mL), and the mixture was purged three times with nitrogen, and stirred at 100°C for 16 hours. The reaction solution was filtered to remove solids, and the filtrate was diluted with ethyl acetate (250 mL), and washed with water and a sodium chloride aqueous solution. The organic phase was concentrated, and the residue was purified by silica gel column chromatography to obtain **688b** (6.10 g, yield: 80.7%). MS m/z (ESI): 187 [M+1]⁺
Step 2: Lithium aluminium hydride (5.37 mL, 2.5 M) was added dropwise to a solution **of 688b** (2.00 g, 3.31 mmol) in tetrahydrofuran (20 mL) at 0°C. The reaction solution was stirred in an ice bath for 1 hour. Upon completion of the reaction, the reaction solution was quenched with sodium sulphate decahydrate, and filtered to remove solids. The filtrate was concentrated, and the residue was purified by silica gel column chromatography to obtain **688c** (460 mg, yield: 18.0%). MS m/z (ESI): 191 [M+1]⁺
Referring to the synthesis method described in Step 6 to Step 9 of **Example 62, Example 688** (15 mg, yield: 3.2%) was obtained from **688c** (200 mg, 1.04 mmol). MS m/z (ESI): 453 [M+1]⁺
¹H NMR (400 MHz, MeOD) δ 9.27 (s, 1H), 8.31 (d, 2H), 8.06 (d, 1H), 7.48 (d, 1H), 7.38 (s, 1H), 6.60 (m, 1H), 5.95 (m, 1H), 5.15 (d, 1H), 4.64 (d, 1H), 4.01 (t, 1H), 3.44 (d, 1H), 2.82 (m, 1H), 2.69 (d, 3H), 2.47 (d, 1H).

### Biological assay and evaluation

The present invention will be further described and explained below in conjunction with test examples, but these examples are not meant to limit the scope of the present invention.

### Test Example 1: Determination of the ability of the compounds of the present invention to improve the thermal stability (melting temperature) of the PRMT5/MEP50-MTA protein complex.

1 Experimental objective: The objective of this test example is to determine the ability of the compound to improve the thermal stability of the PRMT5/MEP50-MTA protein complex, resulting in an increase in the protein melting temperature.
2 Experimental instruments: Quantitative PCR instrument (Quantstudio6 Flex) was purchased from Life Technologies; pipettes were purchased from Eppendorf or Rainin.
3 Experimental reagents and consumables: PRMT5/MEP50 protein was prepared by Viva Biotech; HEPES was purchased from Thermo Fisher Scientific (Cat. No. 15630080); DTT was purchased from Sigma (Cat. No. 43816-10ml); sodium chloride was purchased from Invitrogen (Cat. No. AM9760G); Protein Thermal Shift^{™} Dye Kit was purchased from Thermo Fisher Scientific (Cat. No. 4461146); methylthioadenosine (MTA) was purchased from Sigma (Cat. No. D5011).
4 Experimental method: In this experiment, the thermal shift method was used to measure the change in the melting temperature (Tm) of the PRMT5/MEP50-MTA protein complex before and after compound binding, thereby characterising the ability of the compound to improve the thermal stability of the PRMT5/MEP50-MTA protein complex. A solution containing 50 mM HEPES, 10 mM DTT, 2 µM MTA, SYPRO Orange and 250 mM NaCl was prepared as the experimental buffer. Human PRMT5/MEP50 protein was added to a final concentration of 2 µM, and the mixture was incubated at room temperature for 30 minutes. The above reaction mixture was aliquoted into 8-strip PCR tubes at 19.5 µL per tube. Then 0.5 µL of test compound or DMSO was added to each tube, providing a total reaction volume of 20 µL and a final compound concentration of 8 µM. 2.5% DMSO was set as the vehicle control. After incubation at room temperature for 10 minutes, the PCR tubes were placed in a PCR instrument, and the melt curve function was selected to measure the melting temperature of the PRMT5/MEP50-MTA protein complex in different treatment groups (heated from 25°C to 95°C, 0.03°C/s).
5 Experimental data processing method: The experimental data files from the PCR instrument were imported into the thermal shift software to obtain the melting temperature (Tm) for each treatment group. The Tm of the DMSO vehicle control group was subtracted to yield the change in melting temperature (ΔTm) as shown in the table below:

| **Example** | **ΔTm (°C)** | **Example** | **ΔTm (°C)** |
|---|---|---|---|
| **15-P1** | 10.07 | **313-P1** | 11.05 |
| **48** | 10.91 | **352** | 11.02 |
| **62-P1** | 10.78 | **391** | 10.88 |
| **77** | 11.00 | **414** | 12.31 |
| **157** | 11.06 | **415** | 10.33 |
| **158** | 11.33 | **416** | 12.42 |
| **158-P1** | 12.03 | **417** | 11.05 |
| **159** | 10.49 | **418** | 11.47 |
| **164** | 11.19 | **419** | 12.73 |
| **164-P1** | 11.16 | **427** | 13.72 |
| **165-P1** | 11.90 | **431** | 12.31 |
| **167** | 12.31 | **434** | 12.17 |
| **171** | 12.03 | **435** | 12.75 |
| **173** | 11.61 | **437** | 12.17 |
| **176** | 11.47 | **437-P1** | 12.61 |
| **177** | 11.19 | **484** | 11.90 |
| **178** | 11.19 | **502** | 10.63 |
| **184** | 11.05 | **510** | 11.34 |
| **190** | 12.17 | **514** | 10.91 |
| **214** | 12.31 | **514-P1** | 11.33 |
| **270** | 10.77 | **514-P2** | 11.61 |
| **298** | 12.46 | **638** | 12.31 |
| **311** | 11.06 | **688** | 11.61 |
| **311-P1** | 10.92 | | |

6 Experimental conclusion: The compounds of the present invention exhibit good binding ability to the PRMT5/MEP50-MTA protein complex.

### Test Example 2: Determination of the inhibitory effect of the compounds of the present invention on the proliferation activity of HCT116 and MTAP Knockout HCT116 cells.

1 Experimental objective: The objective of this test example is to determine the inhibitory effect of the compound on the proliferation activity of HCT116 wild-type and HCT116 MTAP knockout cells.
2 Experimental instruments: Centrifuge (Eppendorf 5810R); microplate reader (BioTek Synergy H1 or PerkinElmer Envision); pipette (Eppendorf or Rainin).
3 Experimental reagents: HCT116 and MTAP Knockout HCT116 cells were purchased from Nanjing Cobioer; Cell Titer-Glo was purchased from Promega (Cat. No. G7573); McCoy'5A was purchased from Gibco (Cat. No. 12330031); FBS was purchased from Gibco (Cat. No. 10091148); PBS was purchased from Gibco (Cat. No. 10010023); trypsin was purchased from Gibco (Cat. No. 25200056); cell culture plates were purchased from Corning (Cat. No. 3610).
4 Experimental method: HCT116 and MTAP Knockout HCT116 cells were cultured in McCoy'5A medium containing 10% FBS until an appropriate cell density was reached. The cells were then collected and adjusted to a suitable concentration using complete medium. The cell suspension was seeded into a 96-well plate at 90 µL per well and allowed to adhere overnight in a 37°C, 5% CO₂ incubator. Compound solutions of various concentrations were prepared using DMSO and culture medium, with a vehicle control set. The compound solutions were added to the 96-well plate at 10 µL per well, and the plate was incubated in a 37°C, 5% CO₂ incubator for an additional 72-240 hours. After incubation, CellTiter-Glo solution was added, and the mixture was mixed uniformly by shaking, followed by incubation in the dark for 10 to 30 minutes. Luminescence was measured using a Synergy H1 or Envision microplate reader.
5 Experimental data processing method: The inhibition rate was calculated using the luminescence signal values, and the concentrations along with the inhibition rates were subjected to non-linear regression curve fitting using Graphpad Prism software to obtain the IC₅₀ values.

| **Example** | **MTAP Knockout HCT116 IC₅₀ nM** | **HCT116 IC₅₀ nM** | **Selectivity** |
|---|---|---|---|
| **77** | 12.0 | 236 | 20 |
| **157** | 23.0 | 1736 | 77 |
| **158-P1** | 8.7 | 708 | 81 |
| **164** | 16.0 | 6630 | 406 |
| **164-P1** | 14.0 | 556 | 40 |
| **165-P1** | 5.2 | 682 | 132 |
| **171** | 2.6 | 236 | 90 |
| **173** | 3.5 | 279 | 80 |
| **176** | 15.0 | 946 | 62 |
| **177** | 18.0 | 1506 | 84 |
| **178** | 18.0 | 2379 | 131 |
| **184** | 21.0 | 2293 | 108 |
| **190** | 6.2 | 763 | 123 |
| **214** | 5.3 | 106 | 20 |
| **270** | 17.0 | 2446 | 147 |
| **298** | 2.7 | 219 | 81 |
| **311** | 23.0 | 1736 | 77 |
| **313-P1** | 17.0 | 1024 | 59 |
| **414** | 4.9 | 229 | 47 |
| **416** | 2.4 | 222 | 93 |
| **417** | 11.0 | 3799 | 346 |
| **418** | 20.0 | 1089 | 54 |
| **419** | 3.6 | 51 | 14 |
| **427** | 0.9 | 27 | 30 |
| **431** | 3.3 | 200 | 61 |
| **434** | 6.7 | 578 | 87 |
| **435** | 1.9 | 78 | 41 |
| **437** | 3.0 | 191 | 64 |
| **437-P1** | 2.2 | 187 | 85 |
| **484** | 9.9 | 379 | 38 |
| **510** | 12.0 | 771 | 66 |
| **514** | 15.0 | 807 | 55 |
| **514-P2** | 14.0 | 1001 | 71 |
| **638** | 4.5 | 318 | 71 |

6 Experimental conclusion: The compounds of the present invention exhibit good proliferation inhibitory activity against HCT116 MTAP knockout cells, and good selectivity against HCT116 wild-type cells.

### Test Example 3: Determination of the pharmacokinetics of the compounds of the present invention in mice (plasma) after oral administration.

1 Study objective: Balb/c mice were used as test animals to study the pharmacokinetic behaviour of the compounds in mice (plasma) after oral administration.
2 Test scheme
   2.1 Test drugs: The example compounds of the present invention, made in house.
   2.2 Test animals: Balb/c mice, male, purchased from Shanghai Jiesijie Experimental Animal Co., Ltd., with animal production license number (SCXK (Shanghai) 2013-0006, No.311620400001794).
   2.3 Drug formulation: Formulation of drug for oral administration: 0.5% CMC-Na (1% Tween 80).
   5 g of hydroxyethyl cellulose (HEC, CMC-Na, viscosity: 800-1200 Cps) was weighed and dissolved in 1000 mL of purified water, and 10 g of Tween 80 was added. These materials were uniformly mixed to form a clear solution.
   4 mg of the example compound was weighed and dissolved in the above solution. The mixture was shaken well, homogenised using a cell disruptor for 1 min, and ultrasonicated for 10 minutes to obtain a suspension with a concentration of 3 mg/mL.

The example compound was weighed, and 5% DMSO was added based on the total administration volume; the mixture was vortexed and ultrasonicated for 2 min to ensure complete dissolution; then, 10% Solutol HS15 was added, and the mixture was vortexed and ultrasonicated for 2 min to achieve complete dissolution; finally, 85% PBS was added, and the mixture was vortexed and ultrasonicated for 5 min; the resulting mixture was filtered through a 0.22 um filter membrane to obtain a colourless, transparent, and clear solution with a concentration of 0.2 mg/mL.

2.4 Administration: 3 Balb/c mice, male, were fasted overnight and then respectively administered PO at a dose of 30 mg/kg and a volume of 10 mL/kg.

2.5 Sample collection: 0.04 mL of blood was collected from the orbit at 0.083 h, 0.25 h, 0.5 h, 1 h, 2 h, 4 h, 6 h, 8 h, and 24 h after administration and placed in EDTA-2K test tubes, and the test tubes were centrifuged at 6,000 rpm for 6 min at 4°C to separate the plasma, which was stored at -20°C. The animals were fed 4 h after administration.

3 Determination results: The final determination results were obtained using the LCMS/MS method, which showed that the example compounds exhibited high oral exposure.

### Test Example 4: Determination of the pharmacokinetics of the compounds of the present invention in rats (plasma) after oral administration.

1 Study objective: SD rats were used as test animals to study the pharmacokinetic behaviour of the following example compounds in rats (plasma) after oral administration at a dose of 5 mg/kg.
2 Test scheme
2.1 Test drugs: Vehicle formula: 0.5% CMC-Na (1% Tween 80); the examples of the present invention, made in house.
2.2 Test animals: 3 SD rats per group, male, purchased from Shanghai Bikai Keyi Biotechnology Co., Ltd., with animal license number (SCXK (Shanghai) 2018-0006, No.20180006037467).
2.3 Administration: 3 SD rats per group, male, were fasted overnight and then respectively administered PO at a dose of 5 mg/kg and a volume of 10 mL/kg.
2.4 Sample collection: 0.2 mL of blood was collected from the jugular vein of rats before administration and at 0, 0.25, 0.5, 1, 2, 4, 6, 8 and 24 h after administration, and placed in EDTA-K₂test tubes, and the test tubes were centrifuged at 6,000 rpm at 4°C for 6 min to separate the plasma, which was stored at -80°C. The animals were fed 4 h after administration.
2.5 Sample treatment
   1) 160 uL of acetonitrile was added to 40 uL of plasma sample for precipitation, and after mixing, the mixture was centrifuged at 3,500 × g for 5-20 minutes.
   2) 100 uL of the treated supernatant solution was taken for LC/MS/MS analysis to determine the concentration of the test compounds.
2.6 Liquid phase analysis
   - Liquid phase conditions: Shimadzu LC-20AD pump;mass spectrometry conditions: AB Sciex API 4000 mass spectrometer
   - Chromatographic column: Phenomenex Gemiu 5 um C18 50 × 4.6 mm
   - Mobile phase: solution A: 0.1% formic acid in water, solution B: methanol
   - Flow rate: 1.0 mL/min, elution time: 0-4.0 min, with eluents as follows:

| Time/min | Solution A | Solution B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis: The main pharmacokinetic parameters were calculated using WinNonlin 8.2. The results of pharmacokinetic experiments in rats are as shown in the table below:

| **Example No.** | Pharmacokinetic experiments in rats (p.o. 5 mg/kg) | | | | |
|---|---|---|---|---|---|
| | tₘₐₓ (h) | Cₘₐₓ (ng/mL) | AUC _{0-∞}(ng/mL*h) | t_{1/2} (h) | MRT_{0-∞}(h) |
| **62-P1** | 1.0 | 201 | 800 | 2.2 | 3.8 |
| **158** | 0.5 | 287 | 712 | 1.3 | 2.0 |
| **164** | 4.0 | 183 | 2515 | 4.7 | 7.6 |
| **165-P1** | 1.0 | 321 | 999 | 1.5 | 2.5 |
| **171** | 1.0 | 283 | 1058 | 1.6 | 2.9 |
| **178** | 1.7 | 544 | 2786 | 1.9 | 3.7 |
| **311-P1** | 2.3 | 330 | 1775 | 1.8 | 3.7 |
| **414** | 2.0 | 208 | 949 | 1.3 | 3.3 |
| **416** | 2.0 | 395 | 1519 | 1.3 | 2.9 |
| **434** | 2.0 | 273 | 2334 | 3.1 | 5.3 |
| **437-P1** | 2.0 | 318 | 2047 | 2.4 | 3.7 |
| **638** | 1.7 | 241 | 929 | 1.8 | 3.5 |

4 Experimental conclusion: After oral administration at a dose of 5 mg/kg, the example compounds of the present invention exhibit favourable metabolic properties, with good exposure (AUC) and maximum plasma concentration (Cₘₐₓ).

### Test Example 5: hERG potassium channel inhibitory activity assay

1 Study objective: The objective of this test example is to determine the inhibitory effect of the compound on the hERG potassium channel activity.
2 Experimental instruments and reagents:
2.1 Reagents

| Reagent name | Manufacturer | Cat. No. | Specification (mL) |
|---|---|---|---|
| Hams'F-12 nutrient mixture, GlutaMAXTM supplement | ThermoFisher | 31765-092 | 10 x 500 |
| Foetal bovine serum (FBS) | ThermoFisher | 10099-141 | 500 |
| GeneticinTM selective antibiotic (G418 sulphate) (50 mg/mL) | ThermoFisher | 10131027 | 100 |
| Hygromycin B (50 mg/mL) | ThermoFisher | 10687010 | 20 |
| TrypLE recombinant trypsin | ThermoFisher | 12605036 | 100 |

2.2. Instruments and consumables

| Name | Model | Manufacturer |
|---|---|---|
| pH meter | PB-21 | Sartorius |
| Osmometer | VAPRO 5520 | WESCOR |
| Amplifier | EPC10 | HEKA Elektronik |
| Motorised micromanipulator | MP-225 | SUTTER |
| Inverted microscope | ECLIPSE-Ti | Nikon |
| Electrode puller | PC-10 | NARISHIGE |
| Glass microelectrode | GC150tF-10 | Harvard Apparatus |
| 35 mm culture dish | 430165 | Corning |

3. Experimental method
3.1 Cell culture: The CHO cells stably expressing the hERG potassium channel (CHO-hERG) used in the experiment were obtained from Sophion Bioscience (Ballerup, Denmark), and passaged and cryopreserved at WuXi AppTec Co., Ltd. in Shanghai. The CHO-hERG cells were cultured and passaged in Ham's F-12 medium containing 1 × GlutaMAX, 10% foetal bovine serum, 100 µg/mL G418 and 100 µg/mL hygromycin B at 37°C with 5% CO₂.
3.2 Preparation of intracellular and extracellular solutions: The extracellular solution was prepared once a month, and aliquoted and stored in 1 L storage bottles. The intracellular solution was prepared once every three months, and aliquoted and cryopreserved at -20°C. The intracellular solution was thawed in a 37°C water bath before the experiment and then placed in an ice bath for later use.
3.3 Preparation of compounds: The positive control drug amitriptyline hydrochloride and the compounds were dissolved in 100% DMSO (Sigma-Aldrich, D2650) to prepare 10 mM or 30 mM stock solutions. Before the experiment, the stock solutions of the test compounds were diluted with DMSO to prepare solutions 1000-fold or 333-fold higher than the desired test concentrations, and then further diluted 1000-fold or 333-fold with the extracellular solution to achieve the desired concentrations.
3.4 Cell preparation: The CHO-hERG cells used in the experiment were cultured for at least two days. When the cell density reached more than 75%, the cells were digested with TrypLE, then resuspended in the extracellular solution, and centrifuged. The supernatant was discarded, and then the cells were further resuspended in 2 mL of extracellular solution for later use.
3.4 Experimental data processing: In data processing, the peak tail current was baseline-corrected when evaluating the hERG blocking effect. The effect of each compound at various concentrations was expressed as the inhibition rate of the tail current. IC₅₀ values were obtained by fitting the data to the Hill equation: I/Icontrol =Bottom + (Top-Bottom)/(1+10^((LogIC₅₀-X) * HillSlope))
   Top: maximum effect; Bottom: minimum effect; Hillslope: slope; X: concentration of the test compound.
   IC₅₀: half-maximal inhibitory concentration of the test compound on hERG. If the inhibition rate at the lowest concentration exceeded 50%, or the inhibition rate at the highest concentration failed to reach 50%, the corresponding IC₅₀ of the compound would be lower than the lowest concentration or greater than the highest concentration, respectively.

4 Experimental conclusion: The advantageous compounds of the present invention exhibit inhibitory activity (IC₅₀) against the cardiac hERG potassium channel of greater than 5 µM, or even greater than 15 µM, and in particular, the inhibitory activity (IC₅₀) of the compounds in **Examples 176, 178, 298 and 417** is greater than 30 µM, thereby avoiding cardiotoxic side effects at high doses.

### Test Example 6: In vivo pharmacodynamic study of the compounds of the present invention in subcutaneous graft tumour model of human lung cancer cell line LU99 in nude mice

1 Study objective: To evaluate the in vivo efficacy of the compounds in a subcutaneous graft tumour model of human lung cancer cell line LU99 in nude mice.
2 Experimental instruments and reagents
   2.1 Instruments: Refrigerator (BCD-268TN, Haier); biosafety cabinet (BSC-1300II A2, Medical Equipment Factory, Shanghai Boxun Industrial Co., Ltd.); ultraclean workbench (CJ-2F, Suzhou Fengshi Experimental Animal Equipment Co., Ltd.); electric pipette-aid (Easypet 3, Eppendorf); thermostatic water bath (HWS-12, Shanghai Yiheng Scientific Instruments Co., Ltd.); CO₂ incubator (Thermo-311, Thermo); centrifuge (Centrifuge 5720R, Eppendorf); automated cell counter (Countess II, Life Technologies); vernier calliper (CD-6"AX, Mitutoyo, Japan); cell culture flask (T25/T75/T225, Corning); electronic balance (CPA2202S, Sartorius); electronic balance (BSA2202S-CW, Sartorius); ultrasonic cleaner (115F0032, Shanghai Kudos); water purification system (Pacific TII, Thermo); magnetic stirrer (08-2G, Chijiu).
   2.2 Reagents: RPMI-1640 medium (22400-089, Gibco); foetal bovine serum (FBS) (A5669701, Gibco); phosphate buffer solution (PBS) (10010-023, Gibco); Matrigel (356234, Corning); sodium carboxymethylcellulose (30036365, Sinopharm Chemical Reagent Co., Ltd.); Tween-80 (30189828, Sinopharm Chemical Reagent Co., Ltd.).
3 Experimental operations and data processing
   3.1 Animals: BALB/c nude mice, 6-8 weeks old, female, purchased from Beijing Vital River Laboratory Animal Technology Co., Ltd.
   3.2 Cell culture and cell suspension preparation
      a) A vial of LU99 cells was taken from a cell bank and thawed using RPMI-1640 medium (RPMI-1640 + 10% FBS), and the thawed cells were placed in a cell culture flask and cultured in a CO₂ incubator (incubator temperature at 37°C, and CO₂ concentration at 5%).
      b) The cells were passaged every three days, and after passage, the cells were further cultured in the CO₂ incubator. This process was repeated until the cell number met the requirements for *in vivo* efficacy.
      c) The cells in the exponential growth phase were collected, counted using anautomated cell counter, and resuspended in PBS based on the counting results. The resuspended cells were mixed with Matrigel at a 1 : 1 ratio to achieve a concentration of 5 × 10⁷ cells/mL, and then placed in an ice box for later use.
   3.3 Cell inoculation
      a) Before inoculation, the nude mice were marked with disposable universal ear tags for mice and rats.
      b) At the time of inoculation, the cell suspension was mixed uniformly, 0.1-1 mL of the cell suspension was drawn into a 1 mL syringe with air bubbles expelled, and the syringe was then placed on an ice pack for later use.
      c) The nude mouse was restrained with the left hand, and the inoculation site (the right dorsal side near the right shoulder) was disinfected with a 75% alcohol cotton swab; inoculation was performed 30 seconds later.
      d) The experimental nude mice were inoculated sequentially (0.1 mL of cell suspension per mouse).
   3.4 Tumour volume measurement, grouping and administration in tumour-bearing mice
      a) Based on tumour growth, on days 9-12 post-inoculation, tumour volumes were measured and tumour sizes were calculated. Tumour volume calculation: $tumor volume \left({mm}^{3}\right) = length \left(mm\right) \times width \left(mm\right) \times width \left(mm\right) / 2 .$
      b) Based on the body weight and tumour size of the tumour-bearing mice, the mice were randomly assigned to groups.
      c) Based on the grouping results, administration of the test drug was started (mode of administration: oral administration; administration volume: 10 mL/kg; administration frequency: once/day or twice/day; administration cycle: 28 days; vehicle: 0.5% CMC-Na (1% Tween 80)).
      d) After the start of administration, tumour volume and body weight were measured twice a week.
      e) The animals were euthanised at the end of the experiment.
      f) The data was processed with software such as Excel. Calculation of tumour growth inhibition (TGI) rate (%) for the compound: When there is no tumour regression, TGI (%) = [(1 - (average tumour volume at the end of administration in a treatment group - average tumour volume at the start of administration in the treatment group) / (average tumour volume at the end of treatment in the solvent control group - average tumour volume at the start of treatment in the solvent control group)] × 100%. When there is tumour regression, TGI (%) = [1 - (average tumour volume at the end of administration in a treatment group - average tumour volume at the start of administration in the treatment group) / average tumour volume at the start of administration in the treatment group] × 100%.
4 Experimental conclusion:
   The advantageous compounds of the present invention exhibit a tumour growth inhibition (TGI) rate of greater than 80%, even greater than 90%, at low doses, demonstrating significant tumour growth inhibitory effects. The compounds exhibit a tumour growth inhibition (TGI) rate of greater than 100%, even greater than 150%, at high doses, demonstrating significant tumour regression effects.

## Claims

1. A compound represented by general formula (I), or a stereoisomer or a pharmaceutically acceptable salt thereof: 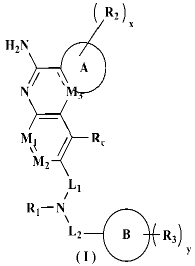
M₁ is selected from -N- or -CRₐ-;
M₂ is selected from -N- or -CR_{b}-; preferably -CR_{b}-;
M₃ is selected from N or C;
ring A is selected from C₃₋₈ cycloalkyl, 3- to 8-membered heterocyclyl, C₆₋₁₀ aryl or 5- to 10-membered heteroaryl; preferably C₃₋₆ cycloalkyl, 3- to 6-membered heterocyclyl, phenyl or 5- to 6-membered heteroaryl;
ring B is selected from C₃₋₁₄ cycloalkyl, 3- to 14-membered heterocyclyl, C₆₋₁₄ aryl or 5- to 14-membered heteroaryl; preferably C₃₋₁₂ cycloalkyl, 3- to 12-membered heterocyclyl, C₆₋₁₂ aryl or 5- to 12-membered heteroaryl; preferably C₃₋₆ cycloalkyl, 3- to 6-membered heterocyclyl, C₆₋₁₀ fused cycloalkyl, 6- to 10-membered fused heterocyclyl, C₆₋₁₀ aryl or 5- to 10-membered heteroaryl;
L₁ is selected from a bond, -(CRₐₐR_{bb})ₘ₂-, -(CRₐₐR_{db})ₘ₂C(O)-, - (CRₐₐR_{bb})ₘ₂C(S)-, -(CRₐₐR_{bb})ₘ₂C(NR_{cc})-, -(CRₐₐR_{bb})ₘ₂NR_{cc}C(O)-, - (CRₐₐR_{bb})ₘ₂S(O)ₘ₁-, -(CRₐₐR_{bb})ₘ₂NR_{cc}-, -(CRₐₐR_{bb})ₘ₂P(O)₂-, - (CRₐₐR_{bb})ₘ₂P(O)(OR_{cc})-, C₃₋₁₂ cycloalkylene, 3- to 12-membered heterocyclylene, C₆₋₁₂ arylene or 5- to 12-membered heteroarylene, wherein the C₃₋₁₂ cycloalkylene, 3- to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably -CRₐₐR_{bb}-, - C(O)-, -S(O)ₘ₁- or -NR_{cc}-;
L₂ is selected from a bond, -(CRₐₐR_{bb})ₘ₂-, -(CRₐₐR_{db})ₘ₂C(O)-, - (CRₐₐRbb)m2NR_{cc}C(O), -(CRₐₐR_{db})ₘ₂S(O)ₘ₁- or -(CRₐₐR_{bb})ₘ₂NR_{ec}-; preferably - CRₐₐR_{bb}-, -C(O)-, -S(O)ₘ₁- or NR_{cc};
R₁ is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, -(CR_{cc}R_{dd})ₙ₁-C₃₋₁₂ cycloalkyl, - (CR_{cc}R_{dd})ₙ₁-3- to 12-membered heterocyclyl, -(CR_{cc}R_{dd})ₙ₁-C₆₋₁₂ aryl, -(CR_{cc}R_{dd})ₙ₁-5- to 12-membered heteroaryl, -SF₅, -ORₑ, -NRₑR_{f}, -C(O)Rₑ, -C(O)ORₑ, - C(O)NRₑR_{f}, -N=S(O)RₑR_{f}, -S(O)Rₑ(=NR_{f}) or -P(O)RₑR_{f}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, -(CR_{cc}R_{dd})ₙ₁-C₃₋₈ cycloalkyl, -(CR_{cc}R_{dd})ₙ₁-3- to 8-membered heterocyclyl, -(CR_{cc}R_{dd})ₙ₁-C₆₋₁₀ aryl, -(CR_{cc}R_{dd})ₙ₁-5- to 10-membered heteroaryl, -ORₑ, -NRₑR_{f}, -C(O)Rₑ, -C(O)NRₑR_{f} or -P(O)RₑR_{f}, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3-to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
alternatively, R₁ and Rₐ, R_{b} or R_{c} are connected to form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably form C₃₋₈ cycloalkyl, 3- to 8-membered heterocyclyl, C₆₋₁₀ aryl and 5- to 10-membered heteroaryl, which are optionally further substituted with one or more substituents selected from deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R₂ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C6-12 aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff}, -(CH₂)ₙ₂P(O)RₑₑR_{ff} or =CRₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -Y₁-C₃₋₁₂ cycloalkyl, -Y₁-3- to 12-membered heterocyclyl, -Y₁-C₆₋₁₂ aryl, -Y₁-5- to 12-membered heteroaryl, -SF₅, -OR_{g}, -NR_{g}Rₕ, -C(O)R_{g}, -C(O)OR_{g}, -C(O)NR_{g}Rₕ, - N=S(O)R_{g}Rₕ, -S(O)R_{g}(=NRₕ), -P(O)R_{g}Rₕ, -C(=NRᵢ)NR_{g}Rₕ or =R_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f}; preferably hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, - C(O)NR_{g}Rₕ, -N=S(O)R_{g}Rₕ, -S(O)R_{g}(=NRₕ), -P(O)R_{g}Rₕ or =R_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f};
alternatively, any two R₃, together with adjacent atoms to which they are attached, form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably form C₃₋₈ cycloalkyl, 3- to 8-membered heterocyclyl, C₆₋₁₀ aryl and 5- to 10-membered heteroaryl, which are optionally further substituted with one or more substituents selected from deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₐ, R_{b}, Rₑ, Rₑ and R_{f} are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, - (CH₂)ₙ₂NRₑₑR_{ff}, -(CH₂)ₙ₂C(O)Rₑₑ, -(CH₂)ₙ₂C(O)ORₑₑ, -(CH₂)ₙ₂C(O)NRₑₑR_{ff}, - (CH₂)ₙ₂N=S(O)RₑₑR_{ff}, -(CH₂)ₙ₂S(O)Rₑₑ(=NR_{ff}) or -(CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
alternatively, Rₐ and R_{b} are connected to form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Y₁ is selected from a bond, -O-, -S-, -C(O), -NRⱼ-, -C(O)NRⱼ-, -NRⱼC(O)-, - S(O)₂NRj-, -NRjS(O)₂-, C₁₋₆ alkylene, -O-C₁₋₆ alkylene-, -C₁₋₆ alkylene-O-, -NRⱼ-C₁₋₆ alkylene-, -C₁₋₆ alkylene-NRⱼ-, C₂₋₆ alkenylene or C₂₋₆ alkynylene, wherein the C₁₋₆ alkylene, C₂₋₆ alkenylene and C₂₋₆ alkynylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{g}, Rₕ, Rᵢ and Rⱼ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
alternatively, R_{g} and Rₕ are connected to form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Rₐₐ, R_{bb}, R_{cc} and R_{dd} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₑₑ and R_{ff} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R_{gg} and Rₕₕ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
x is selected from 0, 1, 2, 3, 4, 5 or 6;
y is selected from 0, 1, 2, 3, 4, 5 or 6;
m1 is selected from 0, 1 or 2;
m2 is selected from 0, 1 or 2;
n1 is selected from 0, 1, 2, 3 or 4; and
n2 is selected from 0, 1, 2, 3 or 4.

2. The compound, or the stereoisomer or the pharmaceutically acceptable salt thereof according to claim 1, **characterised in that** the 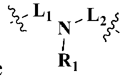 is selected from

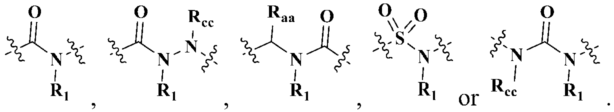

3. A compound represented by general formula (A), or a stereoisomer or a pharmaceutically acceptable salt thereof: 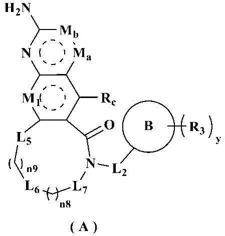
M₁ is selected from -N- or -CRₐ-;
Mₐ is selected from CR₂ₐ, NR₂ₐ or N;
M_{b} is selected from CR_{2b}, NR_{2b} or N;
alternatively, R₂ₐ and R_{2b} are connected to form ring A;
ring A is selected from C₃₋₈ cycloalkyl, 3- to 8-membered heterocyclyl, C₆₋₁₀ aryl or 5- to 10-membered heteroaryl, which is optionally substituted with 0, 1, 2, 3, 4, 5 or 6 R_{2c};
ring B is selected from C₃₋₁₄ cycloalkyl, 3- to 14-membered heterocyclyl, C₆₋₁₄ aryl or 5- to 14-membered heteroaryl;
L₂ is selected from a bond, -(CRₐₐR_{bb})ₘ₂-, -(CRₐₐR_{bb})ₘ₂C(O)-, - (CRₐₐR_{bb})ₘ₂NR_{cc}C(O), -(CRₐₐR_{bb})ₘ₂S(O)ₘ₁- or -(CRₐₐR_{bb})ₘ₂NR_{cc}-;
L₅ is selected from a bond, -O(CR₉ₐR_{9b})ₙ₁₀-, -(CR₉ₐR_{9b})ₙ₁₀O-, -S(CR₉ₐR_{9b})ₙ₁₀-, -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, =CR₉ₐR_{9b}, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
L₆ is selected from a bond, -O(CR₉ₐR_{9b})ₙ₁₀-, -(CR₉ₐR_{9b})ₙ₁₀O-, -S(CR₉ₐR_{9b})ₙ₁₀-, -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, =CR₉ₐR_{9b}, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
L₇ is selected from a bond, -O(CR₉ₐR_{9b})ₙ₁₀-, -(CR₉ₐR_{9b})ₙ₁₀O-, -S(CR₉ₐR_{9b})ₙ₁₀-, -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, =CR₉ₐR_{9b}, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₂ₐ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{2b} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{2c} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C6-12 aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff}, -(CH₂)ₙ₂P(O)RₑₑR_{ff} or =CRₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₃ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, -C(O)NR_{g}Rₕ, -N=S(O)R_{g}Rₕ, - S(O)R_{g}(=NRₕ), -P(O)R_{g}Rₕ or =R_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f};
R₉ₐ and R_{9b} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Rₐ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff} or -(CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{c} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff} or -(CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Rₑ and R_{f} are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{g} and Rₕ are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{N} is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, - C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Rₐₐ, R_{bb} and R_{cc} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₑₑ and R_{ff} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
y is selected from 0, 1, 2, 3, 4, 5 or 6;
m1 is selected from 0, 1 or 2;
m2 is selected from 0, 1 or 2;
n2 is selected from 0, 1, 2, 3 or 4;
n8 is selected from 0, 1, 2, 3 or 4;
n9 is selected from 0, 1, 2, 3 or 4;
n10 is selected from 0, 1, 2, 3 or 4.

4. The compound, or the stereoisomer or the pharmaceutically acceptable salt thereof according to claim 1 or 3, **characterised in that** the compound is further a compound represented by general formula (III-E), or a stereoisomer or a pharmaceutically acceptable salt thereof: 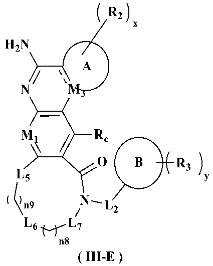
L₆ is selected from a bond, -O(CR₉ₐR_{9b})ₙ₁₀-, -(CR₉ₐR_{9b})ₙ₁₀O-, -S(CR₉ₐR_{9b})ₙ₁₀-, -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, =CR₉ₐR_{9b}, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably a bond, -O-, - S-, -C(O), -NR_{N}-, -C(O)NR_{N}-, -NR_{N}C(O)-, -S(O)₂NR_{N}-, -NR_{N}S(O)₂-, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3- to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; more preferably -O-, -S-, -C(O), -NR_{N}-, C₁₋₃ alkylene or C₂₋₄ alkenylene, wherein the C₁₋₃ alkylene and C₂₋₄ alkenylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
L₇ is selected from a bond, -O(CR₉ₐR_{9b})ₙ₁₀-, -(CR₉ₐR_{9b})ₙ₁₀O-, -S(CR₉ₐR_{9b})ₙ₁₀-, -(CR₉ₐR_{9b})ₙ₁₀S-, -C(O)(CR₉ₐR_{9b})ₙ₁₀-, -(CR₉ₐR_{9b})ₙ₁₀C(O)-, -NR_{N}(CR₉ₐR_{9b})ₙ₁₀-, - (CR₉ₐR_{9b})ₙ₁₀NR_{N}-, -C(O)NR_{N}-, -NR_{N}C(O)-, -S(O)₂NR_{N}-, -NR_{N}S(O)₂-, =CR₉ₐR_{9b}, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3-to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably a bond, -O-, - S-, -C(O), -NR_{N}-, -C(O)NR_{N}-, -NR_{N}C(O)-, -S(O)₂NR_{N}-, -NR_{N}S(O)₂-, C₁₋₆ alkylene, C₂₋₆ alkenylene, C₂₋₆ alkynylene, C₃₋₁₂ cycloalkylene, 3- to 12-membered heterocyclylene, C₆₋₁₂ arylene or 5- to 12-membered heteroarylene, wherein the C₁₋₆ alkylene, C₂₋₆ alkenylene, C₂₋₆ alkynylene, C₃₋₁₂ cycloalkylene, 3- to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
n8 is selected from 0, 1, 2, 3 or 4; preferably 1, 2 or 3;
n9 is selected from 0, 1, 2, 3 or 4; preferably 0, 1 or 2;
n10 is selected from 0, 1, 2, 3 or 4;
ring A, ring B, L₂, M₁, M₃, R_{c}, R₂, R₃, x and y are as defined in claim 1.

5. The compound, or the stereoisomer or the pharmaceutically acceptable salt thereof according to any one of claims 1-4, **characterised in that** the compound is further a compound represented by general formula (IV-E), or a stereoisomer or a pharmaceutically acceptable salt thereof: 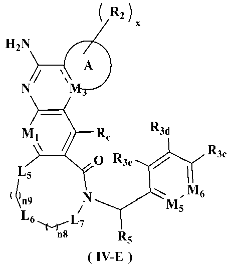
M₅ is selected from N or CR₃ₐ;
M₆ is selected from N or CR_{3b};
R₅ is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, - C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₃ₐ, R_{3b}, R_{3d} and R₃ₑ are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, -C(O)NR_{g}Rₕ, -N=S(O)R_{g}Rₕ, -S(O)R_{g}(=NRₕ), -P(O)R_{g}Rₕ or - C(=NRᵢ)NR_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5-to 12-membered heteroaryl and =CRₑR_{f};
R_{3c} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, -C(O)NR_{g}Rₕ, -N=S(O)R_{g}Rₕ, - S(O)R_{g}(=NRₕ), -P(O)R_{g}Rₕ or -C(=NRᵢ)NR_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f};
L₅, L₆, L₇, n8 and n9 are as defined in claim 3;
ring A, M₁, M₃, R_{c}, R₂ and x are as defined in claim 1.

6. The compound, or the stereoisomer or the pharmaceutically acceptable salt thereof according to any one of claims 1-5, **characterised in that** ring A is selected from 3- to 12-membered heterocyclyl or 5- to 12-membered heteroaryl; preferably 5-membered heterocyclyl, 6-membered heterocyclyl, 5-membered heteroaryl or 6-membered heteroaryl; more preferably

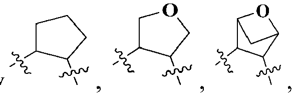

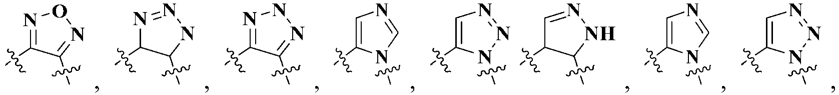

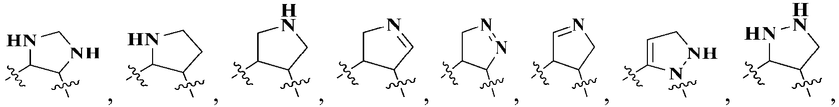

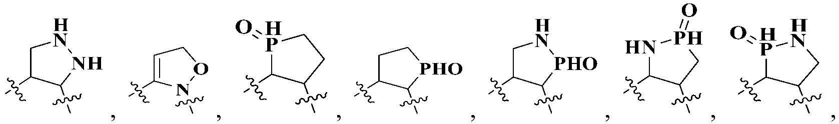

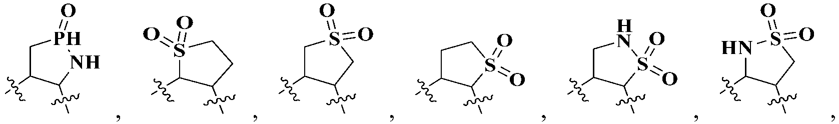

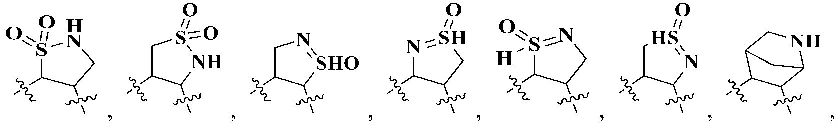

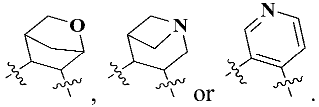

7. The compound, or the stereoisomer or the pharmaceutically acceptable salt thereof according to any one of claims 1-4, **characterised in that** ring B is selected from 3- to 6-membered heterocyclyl fused phenyl or 3- to 6-membered heterocyclyl fused 5- to 6-membered heteroaryl; preferably 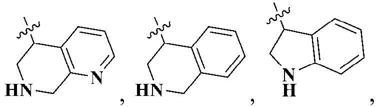 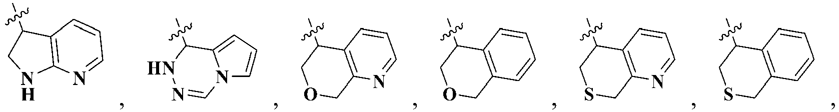 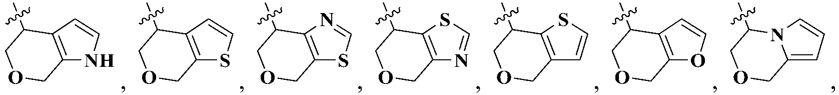 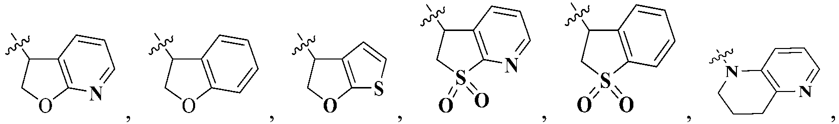 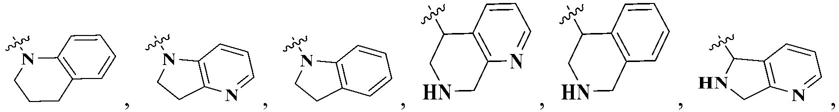 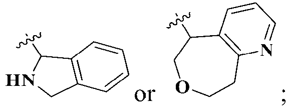 alternatively, ring B is 6- to 14-membered tricyclic heterocyclyl; preferably 6-to 14-membered tricyclic spiro heterocyclyl or 6- to 14-membered tricyclic fused heterocyclyl; more preferably 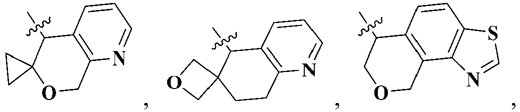 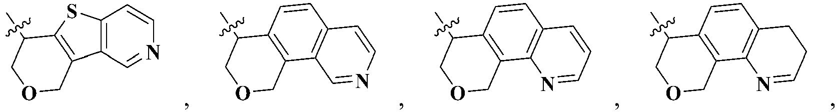 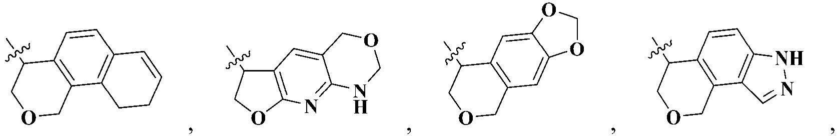 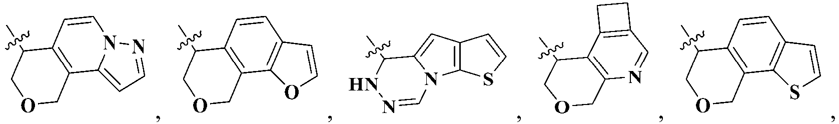 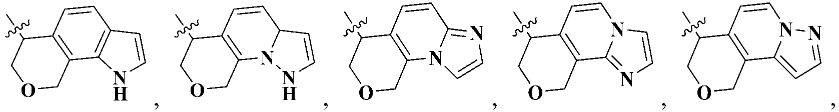 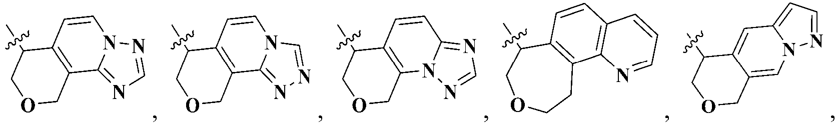 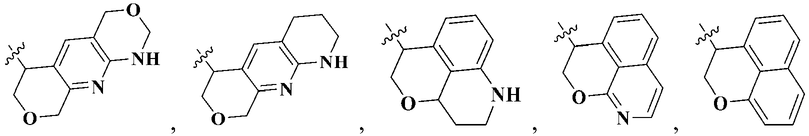 or

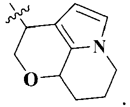

8. The compound, or the stereoisomer or the pharmaceutically acceptable salt thereof according to any one of claims 1-7, **characterised in that** the compound is further as represented by general formula (VI-A) or (VI-B): 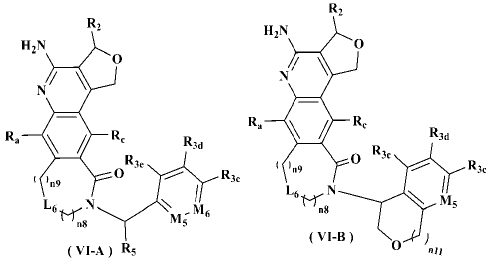 n11 is selected from 0, 1 or 2.

9. The compound, or the stereoisomer or the pharmaceutically acceptable salt thereof according to any one of claims 1-8, **characterised in that** each R₂ or R_{2c} is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl, C₃₋₈ cycloalkyl or -C(O)NRₑₑR_{ff}, wherein the amino, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl and C₃₋₈ cycloalkyl are optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl and C₃₋₈ cycloalkyl;
Rₑₑ and R_{ff} are each independently selected from hydrogen, deuterium, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl or cyano-substituted C₁₋₃ alkyl;
preferably hydrogen, deuterium, fluorine, chlorine, methyl, deuterated methyl, difluoromethyl, trifluoromethyl, 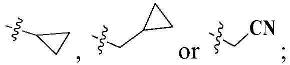
R_{c} is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl or cyano-substituted C₁₋₃ alkyl; preferably hydrogen, deuterium, fluorine, chlorine, methyl, deuterated methyl, difluoromethyl or trifluoromethyl;
Rₐ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl or cyano-substituted C₁₋₃ alkyl; preferably hydrogen, deuterium, fluorine, chlorine, methyl, deuterated methyl, difluoromethyl or trifluoromethyl.

10. The compound, or the stereoisomer or the pharmaceutically acceptable salt thereof according to any one of claims 1-9, **characterised in that** R₃ and R_{3c} are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl, 5- to 10-membered heteroaryl or -SF₅, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₃ₐ, R_{3b}, R_{3d} and R₃ₑ are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl.

11. The compound, or the stereoisomer or the pharmaceutically acceptable salt thereof according to any one of claims 1-10, **characterised in that** the compound has the following structure:

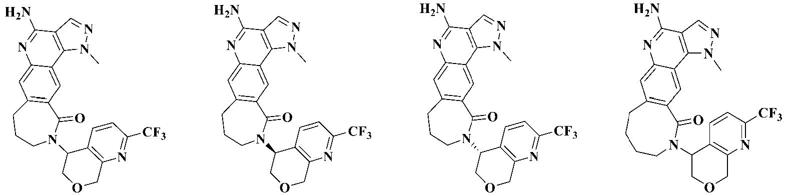

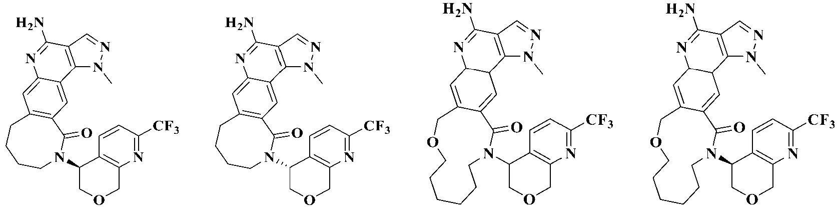

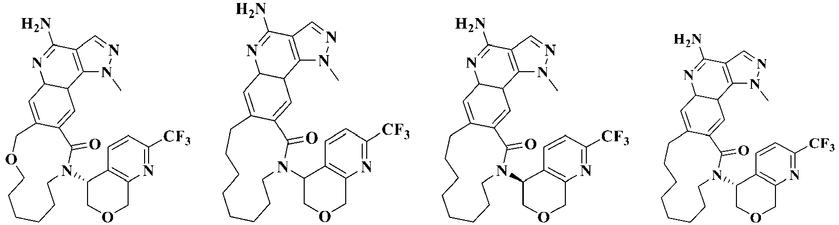

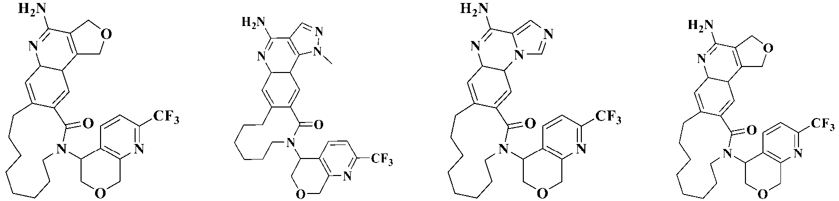

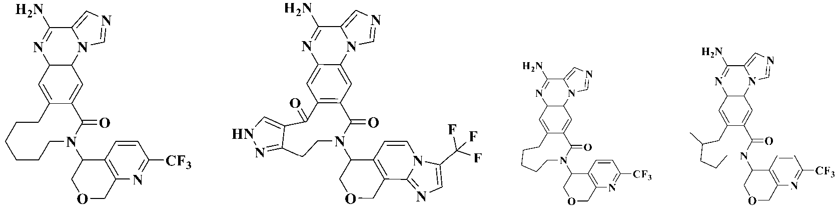

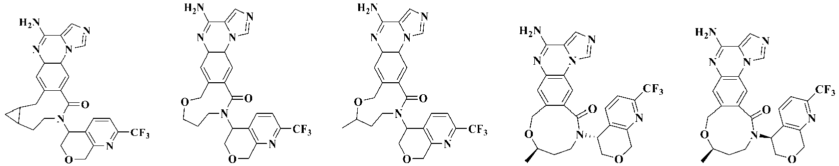

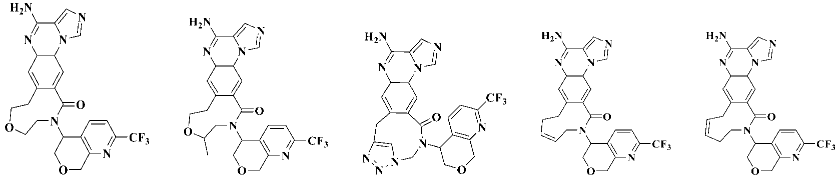

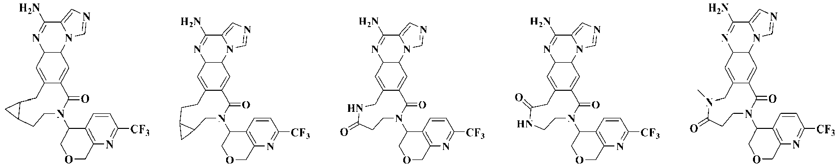

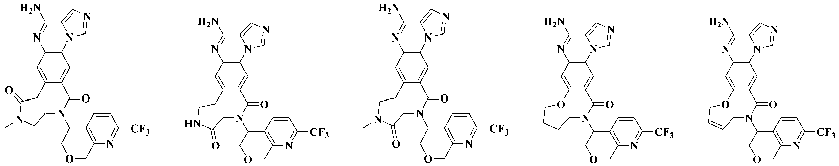

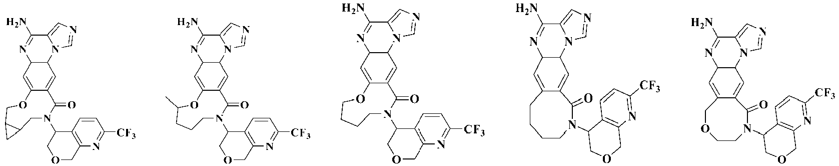

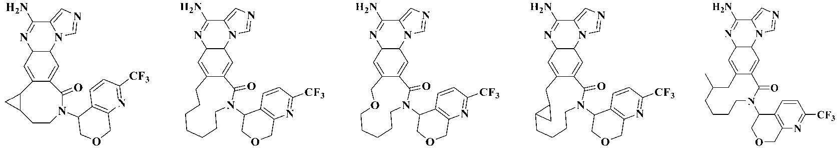

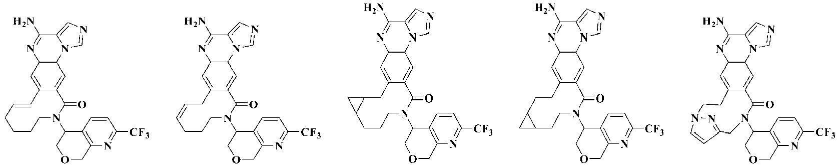

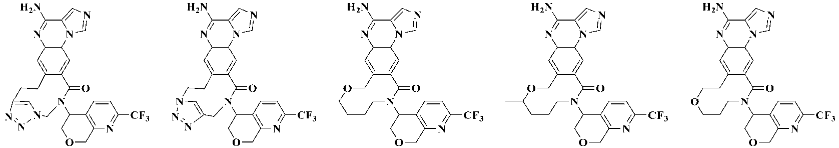

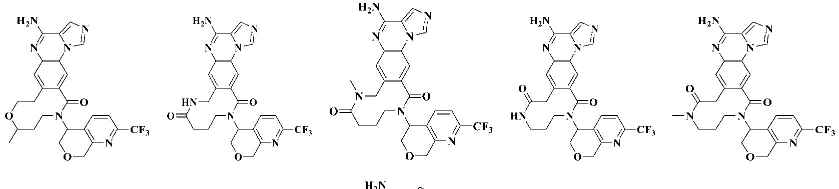

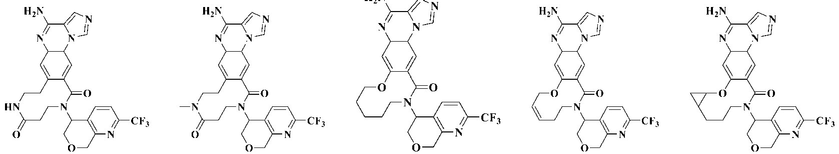

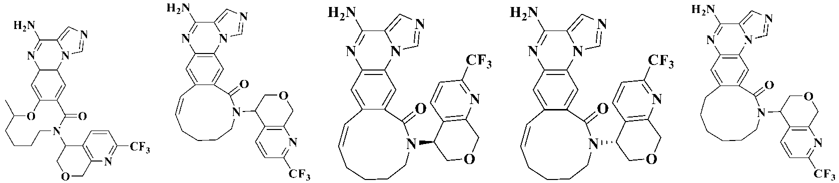

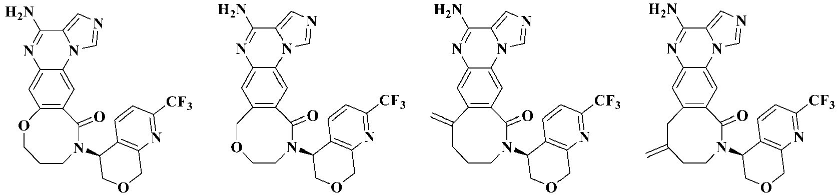

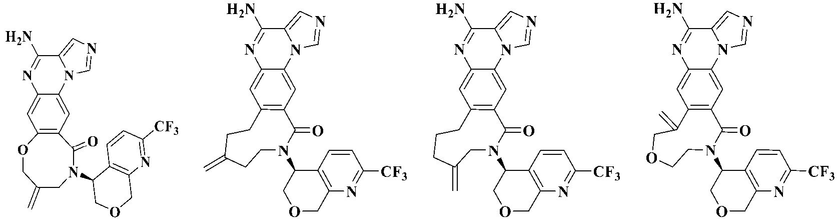

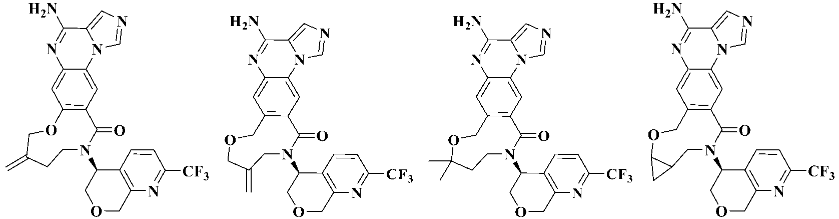

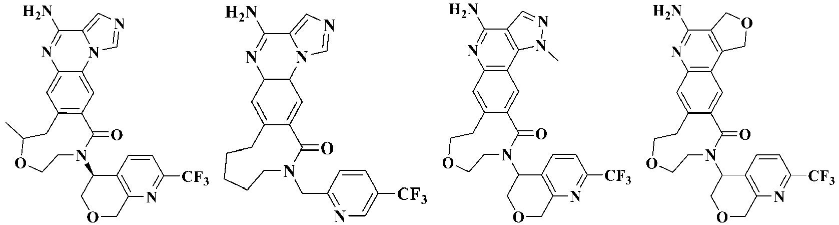

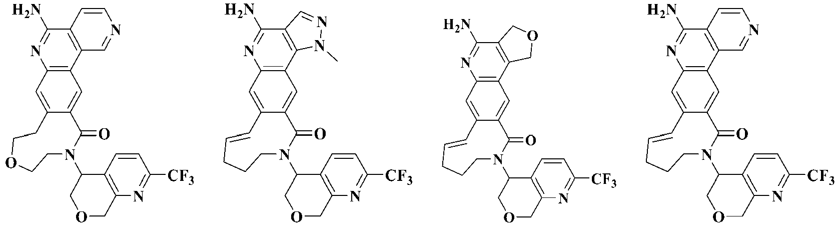

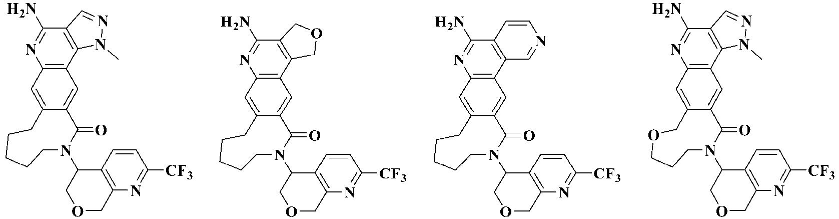

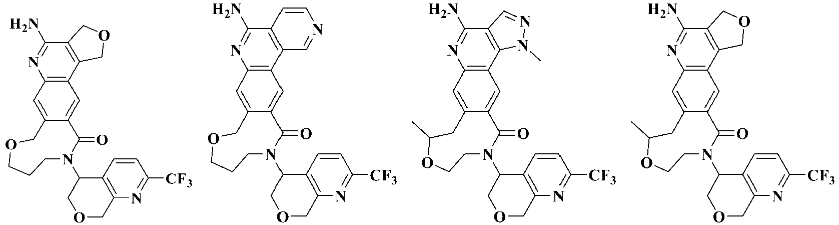

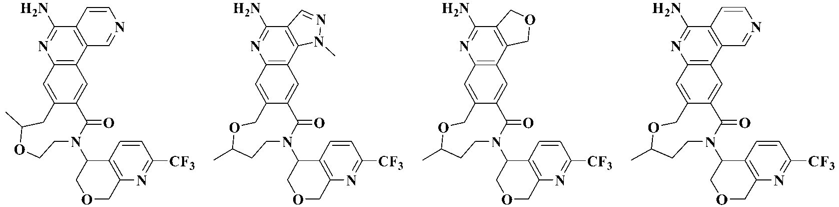

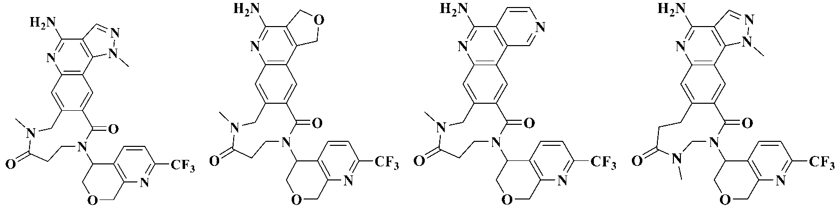

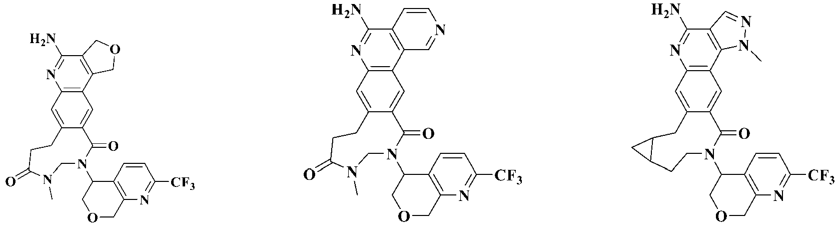

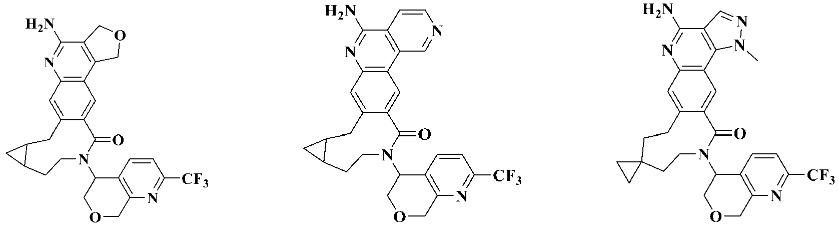

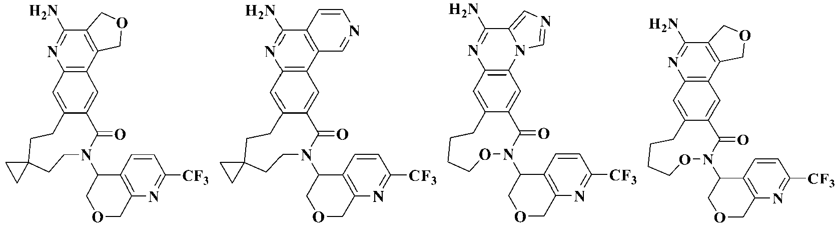

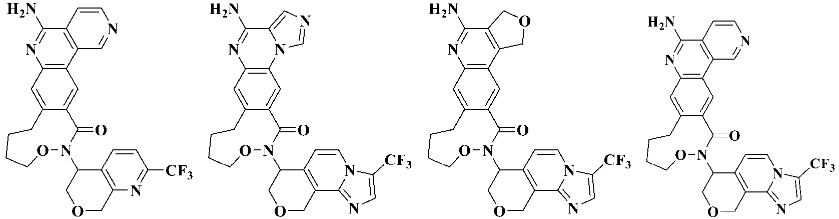

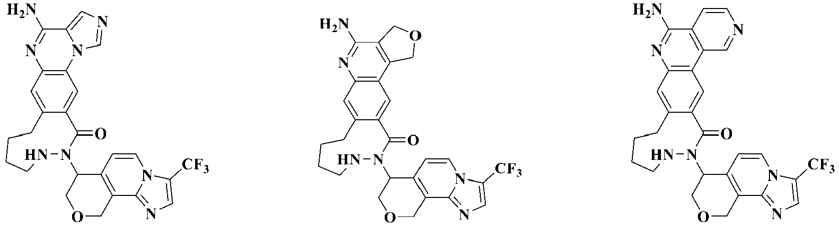

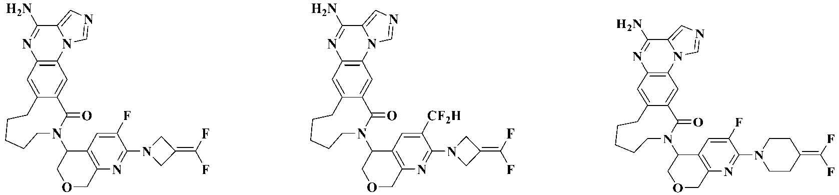

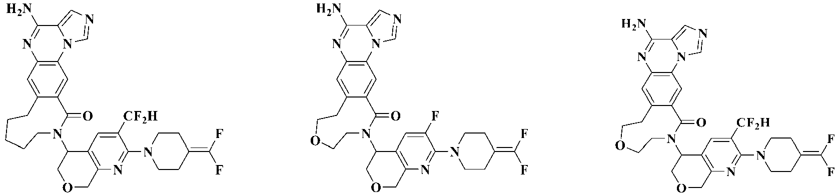

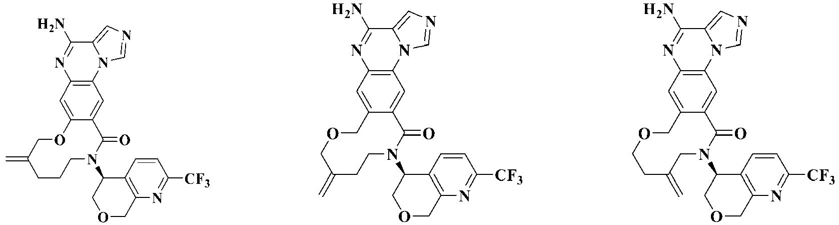

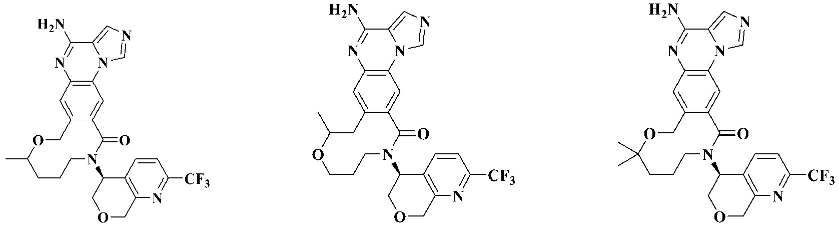

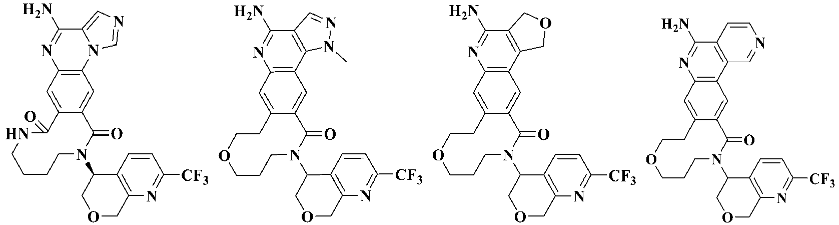

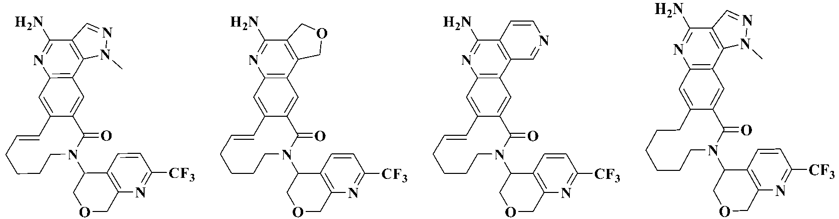

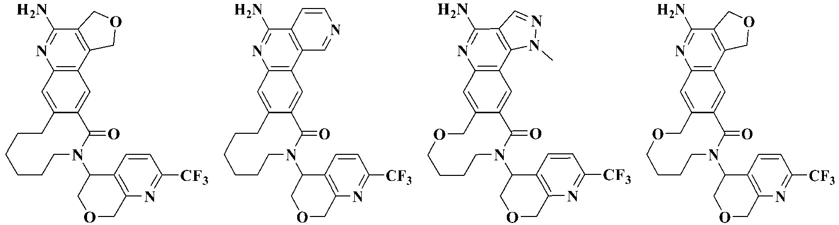

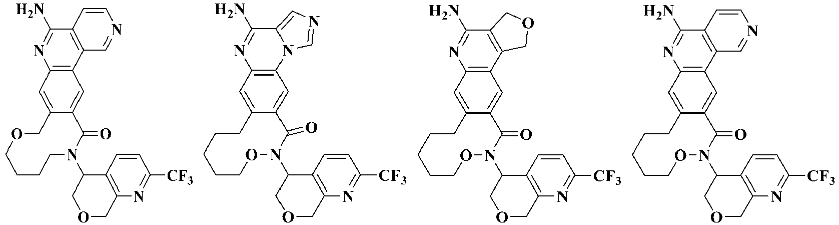

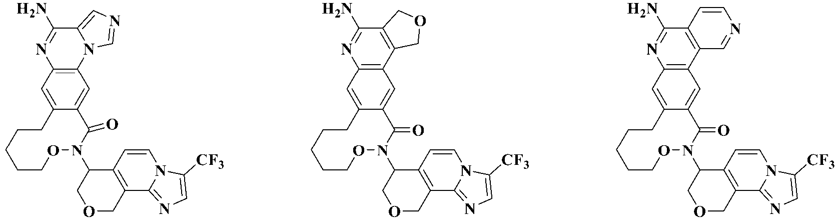

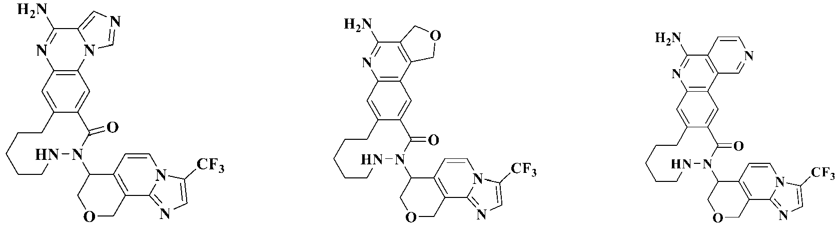

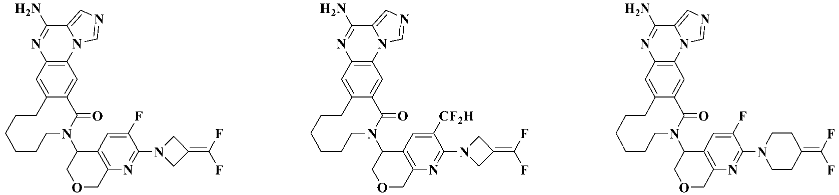

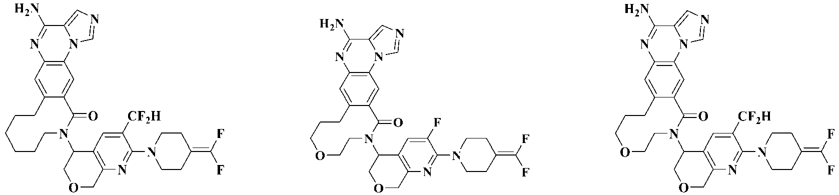

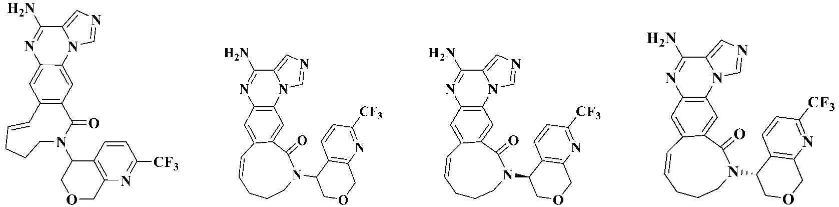

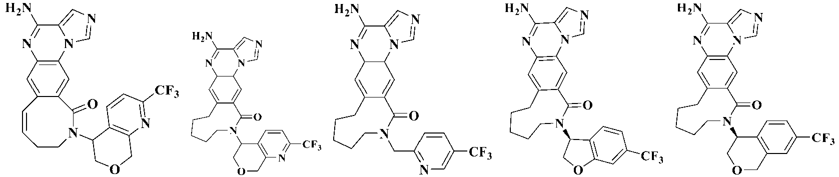

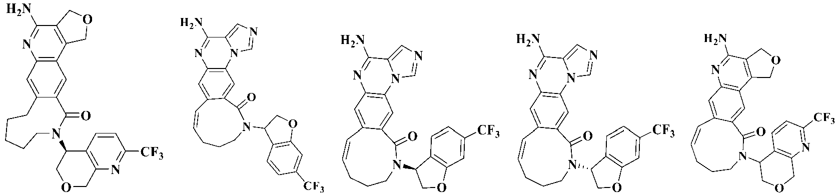

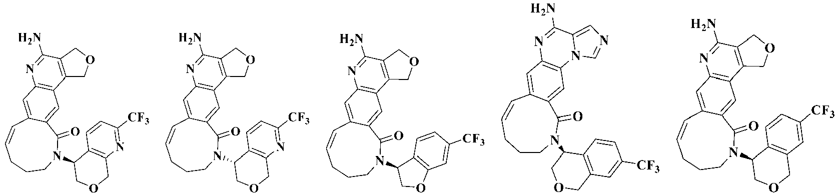

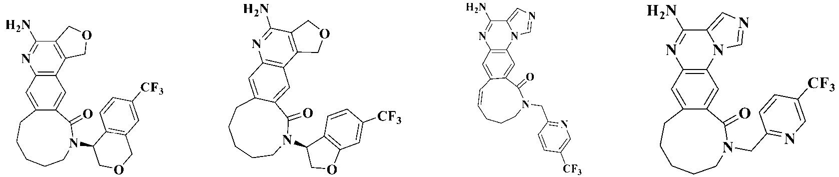

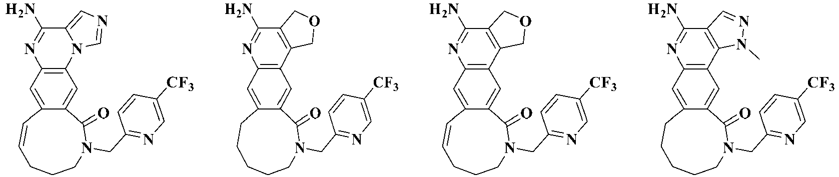

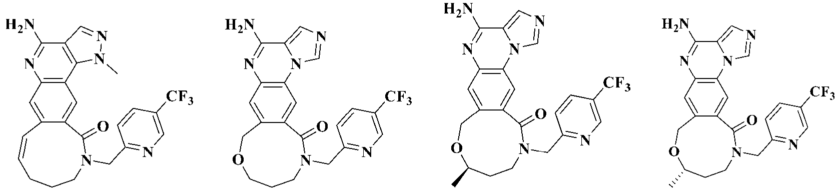

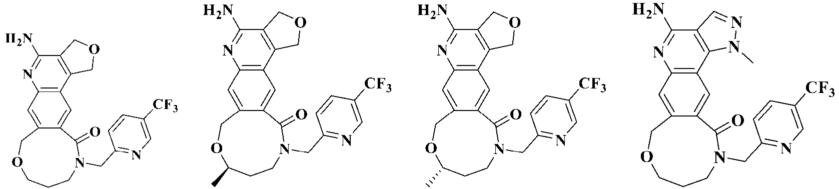

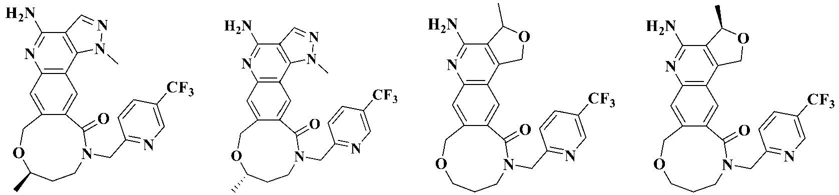

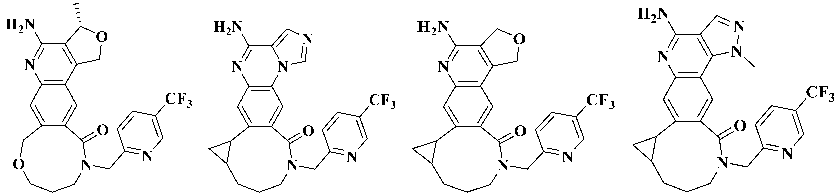

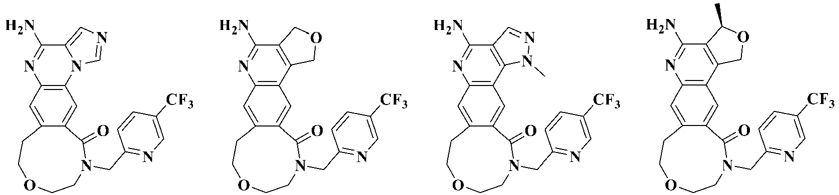

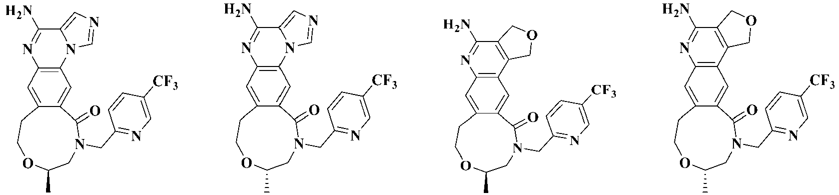

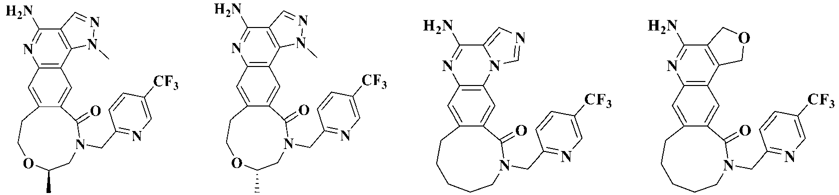

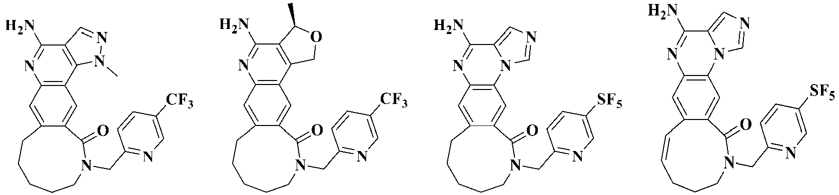

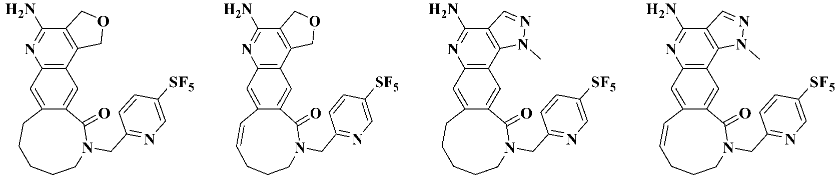

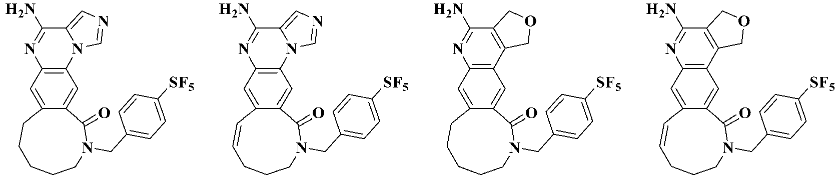

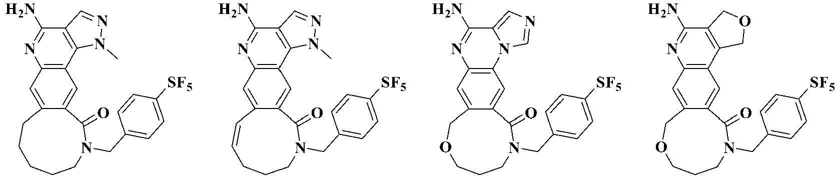

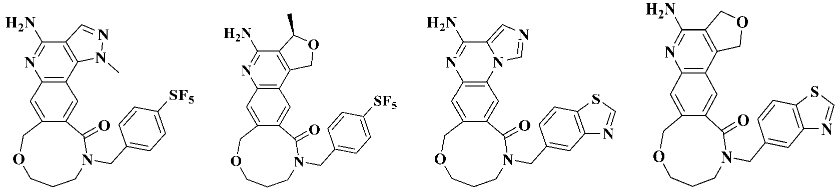

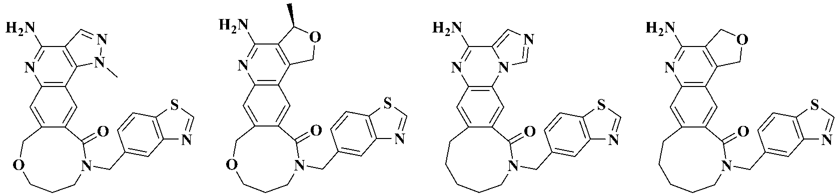

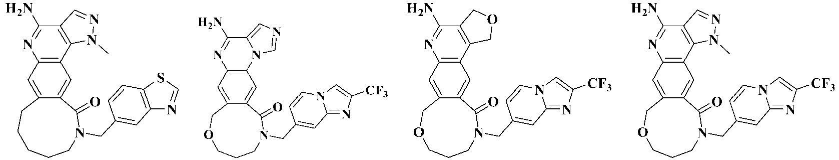

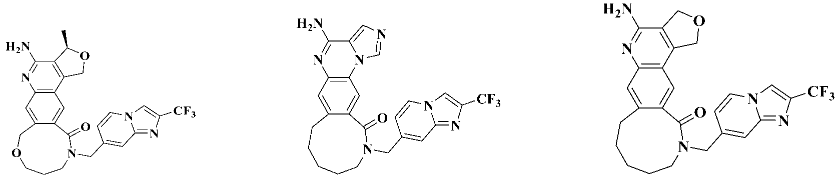

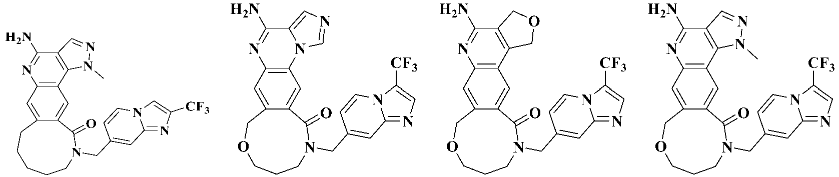

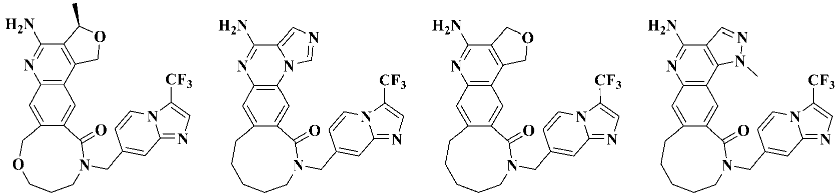

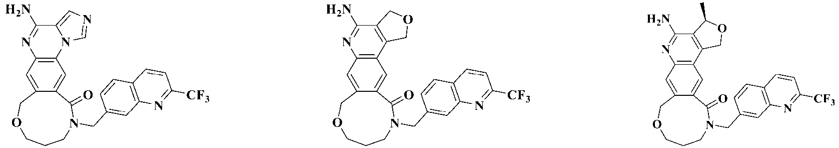

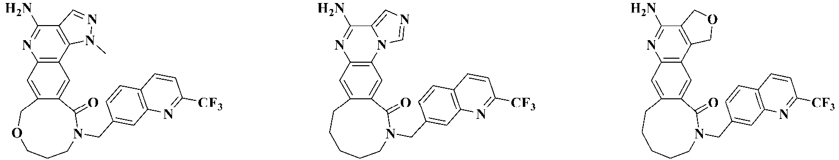

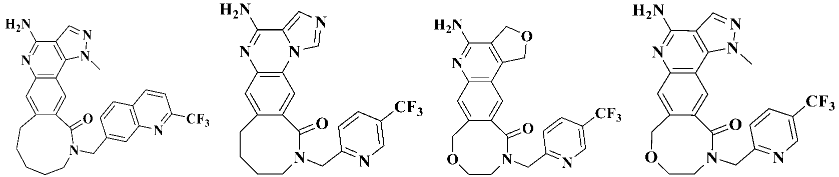

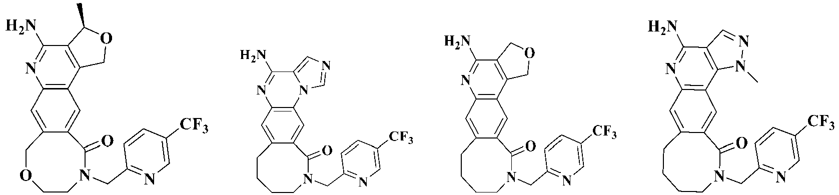

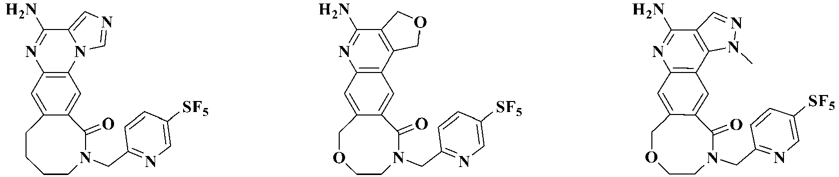

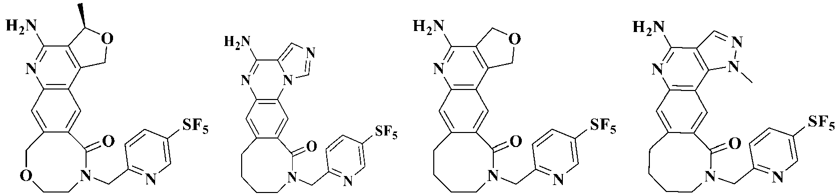

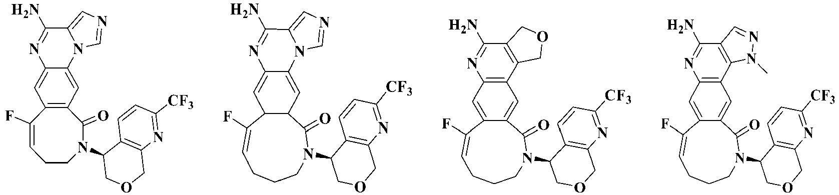

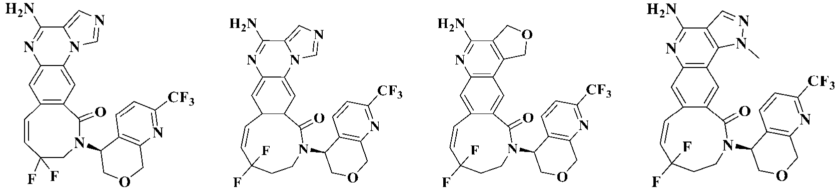

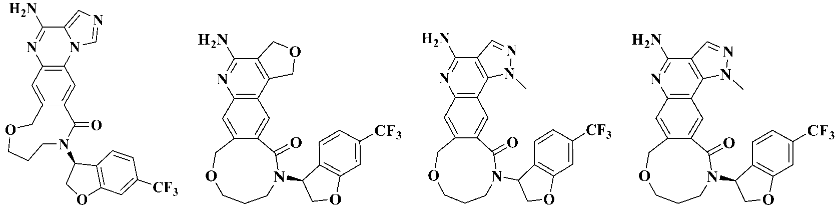

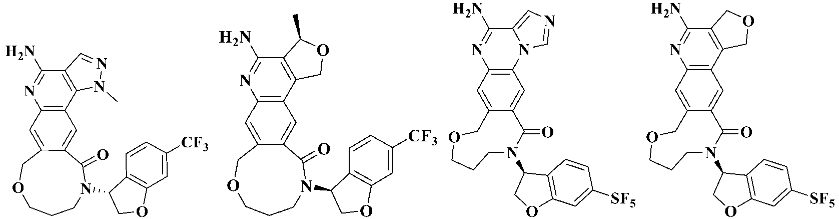

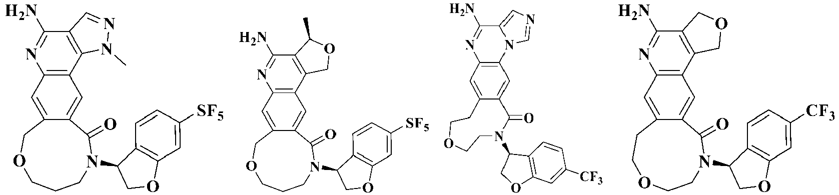

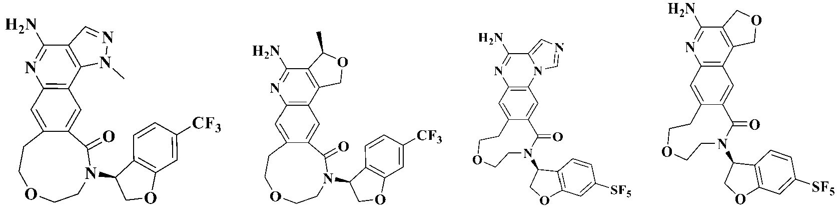

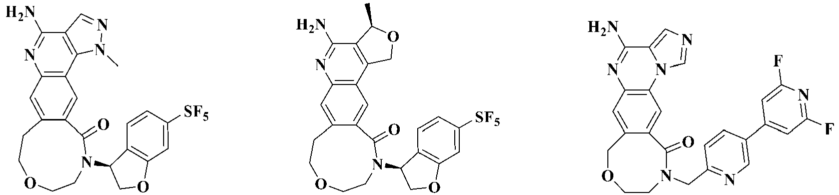

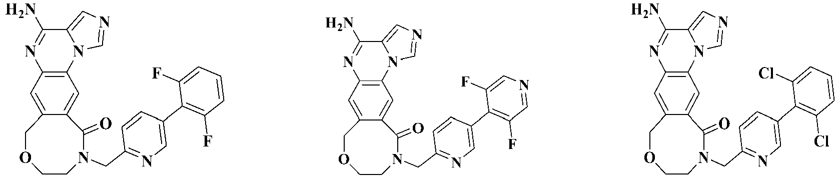

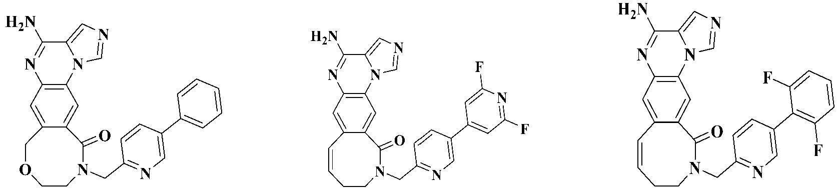

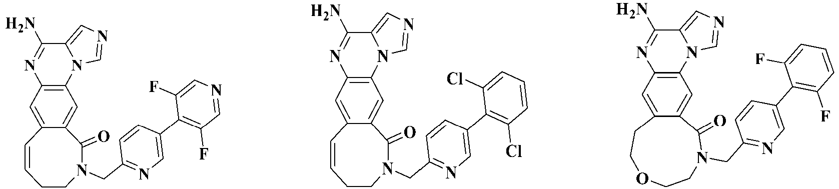

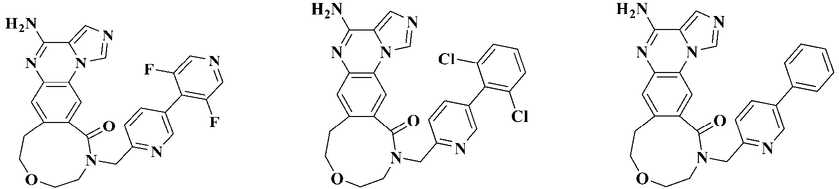

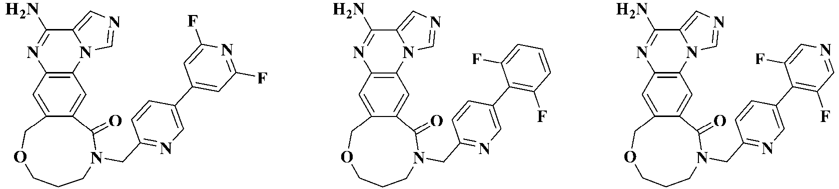

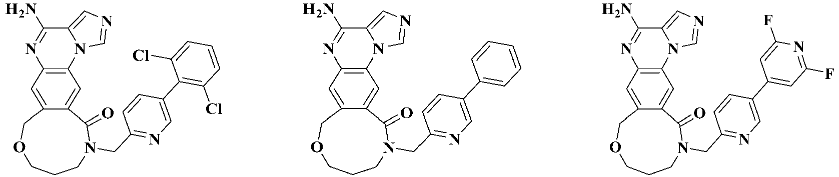

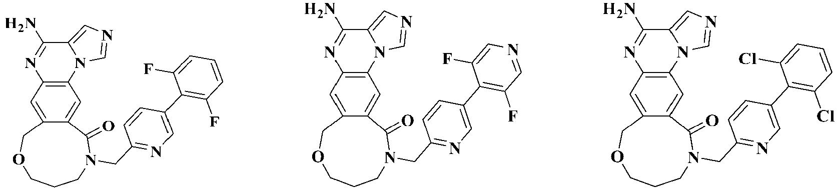

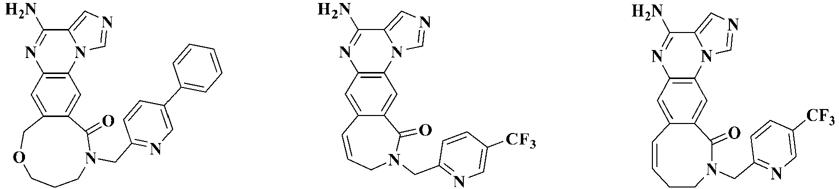

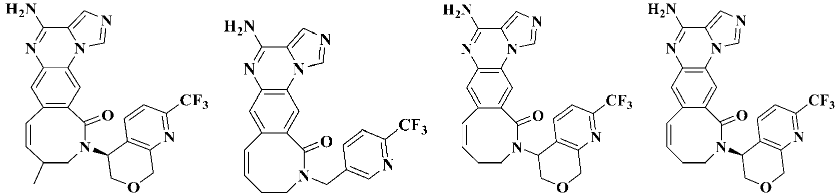

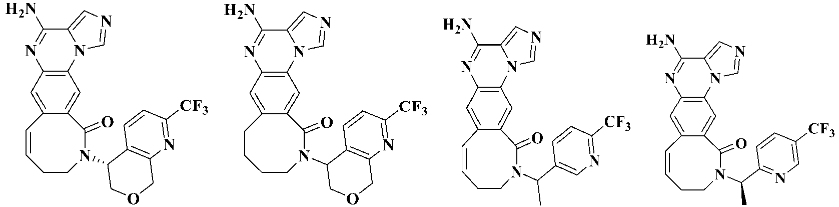

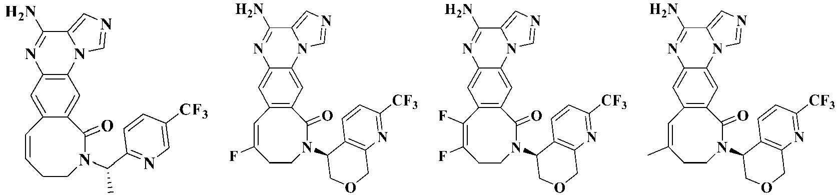

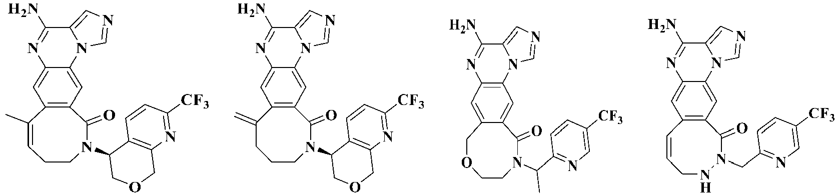

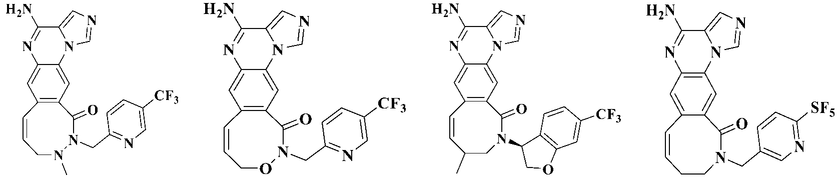

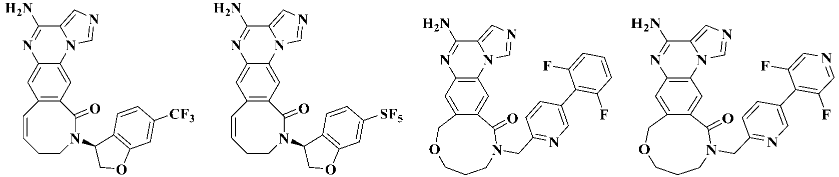

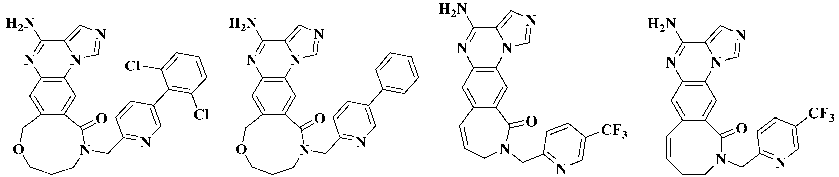

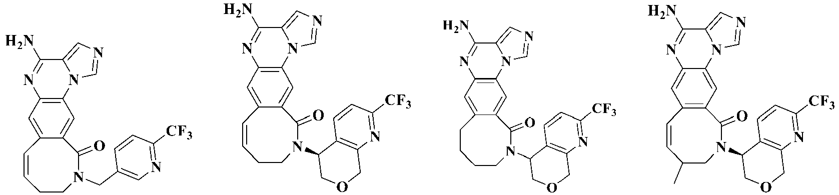

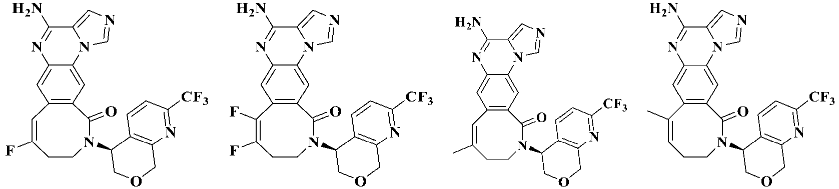

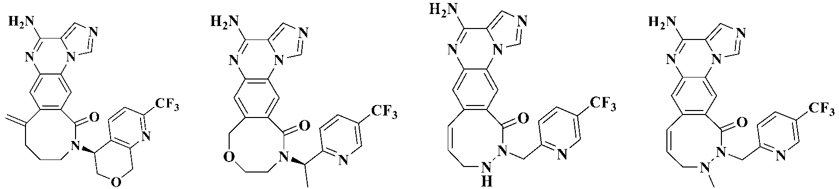

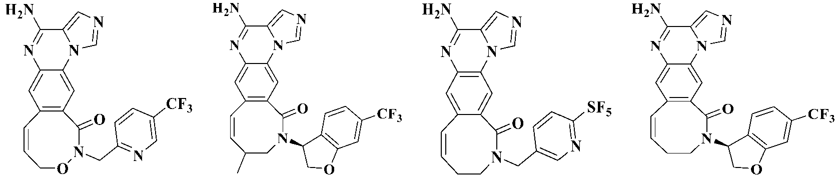

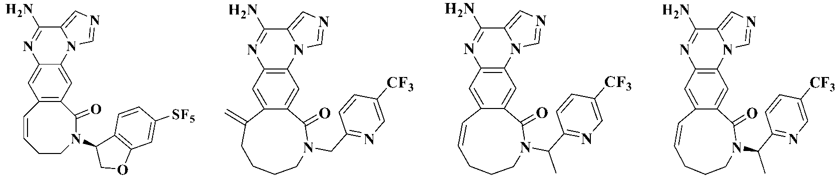

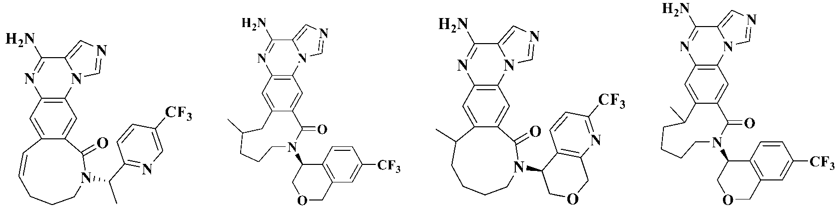

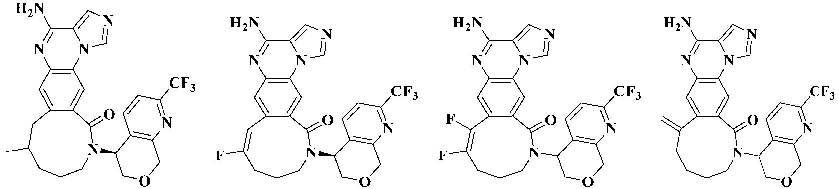

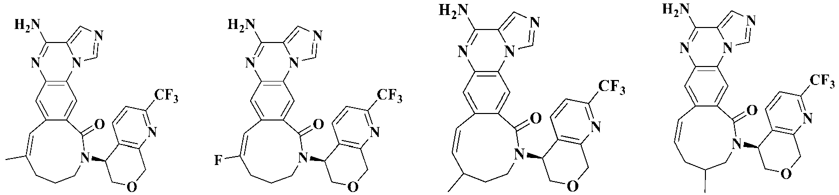

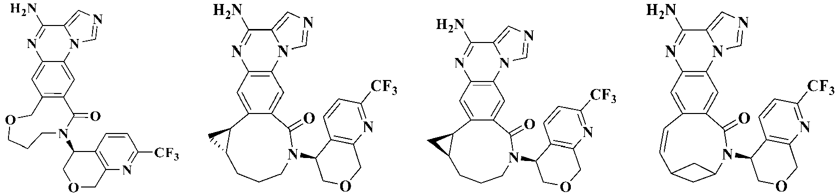

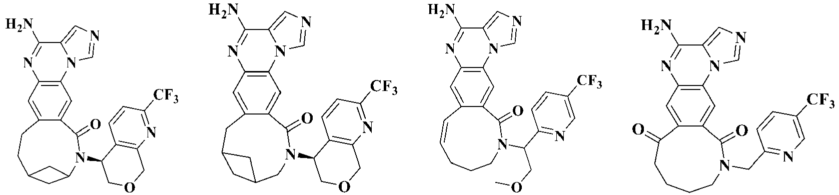

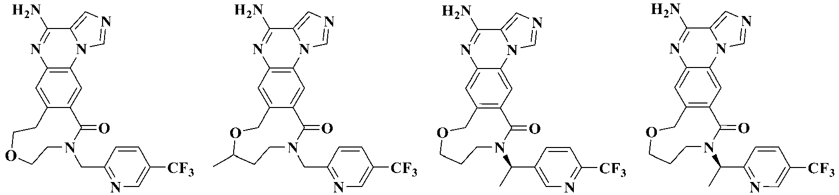

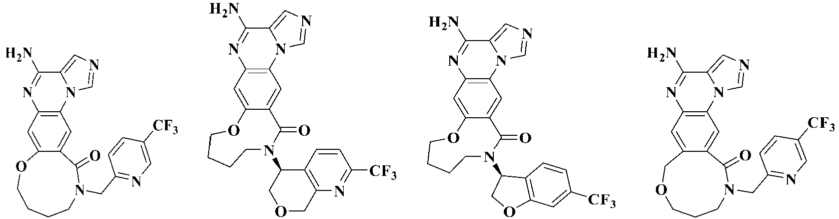

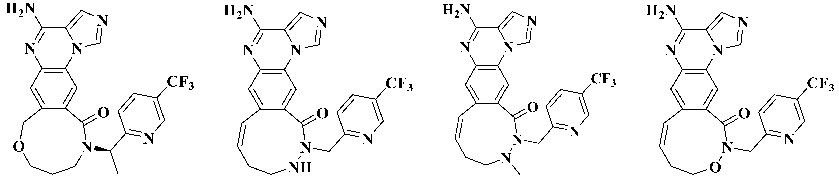

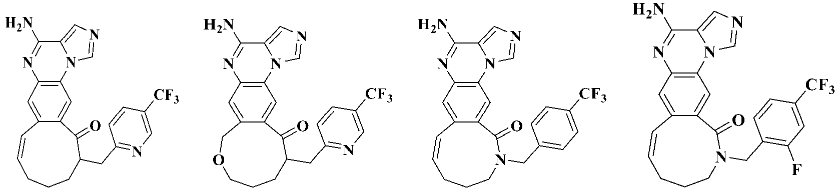

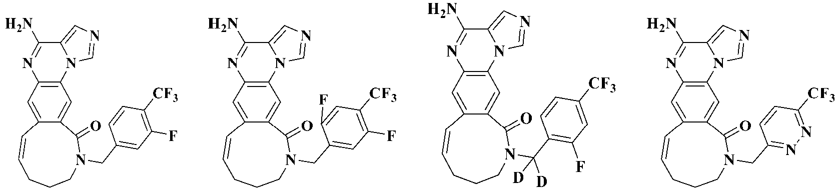

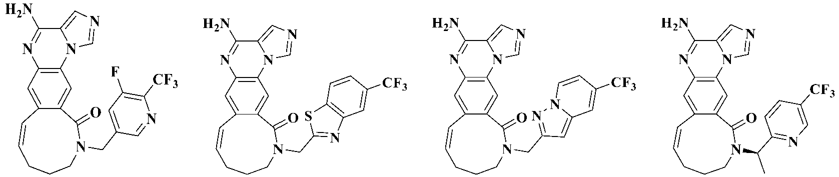

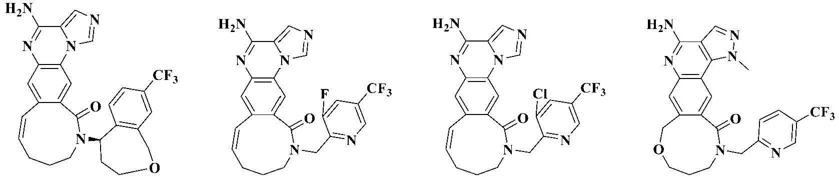

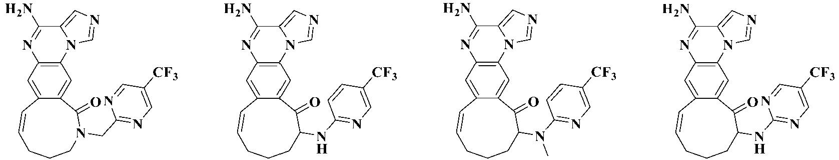

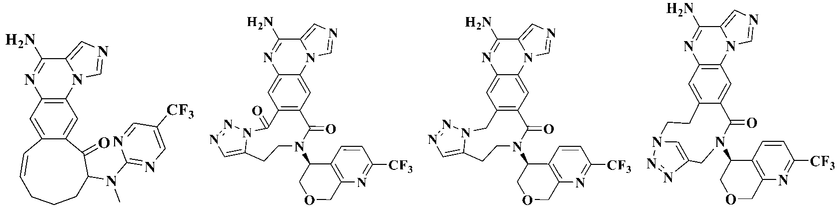

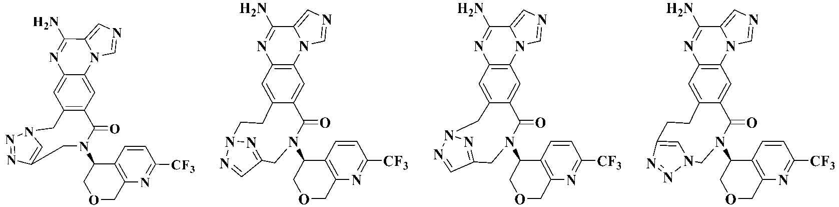

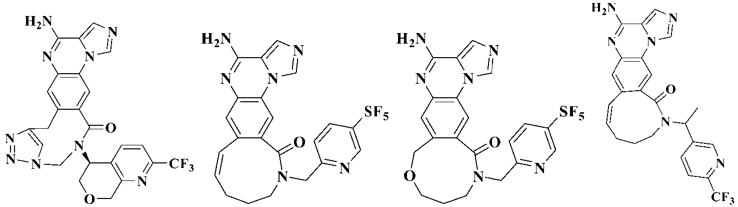

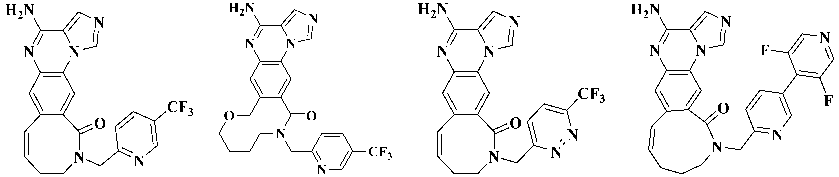

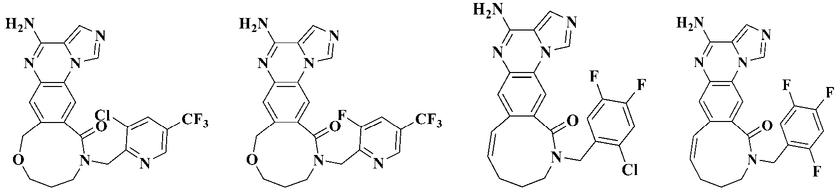

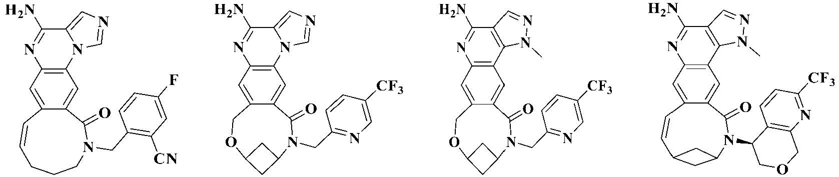

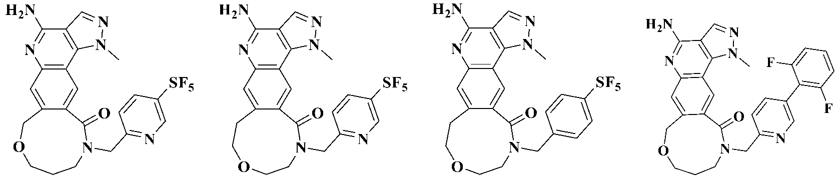

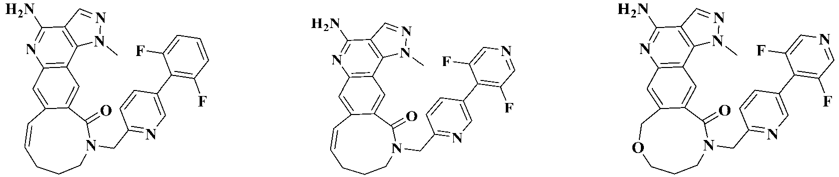

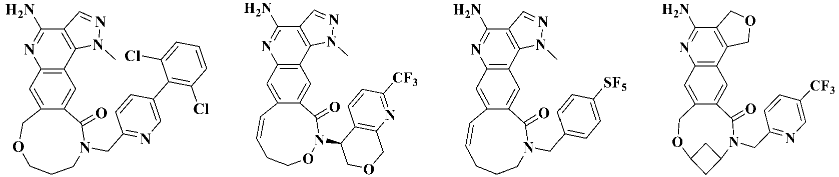

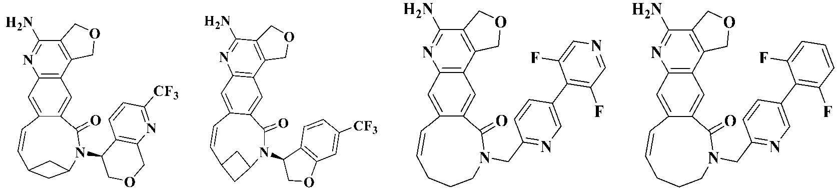

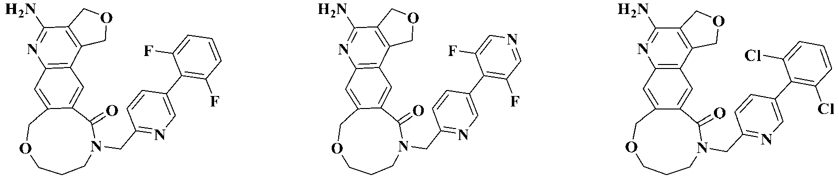

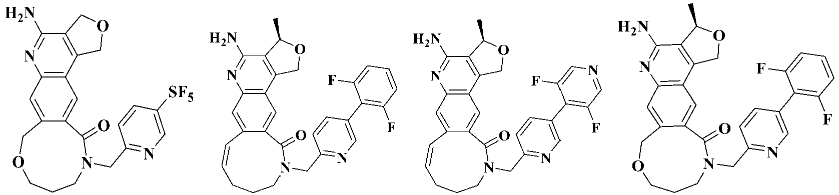

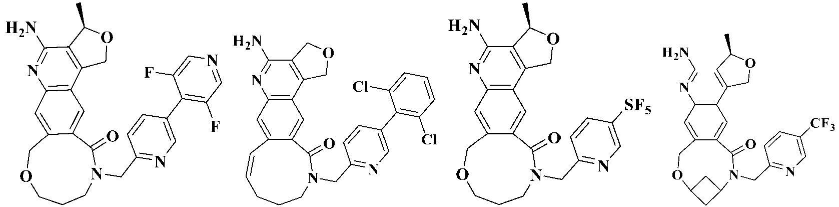

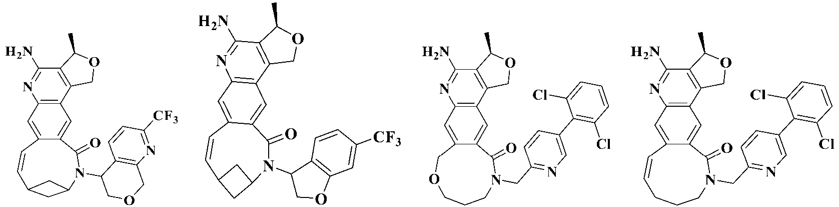

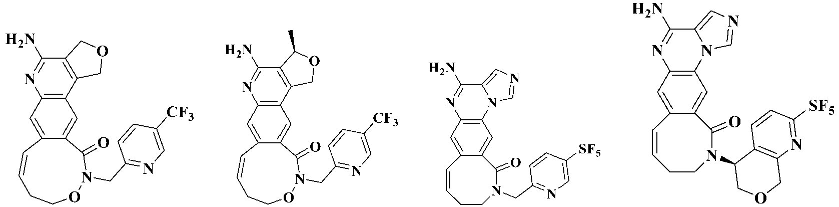

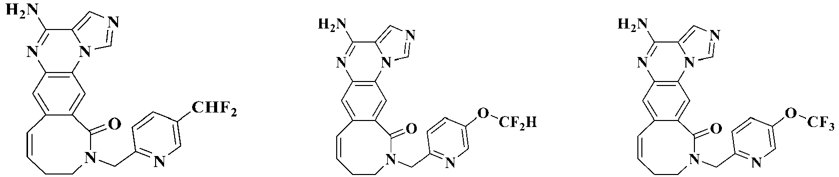

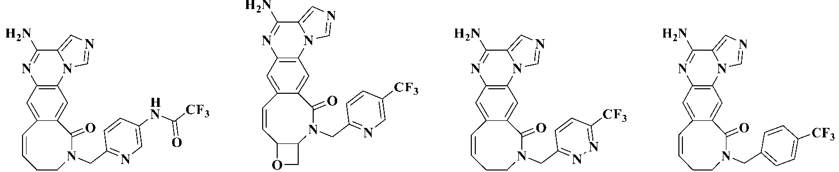

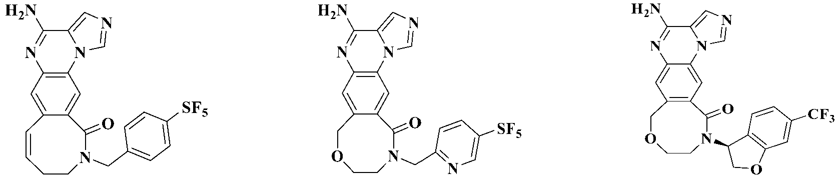

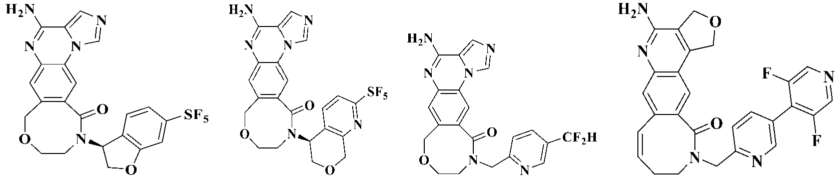

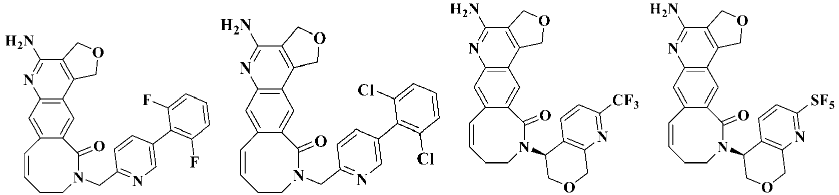

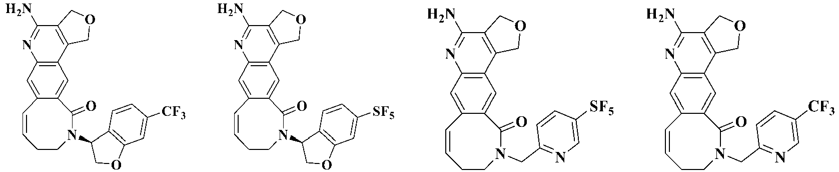

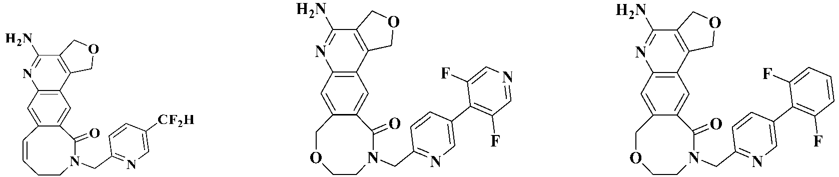

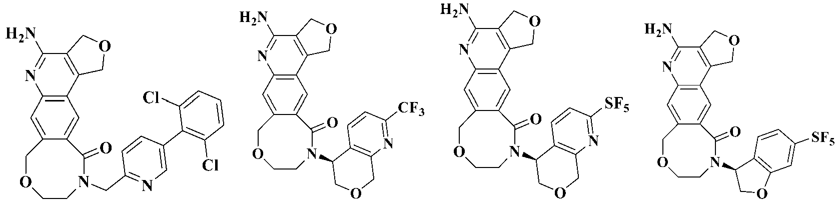

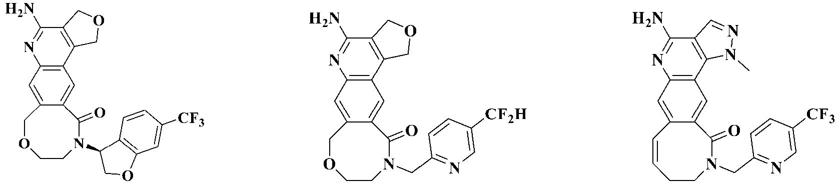

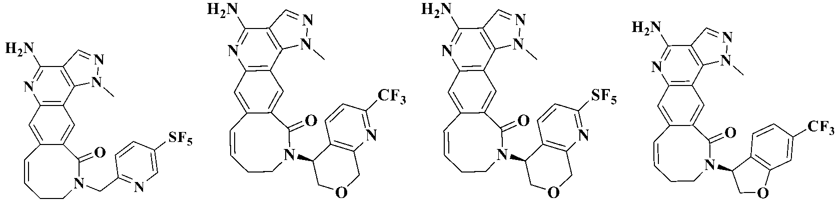

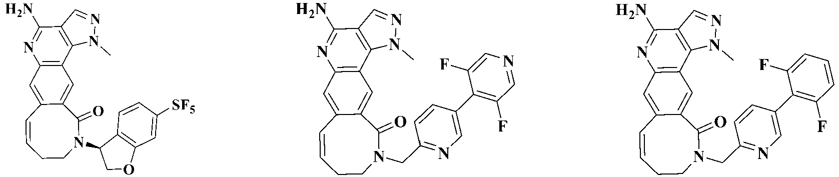

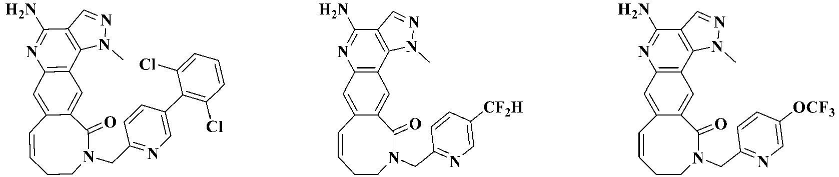

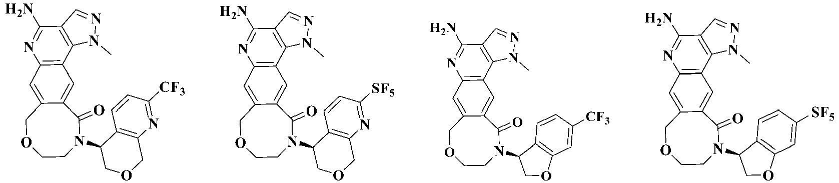

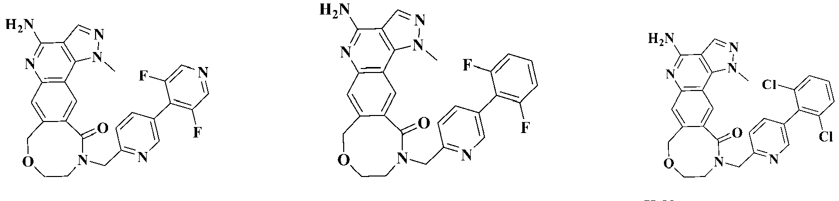

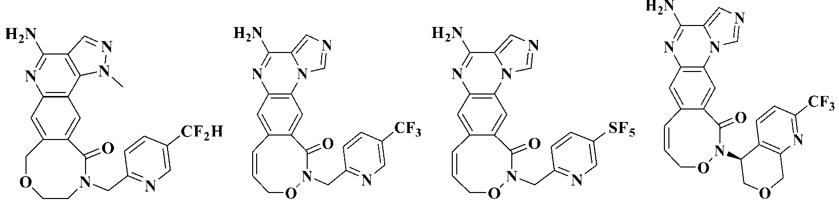

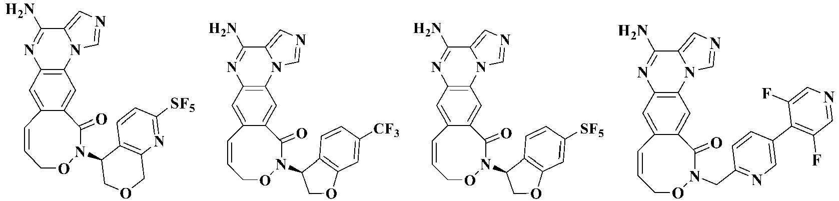

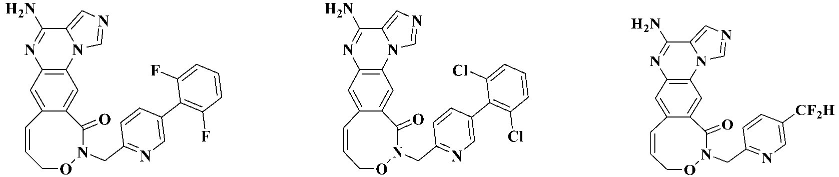

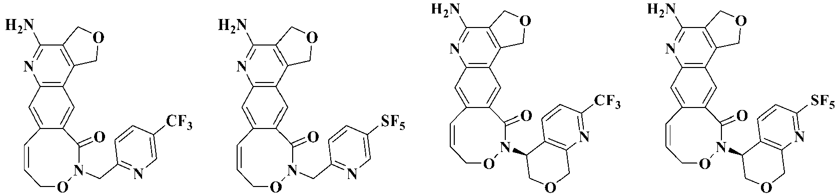

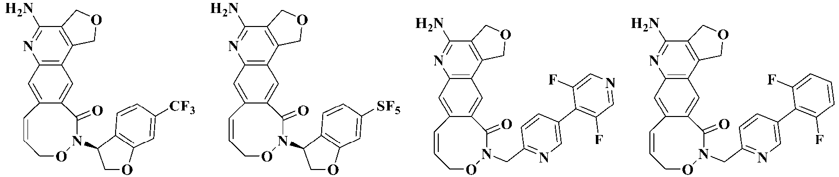

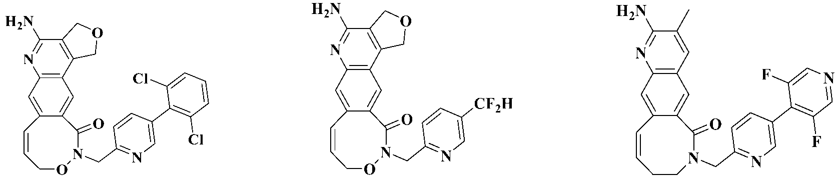

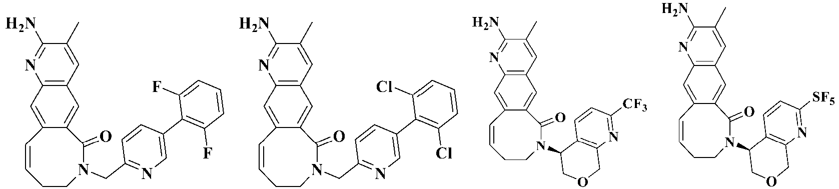

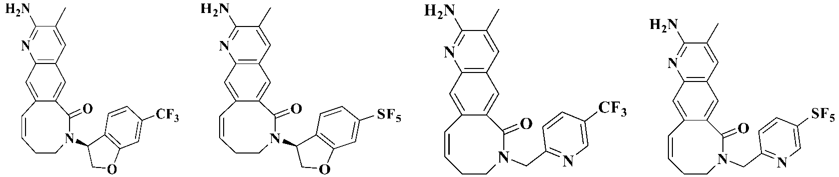

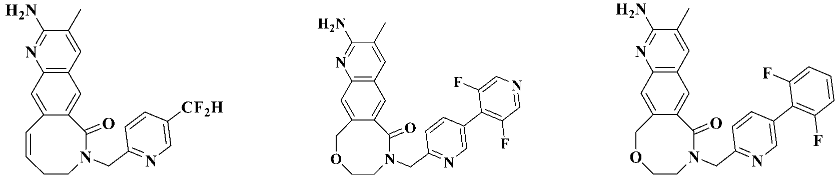

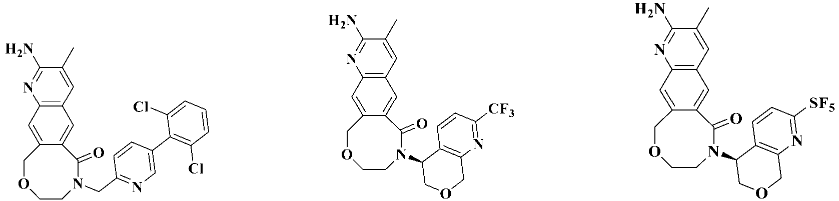

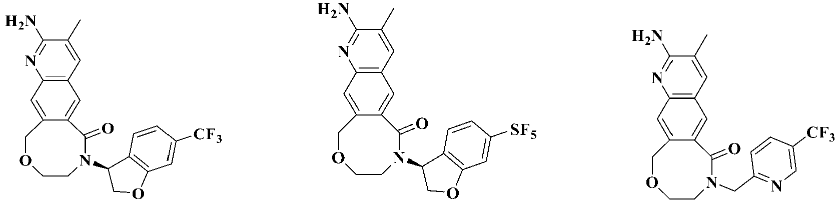

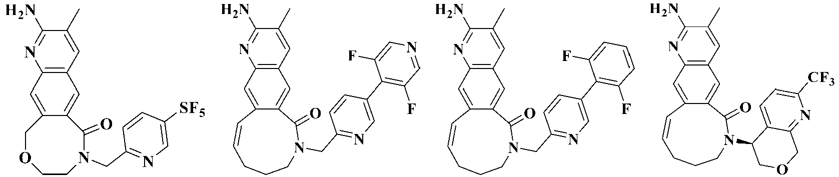

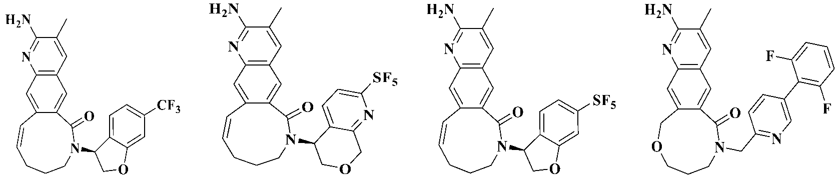

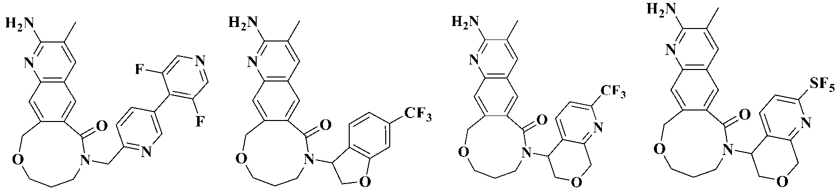

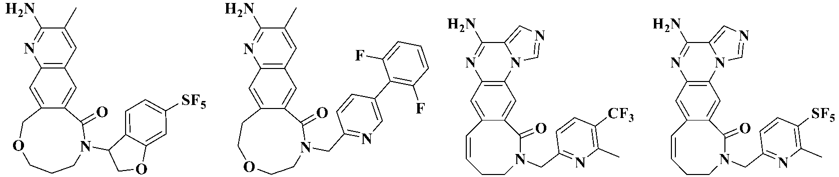

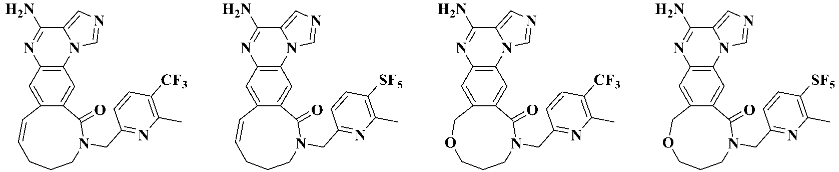

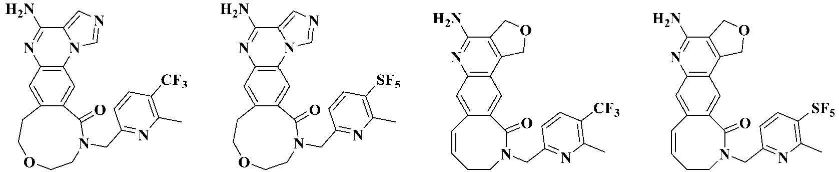

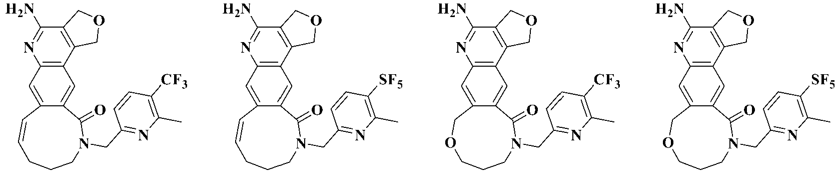

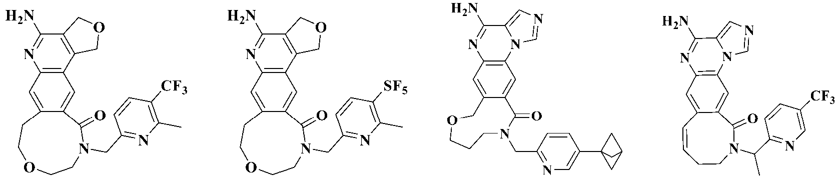

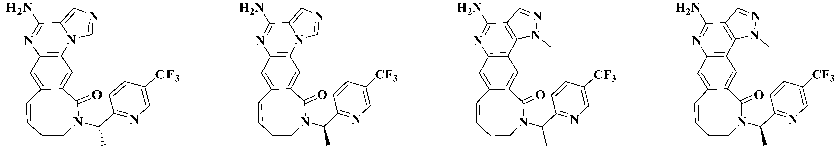

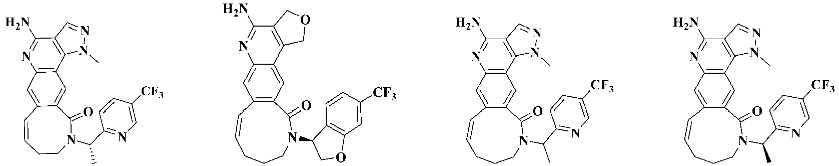

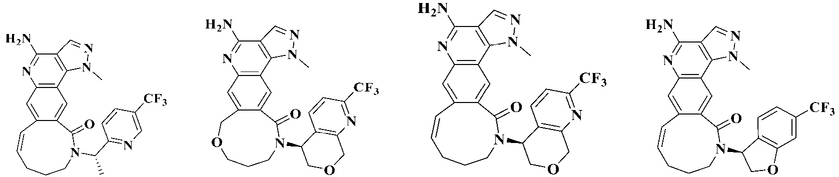

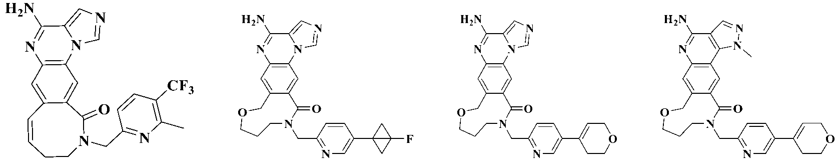

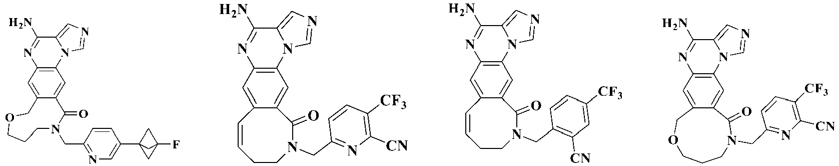

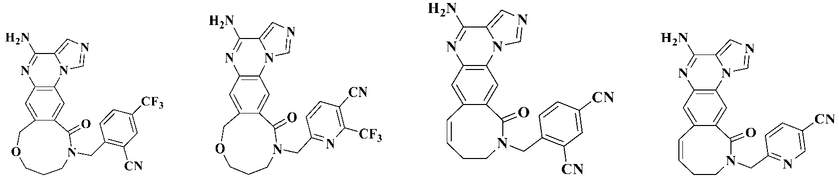

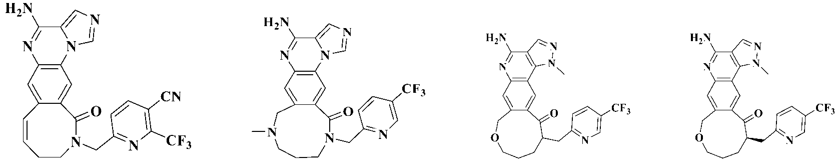

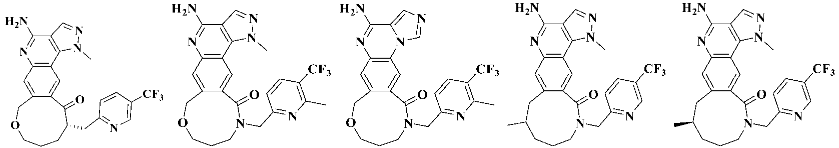

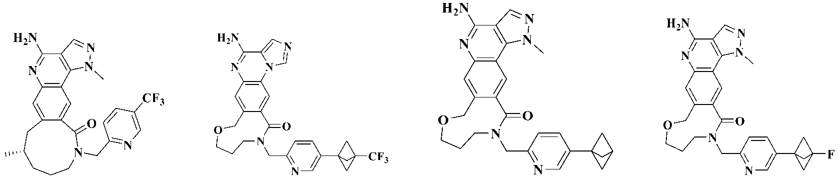

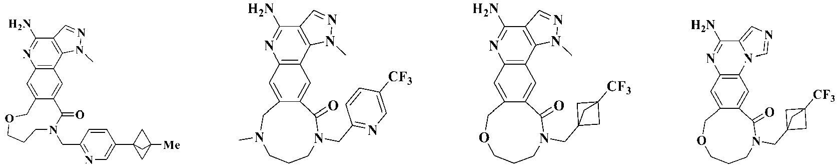

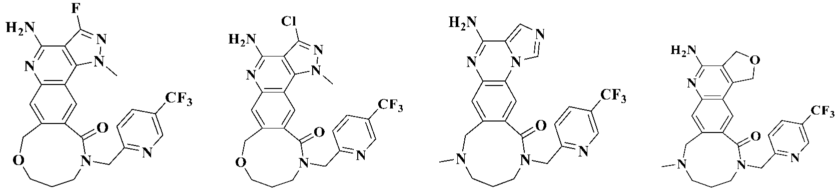

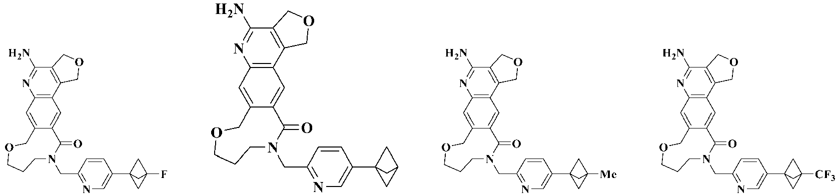

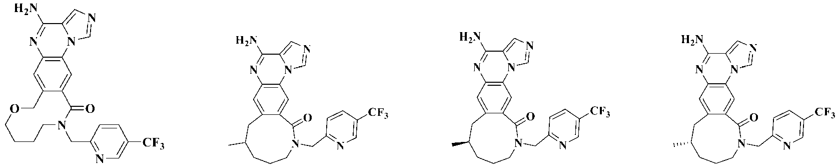

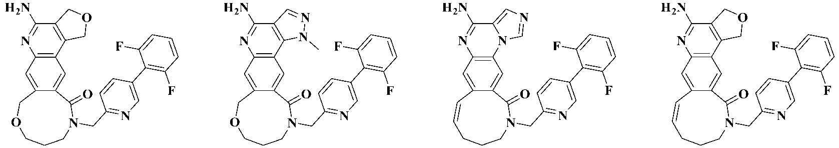

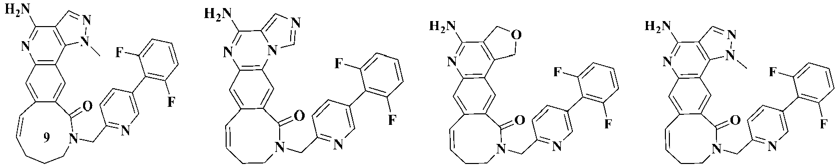

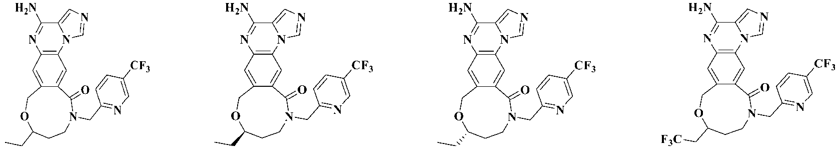

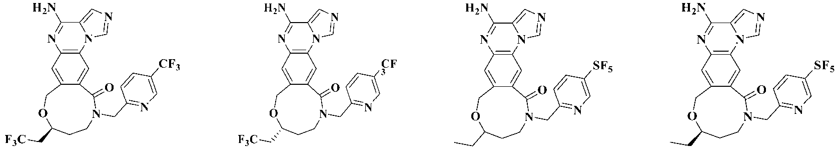

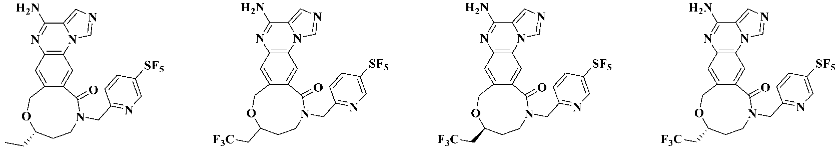

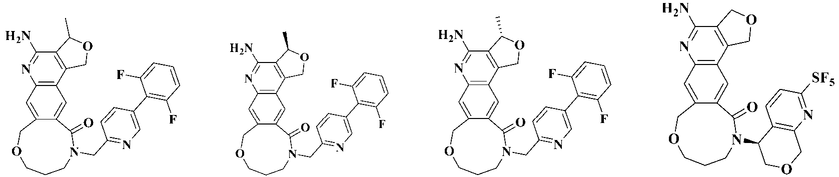

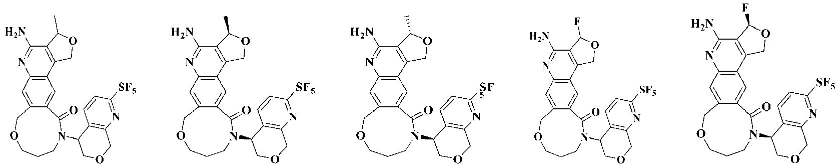

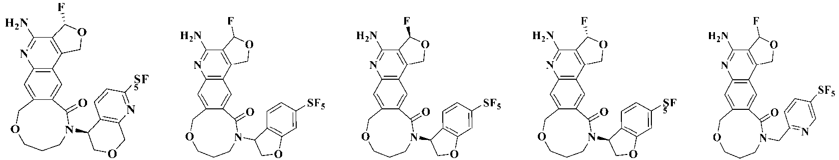

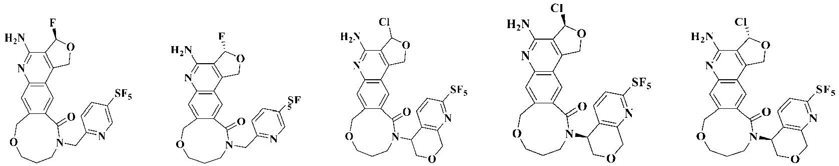

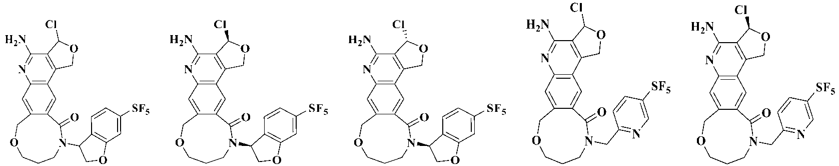

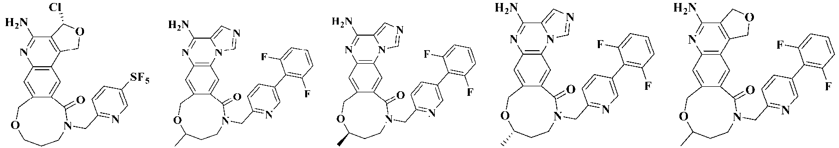

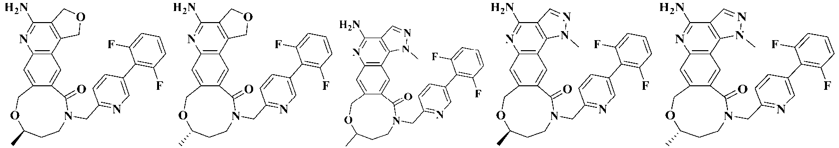

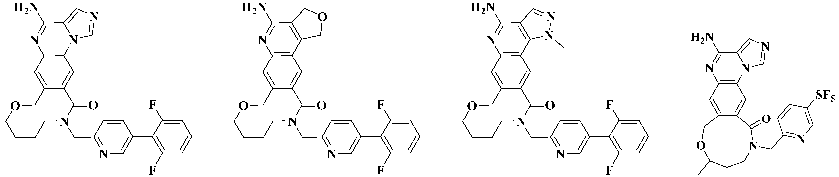

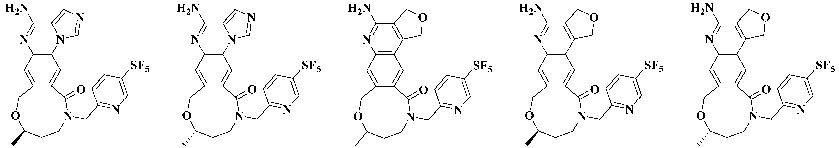

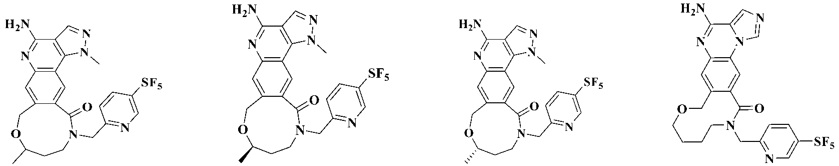

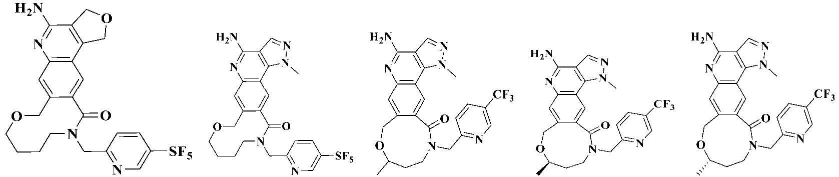

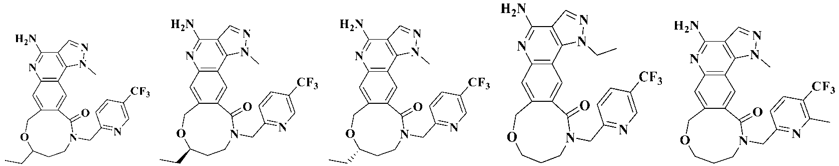

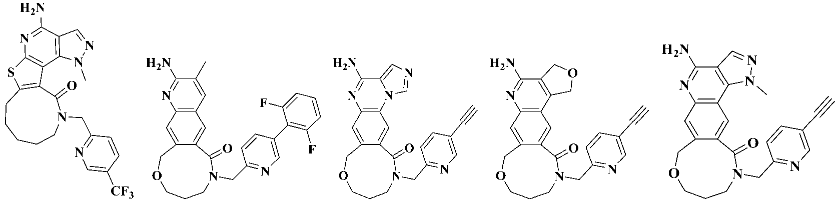

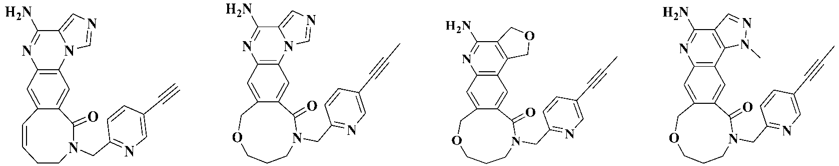

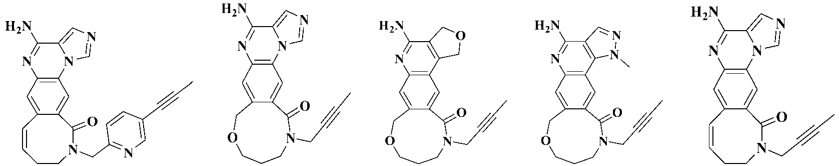

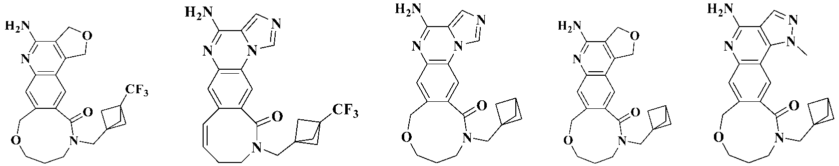

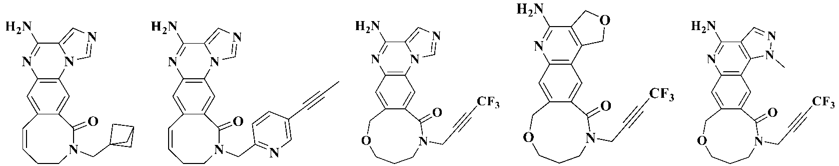

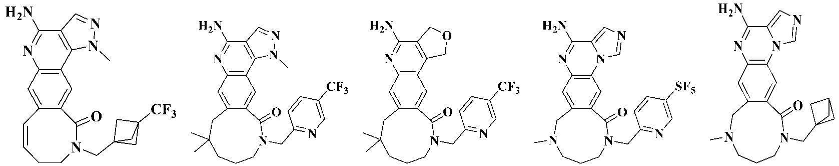

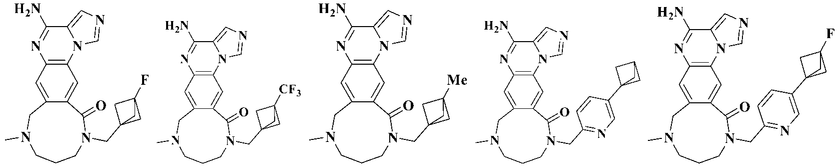

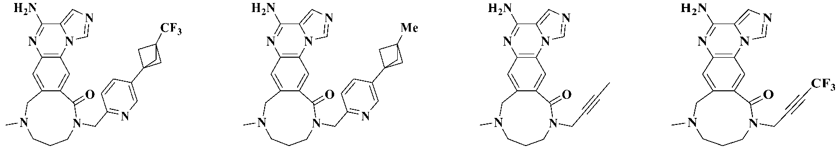

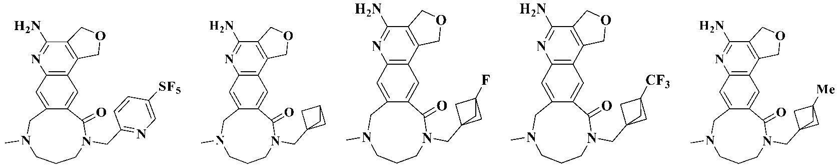

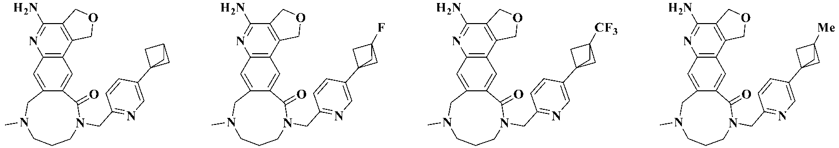

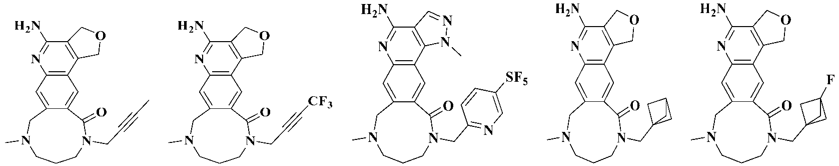

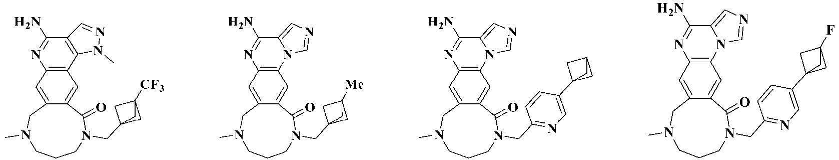

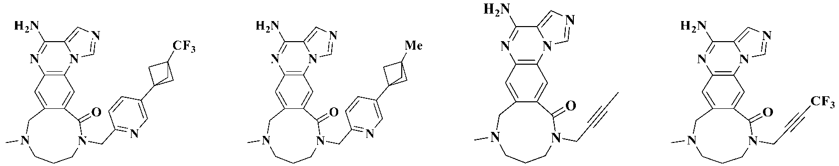

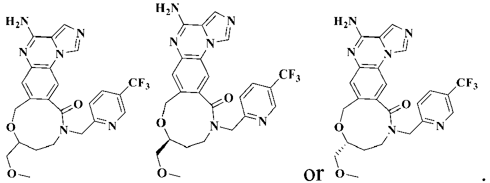

12. A compound represented by general formula (A-I), or a stereoisomer or a pharmaceutically acceptable salt thereof: 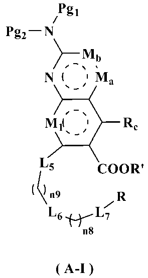
R' is selected from hydrogen, C₁₋₆ alkyl, C₁₋₆ deuteroalkyl, C₁₋₆ haloalkyl, C₁₋₆ alkoxy, halogenated C₁₋₆ alkoxy or C₁₋₆ hydroxyalkyl;
R is selected from halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl or 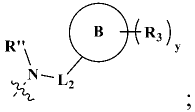
R'' is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, - C(O)-C₁₋₆ alkyl, -C(O)O-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
preferably a compound represented by general formula (A-II): 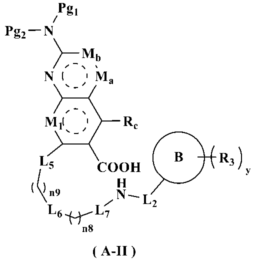
preferably, the compound represented by general formula (A-II) is further as represented by general formula (IV-E-I), general formula (IV-A-I) or general formula (IV-B-I): 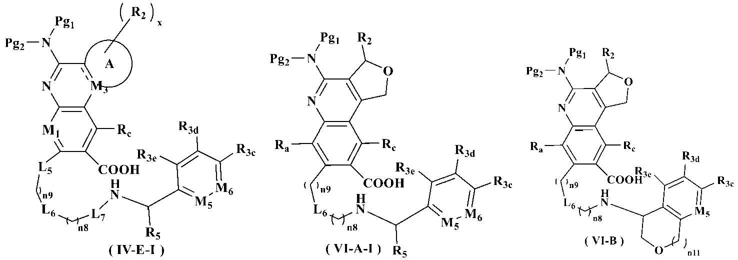
Pg₁ is selected from hydrogen, allyloxycarbonyl, trifluoroacetyl, tert-butylsulphinyl 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl or p-methoxyphenyl;
Pg₂ is selected from hydrogen, allyloxycarbonyl, trifluoroacetyl, tert-butylsulphinyl 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl or p-methoxyphenyl;
M₁, Mₐ, M_{b}, ring A, ring B, L₂, L₅, L₆, L₇, Rₐ, R_{b}, R_{c}, R₃, n8, n9, x and y are as defined in any one of claims 3-7;
M₅, M₆, R₃ₐ, R_{3b}, R_{3c}, R_{3d}, R₃ₑ, R₅ and n11 are as defined in claim 5 or 8.

13. The compound, or the stereoisomer or the pharmaceutically acceptable salt thereof according to claim 12, **characterised in that** the compound has the following structure:

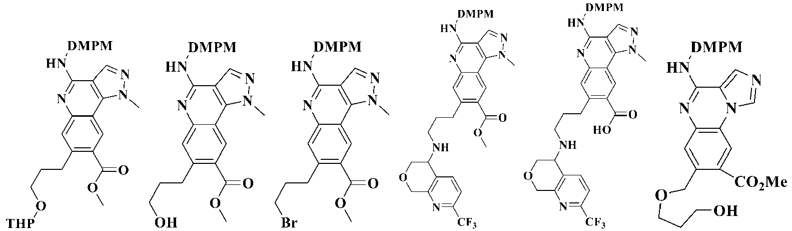

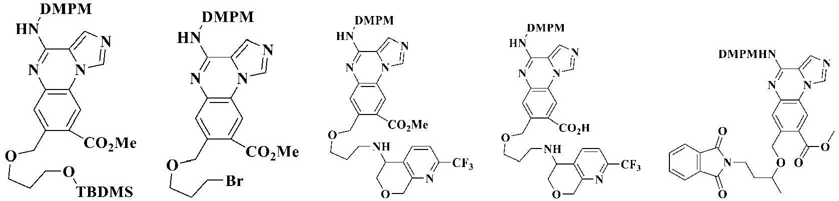

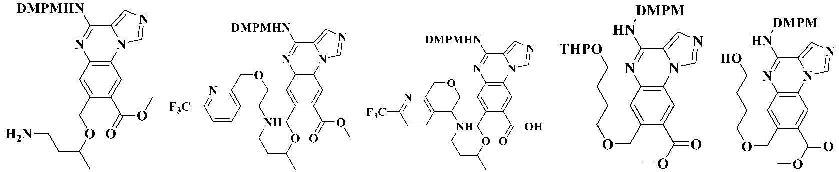

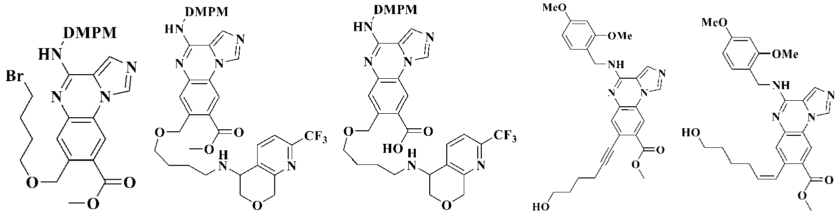

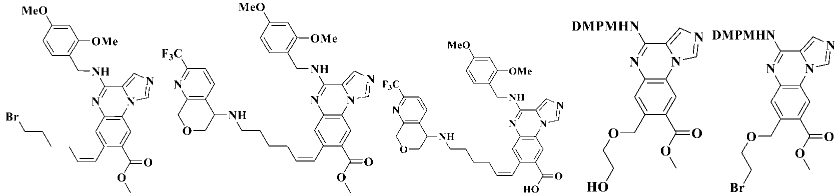

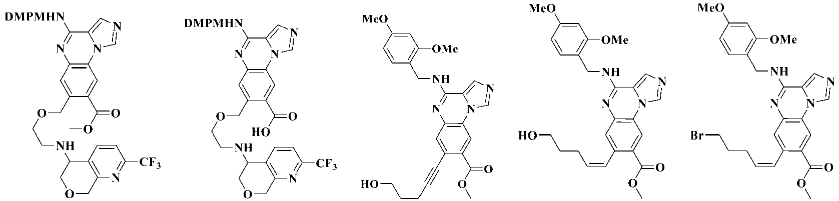

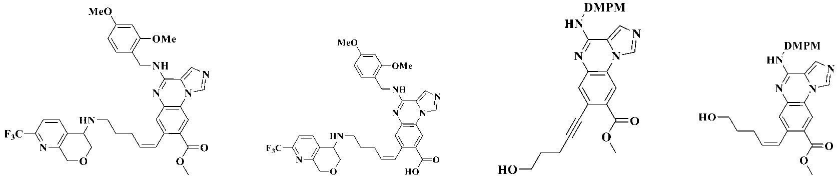

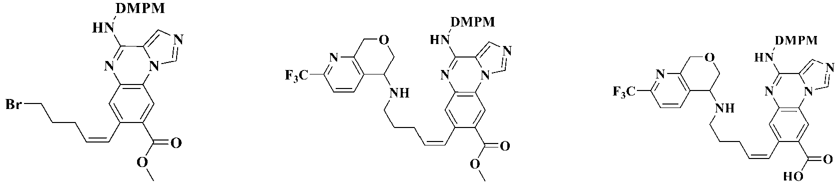

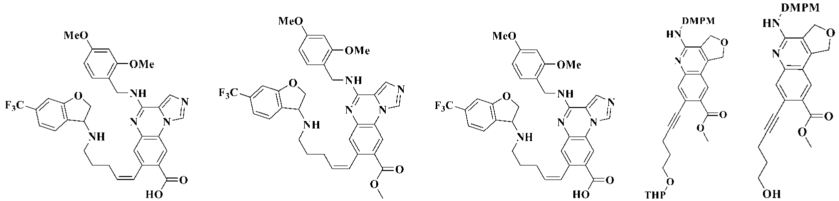

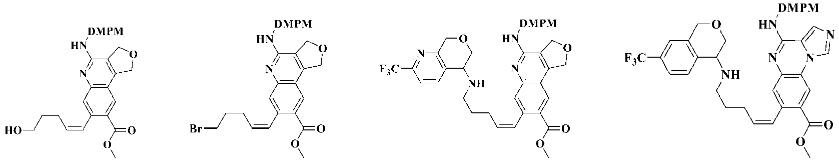

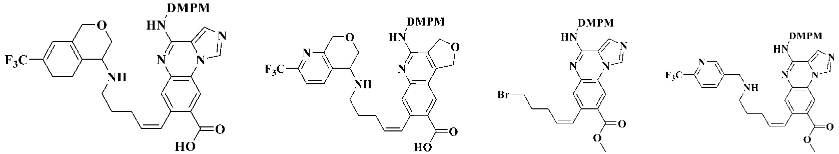

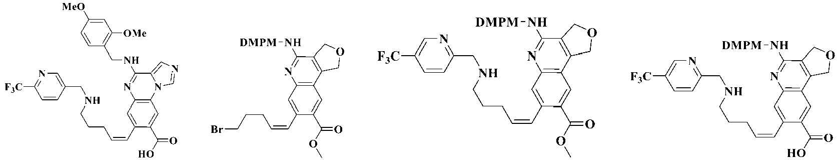

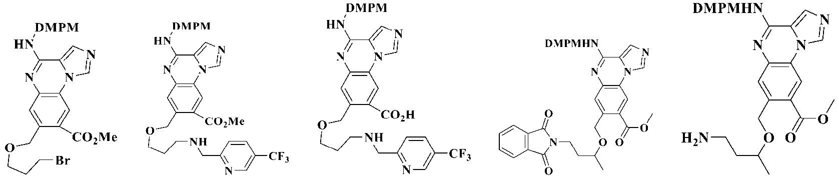

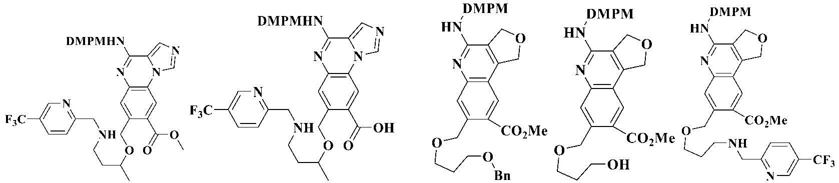

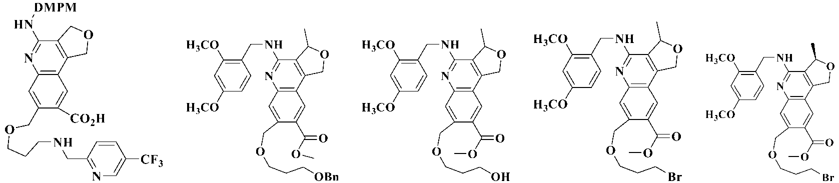

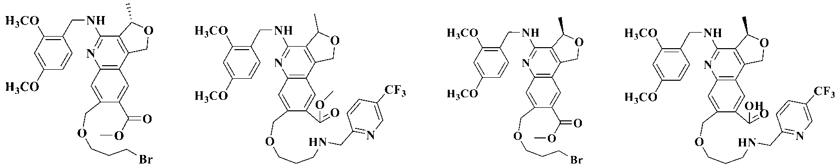

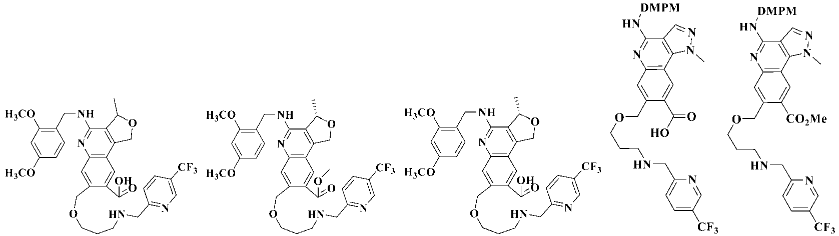

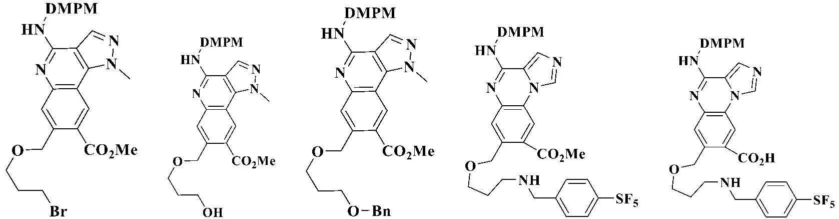

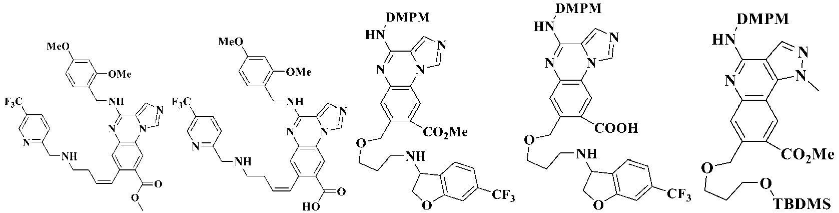

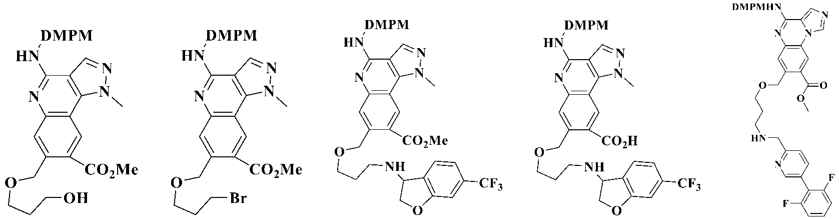

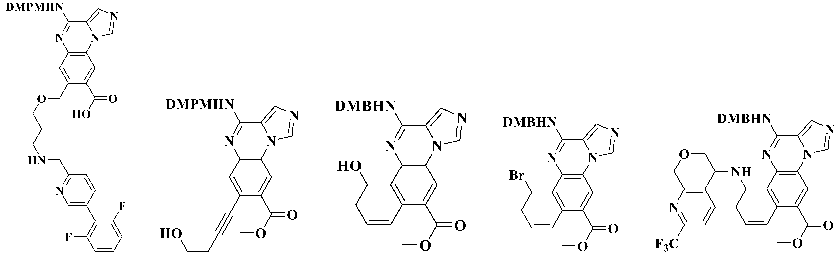

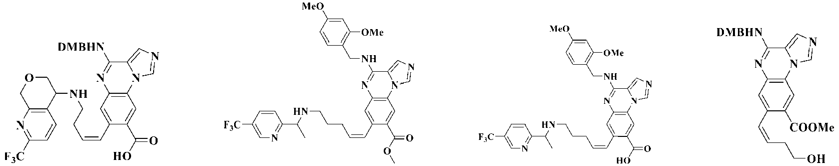

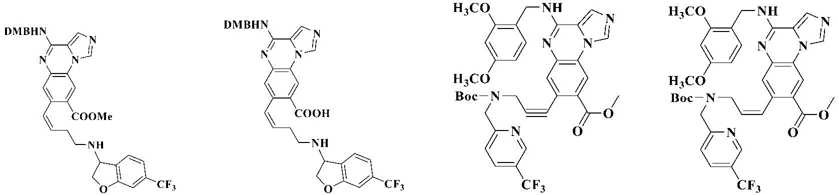

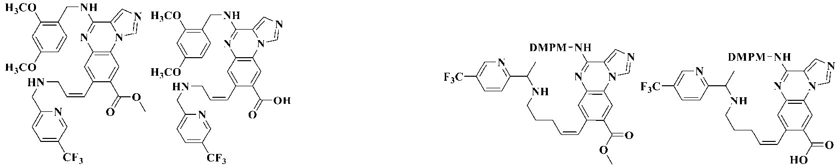

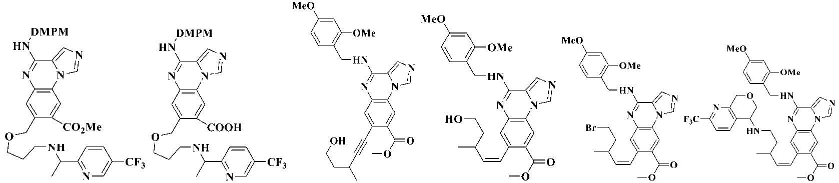

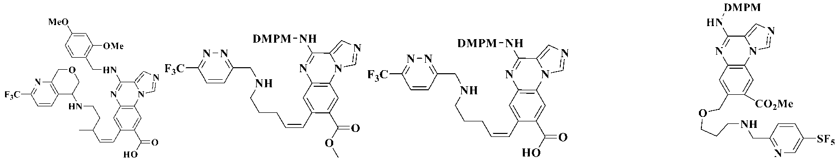

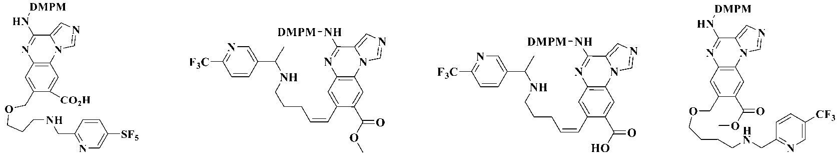

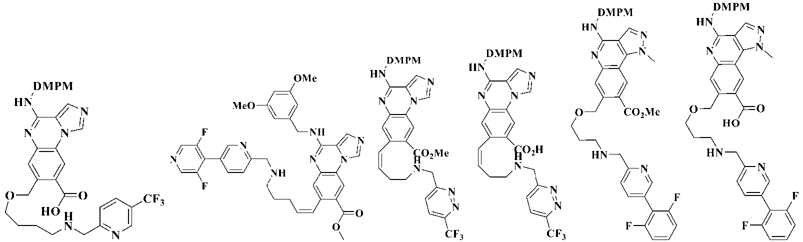

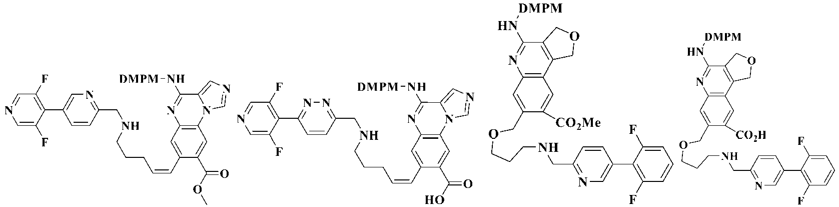

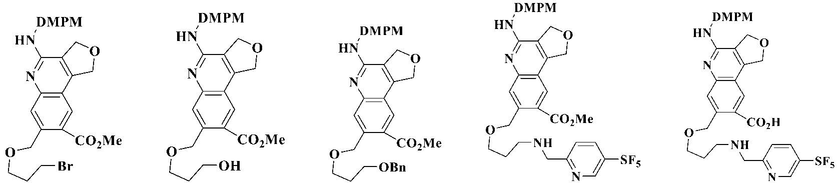

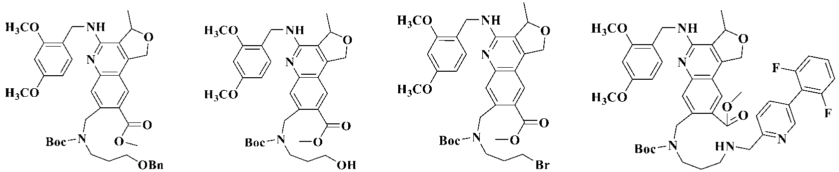

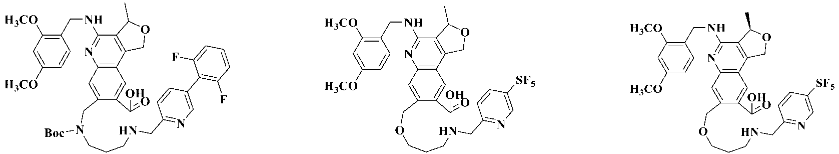

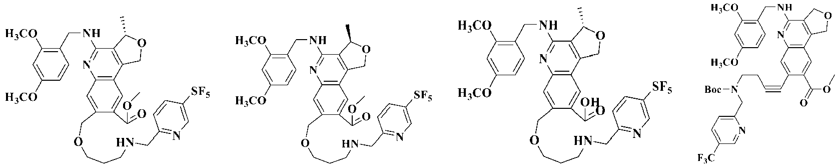

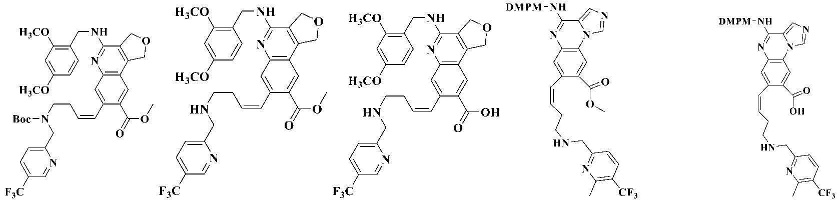

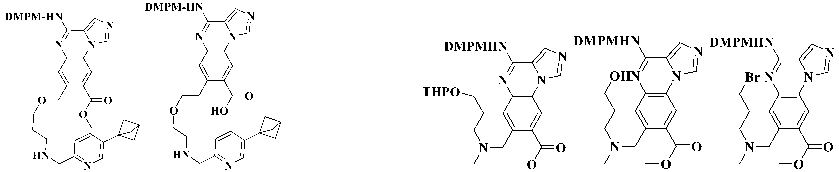

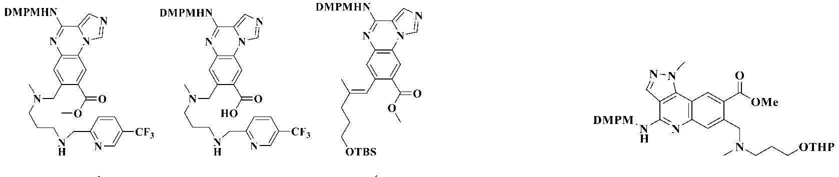

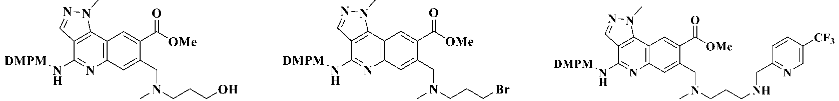

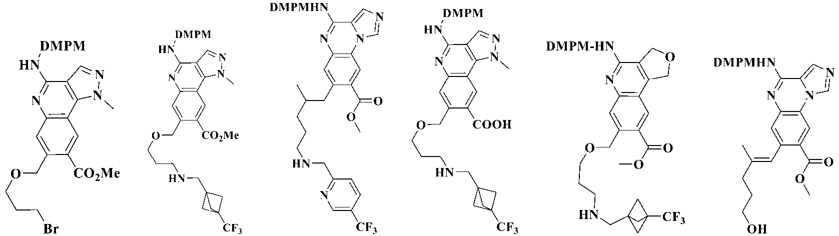

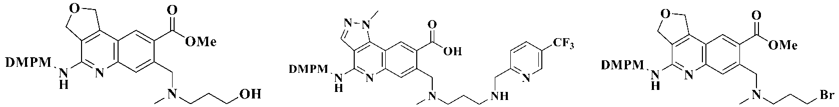

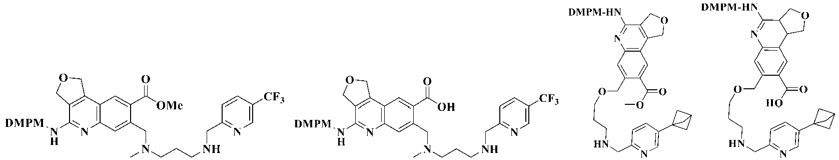

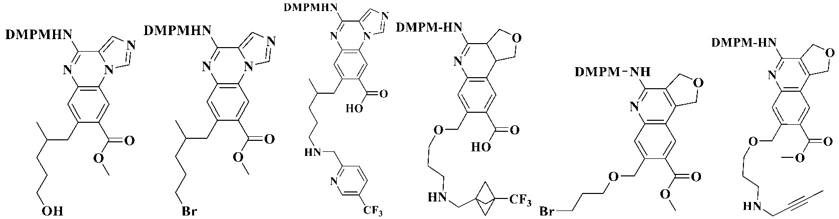

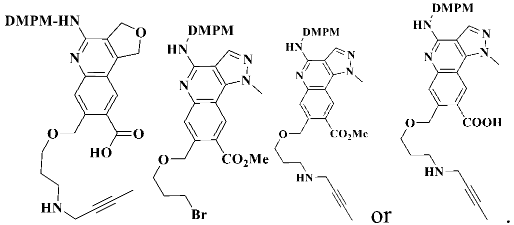

14. A method for preparing a compound represented by general formula (A), or a stereoisomer or a pharmaceutically acceptable salt thereof, **characterised in that** 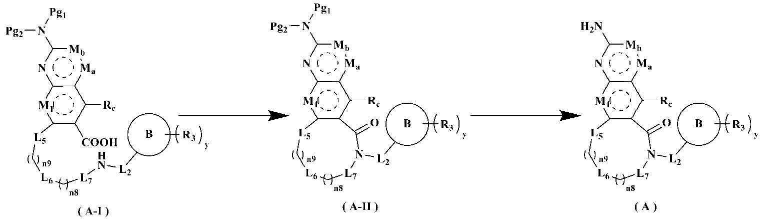
a compound represented by general formula (A-I) is reacted in the presence of a condensing agent and a base to obtain a compound represented by general formula (A-II), which is further deprotected to obtain the compound represented by general formula (A);
preferably, the method is a method for preparing a compound represented by general formula (IV-E), or a stereoisomer or a pharmaceutically acceptable salt thereof: 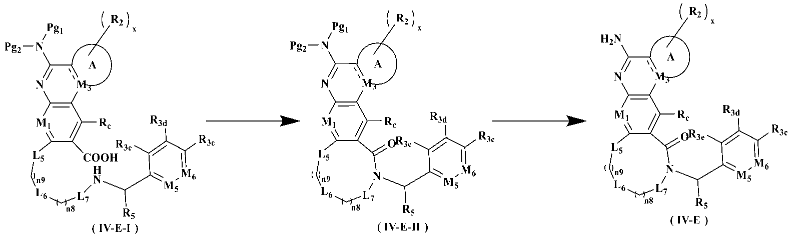
a compound represented by general formula (IV-E-I) is reacted in the presence of a condensing agent and a base to obtain a compound represented by general formula (IV-E-II), which is further deprotected to obtain the compound represented by general formula (IV-E);
alternatively, the method is a method for preparing a compound represented by general formula (VI-A), or a stereoisomer or a pharmaceutically acceptable salt thereof: 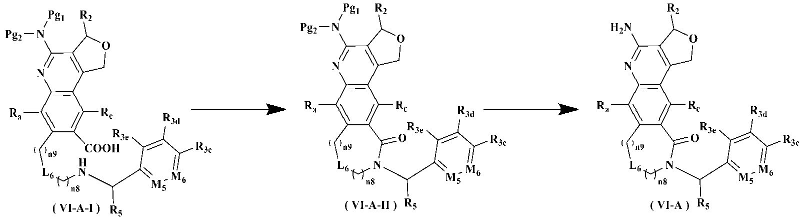
a compound represented by general formula (VI-A-I) is reacted in the presence of a condensing agent and a base to obtain a compound represented by general formula (VI-A-II), which is further deprotected to obtain the compound represented by general formula (VI-A);
alternatively, the method is a method for preparing a compound represented by general formula (VI-B), or a stereoisomer or a pharmaceutically acceptable salt thereof: 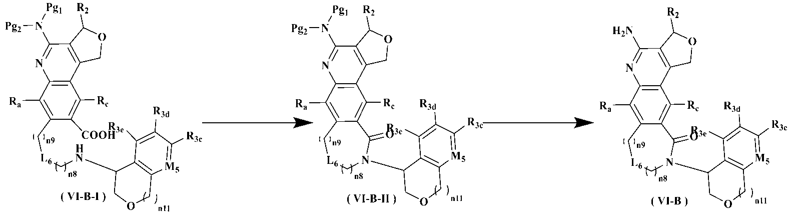
a compound represented by general formula (VI-B-I) is reacted in the presence of a condensing agent and a base to obtain a compound represented by general formula (VI-B-II), which is further deprotected to obtain the compound represented by general formula (VI-B);
Pg₁ is selected from hydrogen, allyloxycarbonyl, trifluoroacetyl, tert-butylsulphinyl 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl or p-methoxyphenyl;
Pg₂ is selected from hydrogen, allyloxycarbonyl, trifluoroacetyl, tert-butylsulphinyl 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl or p-methoxyphenyl;
M₁, Mₐ, M_{b}, ring A, ring B, L₂, L₅, L₆, L₇, Rₐ, R_{b}, R_{c}, R₃, n8, n9, x and y are as defined in any one of claims 3-7;
M₅, M₆, R₃ₐ, R_{3b}, R_{3c}, R_{3d}, R₃ₑ, R₅ and n11 are as defined in claim 5 or 8.

15. A pharmaceutical composition, **characterised by** comprising a therapeutically effective dose of the compound, or the stereoisomer or the pharmaceutically acceptable salt thereof according to any one of claims 1-11, and one or more pharmaceutically acceptable carriers, diluents, or excipients.

16. Use of the compound, or the stereoisomer or the pharmaceutically acceptable salt thereof according to any one of claims 1-11, or the pharmaceutical composition according to claim 15 in the preparation of a PRMT5 inhibitor drug.

17. Use of the compound, or the stereoisomer or the pharmaceutically acceptable salt thereof according to any one of claims 1-11, or the pharmaceutical composition according to claim 15 in the preparation of a drug for the treatment of a cancer.

18. The use according to claim 17, **characterised in that** the cancer is selected from lung cancer, hepatocellular carcinoma, breast cancer, skin cancer, bladder cancer, liver cancer, pancreatic cancer, head and neck cancer, glioma, glioblastoma, oesophageal cancer, pancreatic cancer, mesothelioma, melanoma, astrocytoma, undifferentiated pleomorphic sarcoma, leukaemia, gastric adenocarcinoma, myxofibrosarcoma, cholangiosarcoma, brain cancer, gastric cancer, renal cancer, endometrial cancer, ovarian tumour, prostate cancer, diffuse large B-cell lymphoma, non-Hodgkin lymphoma, urinary tract cancer, soft tissue cancer, pleural cancer, large intestine cancer, colourectal cancer, biliary tract cancer or cholangiocarcinoma; the lung cancer is selected from non-small cell lung cancer, lung squamous cell carcinoma or lung adenocarcinoma; the oesophageal cancer is selected from oesophageal squamous cell carcinoma or oesophageal adenocarcinoma.
